(19) Europäisches Patentamt
European Patent Office
Office européen des brevets

(11) EP 1 480 944 B1

(12) # EUROPÄISCHE PATENTSCHRIFT

(45) Veröffentlichungstag und Bekanntmachung des Hinweises auf die Patenterteilung:
**10.08.2016 Patentblatt 2016/32**

(21) Anmeldenummer: **03706551.3**

(22) Anmeldetag: **21.02.2003**

(51) Int Cl.:
***C07C 239/20*** (2006.01) ***C07C 311/04*** (2006.01)
***C07C 311/17*** (2006.01) ***C07C 317/24*** (2006.01)
***C07D 409/12*** (2006.01) ***C07D 333/34*** (2006.01)
***C07D 233/80*** (2006.01) ***C07D 213/30*** (2006.01)
***C07D 335/02*** (2006.01) ***A01N 33/08*** (2006.01)
***A01N 35/06*** (2006.01) ***A01N 41/06*** (2006.01)
***A01N 43/56*** (2006.01) ***C07D 213/55*** (2006.01)

(86) Internationale Anmeldenummer:
**PCT/EP2003/001800**

(87) Internationale Veröffentlichungsnummer:
**WO 2003/074475 (12.09.2003 Gazette 2003/37)**

(54) **SUBSTITUIERTE ARYLKETONE**
SUBSTITUTED ARYL KETONES
ARYLCETONE SUBSTITUEE

(84) Benannte Vertragsstaaten:
**AT BE BG CH CY CZ DE DK EE ES FI FR GB GR HU IE IT LI LU MC NL PT SE SI SK TR**

(30) Priorität: **05.03.2002 DE 10209645**

(43) Veröffentlichungstag der Anmeldung:
**01.12.2004 Patentblatt 2004/49**

(73) Patentinhaber: **Bayer Intellectual Property GmbH
40789 Monheim am Rhein (DE)**

(72) Erfinder:
- **HOISCHEN, Dorothee
40474 Düsseldorf (DE)**
- **HERRMANN, Stefan
40764 Langenfeld (DE)**
- **KATHER, Kristian
40764 Langenfeld (DE)**
- **MÜLLER, Klaus-Helmut
40593 Düsseldorf (DE)**
- **SCHWARZ, Hans-Georg
40764 Langenfeld (DE)**
- **SCHALLNER, Otto
40789 Monheim (DE)**
- **DREWES, Mark, Wilhelm
40764 Langenfeld (DE)**
- **DAHMEN, Peter
41470 Neuss (DE)**
- **FEUCHT, Dieter
65760 Eschborn (DE)**
- **PONTZEN, Rolf
42799 Leichlingen (DE)**

(74) Vertreter: **BIP Patents
c/o Bayer Intellectual Property GmbH
Alfred-Nobel-Straße 10
40789 Monheim am Rhein (DE)**

(56) Entgegenhaltungen:
**WO-A-95/31446 DE-A- 19 846 792**

Bemerkungen:
Die Akte enthält technische Angaben, die nach dem Eingang der Anmeldung eingereicht wurden und die nicht in dieser Patentschrift enthalten sind.



Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft neue substituierte Arylketone, Verfahren zu ihrer Herstellung und ihre Verwendung als Pflanzenbehandlungsmittel, insbesondere als Herbizide.

Es ist bereits bekannt, dass bestimmte substituierte Arylketone herbizide Eigenschaften aufweisen (vgl. EP-A-090 262, EP-A-135 191, EP-A-186118, EP-A-186 119, EP-A-186120, EP-A-319075, EP-A-352 543, EP-A-418 175, EP-A-487 357, EP-A-527036, EP-A-527037, EP-A-560 483, EP-A-609 797, EP-A-609 798, EP-A-625505, EP-A-625508, EP-A-636 622, US-A-5804532, US-A-5 834 402, US-A-5846906, US-A-5 863 865, WO-A-95/31446, WO-A-96/26192, WO-A-96/26193, WO-A-96/26200, WO-A-96/26206, WO-A-97/27187, WO-A-97/35850, WO-A-97/41105, WO-A-97/41116, WO-A-97/41117, WO-A-97/41118, WO-A-97/43270, WO-A-97/46530, WO-A-98/28981, WO-A-98/31681, WO-A-98/31682, WO-A-99/03856, WO-A-99/07688, WO-A-99/07697, WO-A-99/10327, WO-A-99/10328, WO-A-00/05221, WO-A-00/21924). Die Wirkung dieser Verbindungen ist jedoch nicht in allen Belangen zufriedenstellend.

Es wurden nun die neuen substituierten Arylketone der Formel (I-2) gefunden:

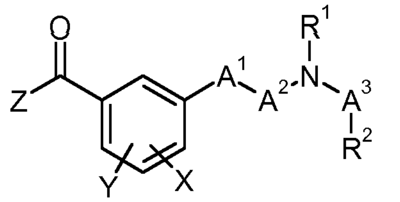

(I-2)

in welcher

$A^1$ für O (Sauerstoff) steht, wobei

$A^3$ für O (Sauerstoff), S (Schwefel), die Gruppierung

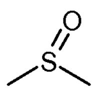 , 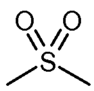 , 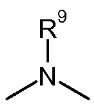 , 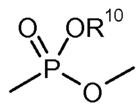 oder 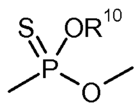

steht, wobei

Z für eine der nachstehenden Gruppierungen steht

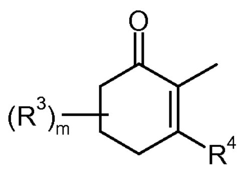 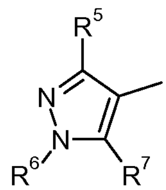

In den vorstehenden und nachfolgenden Definitionen gelten, sofern nicht anders ausgeführt, die nachfolgenden Definitionen:

Gesättigte oder ungesättigte Kohlenwasserstoffketten, wie Alkyl, Alkandiyl, Alkenyl oder Alkinyl, auch in Verknüpfung mit Heteroatomen, wie beispielsweise in Alkoxy, Alkylthio oder Alkylamino sind jeweils geradkettig oder verzweigt. Bevorzugt sind, falls nicht anders angegeben, Kohlenwasserstoffketten mit 1 bis 6 Kohlenstoffatomen.

Cycloalkyl steht für gesättigte, carbocyclische Verbindungen, die gegebenenfalls mit weiteren carbocyclischen, ankondensierten oder überbrückten Ringen ein polycyclisches Ringsystem bilden. Bevorzugt sind, falls nicht anders angegeben, Cyclopropyl, Cyclopentyl und Cyclohexyl.

Aryl steht für aromatische, mono- oder polycyclische Kohlenwasserstoffringe, wie z.B. Phenyl, Naphthyl, Anthranyl, Phenanthryl, vorzugsweise Phenyl oder Naphthyl, insbesondere Phenyl.

Heterocyclyl steht für gesättigte, ungesättigte oder aromatische, ringförmige Verbindungen, in denen mindestens ein Ringglied ein Heteroatom, d.h. ein von Kohlenstoff verschiedenes Atom, ist. Enthält der Ring mehrere Heteroatome, können diese gleich oder verschieden sein. Heteroatome sind bevorzugt Sauerstoff, Stickstoff oder Schwefel. Enthält dieser Ring mehrere Sauerstoffatome, stehen diese nicht benachbart. Gegebenenfalls bilden die ringförmigen Verbindungen mit weiteren carbocyclischen oder heterocyclischen, ankondensierten oder überbrückten Ringen gemeinsam ein polycyclisches Ringsystem. Ein polycyclisches Ringsystem kann über den heterocyclischen Ring oder einen ankondensierten carbocyclischen Ring verknüpft sein. Bevorzugt sind mono- oder bicyclische Ringsysteme, insbesondere monocyclische Ringsysteme mit 5 oder 6 Ringgliedern und bicyclischen Ringsysteme mit 7 bis 9 Ringgliedern.

Soweit die Verbindungen der allgemeinen Formel (I-2) in verschiedenen tautomeren bzw. stereoisomeren Formen existieren können, schließt die Erfindung die jeweils möglichen tautomeren bzw. stereoisomeren Formen mit ein.

Substituenten bzw. Bereiche der in den oben und nachstehend aufgeführten Formeln vorhandenen Reste werden im Folgenden definiert.

$A^2$ steht für Alkandiyl mit 1 bis 6 Kohlenstoffatomen, Alkendiyl oder Alkindiyl mit jeweils 2 bis 6 Kohlenstoffatomen.

$R^1$ steht für Wasserstoff, für gegebenenfalls durch Hydroxy, Amino, Cyano, Carbamoyl, Halogen, $C_1$-$C_4$-Alkoxy, $C_1$-$C_4$-Alkyl-carbonyl, $C_1$-$C_4$-Alkoxy-carbonyl, $C_1$-$C_4$-Alkylamino-carbonyl, Di-($C_1$-$C_4$-alkyl)-amino, Di-($C_1$-$C_4$-alkyl)-amino-carbonyl oder N-($C_1$-$C_4$-Alkoxy)-N-($C_1$-$C_4$-alkyl)-amino-carbonyl substituiertes Alkyl mit 1 bis 6 Kohlenstoffatomen, für jeweils gegebenenfalls durch Halogen substituiertes Alkylthio, Alkylsulfinyl oder Alkylsulfonyl mit jeweils 1 bis 6 Kohlenstoff-atomen, für jeweils gegebenenfalls durch Halogen substituiertes Alkenyl oder Alkinyl, mit jeweils 2 bis 6 Kohlenstoffatomen für jeweils gegebenenfalls durch Cyano, Halogen oder $C_1$-$C_4$-Alkyl substituiertes Cycloalkyl, Cycloalkylalkyl, mit jeweils 3 bis 6 Kohlenstoffatomen in den Cycloalkylgruppen und gegebenenfalls 1 bis 4 Kohlenstoffatomen im Alkylteil, für jeweils gegebenenfalls durch Cyano, Nitro, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Aryl, Arylalkyl, oder Arylcarbonylalkyl mit jeweils 6 oder 10 Kohlenstoffatomen in den Arylgruppen und gegebenenfalls 1 bis 4 Kohlenstoffatomen im Alkylteil, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Heterocyclyl, oder Heterocyclylalkyl, wobei jeweils die Heterocyclyl-Gruppierung bis zu 10 Kohlenstoffatome und zusätzlich mindestens ein Heteroatom ausgewählt aus der Reihe N (Stickstoff, höchstens jedoch 5 N-Atome), O (Sauerstoff, höchstens jedoch 2 O-Atome), S (Schwefel, höchstens jedoch 2 S-Atome), SO oder $SO_2$, sowie gegebenenfalls zusätzlich eine Gruppe ausgewählt aus Oxo (C=O), Thioxo (C=S), Imino (C=NH), Cyanoimino (C=N-CN), Nitroimino (C=N-$NO_2$) enthält.

$R^2$ steht für Wasserstoff, für gegebenenfalls durch Cyano, Halogen, $C_1$-$C_4$-Alkoxy, $C_1$-$C_4$-Alkyl-carbonyl, $C_1$-$C_4$-Alkoxy-carbonyl, $C_1$-$C_4$-Alkylaminocarbonyl oder Di-($C_1$-$C_4$-alkyl)-amino-carbonyl substituiertes Alkyl mit 1 bis 6 Kohlenstoffatomen, für jeweils gegebenenfalls durch Cyano, Halogen oder $C_1$-$C_4$-Alkoxy substituiertes Alkylcarbonyl, Alkoxycarbonyl oder Alkylaminocarbonyl mit jeweils 1 bis 6 Kohlenstoffatomen in den Alkylgruppen, für Dialkylaminocarbonyl mit jeweils 1 bis 4 Kohlenstoffatomen in den Alkylgruppen, für jeweils gegebenenfalls durch Halogen substituiertes Alkenyl, Alkenylcarbonyl, Alkenyloxycarbonyl, Alkinyl, Alkinylcarbonyl oder Alkinyloxycarbonyl mit jeweils 3 bis 6 Kohlenstoffatomen in den Alkenyl- bzw. Alkinylgruppen, für jeweils gegebenenfalls durch Cyano, Halogen oder $C_1$-$C_4$-Alkyl substituiertes Cycloalkyl, Cycloalkylcarbonyl, Cycloalkyloxycarbonyl, Cycloalkylalkyl, Cycloalkylalkylcarbonyl oder Cycloalkylalkoxycarbonyl mit jeweils 3 bis 6 Kohlenstoffatomen in den Cycloalkylgruppen und gegebenenfalls 1 bis 4 Kohlenstoffatomen im Alkylteil, für jeweils gegebenenfalls durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Aryl, Arylcarbonyl, Aryloxycarbonyl, Arylaminocarbonyl, Arylalkyl, Arylalkylcarbonyl, Arylalkoxycarbonyl oder Arylalkylaminocarbonyl mit jeweils 6 oder 10 Kohlenstoffatomen in den Arylgruppen und gegebenenfalls 1 bis 4 Kohlenstoffatomen im Alkylteil, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Heterocyclyl, Heterocyclylcarbonyl, oder Heterocyclylalkyl, wobei jeweils die Heterocyclyl-Gruppierung bis zu 10 Kohlenstoffatome und zusätzlich mindestens ein Heteroatom ausgewählt aus der Reihe N (Stickstoff, höchstens jedoch 5 N-Atome), O (Sauerstoff, höchstens jedoch 2 O-Atome), S (Schwefel, höchstens jedoch 2 S-Atome), SO oder $SO_2$, sowie gegebenenfalls zusätzlich eine Gruppe ausgewählt aus Oxo (C=O), Thioxo (C=S), Imino (C=NH), Cyanoimino (C=N-CN), Nitroimino (C=N-$NO_2$) enthält.

$R^3$ steht für Wasserstoff, Halogen, für jeweils gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl oder Alkylthio mit jeweils 1 bis 6 Kohlenstoffatomen, oder für Phenyl, oder - für den Fall, dass m für 2 steht - gegebenenfalls auch zusammen mit einem zweiten Rest $R^3$ für Sauerstoff oder Alkandiyl (Alkylen) mit 3 bis 5 Kohlenstoffatomen.

$R^4$ steht für Hydroxy, Formyloxy, Halogen, für jeweils gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkoxy, Alkylthio, Alkylsulfinyl, Alkylsulfonyl,

Alkylcarbonyloxy, Alkoxycarbonyloxy, Alkylaminocarbonyloxy oder Alkylsulfonyloxy mit jeweils 1 bis 6 Kohlenstoffatomen, für jeweils gegebenenfalls durch Halogen substituiertes Alkenyloxy oder Alkinyloxy mit jeweils 3 bis 6 Kohlenstoffatomen, für jeweils gegebenenfalls durch Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl substituiertes Aryloxy, Arylthio, Arylsulfinyl, Arylsulfonyl, Arylcarbonyloxy, Arylcarbonylalkoxy, Arylsulfonyloxy, Arylalkoxy, Arylalkylthio, Arylalkylsulfinyl oder Arylalkylsulfonyl mit jeweils 6 oder 10 Kohlenstoffatomen in der Arylgruppe und gegebenenfalls 1 bis 4 Kohlenstoffatomen im Alkylteil, für gegebenenfalls durch Cyano, Halogen, Oxo-, Hydroxy-, $C_1$-$C_4$-Alkoxy, $C_1$-$C_4$-Alkyl substituiertes Heterocyclyl mit 5 oder 6 Ringatomen, wobei mindestens 1 Stickstoffatom und gegebenenfalls bis zu 2 Sauerstoffatome, Schwefelatome und 3 Stickstoffatome enthalten sind, wobei insgesamt nicht mehr als 4 Heteroatome vorliegen und der Heterocyclus über den Stickstoff gebunden ist.

$R^5$ steht für Wasserstoff, Cyano, Carbamoyl, Thiocarbamoyl, Halogen, für jeweils gegebenenfalls durch Cyano, Halogen, $C_1$-$C_4$-Alkoxy, $C_1$-$C_4$-Alkylthio, $C_1$-$C_4$-Alkylsulfmyl oder $C_1$-$C_4$-Alkylsulfonyl substituiertes Alkyl, Alkoxy, Alkylthio, Alkylsulfinyl, Alkylsulfonyl oder Alkoxycarbonyl mit jeweils 1 bis 6 Kohlenstoffatomen in den Alkylgruppen oder für gegebenenfalls durch Cyano, Halogen oder $_C1$-$_C4$-Alkyl substituiertes Cycloalkyl mit 3 bis 6 Kohlenstoffatomen.

$R^6$ steht für Wasserstoff, für gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl mit 1 bis 6 Kohlenstoffatomen, für jeweils gegebenenfalls durch Cyano oder Halogen substituiertes Alkenyl oder Alkinyl mit jeweils 3 bis 6 Kohlenstoffatomen, für jeweils gegebenenfalls durch durch Cyano, Halogen oder $C_1$-$C_4$-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl substituiertes Aryl oder Arylalkyl mit jeweils 6 oder 10 Kohlenstoffatomen in der Arylgruppe und gegebenenfalls 1 bis 4 Kohlenstoffatomen im Alkylteil.

$R^7$ steht für Hydroxy, Formyloxy, für jeweils gegebenenfalls durch Alkyl, Cyano, Halogen oder $C_1$-$C_4$-Alkoxy substituiertes Alkoxy, Alkylcarbonyloxy, Alkoxycarbonyloxy, Alkoxycarbonylalkoxy, Alkylaminocarbonyloxy oder Alkylsulfonyloxy mit jeweils 1 bis 6 Kohlenstoffatomen in den Alkylgruppen, für jeweils gegebenenfalls durch Cyano oder Halogen substituiertes Alkenyloxy oder Alkinyloxy mit jeweils 3 bis 6 Kohlenstoffatomen, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl substituiertes Arylalkoxy, Arylcarbonyloxy, Arylcarbonylalkoxy oder Arylsulfonyloxy mit jeweils 6 oder 10 Kohlenstoffatomen in der Arylgruppe und gegebenenfalls 1 bis 4 Kohlenstoffatomen im Alkylteil.

$R^9$ steht für Wasserstoff, für gegebenenfalls durch Hydroxy, Amino, Cyano, Carbamoyl, Halogen, $C_1$-$C_4$-Alkoxy, $C_1$-$C_4$-Alkyl-carbonyl, $C_1$-$C_4$-Alkoxy-carbonyl, $C_1$-$C_4$-Alkylaminocarbonyl oder Di-($C_1$-$C_4$-alkyl)-amino-carbonyl substituiertes Alkyl mit 1 bis 6 Kohlenstoffatomen, für jeweils gegebenenfalls durch Cyano, Halogen oder $C_1$-$C_4$-Alkoxy substituiertes Alkylcarbonyl oder Alkoxycarbonyl mit jeweils 1 bis 6 Kohlenstoffatomen in den Alkylgruppen, für jeweils gegebenenfalls durch Halogen substituiertes Alkylthio, Alkylsulfinyl oder Alkylsulfonyl mit jeweils 1 bis 6 Kohlenstoffatomen, für jeweils gegebenenfalls durch Halogen substituiertes Alkenyl, Alkenylcarbonyl, Alkenyloxycarbonyl, Alkinyl, Alkinylcarbonyl oder Alkinyloxycarbonyl mit jeweils 2 bis 6 Kohlenstoffatomen in den Alkenyl- bzw. Alkinylgruppen, für jeweils gegebenenfalls durch Cyano, Halogen oder $C_1$-$C_4$-Alkyl substituiertes Cycloalkyl, Cycloalkylcarbonyl, Cycloalkyloxycarbonyl, Cycloalkylalkyl, Cycloalkylalkylcarbonyl oder Cycloalkylalkoxycarbonyl mit jeweils 3 bis 6 Kohlenstoffatomen in den Cycloalkylgruppen und gegebenenfalls 1 bis 4 Kohlenstoffatomen im Alkylteil, für jeweils gegebenenfalls durch Cyano, Nitro, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Aryl, Arylcarbonyl, Aryloxycarbonyl, Arylalkyl, Arylalkylcarbonyl oder Arylalkoxycarbonyl mit jeweils 6 oder 10 Kohlenstoffatomen in den Arylgruppen und gegebenenfalls 1 bis 4 Kohlenstoffatomen im Alkylteil, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Heterocyclyl, Heterocyclylcarbonyl oder Heterocyclylalkyl, wobei jeweils die Heterocyclyl-Gruppierung bis zu 10 Kohlenstoffatome und zusätzlich mindestens ein Heteroatom ausgewählt aus der Reihe N (höchstens jedoch 5 N-Atome), O (höchstens jedoch 2 O-Atome), S (höchstens jedoch 2 S-Atome), SO oder $SO_2$, sowie gegebenenfalls zusätzlich eine Gruppe ausgewählt aus Oxo (C=O), Thioxo (C=S), Imino (C=NH), Cyanoimino (C=N-CN), Nitroimino (C=N-$NO_2$) enthält, oder gemeinsam mit $R^2$ und dem Stickstoff an welches sie gebunden sind für einen gegebenenfalls

durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituierten Heterocyclus steht, welcher 1 Stickstoffatom und 1 bis 10 Kohlenstoffatome und gegebenenfalls ein weiteres Heteroatom aus der Reihe N (höchstens jedoch 4 weitere N-Atome), O (höchstens jedoch 2 O-Atome), S (höchstens jedoch 2 S-Atome), SO oder $SO_2$, enthält sowie gegebenenfalls zusätzlich eine Gruppe ausgewählt aus Oxo (C=O), Thioxo (C=S), Imino (C=NH), Cyanoimino (C=N-CN), Nitroimino (C=N-$NO_2$).

$R^{10}$ steht für Wasserstoff, Formyl, für gegebenenfalls durch Cyano, Halogen, $C_1$-$C_4$-Alkoxy, $C_1$-$C_4$-Alkylcarbonyl, $C_1$-$C_4$-Alkoxy-carbonyl, $C_1$-$C_4$-Alkylamino-carbonyl oder Di-($C_1$-$C_4$-alkyl)-amino-carbonyl substituiertes Alkyl mit 1 bis 6 Kohlenstoffatomen.

X steht für Wasserstoff, Nitro, Cyano, Carboxy, Carbamoyl, Thiocarbamoyl, Halogen, oder für jeweils gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl, Alkoxy, Alkylthio, Alkylsulfinyl, Alkylsulfonyl, Alkylamino, Dialkylamino oder Dialkylaminosulfonyl mit jeweils 1 bis 6 Kohlenstoffatomen in den Alkylgruppen.

Y steht für Wasserstoff, Nitro, Cyano, Carboxy, Carbamoyl, Thiocarbamoyl, Halogen, oder für jeweils gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl, Alkoxy, Alkylthio, Alkylsulfinyl, Alkylsulfonyl, Alkylamino, Dialkylamino oder Dialkylaminosulfonyl mit jeweils 1 bis 6 Kohlenstoffatomen in den Alkylgruppen.

m steht für die Zahlen 0 bis 3.

$A^2$ steht besonders bevorzugt für Methylen (-$CH_2$-), Ethan-1,1-diyl-(-$CH(CH_3)$-), Ethan-1,2-diyl (Dimethylen, -$CH_2CH_2$-), Propan-1,1-diyl-(-$CH(C_2H_5)$-), Propan-1,2-diyl (-$CH(CH_3)CH_2$-), Propan-1,3-diyl-(-$CH_2CH_2CH_2$-), Butan1,3-diyl (-$CH(CH_3)CH_2CH_2$-), Butan-1,4-diyl (-$CH_2CH_2CH_2CH_2$-), Ethendiyl, Propendiyl, Butendiyl, Ethindiyl, Propindiyl oder Butindiyl.

$R^1$ steht besonders bevorzugt für Wasserstoff, für jeweils gegebenenfalls durch Hydroxy, Amino, Cyano, Carbamoyl, Fluor, Chlor, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, Dimethylamino, Diethylamino, Dimethylaminocarbonyl, Diethylaminocarbonyl oder N-Methoxy-N-methylaminocarbonyl substituiertes Methyl, Ethyl, n-oder i-Propyl, n-, i- oder s-Butyl, n-, i- oder s-Pentyl, für jeweils gegebenenfalls durch Fluor und/oder Chlor substituiertes Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, für jeweils gegebenenfalls durch Fluor, Chlor und/oder Brom substituiertes Propenyl, Butenyl, Propinyl, oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl, oder Cyclohexylmethyl, für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, s- oder i-Propoxy, n-, i-, s- oder t-Butoxy, Difluormethoxy oder Trifluormethoxy substituiertes Phenyl, Benzoyl, Benzyl, Phenylethyl, oder Phenylcarbonylmethyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Heterocyclyl, oder Heterocyclylalkyl aus der Reihe Furyl, , Furylmethyl, Thienyl, Thienylmethyl, Pyrrolidinyl, Oxopyrrolidinyl, Pyrrolyl, Indolyl, Pyrrolylmethyl, Pyrazolyl, Pyrazolylmethyl, Oxazolyl, Oxazolylmethyl, Isoxazolyl, Thiazolyl, Thiazolylmethyl, Dihydropyranyl, Dihydropyranylmethyl, Piperidinyl, Oxopiperidinyl, Morpholinyl, Piperazinyl, Pyridinyl, Pyridinylcarbonyl oder Pyridinylmethyl.

$R^2$ steht besonders bevorzugt für Wasserstoff, für jeweils gegebenenfalls durch Cyano, Fluor, Chlor, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl, Methylaminocarbonyl, Ethyl-aminocarbonyl, n- oder i-Propylaminocarbonyl, Dimethylaminocarbonyl oder Diethylaminocarbonyl substituiertes Methyl, Ethyl, n- oder i-Propyl, n-, i-, s-oder t-Butyl, n-, i-, s- oder t-Pentyl, für jeweils gegebenenfalls durch Cyano, Fluor, Chlor, Methoxy, Ethoxy, n-oder i-Propoxy substituiertes Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, für Dimethylaminocarbonyl, Diethylaminocarbonyl oder Dipropylaminocarbonyl, für jeweils gegebenenfalls durch Fluor, Chlor und/oder Brom substituiertes Propenyl, Butenyl, Propenylcarbonyl, Butenylcarbonyl, Propenyloxycarbonyl, Butenyloxycarbonyl, Propenylaminocarbonyl, Butenylaminocarbonyl, Propinyl, Butinyl, Propinylcarbonyl, Butinylcarbonyl, Propinyloxycarbonyl oder Butinyloxycarbonyl, für jeweils gegebenenfalls durch Cyano, Fluor, Chlor, Methyl oder Ethyl substituiertes

Cyclopropyl, Cyclobutyl, Cyclopentyl, Cyclohexyl, Cyclopropylcarbonyl, Cyclobutylcarbonyl, Cyclopentylcarbonyl, Cyclohexylcarbonyl, Cyclopropyloxycarbonyl, Cyclobutyloxycarbonyl, Cyclopentyloxycarbonyl, Cyclohexyloxycarbonyl, Cyclopropylmethyl, Cyclobutylmethyl, Cyclopentylmethyl, Cyclohexylmethyl, Cyclopropylmethylcarbonyl, Cyclobutylmethylcarbonyl, Cyclopentylmethylcarbonyl, Cyclohexylmethylcarbonyl, Cyclopropylmethoxycarbonyl, Cyclobutylmethoxycarbonyl, Cyclopentylmethoxycarbonyl, Cyclohexylmethoxycarbonyl, für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Naphthyl, Benzoyl, Phenoxycarbonyl, Phenylaminocarbonyl, Benzyl, Phenylethyl, Phenylmethylcarbonyl oder Phenylmethoxycarbonyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Furyl, Furylcarbonyl, Furylmethyl, Thienyl, Thienylcarbonyl, Thienylmethyl, Pyrrolidinyl, Oxopyrrolidinyl, Pyrrolyl, Indolyl, Pyrrolylmethyl, Pyrazolyl, Pyrazolylcarbonyl, Pyrazolylmethyl, Oxazolyl, Oxazolylmethyl, Isoxazolyl, Isoxazolylcarbonyl, Thiazolyl, Thiazolylmethyl, 2-Oxo-1,3-diazacyclopentyl (2-Oxo-imidazolidinyl), Piperidinyl, Oxopiperidinyl, 2-Oxo-1,3-di-aza-cyclohexyl, Morpholinyl, Thiomorpholinyl, 3-Oxo-morpholinyl, 3-Oxo-thiomorpholinyl, Piperazinyl, Pyridinyl, Pyridinylcarbonyl oder Pyridinyl-methyl.

$R^3$ steht besonders bevorzugt für Wasserstoff, Fluor, Chlor oder Brom, für jeweils gegebenenfalls durch Cyano, Fluor, Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n-oder i-Propyl, n-, i-, s- oder t-Butyl, Methylthio, Ethylthio, n- oder i-Propyl-thio, n-, i-, s- oder t-Butylthio, oder für Phenyl, oder - für den Fall, dass m für 2 steht - gegebenenfalls auch zusammen mit einem zweiten Rest $R^3$ für Sauerstoff, Propan-1,3-diyl oder Butan-1,4-diyl.

$R^4$ steht besonders bevorzugt für Hydroxy, Formyloxy, Fluor oder Chlor, für jeweils gegebenenfalls durch Cyano, Fluor, Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methoxy, Ethoxy, n-oder i-Propoxy, Methylthio, Ethylthio, n- oder i-Propylthio, Methylsulfinyl, Ethylsulfinyl, Methylsulfonyl, Ethylsulfonyl, Acetyloxy, Propionyloxy, n- oder i-Butyroyloxy, Methoxycarbonyloxy, Ethoxycarbonyloxy, n- oder i-Propoxy-carbonyloxy, Methylaminocarbonyloxy, Ethylaminocarbonyloxy, n- oder i-Propylaminocarbonyloxy, Methylsulfonyloxy, Ethylsulfonyloxy, n- oder i-Propylsulfonyloxy, für jeweils gegebenenfalls durch Fluor, Chlor und/oder Brom substituiertes Propenyloxy, Butenyloxy, Propinyloxy oder Butinyloxy, für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n- oder i-Propylthio, Difluormethylthio, Trifluormethylthio, Methyl-sulfinyl, Ethylsulfinyl, Trifluormethylsulfinyl, Methylsulfonyl, Ethylsulfonyl oder Trifluormethylsulfonyl substituiertes Phenyloxy, Phenylthio, Phenylsulfinyl, Phenylsulfonyl, Phenylcarbonyloxy, Phenylcarbonylmethoxy, Phenylsulfonyloxy, Phenylmethoxy, Phenylmethylthio, Phenylmethylsulfinyl oder Phenylmethylsulfonyl, für jeweils gegebenenfalls durch Cyano, Oxo, Fluor, Chlor, Methyl, Ethyl, Methoxy, Ethoxy, Methylthio, Ethylthio substituiertes Pyrrolyl, Pyrrolinyl, Pyrrolidinyyl, Pyrazolyl, Pyrazolinyl, Pyrazolidinyl, Imidazolyl, Imidazolinyl, Imidazolidinyl, Triazolyl, Triazolinyl, Triazolidinyl, Tetrazolyl, Tetrazolinyl, Tetrazolidinyl, Oxazolyl, Oxazolinyl, Oxazolidinyl, Isoxazolyl, Isoxazolinyl, Isoxazolidinyl, Thiazolyl, Thiazolinyl, Thiazolidinyl, Thiadiazolyl, Indolyl, Piperidinyl, Piperazinyl, Oxazinyl, Thiazinyl, Morpholinyl.

$R^5$ steht besonders bevorzugt für Wasserstoff, Cyano, Carbamoyl, Thiocarbamoyl, Fluor, Chlor oder Brom, für jeweils gegebenenfalls durch Cyano, Fluor, Chlor, Methoxy, Ethoxy, n- oder i-Propoxy, Methylthio, Ethylthio, n-oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, Methoxy, Ethoxy, n- oder i-Propoxy, Methylthio, Ethylthio, n- oder i-Propyl-thio, Methylsulfinyl, Ethylsulfinyl, Methylsulfonyl, Ethylsulfonyl, Methoxycarbonyl, Ethoxycarbonyl, n- oder i-Propoxycarbonyl, oder für jeweils gegebenenfalls durch Cyano, Fluor, Chlor, Methyl oder Ethyl substituiertes Cyclopropyl, Cyclobutyl, Cyclopentyl oder Cyclohexyl.

$R^6$ steht besonders bevorzugt für Wasserstoff, für jeweils gegebenenfalls durch Cyano, Fluor, Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls Cyano, Fluor, Chlor oder Brom substituiertes Propenyl, Butenyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Nitro, Cyano, Fluor, Chlor, Brom, Methyl, Ethyl, n- oder i-Propyl, n-, i-, s-oder t-Butyl, Trifluormethyl, Methoxy, Ethoxy, n- oder i-Propoxy, Difluormethoxy, Trifluormethoxy, Methylthio, Ethylthio, n- oder i-Propylthio, n-, i-, s-

oder t-Butylthio, Difluormethylthio, Trifluormethylthio, Methylsulfinyl, Ethylsulfinyl, Trifluormethylsulfinyl, Methylsulfonyl, Ethylsulfonyl oder Trifluormethylsulfonyl substituiertes Phenyl oder Phenylmethyl.

$R^7$ steht besonders bevorzugt für Hydroxy, Formyloxy, für jeweils gegebenenfalls durch Alkyl, Cyano, Fluor, Chlor, Methoxy, Ethoxy, n- oder i-Propoxy substituiertes Methoxy, Ethoxy, n- oder i-Propoxy, Acetyloxy, Propionyloxy, n- oder i-Butyroyloxy, Methoxycarbonyloxy, Ethoxycarbonyloxy, n- oder i-Propoxycarbonyloxy, Methylaminocarbonyloxy, Ethylaminocarbonyloxy, n- oder i-Propylaminocarbonyloxy, Ethoxycarbonylmethoxy, Methoxycarbonylmethoxy, Methylsulfonyloxy, Ethylsulfonyloxy, n- oder i-Propylsulfonyloxy, für jeweils gegebenenfalls durch Cyano, Fluor, Chlor oder Brom substituiertes Propenyloxy, Butenyloxy, Propinyloxy oder Butinyloxy, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n- oder i-Propylthio, Difluormethylthio, Trifluormethylthio, Methylsulfinyl, Ethylsulfinyl, Trifluormethylsulfinyl, Methylsulfonyl, Ethylsulfonyl oder Trifluormethylsulfonyl substituiertes Phenylmethoxy, Phenylcarbonyloxy, Phenylcarbonylmethoxy oder Phenylsulfonyloxy.

$R^9$ steht besonders bevorzugt für Wasserstoff, für jeweils gegebenenfalls durch Hydroxy, Amino, Cyano, Carbamoyl, Fluor, Chlor, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, Dimethylaminocarbonyl oder Di-ethylaminocarbonyl substituiertes Methyl, Ethyl, n- oder i-Propyl, n-, i- oder s-Butyl, n-, i- oder s-Pentyl, für jeweils gegebenenfalls durch Cyano, Fluor, Chlor, Methoxy, Ethoxy, n-oder i-Propoxy substituiertes Acetyl, Propionyl, n- oder i-Butyroyl, Methoxycarbonyl, Ethoxycarbonyl, n- oder i-Propoxycarbonyl, für jeweils gegebenenfalls durch Fluor und/oder Chlor substituiertes Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, für jeweils gegebenenfalls durch Fluor, Chlor und/oder Brom substituiertes Propenyl, Butenyl, Propenylcarbonyl, Butenylcarbonyl, Propenyloxycarbonyl, Butenyloxycarbonyl, Propinyl, Butinyl, Propinylcarbonyl, Butinylcarbonyl, Propinyloxycarbonyl oder Butinyloxycarbonyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylcarbonyl, Cyclobutylcarbonyl, Cyclopentylcarbonyl, Cyclohexylcarbonyl, Cyclopropyloxycarbonyl, Cyclobutyloxycarbonyl, Cyclopentyloxycarbonyl, Cyclohexyloxycarbonyl, Cyclopropylmethyl, Cyclobutylmethyl, Cyclopentylmethyl, Cyclohexylmethyl, Cyclopropylmethylcarbonyl, Cyclobutylmethylcarbonyl, Cyclopentylmethylcarbonyl, Cyclohexylmethylcarbonyl, Cyclopropylmethoxycarbonyl, Cyclobutylmethoxycarbonyl, Cyclopentylmethoxycarbonyl, Cyclohexylmethoxycarbonyl, für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, s- oder i-Propoxy, n-, i-, s- oder t-Butoxy, Difluormethoxy oder Trifluormethoxy substituiertes Phenyl, Benzoyl, Phenoxycarbonyl, Benzyl, Phenylethyl, Phenylmethylcarbonyl oder Phenylmethoxycarbonyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Heterocyclyl, Heterocyclylcarbonyl oder Heterocyclylalkyl aus der Reihe Furyl, Furylcarbonyl, Furylmethyl, Thienyl, Thienylcarbonyl, Thienylmethyl, Pyrrolidinyl, Oxopyrrolidinyl, Pyrrolyl, Indolyl, Pyrrolylmethyl, Pyrazolyl, Pyrazolylcarbonyl, Pyrazolylmethyl, Oxazolyl, Oxazolylmethyl, Isoxazolyl, Isoxazolylcarbonyl, Thiazolyl, Thiazolylmethyl, Piperidinyl, Oxopiperidinyl, Morpholinyl, Piperazinyl, Pyridinyl, Pyridinylcarbonyl oder Pyridinylmethyl steht, oder gemeinsam mit $R^2$ und dem Stickstoff an welches sie gebunden sind für jeweils gegebenenfalls durch Nitro, Cyano, Fluor, Chlor, Brom, Methyl, Ethyl, n-oder i-Propyl, n-, i-, s- oder t-Butyl, Trifluormethyl, Methoxy, Ethoxy, n- oder i-Propoxy, Difluormethoxy oder Trifluormethoxy substituiertes, Pyrrolidinyl, Oxopyrrolidinyl, Pyrrolyl, Indolyl, Pyrazolyl, Oxazolyl, Isoxazolyl, Dihydropyranyl, Piperidinyl, Thiomorpholinyl, 3-Oxo-morpholinyl, 3-Oxothiomorpholinyl, Oxopiperidinyl, Morpholinyl, Piperazinyl, Imidazolyl, Imidazolidinyl, Oxo-imidazolidinyl, Triazol, Triazoliniyl, Tetrazolinyl oder Pyridinyl.

$R^{10}$ steht besonders bevorzugt für Wasserstoff, Formyl, für gegebenenfalls durch Cyano, Halogen, $C_1$-$C_4$-Alkoxy, $C_1$-$C_4$-Alkylcarbonyl, $C_1$-$C_4$-Alkoxy-carbonyl, $C_1$-$C_4$-Alkylamino-carbonyl oder Di-($C_1$-$C_4$-alkyl)-amino-carbonyl substituiertes Alkyl mit 1 bis 6 Kohlenstoffatomen.

X steht besonders bevorzugt für Wasserstoff, Nitro, Cyano, Carboxy, Carbamoyl, Thiocarbamoyl, Fluor, Chlor, Brom, Iod, oder für jeweils gegebenenfalls durch Cyano, Fluor, Chlor, Methoxy, Ethoxy, Methylthio, Ethylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Dimethylaminosulfonyl oder Diethylaminosulfonyl.

Y steht besonders bevorzugt für Wasserstoff, Nitro, Cyano, Carboxy, Carbamoyl, Thiocarbamoyl, Fluor, Chlor, Brom, Iod, oder für jeweils gegebenenfalls durch Cyano, Fluor, Chlor, Methoxy, Ethoxy, Methylthio, Ethylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Dimethylaminosulfonyl oder Diethylaminosulfonyl.

m steht besonders bevorzugt für die Zahlen 0, 1 oder 2.

$A^2$ steht ganz besonders bevorzugt für Methylen ($-CH_2-$), Ethan-1,1-diyl ($-CH(CH_3)-$), Ethan-1,2-diyl (Dimethylen, $-CH_2CH_2-$), Propan-1,2-diyl ($-CH(CH_3)CH_2-$) oder Propan-1,3-diyl ($-CH_2CH_2CH_2-$).

$R^1$ steht ganz besonders bevorzugt für Wasserstoff, für jeweils gegebenenfalls durch Cyano, Fluor, Chlor, Methoxy, Ethoxy, n- oder i-Propoxy, Acetyl, Propionyl, Methoxycarbonyl oder Ethoxycarbonyl substituiertes Methyl, Ethyl, n- oder i-Propyl, für jeweils gegebenenfalls durch Cyano, Fluor, Chlor, Methoxy, Ethoxy, n- oder i-Propoxy substituiertes Acetyl, Propionyl, Methoxycarbonyl oder Ethoxycarbonyl, für jeweils gegebenenfalls durch Fluor und/oder Chlor substituiertes Methylthio, Ethylthio, n- oder i-Propylthio, n-, i-, s- oder t-Butylthio, Methylsulfinyl, Ethylsulfinyl, Methylsulfonyl oder Ethylsulfonyl, für jeweils gegebenenfalls durch Fluor, Chlor und/oder Brom substituiertes Propenyl, Butenyl, Propinyl oder Butinyl, für jeweils gegebenenfalls durch Fluor, Chlor oder Methyl substituiertes Cyclopropyl, Cyclopropylcarbonyl oder Cyclopropylmethyl, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, s- oder i-Propoxy, Difluormethoxy oder Trifluormethoxy substituiertes Phenyl, Benzoyl, Phenoxycarbonyl, Benzyl, Phenylmethylcarbonyl oder Phenylmethoxycarbonyl.

$R^2$ steht ganz besonders bevorzugt für Wasserstoff, für jeweils gegebenenfalls durch Cyano, Fluor, Chlor, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl, Ethyl, n- oder i-Propyl, n-, i-, s- oder t-Butyl, n-, i-, s- oder t-Pentyl, für jeweils gegebenenfalls durch Cyano, Fluor, Chlor, Methoxy, Ethoxy, n- oder i-Propoxy substituiertes Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, für Dimethylaminocarbonyl, für jeweils gegebenenfalls durch Fluor und/oder Chlor substituiertes Methylthio, Ethylthio, n- oder i-Propylthio, n-, i-, s- oder t-Butylthio, Methylsulfinyl, Ethylsulfinyl, Methylsulfonyl oder Ethylsulfonyl, für jeweils gegebenenfalls durch Fluor, Chlor und/oder Brom substituiertes Propenyl, Butenyl, Propinyl oder Butinyl, für jeweils gegebenenfalls durch Fluor, Chlor oder Methyl substituiertes Cyclopropyl, Cyclopropylcarbonyl oder Cyclopropylmethyl, für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Benzoyl, Phenoxycarbonyl, Phenylaminocarbonyl, Benzyl, Phenylmethylcarbonyl oder Phenylmethoxycarbonyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Heterocyclyl, Heterocyclylcarbonyl oder Heterocyclylalkyl aus der Reihe Furyl, Furylcarbonyl, Furylmethyl, Thienyl, Thienylcarbonyl, Thienylmethyl, Pyrrolidinyl, Pyrrolyl, Indolyl, Pyrrolylmethyl, Pyrazolyl, Pyrazolylmethyl, Isoxazolyl, Piperidinyl, Morpholinyl, Thiomorpholinyl, 3-Oxo-morpholinyl, 3-Oxo-thiomorpholinyl, Piperazinyl, Pyridinyl, Pyridinylmethyl.

$R^3$ steht ganz besonders bevorzugt für Wasserstoff, für jeweils gegebenenfalls durch Fluor und/oder Chlor substituiertes Methyl, Ethyl, n- oder i-Propyl, Methylthio, Ethylthio, n- oder i-Propylthio, oder für Phenyl, oder - für den Fall, dass m für 2 steht - gegebenenfalls auch zusammen mit einem zweiten Rest $R^3$ für Sauerstoff, Propan-1,3-diyl oder Butan-1,4-diyl.

$R^4$ steht ganz besonders bevorzugt für Hydroxy, für Formyloxy, für jeweils gegebenenfalls durch Fluor und/oder Chlor substituiertes Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Acetyloxy, Propionyloxy, n- oder i-Butyroyloxy, Methoxycarbonyloxy, Ethoxycarbonyloxy, n- oder i-Propoxy-carbonyloxy, Methylaminocarbonyloxy, Ethylaminocarbonyloxy, n- oder i-Propylaminocarbonyloxy, Methylsulfonyloxy, Ethylsulfonyloxy, n- oder i-Propylsulfonyloxy, für jeweils gegebenenfalls durch Fluor und/oder Chlor substituiertes Propenyloxy, Butenyloxy, Propinyloxy oder Butinyloxy, für jeweils gegebenenfalls durch Nitro, Cyano, Fluor, Chlor, Brom, Methyl, Ethyl, n-oder i-Propyl, Trifluormethyl, Methoxy, Ethoxy, n- oder i-Propoxy, Difluormethoxy oder Trifluormethoxy substituiertes Phenyloxy, Phenylthio, Phenylsulfinyl, Phenylsulfonyl, Phenylcarbonyloxy, Phenylcarbonylmethoxy, Phenylsulfonyloxy, Phenylmethoxy,

Phenylmethylthio, Phenylmethylsulfinyl oder Phenylmethylsulfonyl, für jeweils gegebenenfalls durch Cyano, Oxo, Fluor, Chlor, Methyl, Ethyl, Methoxy, Ethoxy, Methylthio, Ethylthio substituiertes Pyrrolyl, Pyrrolinyl, Pyrrolidinyl, Pyrazolyl, Pyrazolinyl, Pyrazolidinyl, Imidazolyl, Imidazolinyl, Imidazolidinyl, Triazolyl, Triazolinyl, Triazolidinyl, Tetrazolyl, Tetrazolinyl, Tetrazolidinyl, Oxazolyl, Oxazolinyl, Oxazolidinyl, Isoxazolyl, Isoxazolinyl, Isoxazolidinyl, Thiazolyl, Thiazolinyl, Thiazolidinyl, Thiadiazolyl, Indolyl Piperidinyl, Piperazinyl, Oxazinyl, Thiazinyl, Morpholinyl.

$R^5$ steht ganz besonders bevorzugt für Wasserstoff, Cyano, Fluor, Chlor, für jeweils gegebenenfalls durch Fluor und/oder Chlor substituiertes Methyl, Ethyl, n- oder i-Propyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Methoxycarbonyl, Ethoxycarbonyl, n- oder i-Propoxycarbonyl, oder für gegebenenfalls durch Cyano, Fluor, Chlor oder Methyl substituiertes Cyclopropyl.

$R^6$ steht ganz besonders bevorzugt für Wasserstoff, für jeweils gegebenenfalls durch Cyano, Fluor, Chlor, Methoxy oder Ethoxy substituiertes Methyl, Ethyl, n- oder i-Propyl, n-, i-, s- oder t-Butyl, für jeweils gegebenenfalls durch Fluor oder Chlor substituiertes Propenyl, Butenyl, Propinyl oder Butinyl, für jeweils gegebenenfalls durch Fluor, Chlor oder Methyl substituiertes Cyclopropyl, Cyclobutyl, Cyclopentyl oder Cyclohexyl, oder für jeweils gegebenenfalls durch Fluor, Chlor, Brom, Methyl, Ethyl, n- oder i-Propyl, Trifluormethyl, Methoxy, Ethoxy, n- oder i-Propoxy, Difluormethoxy oder Trifluormethoxy substituiertes Phenyl oder Phenylmethyl.

$R^7$ steht ganz besonders bevorzugt für Hydroxy, für Formyloxy, für jeweils gegebenenfalls durch Alkyl, Cyano, Fluor, Chlor, Methoxy, Ethoxy, n- oder i-Propoxy substituiertes Methoxy, Ethoxy, n- oder i-Propoxy, Acetyloxy, Propionyloxy, n- oder i-Butyroyloxy, Methoxycarbonyloxy, Ethoxycarbonyloxy, n- oder i-Propoxycarbonyloxy, Methylaminocarbonyloxy, Ethylaminocarbonyloxy, n- oder i-Propylaminocarbonyloxy, Ethoxycarbonylmethoxy, Methoxycarbonylmethoxy, Methylsulfonyloxy, Ethylsulfonyloxy, n- oder i-Propylsulfonyloxy, für jeweils gegebenenfalls durch Fluor und/oder Chlor substituiertes Propenyloxy, Butenyloxy, Propinyloxy oder Butinyloxy, oder für jeweils gegebenenfalls durch Nitro, Cyano, Fluor, Chlor, Brom, Methyl, Ethyl, n- oder i-Propyl, Trifluormethyl, Methoxy, Ethoxy, n- oder i-Propoxy, Difluormethoxy oder Trifluormethoxy substituiertes Phenylmethoxy, Phenylcarbonyloxy, Phenylcarbonylmethoxy oder Phenylsulfonyloxy.

$R^9$ steht ganz besonders bevorzugt für Wasserstoff, für jeweils gegebenenfalls durch Cyano, Fluor, Chlor, Methoxy, Ethoxy, n- oder i-Propoxy, Acetyl, Propionyl, Methoxycarbonyl oder Ethoxycarbonyl substituiertes Methyl, Ethyl, n- oder i-Propyl, für jeweils gegebenenfalls durch Cyano, Fluor, Chlor, Methoxy, Ethoxy, n- oder i-Propoxy substituiertes Acetyl, Propionyl, Methoxycarbonyl oder Ethoxycarbonyl, für jeweils gegebenenfalls durch Fluor und/oder Chlor substituiertes Methylthio, Ethylthio, n- oder i-Propylthio, n-, i-, s- oder t-Butylthio, Methylsulfinyl, Ethylsulfinyl, Methylsulfonyl oder Ethylsulfonyl, für jeweils gegebenenfalls durch Fluor, Chlor und/oder Brom substituiertes Propenyl, Butenyl, Propinyl oder Butinyl, für jeweils gegebenenfalls durch Fluor, Chlor oder Methyl substituiertes Cyclopropyl, Cyclopropylcarbonyl oder Cyclopropylmethyl, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, s- oder i-Propoxy, Difluormethoxy oder Trifluormethoxy substituiertes Phenyl, Benzoyl, Phenoxycarbonyl, Benzyl, Phenylmethylcarbonyl oder Phenylmethoxycarbonyl oder gemeinsam mit $R^2$ und dem Stickstoff an welches sie gebunden sind für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes, Pyrrolidinyl, Oxopyrrolidinyl, Pyrrolyl, Indolyl, Pyrazolyl, Oxazolyl, Isoxazolyl, Dihydropyranyl, Piperidinyl, Oxopiperidinyl, Morpholinyl, Thiomorpholinyl, 3-Oxo-morpholinyl, 3-Oxo-thiomorpholinyl, Piperazinyl, Imidazolyl, Imidazolidinyl, Oxo-imidazolidinyl, Triazol, Triazolinyl, Tetrazolinyl oder Pyridinyl.

$R^{10}$ steht ganz besonders bevorzugt für Wasserstoff, Formyl, für jeweils gegebenenfalls durch Cyano, Fluor, Chlor, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl, Ethyl, n- oder i-Propyl, n-, i-, s- oder t-Butyl, n-, i-, s- oder t-Pentyl, für jeweils gegebenenfalls durch Cyano, Fluor, Chlor, Methoxy, Ethoxy, n- oder i-Propoxy substituiertes Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, für Dimethylaminocarbonyl, für jeweils gegebenenfalls durch Fluor und/oder Chlor substituiertes Methylthio, Ethylthio, n- oder i-Propylthio, n-, i-, s- oder t-Butylthio, Methylsulfinyl, Ethylsulfinyl, Methylsulfonyl oder Ethylsulfonyl, für jeweils gegebenenfalls durch Fluor, Chlor und/oder Brom substituiertes Propenyl, Butenyl, Propinyl oder Butinyl, für jeweils gegebenenfalls durch Fluor, Chlor oder Methyl substituiertes Cyclopropyl, Cyclopropylcarbonyl oder Cyclopropylmethyl, für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Benzoyl, Phenoxycarbonyl,

Phenylaminocarbonyl, Benzyl, Phenylmethylcarbonyl oder Phenylmethoxycarbonyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Heterocyclyl, Heterocyclylcarbonyl oder Heterocyclylalkyl aus der Reihe Furyl, Furylcarbonyl, Furylmethyl, Thienyl, Thienylcarbonyl, Thienylmethyl, Pyrrolidinyl, Pyrrolyl, Indolyl, Pyrrolylmethyl, Pyrazolyl, Pyrazolylmethyl, Isoxazolyl, Piperidinyl, Morpholinyl, Piperazinyl, Pyridinyl, Pyridinylmethyl.

X steht ganz besonders bevorzugt für Wasserstoff, Nitro, Cyano, Fluor, Chlor, Brom, Iod, Methyl, Ethyl, Difluormethyl, Trifluormethyl, Dichlormethyl, Trichlormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl.

Y steht ganz besonders bevorzugt für Wasserstoff, Nitro, Cyano, Fluor, Chlor, Brom, Iod, Methyl, Ethyl, Difluormethyl, Trifluormethyl, Dichlormethyl, Trichlormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl.

m steht ganz besonders bevorzugt für die Zahlen 0 oder 2.

Die Verbindungen der Formel (I-2) werden besonders hervorgehoben:

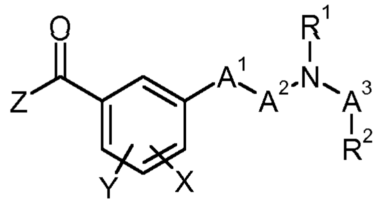

(I-2)

$A^1$, $A^2$, $A^3$, $R^1$, $R^2$, X, Y und Z haben hierbei jeweils die oben als bevorzugt, besonders bevorzugt oder ganz besonders bevorzugt angegebenen Bedeutungen. Weiter werden die Verbindungen der allgemeinen Formeln (I-2A) und (I-2B) besonders hervorgehoben:

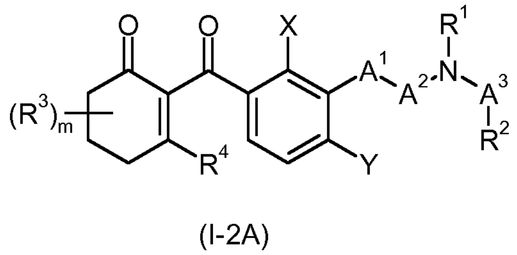

(I-2A)

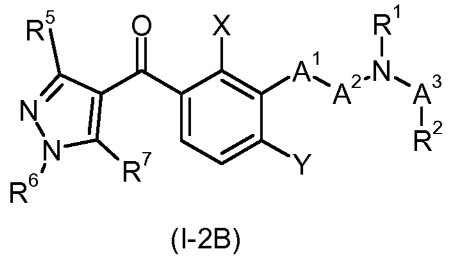

(I-2B)

m $A^1$ $A^2$ $A^3$ $R^1$ $R^2$ $R^3$ $R^4$ $R^5$ $R^6$ $R^7$ X und Y haben hierbei jeweils die oben angegebenen Bedeutungen.

Von den Verbindungen der Formeln (I-2A) und (I-2B) seien weiter auch diejenigen, bei denen $A^1$ für O (Sauerstoff) steht und $A^2$ für Ethan-1,2-diyl (Dimethylen) oder Propan-1,3-diyl (Trimethylen) steht, ganz besonders hervorgehoben.

Von den Verbindungen der Formel (I-2A) seien weiter auch diejenigen, bei denen m für null steht, ganz besonders hervorgehoben.

Von den Verbindungen der Formel (I-2B) seien weiter auch diejenigen, bei denen $R^5$ für Wasserstoff und $R^6$ für Methyl, Ethyl, n-, i- Propyl, n-, i-, s-, t-Butyl steht, ganz besonders hervorgehoben.

Die Restedefmitionen können untereinander, also auch zwischen den angegebenen bevorzugten Bereichen beliebig kombiniert werden. Außerdem können einzelne Restedefinitionen entfallen.

Erfindungsgemäß bevorzugt sind die Verbindungen der Formel (I-2), in welchen eine Kombination der vorstehend als bevorzugt aufgeführten Bedeutungen vorliegt.

Erfindungsgemäß besonders bevorzugt sind die Verbindungen der Formel (I-2), in welchen eine Kombination der vorstehend als besonders bevorzugt aufgeführten Bedeutungen vorliegt.

Erfindungsgemäß ganz besonders bevorzugt sind die Verbindungen der Formel (I-2), in welchen eine Kombination der vorstehend als ganz besonders bevorzugt aufgeführten Bedeutungen vorliegt.

Die oben aufgeführten allgemeinen oder in Vorzugsbereichen aufgeführten Restedefinitionen gelten sowohl für die Endprodukte der Formel (I-2) als auch entsprechend für die jeweils zur Herstellung benötigten Ausgangs- oder Zwischenprodukte.

Die neuen substituierten Arylketone der Formel (I-2) zeichnen sich durch starke und selektive herbizide Wirksamkeit aus.

Man erhält die neuen substituierten Arylketone der Formel (I-2), wenn man

a) Carbonsäuren der Formel (II)

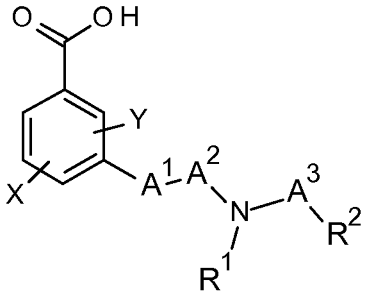

(II)

in welcher

$A^1$, $A^2$, $A^3$, $R^1$, $R^2$, X und Y die oben angegebene Bedeutung haben
- oder deren Alkali-, Erdalkali- oder Ammoniumsalze -
mit Verbindungen der allgemeinen Formel (III)

H-Z (III)

in welcher

Z die oben angegebene Bedeutung hat, oder

b) Carbonsäurederivate der Formel (IX)

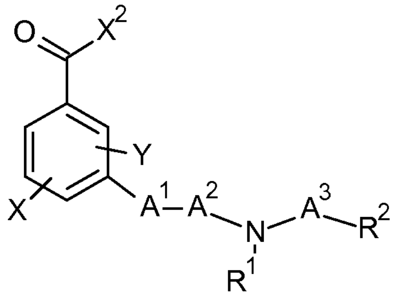

(IX)

in welcher

$A^1$, $A^2$, $A^3$, $R^1$, $R^2$, X und Y die oben angegebene Bedeutung haben und
$X^2$ für CN oder Halogen, bevorzugt Cl, Br, Imidazolyl oder Triazolyl steht

mit Verbindungen der Formel (III)

H-Z (III)

in welcher

Z die oben angegebene Bedeutung hat, oder

c) Verbindungen der Formel (XIII)

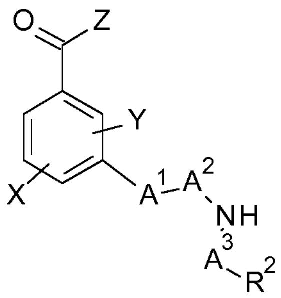

(XIII)

in welcher

$A^1$, $A^2$, $A^3$, $R^2$, X, Y und Z die oben angegebene Bedeutung haben mit Verbindungen der Formel (XI)

$X^1$-$R^1$ (XI)

in welcher

$R^1$ die oben angegebene Bedeutung hat und

$X^1$ für Halogen, Arylsulfonat oder Alkylsulfonat, bevorzugt Chlor, Brom, Jod, Mesylat oder Tosylat steht, oder

d) Verbindungen der Formel (XIV)

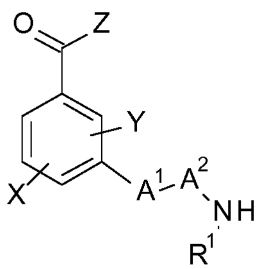

(XIV)

in welcher

$A^1$, $A^2$, $R^1$, X, Y und Z die oben angegebene Bedeutung haben mit Verbindungen der Formel (V)

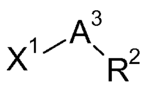

(V)

in welcher

$A^3$ und $R^2$ die oben angegebene Bedeutung haben und

$X^1$ für Halogen oder Tosylat, bevorzugt Chlor, Brom oder Tosylat steht, oder

e) Verbindungen der Formel (XV)

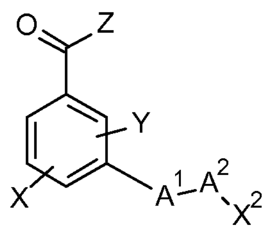

(XV)

in welcher

$A^1$, $A^2$, X, Y und Z die oben angegebene Bedeutung haben und

$X^2$ für Halogen oder Tosylat, bevorzugt Chlor, Brom oder Tosylat steht

mit Verbindungen der Formel (VII)

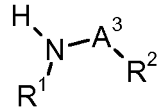

(VII)

in welcher

$A^3$, $R^1$ und $R^2$ die oben angegbene Bedeutung haben,

gegebenenfalls in Gegenwart eines oder mehrerer Reaktionshilfsmittel und gegebenenfalls in Gegenwart eines oder mehrerer Verdünnungsmittel, umsetzt.

Die Ausgangsstoffe der allgemeinen Formel (IX) sind noch nicht bekannt und als neue Stoffe Gegenstand der vorliegenden Anmeldung.
Man erhält die neuen Carbonsäurederivate der Formel (IX), wenn man
f) Carbonsäuren der Formel (II)

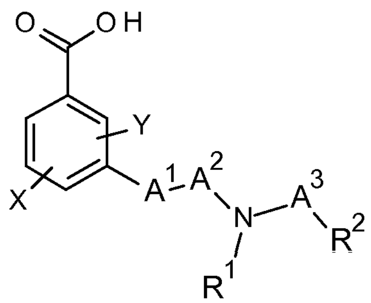

(II)

in welcher
$A^1$, $A^2$, $A^3$, $R^1$, $R^2$, X und Y die oben angegebene Bedeutung haben mit geeigneten Aktivierungsreagenzien, gegebenenfalls in Gegenwart eines oder mehrerer Reaktionshilfsmittel und gegebenenfalls in Gegenwart eines oder mehrerer Verdünnungsmittel, umsetzt.
Die Ausgangsstoffe der allgemeinen Formel (II) sind noch nicht bekannt und als neue Stoffe Gegenstand der vorliegenden Anmeldung. Diese Ausgangsstoffe sind ähnlich zu den Verbindungen 3,4-Difluor-2-(N-methyl-N-methylsulfonyl-amino-methyl)-benzoesäure, 2-(N-Methyl-N-methylsulfonyl-amino-methyl)-4-trifluormethyl-benzoesäure, 4-Fluor-2-(N-methyl-N-methylsulfonyl-amino-methyl)-benzoesäure, 2-(N-Methyl-N-methylsulfonyl-amino-methyl)-benzoesäure, 4-Chlor-2-(N-methyl-N-methylsulfonyl-amino-methyl)-benzoesäure, 4-Chlor-3-fluor-2-(N-methyl-N-methylsulfonyl-amino-methyl)-benzoesäure und 4-Chlor-3-(N-methyl-N-methylsulfonyl-amino-methyl)-2-methylthio-benzoesäure (vgl. WO-A-95/31446) sowie der Verbindung 2-Chlor-3-(methylsulfonyl-aminomethyl)-4-methylsulfonyl-benzoesäure (vgl. WO-A-00/21924).

Bevorzugt sind Verbindungen der Formel (II) in denen $A^1$, $A^2$, $R^1$, $R^2$, X und Y die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $A^1$, $A^2$, $R^1$, $R^2$, X und Y genannten Bedeutungen hat und
$A^3$ für O, S, die Gruppierung

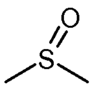 , 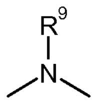 , 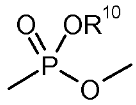 oder 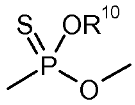

steht.
Man erhält die neuen Carbonsäuren der allgemeinen Formel (II), wenn man
g) Verbindungen der Formel (VIII)

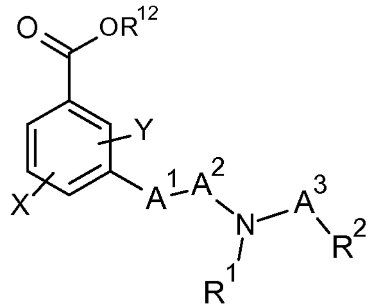

(VIII)

in welcher

$A^1$, $A^2$, $A^3$, $R^1$, $R^2$, X und Y die oben angegebene Bedeutung haben und

$R^{12}$ für $C_1$-$C_4$-Alkyl, insbesondere Methyl, Ethyl, n-, i-Propyl, n-, s-, i-, t-Butyl, für Allyl oder Benzyl steht

unter reduktiven oder alkalischen Bedingungen in Gegenwart eines oder mehrerer Reaktionshilfsmittel und gegebenenfalls in Gegenwart eines oder mehrerer Verdünnungsmittel, umsetzt.

Die Ausgangsstoffe der allgemeinen Formel (VIII) sind noch nicht bekannt und als neue Stoffe Gegenstand der vorliegenden Anmeldung.
Man erhält die neuen Carbonsäuren der allgemeinen Formel (VIII), wenn man

i) Verbindungen der Formel (IV)

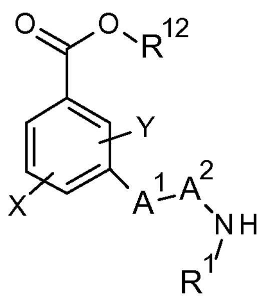

(IV)

in welcher

| | |
|---|---|
| $A^1$, $A^2$, $R^1$, X und Y | die oben angegebenen Bedeutungen haben und |
| $R^{12}$ | für $C_1$-$C_4$-Alkyl, insbesondere Methyl, Ethyl, n-, i-Propyl, n-, s-, i-, t-Butyl, für Allyl oder Benzyl steht, |

mit Verbindungen der Formel (V)

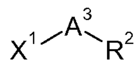

(V)

in welcher

$A^3$ und $R^2$ die oben angegebenen Bedeutungen haben und
$X^1$ für Halogen (vorzugsweise Fluor, Chlor, Brom oder Iod, insbesondere Chlor, Brom oder Iod) steht,

gegebenenfalls in Gegenwart eines oder mehrerer Reaktionshilfsmittel, und gegebenenfalls in Gegenwart eines oder mehrerer Verdünnungsmittel umsetzt,
oder wenn man

j) Verbindungen der Formel (X)

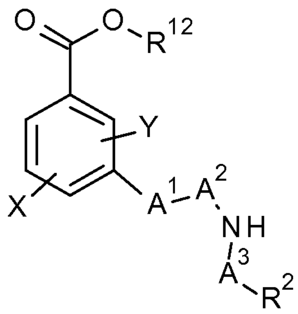

(X)

in welcher

$A^1$, $A^2$, $A^3$, $R^2$, X und Y die oben angegebenen Bedeutungen haben und
$R^{12}$ für $C_1$-$C_4$-Alkyl, insbesondere Methyl, Ethyl, n-, i-Propyl, n-, s-, i-, t-Butyl, für Allyl oder Benzyl steht,

mit Verbindungen der Formel (XI)

$X^1$-$R^1$ (XI)

in welcher

$R^1$ die oben angegebene Bedeutung hat und
$X^1$ für Halogen (vorzugsweise Fluor, Chlor, Brom oder Iod, insbesondere Chlor, Brom oder Iod) steht,

gegebenenfalls in Gegenwart eines oder mehrerer Reaktionshilfsmittel, und gegebenenfalls in Gegenwart eines oder mehrerer Verdünnungsmittel umsetzt,
oder wenn man

k) Verbindungen der Formel (X)

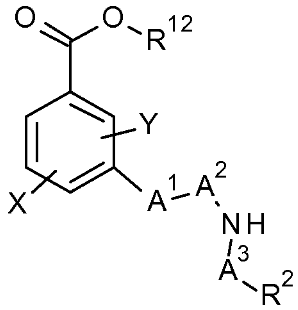

(X)

in welcher

$A^1$, $A^2$, $A^3$, $R^2$, X und Y die oben angegebenen Bedeutungen haben und

$R^{12}$ für $C_1$-$C_4$-Alkyl, insbesondere Methyl, Ethyl, n-, i-Propyl, n-, s-, i-, t-Butyl, steht

mit Verbindungen der Formel (XII)

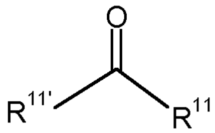

(XII)

in welcher

$R^{11'}$ und $R^{11}$ unabhängig voneinander für Wasserstoff, für gegebenenfalls durch Cyano, Halogen, $C_1$-$C_4$-Alkoxy, $C_1$-$C_4$-Alkylthio, $C_1$-$C_4$-Alkylsulfmyl oder $C_1$-$C_4$-Alkylsulfonyl substituiertes Alkyl mit 1 bis 6 Kohlenstoffatomen, für jeweils gegebenenfalls durch Cyano oder Halogen substituiertes Alkenyl oder Alkinyl mit jeweils 3 bis 6 Kohlenstoffatomen, für jeweils gegebenenfalls durch Cyano, Halogen oder $C_1$-$C_4$-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl substituiertes Aryl oder Arylalkyl mit jeweils 6 oder 10 Kohlenstoffatomen in der Arylgruppe und gegebenenfalls 1 bis 4 Kohlenstoffatomen im Alkylteil,

bevorzugt für Wasserstoff, Methyl, Ethyl, n-Pr, i-Pr, Cyclopropyl, n-Butyl, i-Butyl, Cyclobutyl, Phenyl, Benzyl und Tolyl stehen,
in Gegenwart eines Reduktionsmittels, vorzugsweise eines Borans oder eines $BH_3$-Adduktes,
gegebenenfalls in Gegenwart eines oder mehrerer Reaktionshilfsmittel und gegebenenfalls in Gegenwart eines oder mehrerer Verdünnungsmittel umsetzt.

Gegebenenfalls können im Anschluss an die Durchführung der erfindungsgemäßen Verfahren a) bis e) an den so erhaltenen Verbindungen der allgemeinen Formel (I-2) im Rahmen der Substituentendefinition Folgeumsetzungen (beispielsweise Substitutions-, Oxidations- oder Reduktionsreaktionen) zur Umwandlung in andere Verbindungen der allgemeinen Formel (I-2) nach üblichen Methoden durchführt werden.
Gegebenenfalls können mehrere der vorstehend genannten Verfahrensschritte kombiniert werden, indem die Reaktionsprodukte nicht isoliert sondern im Folgenden Verfahrensschritt direkt eingesetzt werden.
Im Folgenden wird das erfindungsgemäße Verfahren a) näher erläutert:

Die als Ausgangsstoffe zu verwendenden Carbonsäuren sind durch die Formel (II) allgemein definiert. In der Formel (II) haben $A^1$, $A^2$, $A^3$, $R^1$, $R^2$, X und Y vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $A^1$, $A^2$, $A^3$, $R^1$, $R^2$, X und Y angegeben worden sind. Die Ausgangsstoffe der Formel (II) sind beispielsweise nach Verfahren g) erhältlich.

Die weiter als Ausgangsstoffe zu verwendenden Verbindungen sind durch die Formel (III) allgemein definiert. In der Formel (III) hat Z vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für Z, $R^3$, $R^4$, $R^5$, $R^6$, $R^7$ angegeben worden sind. Die Ausgangsstoffe der Formel (III) sind bekannt oder können nach bekannten Verfahren hergestellt werden.

Zur Durchführung des Verfahrens a) werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Als Reaktionshilfsmittel kommen im Allgemeinen die üblichen anorganischen oder organischen Basen oder Säureakzeptoren in Betracht. Hierzu gehören vorzugsweise Alkalimetall- oder Erdalkalimetall-, -acetate, -amide, -carbonate, -hydrogencarbonate, -hydride, -hydroxide oder -alkanolate, wie beispielsweise Natrium-, Kalium- oder Calciumacetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calciumhydroxid, Natrium- oder Kalium-, -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyldiisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethylpyridin, 5-Ethyl-2-methyl-pyridin, 4-Dimethylamino-pyridin, N-Methyl-piperidin, N-Ethyl-piperidin, N-Methyl-morpholin, N-Ethyl-morpholin, 1,4-Diazabicyclo[2.2.2]-octan (DABCO), 1,5-Diazabicyclo[4.3.0]-non-5-en (DBN), oder 1,8-Diazabicyclo-[5.4.0]-undec-7-en (DBU).

Das erfindungsgemäße Verfahren a) wird gegebenenfalls unter Verwendung eines Dehydratisierungsmittels durchgeführt. Es kommen hierbei die üblichen zur Bindung von Wasser geeigneten Chemikalien in Betracht. Als Beispiele hierfür werden Dicyclohexylcarbodiimid, Carbonyl-bis-imidazol und Propanphosphonsäureanhydrid genannt. Als besonders gut geeignete Dehydratisierungsmittel seien Dicyclohexylcarbodiimid und Propanphosphonsäureanhydrid genannt (vgl. WO 99/28282).

Das erfindungsgemäße Verfahren a) wird gegebenenfalls unter Verwendung eines Umlagerungsreagenzes durchgeführt. Es kommen hierbei die üblichen zur Umlagerung geeigneten Chemikalien in Betracht. Als Beispiele hierfür werden Trimethylsilylcyanid, Kaliumcyanid und Acetoncyanhydrin genannt.

Das Verfahren a) zur Herstellung der Verbindungen der allgemeinen Formel (I-2) wird vorzugsweise unter Verwendung eines oder mehrerer Verdünnungsmittel durchgeführt. Als Verdünnungsmittel kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester; Sulfoxide, wie Dimethylsulfoxid.

Die Reaktionstemperaturen können bei der Durchführung des Verfahrens a) in einem größeren Bereich variiert werden. Im Allgemeinen arbeitet man bei Temperaturen zwischen 0°C und 150°C, vorzugsweise zwischen 10°C und 120°C.

Das Verfahren a) wird im Allgemeinen unter Normaldruck durchgeführt. Es ist jedoch auch möglich, erfindungsgemäße Verfahren unter erhöhtem oder vermindertem Druck - im Allgemeinen zwischen 0,1 bar und 10 bar - durchzuführen.

Verwendet man im Verfahren a) beispielsweise 4-Chlor-3-(ethoxyaminomethyl)-benzoesäure und Cyclohexan-1,3-dion als Ausgangsstoffe, so kann der Reaktionsablauf beim erfindungsgemäßen Verfahren durch das folgende Formelschema skizziert werden:

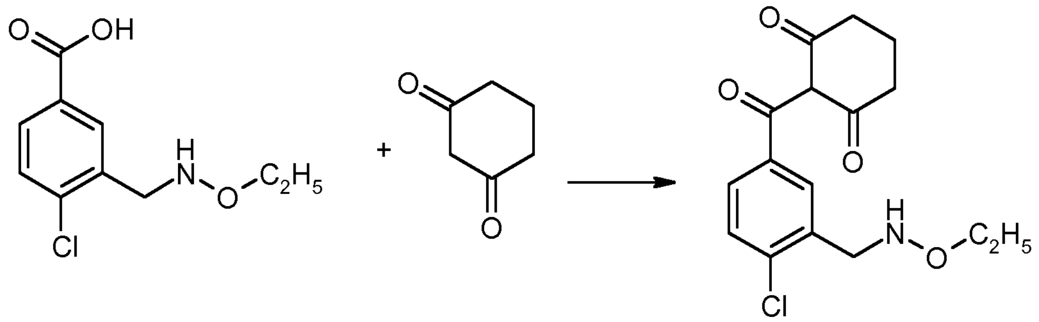

Im Folgenden wird das erfindungsgemäße Verfahren b) näher erläutert:

Die als Ausgangsstoffe zu verwendenden Carbonsäurederivate sind durch die Formel (IX) allgemein definiert. In der Formel (IX) haben $A^1$, $A^2$, $A^3$, $R^1$, $R^2$, X und Y vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $A^1$, $A^2$, $A^3$, $R^1$, $R^2$, X und Y angegeben worden sind. Die Ausgangsstoffe der Formel (IX) sind beispielsweise nach Verfahren f) erhältlich.

Die weiter als Ausgangsstoffe zu verwendenden Verbindungen sind durch die Formel (III) allgemein definiert. In der Formel (III) hat Z vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für Z, $R^3$, $R^4$, $R^5$, $R^6$, $R^7$ angegeben worden sind. Die Ausgangsstoffe der Formel (III) sind bekannt oder können nach bekannten Verfahren hergestellt werden.

Zur Durchführung des Verfahrens b) werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Als Reaktionshilfsmittel kommen im Allgemeinen die üblichen anorganischen oder organischen Basen oder Säureakzeptoren in Betracht. Hierzu gehören vorzugsweise Alkalimetall- oder Erdalkalimetall-, -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium-, -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyldiisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethylpyridin, 5-Ethyl-2-methyl-pyridin, 4-Dimethylamino-pyridin, N-Methyl-piperidin, N-Ethyl-piperidin, N-Methyl-morpholin, N-Ethyl-morpholin, 1,4-Diazabicyclo[2.2.2]-octan (DABCO), 1,5-Diazabicyclo[4.3.0]-non-5-en (DBN), oder 1,8-Diazabicyclo-[5.4.0]-undec-7-en (DBU).

Das erfindungsgemäße Verfahren b) wird gegebenenfalls unter Verwendung eines Umlagerungsreagenzes durchgeführt. Es kommen hierbei die üblichen zur Umlagerung geeigneten Chemikalien in Betracht. Als Beispiele hierfür werden Trimethylsilylcyanid, Kaliumcyanid und Acetoncyanhydrin genannt.

Das Verfahren b) wird vorzugsweise unter Verwendung eines oder mehrerer Verdünnungsmittel durchgeführt. Als Verdünnungsmittel kommen vor allem die in Verfahren a) genannten Lösungsmittel in Betracht.

Die Reaktionstemperaturen können bei der Durchführung des Verfahrens b) in einem größeren Bereich variiert werden. Im Allgemeinen arbeitet man bei Temperaturen zwischen 0°C und 150°C, vorzugsweise zwischen 10°C und 120°C.

Das Verfahren b) wird im Allgemeinen unter Normaldruck durchgeführt. Es ist jedoch auch möglich, erfindungsgemäße Verfahren unter erhöhtem oder vermindertem Druck - im Allgemeinen zwischen 0,1 bar und 10 bar - durchzuführen.

Im Folgenden wird das erfindungsgemäße Verfahren c) näher erläutert:

Die als Ausgangsstoffe zu verwendenden sekundären Amine sind durch die Formel (XIII) allgemein definiert. In der Formel (XIII) haben $A^1$, $A^2$, $A^3$, $R^2$, X, Y und Z vorzugsweise diejenigen Bedeutungen, die bereits oben im Zusammenhang mit der Beschreibung der erfindungsgemäßen Verbindungen der allgemeinen Formel (I-2) als bevorzugt,

besonders bevorzugt, ganz besonders bevorzugt oder am meisten bevorzugt für $A^1$, $A^2$, $A^3$, $R^2$, X, Y und Z angegeben worden sind. Die Ausgangsstoffe der Formel (XIII) sind bekannt oder nach bekannten Verfahren erhältlich.

Die weiter als Ausgangsstoffe zu verwendenden Verbindungen sind durch die Formel (XI) allgemein definiert. In der Formel (XI) hat $R^1$ vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $R^1$ angegeben worden sind. Die Ausgangsstoffe der Formel (XI) sind bekannt oder nach bekannten Verfahren erhältlich.

Zur Durchführung des Verfahrens c) werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt.

Als Reaktionshilfsmittel kommen im Allgemeinen die üblichen anorganischen oder organischen Basen oder Säureakzeptoren in Betracht. Hierzu gehören vorzugsweise Alkalimetall- oder Erdalkalimetall-, -acetate, -amide, -carbonate, -hydrogencarbonate, -hydride, -hydroxide oder -alkanolate, wie beispielsweise Natrium-, Kalium- oder Calciumacetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calciumhydroxid, Natrium- oder Kalium-, -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyldiisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethylpyridin, 5-Ethyl-2-methyl-pyridin, 4-Dimethylamino-pyridin, N-Methyl-piperidin, N-Ethyl-piperidin, N-Methyl-morpholin, N-Ethyl-morpholin, 1,4-Diazabicyclo[2.2.2]-octan (DABCO), 1,5-Diazabicyclo[4.3.0]-non-5-en (DBN), oder 1,8-Diazabicyclo-[5.4.0]-undec-7-en (DBU).

Das Verfahren c) wird vorzugsweise unter Verwendung eines oder mehrerer Verdünnungsmittel durchgeführt. Als Verdünnungsmittel kommen vor allem Wasser und inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester; Sulfoxide, wie Dimethylsulfoxid.

Die Reaktionstemperaturen können bei der Durchführung des Verfahrens c) in einem größeren Bereich variiert werden. Im Allgemeinen arbeitet man bei Temperaturen zwischen 0°C und 150°C, vorzugsweise zwischen 10°C und 120°C.

Das Verfahren c) wird im Allgemeinen unter Normaldruck durchgeführt. Es ist jedoch auch möglich, erfindungsgemäße Verfahren unter erhöhtem oder vermindertem Druck - im Allgemeinen zwischen 0,1 bar und 10 bar - durchzuführen.

Im Folgenden wird das erfindungsgemäße Verfahren d) näher erläutert:

Die als Ausgangsstoffe zu verwendenden sekundären Amine sind durch die Formel (XIV) allgemein definiert. In der Formel (XIV) haben $A^1$, $A^2$, $R^1$, X, Y und Z vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $A^1$, $A^2$, $R^1$, X, Y und Z angegeben worden sind. Die Ausgangsstoffe der Formel (XIV) sind bekannt oder können nach bekannten Verfahren hergestellt werden.

Die weiter als Ausgangsstoffe zu verwendenden Verbindungen sind durch die Formel (V) allgemein definiert. In der Formel (V) hat $R^2$ und $A^3$ vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $R^2$ und $A^3$ angegeben worden sind. Die Ausgangsstoffe der Formel (V) sind bekannt oder können nach bekannten Verfahren hergestellt werden.

Zur Durchführung des Verfahrens d) werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Als Reaktionshilfsmittel kommen im Allgemeinen die üblichen anorganischen oder organischen Basen oder Säureakzeptoren in Betracht. Hierzu gehören vorzugsweise die in Verfahren c) genannten Verbindungen.

Das Verfahren d) zur Herstellung der Verbindungen der allgemeinen Formel (I-2) wird vorzugsweise unter Verwendung eines oder mehrerer Verdünnungsmittel durchgeführt. Als Verdünnungsmittel kommen vorzugsweise die in Verfahren c) genannten Verdünnungsmittel in Betracht.

Die Reaktionstemperaturen können bei der Durchführung des Verfahrens d) in einem größeren Bereich variiert werden. Im Allgemeinen arbeitet man bei Temperaturen zwischen 0°C und 150°C, vorzugsweise zwischen 10°C und 120°C.

Das Verfahren d) wird im Allgemeinen unter Normaldruck durchgeführt. Es ist jedoch auch möglich, erfindungsgemäße Verfahren unter erhöhtem oder vermindertem Druck - im Allgemeinen zwischen 0,1 bar und 10 bar - durchzuführen.

Im Folgenden wird das erfindungsgemäße Verfahren e) näher erläutert:

Die als Ausgangsstoffe zu verwendenden Verbindungen sind durch die Formel (XV) allgemein definiert. In der Formel (XV) haben $A^1$, $A^2$, X, Y und Z vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $A^1$, $A^2$, $R^1$, X, Y und Z angegeben worden sind. Die Ausgangsstoffe der Formel (XV) sind bekannt oder können nach bekannten Verfahren hergestellt werden.

Die weiter als Ausgangsstoffe zu verwendenden Amine sind durch die Formel (VII) allgemein definiert. In der Formel (VII) hat $R^1$, $R^2$ und $A^3$ vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $R^1$, $R^2$ und $A^3$ angegeben worden sind. Die Ausgangsstoffe der Formel (VII) sind bekannt oder können nach bekannten Verfahren hergestellt werden.

Zur Durchführung des Verfahrens e) werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt.

Als Reaktionshilfsmittel kommen im Allgemeinen die üblichen anorganischen oder organischen Basen oder Säureakzeptoren in Betracht. Hierzu gehören vorzugsweise die in Verfahren c) genannten Verbindungen.

Das Verfahren e) zur Herstellung der Verbindungen der Formel (I-2) wird vorzugsweise unter Verwendung eines oder mehrerer Verdünnungsmittel durchgeführt. Als Verdünnungsmittel kommen vorzugsweise die in Verfahren c) genannten Verdünnungsmittel in Betracht.

Die Reaktionstemperaturen können bei der Durchführung des Verfahrens e) in einem größeren Bereich variiert werden. Im Allgemeinen arbeitet man bei Temperaturen zwischen 0°C und 150°C, vorzugsweise zwischen 10°C und 120°C.

Das Verfahren e) wird im Allgemeinen unter Normaldruck durchgeführt. Es ist jedoch auch möglich, erfindungsgemäße Verfahren unter erhöhtem oder vermindertem Druck - im Allgemeinen zwischen 0,1 bar und 10 bar - durchzuführen.

Im Folgenden wird das erfindungsgemäße Verfahren f) näher erläutert:

Die als Ausgangsstoffe zu verwendenden Carbonsäuren sind durch die Formel (II) allgemein definiert. In der Formel (II) haben $A^1$, $A^2$, $A^3$, $R^1$, $R^2$, X und Y vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $A^1$, $A^2$, $A^3$, $R^1$, $R^2$, X und Y angegeben worden sind. Die Ausgangsstoffe der Formel (II) sind beispielsweise nach Verfahren g) erhältlich.

Als Aktivierungsreagenzien seien Halogenierungsmittel, insbesondere Phosphorylchlorid oder -bromid, Sulfurylchlorid, Oxalylchlorid, Phosgen; Cyanierungsmitteln insbesondere Cyanhydrin oder Cyanwasserstoffsäure; Imidazol bzw. Imidazolderivate wie Bisimidazolylcarbonyl und Triazol bzw. Triazolderivate wie beispielsweise Bistriazolylcarbonyl genannt.

Zur Durchführung des Verfahrens f) werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt.

Als Reaktionshilfsmittel kommen im Allgemeinen die üblichen anorganischen oder organischen Basen oder Säureakzeptoren in Betracht. Hierzu gehören vorzugsweise

Alkalimetall- oder Erdalkalimetall-, -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calciumhydroxid, Natrium- oder Kalium-, -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyldiisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethylpyridin, 5-Ethyl-2-methyl-pyridin, 4-Dimethylamino-pyridin, N-Methyl-piperidin, N-Ethyl-piperidin, N-Methyl-morpholin, N-Ethyl-morpholin, 1,4-Diazabicyclo[2.2.2]-octan (DABCO), 1,5-Diazabicyclo[4.3.0]-non-5-en (DBN), oder 1,8-Diazabicyclo-[5.4.0]-undec-7-en (DBU).

Das Verfahren f) zur Herstellung der Verbindungen der allgemeinen Formel (I-2) wird vorzugsweise unter Verwendung eines oder mehrerer Verdünnungsmittel durchgeführt. Als Verdünnungsmittel kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester; Sulfoxide, wie Dimethylsulfoxid.

Die Reaktionstemperaturen können bei der Durchführung des Verfahrens f) in einem größeren Bereich variiert werden. Im Allgemeinen arbeitet man bei Temperaturen zwischen -30°C und 150°C, vorzugsweise zwischen -10°C und 100°C.

Das Verfahren f) wird im Allgemeinen unter Normaldruck durchgeführt. Es ist jedoch auch möglich, erfindungsgemäße Verfahren unter erhöhtem oder vermindertem Druck - im Allgemeinen zwischen 0,1 bar und 10 bar - durchzuführen.

Im Folgenden wird das erfindungsgemäße Verfahren g) näher erläutert:

Die als Ausgangsstoffe zu verwendenden Carbonsäureester sind durch die Formel (VIII) allgemein definiert. In der Formel (VIII) haben $A^1$, $A^2$, $A^3$, $R^1$, $R^2$, X und Y vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $A^1$, $A^2$, $A^3$, $R^1$, $R^2$, X und Y angegeben worden sind. Die Ausgangsstoffe der Formel (VIII) sind beispielsweise nach Verfahren i), j), k) erhältlich.

Die Verfahren g) kann unter alkalischen, sauren oder reduktiven Bedingungen durchgeführt werden.

Als Reaktionshilfsmittel kommen im Allgemeinen die üblichen anorganischen oder organischen Basen oder Säureakzeptoren in Betracht. Hierzu gehören vorzugsweise Alkalimetall- oder Erdalkalimetall-, -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium-, -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyldiisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethylpyridin, 5-Ethyl-2-methyl-pyridin, 4-Dimethylamino-pyridin, N-Methyl-piperidin, N-Ethyl-piperidin, N-Methyl-morpholin, N-Ethyl-morpholin, 1,4-Diazabicyclo[2.2.2]-octan (DABCO), 1,5-Diazabicyclo[4.3.0]-non-5-en (DBN), oder 1,8-Diazabicyclo-[5.4.0]-undec-7-en (DBU).

Als Reaktionshilfsmittel kommen weiterhin die üblichen Hydrierkatalysatoren wie Palladium und Platin auf heterogenen Trägern wie Aktivkohle, Bariumsulfat in Betracht.

Als Reaktionshilfsmittel kommen weiterhin Reduktionsmittel wie Alanate und Boranate, wie Kaliumalanat, Natriumboranat in Betracht.

Das Verfahren g) zur Herstellung der Verbindungen der allgemeinen Formel (II) wird vorzugsweise unter Verwendung eines oder mehrerer Verdünnungsmittel durchgeführt. Als Verdünnungsmittel kommen vor allem Wasser und inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon

oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester; Sulfoxide, wie Dimethylsulfoxid.

Die Reaktionstemperaturen können bei der Durchführung des Verfahrens g) in einem größeren Bereich variiert werden. Im Allgemeinen arbeitet man bei Temperaturen zwischen 0°C und 150°C, vorzugsweise zwischen 10°C und 120°C.

Das Verfahren g) wird im Allgemeinen unter Normaldruck durchgeführt. Es ist jedoch auch möglich, erfindungsgemäße Verfahren unter erhöhtem oder vermindertem Druck - im Allgemeinen zwischen 0,1 bar und 100 bar, bevorzugt zwischen Normaldruck und 20 bar, besonders bevorzugt zwischen Normaldruck und 10 bar - durchzuführen. Wird das Verfahren g) unter erhöhtem Druck durchgeführt, ist ein erhöher Wasserstoffpartialdruck bevorzugt.

Im Folgenden wird das erfindungsgemäße Verfahren i) näher erläutert:

Die als Ausgangsstoffe zu verwendenden Verbindungen sind durch die Formel (IV) allgemein definiert. In der Formel (IV) haben $A^1$, $A^2$, $R^1$, X und Y vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $A^1$, $A^2$, $R^1$, X und Y angegeben worden sind. Die Ausgangsstoffe der Formel (IV) sind bekannt oder nach bekannten Verfahren erhältlich (vgl. WO-A-95/31446, WO-A-01/53275, DE-A-10122445, Herstellungsbeispiele).

Die weiter als Ausgangsstoffe zu verwendenden Verbindungen sind durch die Formel (V) allgemein definiert. In der Formel (V) hat $A^3$ und $R^2$ vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $A^3$ und $R^2$ angegeben worden sind. Die Ausgangsstoffe der Formel (III) sind bekannt oder können nach bekannten Verfahren hergestellt werden.

Zur Durchführung des Verfahrens i) werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Als Reaktionshilfsmittel kommen im Allgemeinen die üblichen anorganischen oder organischen Basen oder Säureakzeptoren in Betracht. Hierzu gehören vorzugsweise Alkalimetall- oder Erdalkalimetall-, -acetate,-amide,-carbonate,-hydrogencarbonate, -hydride, -hydroxide oder-alkanolate, wie beispielsweise Natrium-, Kaliumoder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kaliumoder Calciumhydrid, Lithium-, Natrium-, Kalium- oder Calcium-hydroxid, Natrium-oder Kalium-,-methanolat,-ethanolat,-n-oder-i- propanolat, -n-, -i-,-s-oder-t-butanolat ; weiterhin auch basische organische Stickstoffverbindungen, wie beispielsweise Trimethylamin, Triethylamin, Tripropylamin, Tributylamin, Ethyl-diisopropylamin, N, N-Dimethylcyclohexylamin, Dicyclohexylamin, Ethyl-dicyclohexylamin, N, N-Dimethyl-anilin, N, N-Dimethyl-benzylamin, Pyridin, 2-Methyl-, 3-Methyl-, 4-Methyl-, 2, 4-Dimethyl-, 2,6-Dimethyl-, 3,4-Dimethyl- und 3,5-Dimethyl-pyridin, 5-Ethyl-2-methyl-pyridin, 4-Dimethylaminopyridin, N-Methyl-piperidin, N-Ethyl-piperidin, N-Methyl-morpholin, N-Ethylmorpholin, 1, 4-Diazabicyclo [2.2. 2]-octan (DABCO), 1, 5-Diazabicyclo [4.3. 0]-non-5- en (DBN), oder 1, 8-Diazabicyclo [5.4. 0] -undec-7-en (DBU).

Das Verfahren i) zur Herstellung der Verbindungen der allgemeinen Formel (VIII) wird vorzugsweise unter Verwendung eines oder mehrerer Verdünnungsmittel durchgeführt. Als Verdünnungsmittel kommen vor allem Wasser und inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff ; Ether, wie Diethylether, Diisopropylether, Dioxan, Tetrahydrofuran oder Ethylenglykoldimethyl-oder-diethylether ; Ketone, wie Aceton, Butanon oder Methyl-isobutylketon ; Nitrile, wie Acetonitril, Propionitril oder Butyronitril; Amide, wie N, N-Dimethylformamid, N, N-Dimethylacetamid, N-Methylformanilid, N-Methyl-pyrrolidon oder Hexamethylphosphorsäuretriamid ; Ester wie Essigsäuremethylester oder Essigsäureethylester, Sulfoxide, wie Dimethylsulfoxid. Die Reaktionstemperaturen können bei der Durchführung des Verfahrens i) in einem größeren Bereich variiert werden. Im Allgemeinen arbeitet man bei Temperaturen zwischen 0°C und 150°C, vorzugsweise zwischen 10°C und 120°C.

Das Verfahren i) wird im Allgemeinen unter Normaldruck durchgeführt. Es ist jedoch auch möglich, erfindungsgemäße Verfahren unter erhöhtem oder vermindertem Druck - im Allgemeinen zwischen 0,1 bar und 10 bar - durchzuführen.

Im Folgenden wird das erfindungsgemäße Verfahren j) näher erläutert:

Die als Ausgangsstoffe zu verwendenden Verbindungen sind durch die Formel (X) allgemein definiert. In der Formel

(X) haben $A^1$, $A^2$, $A^3$, $R^2$, X und Y vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $A^1$, $A^2$, $A^3$, $R^2$, X und Y angegeben worden sind. Die Ausgangsstoffe der Formel (X) sind bekannt oder nach bekannten Verfahren erhältlich (vgl. WO-A-95/31446, WO-A-01/53275, DE-A-10122445, Herstellungsbeispiele).

Die weiter als Ausgangsstoffe zu verwendenden Verbindungen sind durch die Formel (XI) allgemein definiert. In der Formel (XI) hat $R^1$ vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $R^1$ angegeben worden sind. Die Ausgangsstoffe der Formel (XI) sind bekannt oder können nach bekannten Verfahren hergestellt werden.

Zur Durchführung des Verfahrens j) werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Als Reaktionshilfsmittel kommen im Allgemeinen die üblichen anorganischen oder organischen Basen oder Säureakzeptoren in Betracht. Hierzu gehören vorzugsweise die in Verfahren i) genannten Reaktionshilfsmittel.

Das Verfahren j) zur Herstellung der Verbindungen der Formel (VIII) wird vorzugsweise unter Verwendung eines oder mehrerer Verdünnungsmittel durchgeführt. Als Verdünnungsmittel kommen vor allem die in Verfahren i) genannten Verdünnungsmittel.

Die Reaktionstemperaturen können bei der Durchführung des Verfahrens j) in einem größeren Bereich variiert werden. Im Allgemeinen arbeitet man bei Temperaturen zwischen 0°C und 150°C, vorzugsweise zwischen 10°C und 120°C.

Das Verfahren j) wird im Allgemeinen unter Normaldruck durchgeführt. Es ist jedoch auch möglich, erfindungsgemäße Verfahren unter erhöhtem oder vermindertem Druck - im Allgemeinen zwischen 0,1 bar und 10 bar - durchzuführen.

Im Folgenden wird das erfindungsgemäße Verfahren k) näher erläutert:

Die als Ausgangsstoffe zu verwendenden Verbindungen sind durch die Formel (X) allgemein definiert. In der Formel (X) haben $A^1$, $A^2$, $A^3$, $R^2$, X und Y vorzugsweise diejenigen Bedeutungen, die bereits oben im Zusammenhang mit der Beschreibung der erfindungsgemäßen Verbindungen der allgemeinen Formel (I-2) als bevorzugt, besonders bevorzugt, ganz besonders bevorzugt oder am meisten bevorzugt für $A^1$, $A^2$, $A^3$, $R^2$, X und Y angegeben worden sind. Die Ausgangsstoffe der Formel (X) sind bekannt oder nach bekannten Verfahren erhältlich (vgl. WO-A-95/31446, WO-A-01/53275, DE-A-10122445, Herstellungsbeispiele).

Die weiter als Ausgangsstoffe zu verwendenden Verbindungen sind durch die Formel (XII) allgemein definiert. In der Formel (XII) haben $R^{11'}$ und $R^{11}$ diejenigen Bedeutungen, die bereits oben im Zusammenhang mit der Beschreibung des Verfahrens k) als bevorzugt, besonders bevorzugt, ganz besonders bevorzugt oder am meisten bevorzugt für $R^{11'}$ und $R^{11}$ angegeben worden sind. Die Ausgangsstoffe der Formel (XII) sind bekannt oder können nach bekannten Verfahren hergestellt werden.

Zur Durchführung des Verfahrens k) werden die Ausgangsstoffe im Allgemeinen in angenähert äquimolaren Mengen eingesetzt. Es ist jedoch auch möglich, eine der Komponenten in einem größeren Überschuss zu verwenden. Die Umsetzung wird im Allgemeinen in einem geeigneten Verdünnungsmittel in Gegenwart eines Reaktionshilfsmittels durchgeführt und das Reaktionsgemisch wird im Allgemeinen mehrere Stunden bei der erforderlichen Temperatur gerührt. Die Aufarbeitung wird nach üblichen Methoden durchgeführt (vgl. die Herstellungsbeispiele).

Als Reaktionshilfsmittel kommen im Allgemeinen die üblichen anorganischen oder organischen Basen oder Säureakzeptoren in Betracht. Hierzu gehören vorzugsweise die in Verfahren i) genannten Reaktionshilfsmittel.

Das Verfahren k) wird vorzugsweise unter Verwendung eines oder mehrerer Verdünnungsmittel durchgeführt. Als Verdünnungsmittel kommen vor allem die in Verfahren i) genannten Verdünnungsmittel in Betracht.

Die Reaktionstemperaturen können bei der Durchführung des Verfahrens k) in einem größeren Bereich variiert werden. Im Allgemeinen arbeitet man bei Temperaturen zwischen -30°C und 150°C, vorzugsweise zwischen -10°C und 80°C.

Das Verfahren k) wird im Allgemeinen unter Normaldruck durchgeführt. Es ist jedoch auch möglich, erfindungsgemäße Verfahren unter erhöhtem oder vermindertem Druck - im Allgemeinen zwischen 0,1 bar und 10 bar - durchzuführen.

Das Verfahren k) wird beispielsweise in Synlett 1997, 859-861 beschrieben.

Die erfindungsgemäßen Wirkstoffe können als Defoliants, Desiccants, Krautabtötungsmittel und insbesondere

als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.

Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I-2) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden, gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit bestimmten Eigenschaften ("Traits"), die durch konventionelle Züchtung, durch Mutagenese, oder auch durch rekombinante DNA-Techniken erhalten worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel - auch in Kombination

mit anderen agrochemischen Wirkstoffen, besseres Wachstum der Kulturpflanzen, erhöhte Toleranz der Kulturpflanzen gegenüber hohen oder niedrigen Temperaturen, erhöhte Toleranz der Kulturpflanzen gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinothricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid-tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinothricin, z.B. Raps), IMI® (Toleranz gegen Imidazolinone) und STS® (Toleranz gegen Sulfonylharnstoffe z.B. Mais) vertrieben werden. Als Herbizidresistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise

Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flufenpyr, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, - meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Ketospiradox, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Penoxysulam, Pentoxazone, Pethoxamid, Phenmedipham, Picolinafen, Piperophos, Pretilachlor, Primisulfuron (-methyl), Profluazol, Profoxydim, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Für die Mischungen kommen weiterhin bekannte Safener in Frage, beispielsweise

AD-67, BAS-145138, Benoxacor, Cloquintocet (-mexyl), Cyometrinil, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Die aufgeführten Pflanzen können besonders vorteilhaft erfindungsgemäß mit den Verbindungen der allgemeinen Formel (I-2) bzw. den erfindungsgemäßen Wirkstoffmischungen behandelt werden, wobei zusätzlich zu der guten Bekämpfung der Unkrautpflanzen die oben genannten synergistischen Effekte mit den transgenen Pflanzen oder Pflanzensorten auftreten. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe geht aus den nachfolgenden Beispielen hervor.

**Herstellungsbeispiele:**

Beispiel 1 (Verfahren a)

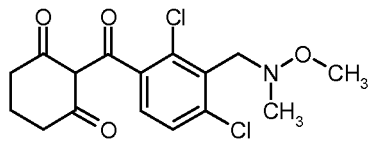

Eine Mischung aus 535 mg (4,77 mMol) 1,3-Cyclohexandion, 1,20 g (4,54 mMol) 2,4-Dichlor-3-(N-methoxy-N-methylamino-methyl)-benzoesäure, 1,25 g (5,45 mMol) Dicyclohexylcarbodiimid und 50 ml Acetonitril wird 15 Stunden bei Raumtemperatur (ca. 20°C) gerührt. Dann werden 0,92 g (9,09 mMol) Triethylamin und 0,18 g (1,82 mMol) Trimethylsilylcyanid zu dieser Mischung gegeben und die Reaktionsmischung wird 15 Stunden bei Raumtemperatur gerührt. Anschließend wird unter vermindertem Druck eingeengt, der Rückstand mit gesättigter wässriger Natriumcarbonat-Lösung verrührt, mit Diethylether versetzt und filtriert. Die wässrige Phase des Filtrats wird abgetrennt, mit 2N-Salzsäure angesäuert und mit Methylenchlorid extrahiert. Die organische Extraktionslösung wird mit Wasser gewaschen, mit Natriumsulfat getrocknet und filtriert. Das Filtrat wird unter vermindertem Druck eingeengt und der Rückstand durch präparative HPLC (high performance liquid chromatography) gereinigt.

Man erhält 0,7 g (43 % der Theorie) 2-[2,4-Dichlor-3-(N-methoxy-N-methyl-aminomethyl)-benzoyl]-1,3-cyclohexandion.
LogP = 3,04.

Beispiel 2 (Verfahren d)

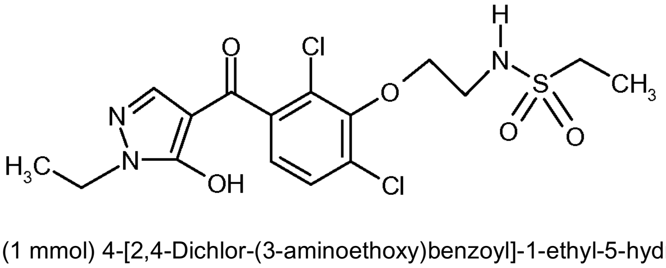

Eine Mischung aus 500 mg (1 mmol) 4-[2,4-Dichlor-(3-aminoethoxy)benzoyl]-1-ethyl-5-hydroxy-pyrazol und 400 mg (3

mmol) Kaliumcarbonat wird für 1h 20 ml Dimethylsulfoxid bei Raumtemperatur (ca. 20°C) gerührt. Dann werden 300 mg (2 mmol) Ethylsulfonsäurechlorid bei Raumtemperatur zugegeben und für 18 h bei dieser Temperatur gerührt. Anschließend werden 40 ml Wasser zugegeben. Die Reaktionsmischung wird dreimal mit 30 ml Dichlormethan extrahiert. Die organische Phase wird zweimal mit 20 ml Wasser gewaschen, mit Natriumsulfat getrocknet und filtriert. Das Filtrat wird unter vermindertem Druck eingeengt und chromatografisch gereinigt (Laufmittel:Cyclohexan:Essigsäureethylester = 3:7).

Man erhält 300 mg 4-[2,4-Dichlor-3-(N-ethylsulfonyl-amino-ethoxy)benzoyl]-1-ethyl-5-hydroxypyrazol.

Analog zu den Beispielen 1 und 2 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I-2) - bzw. der Formeln (I-2A) und (I-2B) - hergestellt werden.

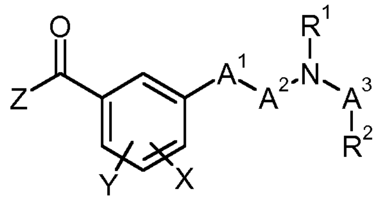

(I-2)

Die in Tabelle 1 verwendeten Kurzzeichen haben die folgende Bedeutung:

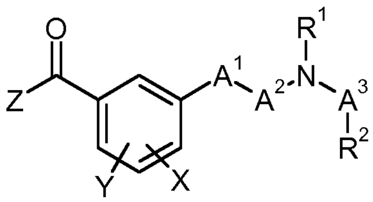

(I-2)

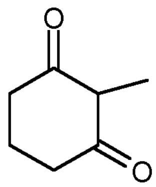

(Z1)

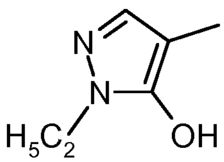

(Z2)

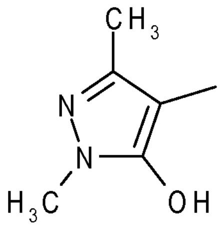

(Z3)

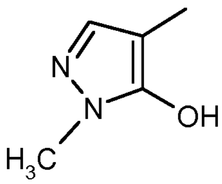

(Z4)

Tabelle 1: Beispiele für die Verbindungen der Formel (I-2)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 2 | O | $(CH_2)_2$ | $SO_2$ | H | $C_2H_5$ | (2)Cl | (4)Cl | Z2 | (I-2) logP = 1,86 [a)] |
| 3 | O | $(CH_2)_2$ | $SO_2$ | H | 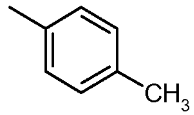 | (2)Cl | (4)Cl | Z2 | (I-2) logP = 2,74 [a)] |
| 4 | O | $(CH_2)_2$ | $SO_2$ | H | $C_2H_5$ | (2)Cl | (4)Cl | Z1 | (I-2) logP = 2,36 [a)] |
| 5 | O | $(CH_2)_2$ | $SO_2$ | H | $CH_3$ | (2)Cl | (4)Cl | Z1 | (I-2) logP = 2,16 [a)] |
| 6 | O | $(CH_2)_2$ | $SO_2$ | H | $CH_3$ | (2)Cl | (4)Cl | Z2 | (I-2) logP = 1,65 [a)] |
| 7 | O | $(CH_2)_2$ | $SO_2$ | H | $CH_2Cl$ | (2)Cl | (4)Cl | Z1 | (I-2) logP = 2,57 [a)] |
| 8 | O | $(CH_2)_2$ | $SO_2$ | H | $CH_2Cl$ | (2)Cl | (4)Cl | Z2 | (I-2) logP = 2,05 [a)] |
| 9 | O | $(CH_2)_2$ | $SO_2$ | H | $CF_3$ | (2)Cl | (4)Cl | Z1 | (I-2) logP = 3,25 [a)] |
| 10 | O | $(CH_2)_2$ | $SO_2$ | H | $CF_3$ | (2)Cl | (4)Cl | Z2 | (I-2) logP = 2,72 [a)] |
| 13 | O | $(CH_2)_2$ | $SO_2$ | H | $CH_3$ | (2)Cl | $(4)SO_2CH_3$ | Z2 | (I-2) logP = 1,23[a)] |
| 14 | O | $(CH_2)_2$ | $SO_2$ | H | $CH_3$ | (2)Cl | $(4)SO_2CH_3$ | Z1 | (I-2) logP = 1,76[a)] |
| 15 | O | $(CH_2)_2$ | $SO_2$ | H | $C_2H_5$ | (2)Cl | $(4)SO_2CH_3$ | Z2 | (I-2) |
| 16 | O | $(CH_2)_2$ | $SO_2$ | H | $C_2H_5$ | (2)Cl | $(4)SO_2CH_3$ | Z1 | (I-2) |
| 17 | O | $(CH_2)_2$ | $SO_2$ | H | $CH_3$ | (2)Br | (4)Br | Z2 | (I-2) logP = 1,71[a)] |
| 18 | O | $(CH_2)_2$ | $SO_2$ | H | $CH_3$ | (2)Br | (4)Br | Z1 | (I-2) logP = 2,23[a)] |
| 19 | O | $(CH_2)_2$ | $SO_2$ | H | $C_2H_5$ | (2)Br | (4)Br | Z2 | (I-2) |
| 20 | O | $(CH_2)_2$ | $SO_2$ | H | $C_2H_5$ | (2)Br | (4)Br | Z1 | (I-2) logP = 2,45[a)] |
| 21 | O | $(CH_2)_2$ | $SO_2$ | H | 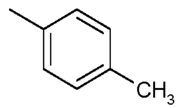 | (2)Cl | (4)Cl | Z1 | (I-2) |
| 22 | O | $(CH_2)_2$ | $SO_2$ | $SO_2CH_3$ | $CH_3$ | (2)Cl | (4)Cl | Z2 | (I-2) |
| 23 | O | $(CH_2)_2$ | $SO_2$ | $SO_2CH_3$ | $CH_3$ | (2)Cl | (4)Cl | Z1 | (I-2) |
| 24 | O | $(CH_2)_2$ | $SO_2$ | H | $C_3H_7$-i | (2)Cl | (4)Cl | Z2 | (I-2) logP = 2,07[a)] |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 25 | O | $(CH_2)_2$ | $SO_2$ | H | $C_3H_7$-i | (2)Cl | (4)Cl | Z1 | (I-2) logP = 2,50[a]) |
| 26 | O | $(CH_2)_2$ | $SO_2$ | H | $CCl_3$ | (2)Cl | (4)Cl | Z2 | (I-2) |
| 27 | O | $(CH_2)_2$ | $SO_2$ | H | $CCl_3$ | (2)Cl | (4)Cl | Z1 | (I-2) |
| 28 | O | $(CH_2)_2$ | $SO_2$ | H | $CH_2CF_3$ | (2)Cl | (4)Cl | Z2 | (I-2) |
| 29 | O | $(CH_2)_2$ | $SO_2$ | H | $CH_2CF_3$ | (2)Cl | (4)Cl | Z1 | (I-2) |
| 30 | O | $(CH_2)_2$ | $SO_2$ | 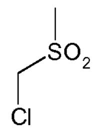 | $CH_2Cl$ | (2)Cl | (4)Cl | Z2 | (I-2) |
| 31 | O | $(CH_2)_2$ | $SO_2$ | 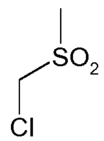 | $CH_2Cl$ | (2)Cl | (4)Cl | Z1 | (I-2) |
| 32 | O | $(CH_2)_2$ | $SO_2$ | H | $CH_2CH_2Cl$ | (2)Cl | (4)Cl | Z2 | (I-2) |
| 33 | O | $(CH_2)_2$ | $SO_2$ | H | $CH_2CH_2Cl$ | (2)Cl | (4)Cl | Z1 | (I-2) |
| 34 | O | $(CH_2)_2$ | $SO_2$ | H | 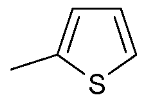 | (2)Cl | (4)Cl | Z2 | (I-2) logP = 2,41 [a]) |
| 35 | **O** | $(CH_2)_2$ | $SO_2$ | H | 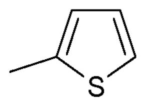 | (2)Cl | (4)Cl | Z1 | (I-2) logP = 2,92 [a]) |
| 36 | O | $(CH_2)_2$ | s | H | $C_4H_9$-t | (2)Cl | (4)Cl | Z2 | (I-2) |
| 37 | O | $(CH_2)_2$ | s | H | $C_4H_9$-t | (2)Cl | (4)Cl | Z1 | (I-2) |
| 38 | O | $(CH_2)_2$ | O | H | $CH_3$ | (2)Cl | (4)Cl | Z2 | (I-2) |
| 39 | O | $(CH_2)_2$ | O | H | $CH_3$ | (2)Cl | (4)Cl | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 40 | O | $(CH_2)_2$ | 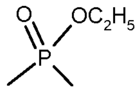 | H | $C_2H_5$ | (2)Cl | (4)Cl | Z2 | (I-2) |
| 41 | O | $(CH_2)_2$ | 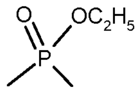 | H | $C_2H_5$ | (2)Cl | (4)Cl | Z1 | (I-2) |
| 42 | O | $(CH_2)_2$ | O | $SO_2CH_3$ | $CH_3$ | (2)Cl | (4)Cl | Z2 | (I-2) |
| 43 | O | $(CH_2)_2$ | O | $SO_2CH_3$ | $CH_3$ | (2)Cl | (4)Cl | Z1 | (I-2) |
| 44 | O | $(CH_2)_2$ | so | H | $CH_3$ | (2)Cl | (4)Cl | Z2 | (I-2) |
| 45 | O | $(CH_2)_2$ | so | H | $CH_3$ | (2)Cl | (4)Cl | Z1 | (I-2) |
| 46 | O | $(CH_2)_2$ | 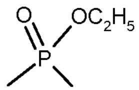 | H | $C_2H_5$ | (2)Cl | $(4)SO_2CH_3$ | Z2 | (I-2) |
| 47 | O | $(CH_2)_2$ | 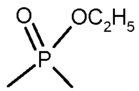 | H | $C_2H_5$ | (2)Cl | $(4)SO_2CH_3$ | Z1 | (I-2) |
| 48 | O | $(CH_2)_2$ | so | H | $CH_3$ | (2)Cl | $(4)SO_2CH_3$ | Z2 | (I-2) |
| 49 | O | $(CH_2)_2$ | so | H | $CH_3$ | (2)Cl | $(4)SO_2CH_3$ | Z1 | (I-2) |
| 50 | O | $(CH_2)_2$ | O | $SO_2CH_3$ | $CH_3$ | (2)Cl | $(4)SO_2CH_3$ | Z2 | (I-2) |
| 51 | O | $(CH_2)_2$ | O | $SO_2CH_3$ | $CH_3$ | (2)Cl | $(4)SO_2CH_3$ | Z1 | (I-2) |
| 52 | O | $(CH_2)_2$ | $SO_2$ | $SO_2CH_3$ | $CH_3$ | (2)Cl | $(4)SO_2CH_3$ | Z2 | (I-2) |
| 53 | O | $(CH_2)_2$ | $SO_2$ | $SO_2CH_3$ | $CH_3$ | (2)Cl | $(4)SO_2CH_3$ | Z1 | (I-2) |
| 54 | O | $(CH_2)_2$ | $SO_2$ | H | $C_3H_7$-i | (2)Cl | $(4)SO_2CH_3$ | Z2 | (I-2) |
| 55 | O | $(CH_2)_2$ | $SO_2$ | H | $C_3H_7$-i | (2)Cl | $(4)SO_2CH_3$ | Z1 | (I-2) |
| 56 | O | $(CH_2)_2$ | $SO_2$ | H | $CF_3$ | (2)Cl | $(4)SO_2CH_3$ | Z2 | (I-2) |
| 57 | O | $(CH_2)_2$ | $SO_2$ | H | $CF_3$ | (2)Cl | $(4)SO_2CH_3$ | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 58 | O | $(CH_2)_2$ | $SO_2$ | H | $CCl_3$ | (2)Cl | $(4)SO_2CH_3$ | Z2 | (I-2) |
| 59 | O | $(CH_2)_2$ | $SO_2$ | H | $CCl_3$ | (2)Cl | $(4)SO_2CH_3$ | Z1 | (I-2) |
| 60 | O | $(CH_2)_2$ | $SO_2$ | H | $CH_2CF_3$ | (2)Cl | $(4)SO_2CH_3$ | Z2 | (I-2) |
| 61 | O | $(CH_2)_2$ | $SO_2$ | H | $CH_2CF_3$ | (2)Cl | $(4)SO_2CH_3$ | Z1 | (I-2) |
| 62 | O | $(CH_2)_2$ | $SO_2$ | H | $CH_2Cl$ | (2)Cl | $(4)SO_2CH_3$ | Z2 | (I-2) |
| 63 | O | $(CH_2)_2$ | $SO_2$ | H | $CH_2Cl$ | (2)Cl | $(4)SO_2CH_3$ | Z1 | (I-2) |
| 64 | O | $(CH_2)_2$ | $SO_2$ | H | $CH_2CH_2Cl$ | (2)Cl | $(4)SO_2CH_3$ | Z2 | (I-2) |
| 65 | O | $(CH_2)_2$ | $SO_2$ | H | $CH_2CH_2Cl$ | (2)Cl | $(4)SO_2CH_3$ | Z1 | (I-2) |
| 66 | O | $(CH_2)_2$ | $SO_2$ | H | 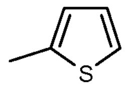 | (2)Cl | $(4)SO_2CH_3$ | Z2 | (I-2) |
| 67 | O | $(CH_2)_2$ | $SO_2$ | H | 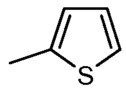 | (2)Cl | $(4)SO_2CH_3$ | Z1 | (I-2) |
| 68 | O | $(CH_2)_2$ | $SO_2$ | H | $C_4H_9$-t | (2)Cl | $(4)SO_2CH_3$ | Z2 | (I-2) |
| 69 | O | $(CH_2)_2$ | $SO_2$ | H | $C_4H_9$-t | (2)Cl | $(4)SO_2CH_3$ | Z1 | (I-2) |
| 70 | O | $(CH_2)_2$ | O | H | $CH_3$ | (2)Cl | $(4)SO_2CH_3$ | Z2 | (I-2) |
| 71 | O | $(CH_2)_2$ | O | H | $CH_3$ | (2)Cl | $(4)SO_2CH_3$ | Z1 | (I-2) |
| 72 | O | $(CH_2)_2$ | O | $CH_3$ | $CH_3$ | (2)Cl | $(4)SO_2CH_3$ | Z2 | (I-2) |
| 73 | O | $(CH_2)_2$ | O | $CH_3$ | $CH_3$ | (2)Cl | $(4)SO_2CH_3$ | Z1 | (I-2) |
| 74 | O | $(CH_2)_2$ | O | $CH_3$ | $CH_3$ | (2)Cl | (4)Cl | Z2 | (I-2) |
| 75 | O | $(CH_2)_2$ | O | $CH_3$ | $CH_3$ | (2)Cl | (4)Cl | Z1 | (I-2) |
| 76 | O | $(CH_2)_2$ | NH | H | 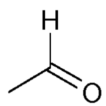 | (2)Cl | (4)Cl | Z2 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 77 | O | $(CH_2)_2$ | NH | H | 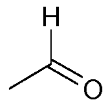 | (2)Cl | (4)Cl | Z1 | (I-2) |
| 78 | O | $(CH_2)_2$ | NH | H | 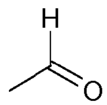 | (2)Cl | $(4)SO_2CH_3$ | Z2 | (I-2) |
| 79 | O | $(CH_2)_2$ | NH | H | 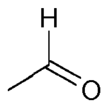 | (2)Cl | $(4)SO_2CH_3$ | Z1 | (I-2) |
| 80 | O | $(CH_2)_2$ | NH | H | $C_4H_9$-t | (2)Cl | (4)Cl | Z2 | (I-2) |
| 81 | O | $(CH_2)_2$ | NH | H | $C_4H_9$-t | (2)Cl | (4)Cl | Z1 | (I-2) |
| 82 | O | $(CH_2)_2$ | NH | H | $C_4H_9$-t | (2)Cl | $(4)SO_2CH_3$ | Z2 | (I-2) |
| 83 | O | $(CH_2)_2$ | NH | H | $C_4H_9$-t | (2)Cl | $(4)SO_2CH_3$ | Z1 | (I-2) |
| 84 | O | $(CH_2)_2$ | NH | H | $SO_2CH_3$ | (2)Cl | (4)Cl | Z2 | (I-2) |
| 85 | O | $(CH_2)_2$ | NH | H | $SO_2CH_3$ | (2)Cl | (4)Cl | Z1 | (I-2) |
| 87 | O | $(CH_2)_2$ | NH | H | $SO_2CH_3$ | (2)Cl | $(4)SO_2CH_3$ | Z1 | (I-2) |
| 88 | O | $(CH_2)_2$ | NH | H | 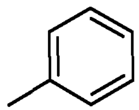 | (2)Cl | (4)Cl | Z2 | (I-2) |
| 89 | O | $(CH_2)_2$ | NH | H | 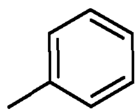 | (2)Cl | (4)Cl | Z1 | (I-2) |
| 90 | O | $(CH_2)_2$ | NH | H | 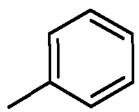 | (2)Cl | $(4)SO_2CH_3$ | Z2 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 91 | O | $(CH_2)_2$ | NH | H | 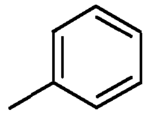 | (2)Cl | (4)$SO_2CH_3$ | Z1 | (I-2) |
| 92 | O | $(CH_2)_2$ | NH | H | 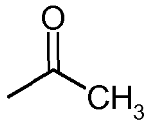 | (2)Cl | (4)Cl | Z2 | (I-2) |
| 93 | O | $(CH_2)_2$ | NH | H | 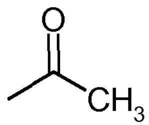 | (2)Cl | (4)Cl | Z1 | (I-2) |
| 94 | O | $(CH_2)_2$ | NH | H | 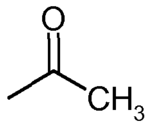 | (2)Cl | (4)$SO_2CH_3$ | Z2 | (I-2) |
| 95 | O | $(CH_2)_2$ | NH | H | 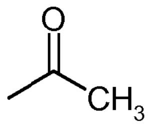 | (2)Cl | (4)$SO_2CH_3$ | Z1 | (I-2) |
| 96 | O | $(CH_2)_2$ | NH | H | 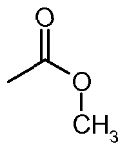 | (2)Cl | (4)Cl | Z2 | (I-2) |
| 97 | O | $(CH_2)_2$ | NH | H | 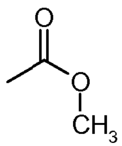 | (2)Cl | (4)Cl | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 98 | O | $(CH_2)_2$ | NH | H | 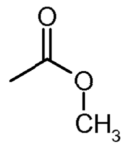 | (2)Cl | (4)$SO_2CH_3$ | Z2 | (I-2) |
| 99 | O | $(CH_2)_2$ | NH | H | 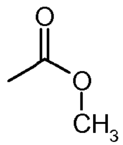 | (2)Cl | (4)$SO_2CH_3$ | Z1 | (I-2) |
| 100 | O | $(CH_2)_2$ | NH | H | 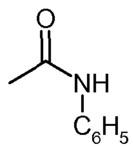 | (2)Cl | (4)Cl | Z2 | (I-2) |
| 101 | O | $(CH_2)_2$ | NH | H | 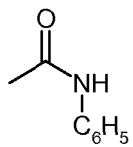 | (2)Cl | (4)Cl | Z1 | (I-2) |
| 102 | O | $(CH_2)_2$ | NH | H | 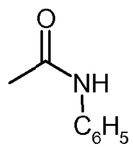 | (2)Cl | (4)$SO_2CH_3$ | Z2 | (I-2) |
| 103 | O | $(CH_2)_2$ | NH | H | 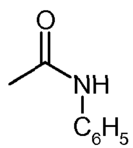 | (2)Cl | (4)$SO_2CH_3$ | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 104 | O | $(CH_2)_2$ | NH | H | 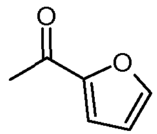 | (2)Cl | (4)Cl | Z2 | (I-2) |
| 105 | O | $(CH_2)_2$ | NH | H | 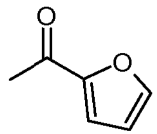 | (2)Cl | (4)Cl | Z1 | (I-2) |
| 106 | O | $(CH_2)_2$ | NH | H | 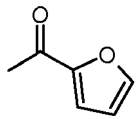 | (2)Cl | $(4)SO_2CH_3$ | Z2 | (I-2) |
| 107 | O | $(CH_2)_2$ | NH | H | 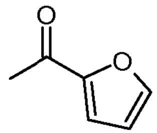 | (2)Cl | $(4)SO_2CH_3$ | Z1 | (I-2) |
| 108 | O | $(CH_2)_2$ | NH | H | 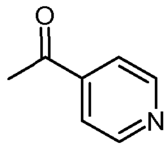 | (2)Cl | (4)Cl | Z2 | (I-2) |
| 109 | O | $(CH_2)_2$ | NH | H | 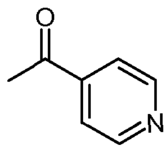 | (2)Cl | (4)Cl | Z1 | (I-2) |
| 110 | O | $(CH_2)_2$ | NH | H | 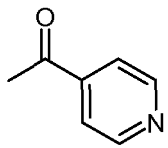 | (2)Cl | $(4)SO_2CH_3$ | Z2 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 111 | O | $(CH_2)_2$ | NH | H | 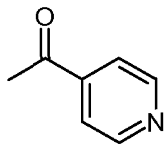 | (2)Cl | (4)$SO_2CH_3$ | Z1 | (I-2) |
| 112 | O | $(CH_2)_2$ | NH | H | 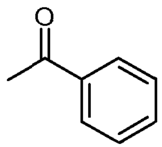 | (2)Cl | (4)Cl | Z2 | (I-2) |
| 113 | O | $(CH_2)_2$ | NH | H | 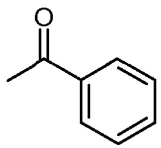 | (2)Cl | (4)Cl | Z1 | (I-2) |
| 114 | O | $(CH_2)_2$ | NH | H | 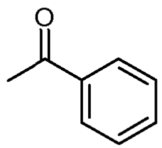 | (2)Cl | (4)$SO_2CH_3$ | Z2 | (I-2) |
| 115 | O | $(CH_2)_2$ | NH | H | 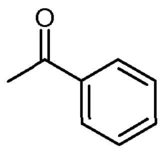 | (2)Cl | (4)$SO_2CH_3$ | Z1 | (I-2) |
| 116 | O | $(CH_2)_2$ | O | H | $C_2H_5$ | (2)Cl | (4)Cl | Z2 | (I-2) |
| 117 | O | $(CH_2)_2$ | O | H | $C_2H_5$ | (2)Cl | (4)Cl | Z1 | (I-2) |
| 118 | O | $(CH_2)_2$ | O | H | $C_2H_5$ | (2)Cl | (4)$SO_2CH_3$ | Z2 | (I-2) |
| 119 | O | $(CH_2)_2$ | O | H | $C_2H_5$ | (2)Cl | (4)$SO_2CH_3$ | Z1 | (I-2) |
| 120 | O | $(CH_2)_2$ | O | H | $C_3H_7$-i | (2)Cl | (4)Cl | Z2 | (I-2) |
| 121 | O | $(CH_2)_2$ | O | H | $C_3H_7$-i | (2)Cl | (4)Cl | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 122 | O | $(CH_2)_2$ | O | H | $C_3H_7$-i | (2)Cl | (4)$SO_2CH_3$ | Z2 | (I-2) |
| 123 | O | $(CH_2)_2$ | O | H | $C_3H_7$-i | (2)Cl | (4)$SO_2CH_3$ | Z1 | (I-2) |
| 124 | O | $(CH_2)_2$ | O | H | $CH_2CH=CH_2$ | (2)Cl | (4)Cl | Z2 | (I-2) |
| 125 | O | $(CH_2)_2$ | O | H | $CH_2CH=CH_2$ | (2)Cl | (4)Cl | Z1 | (I-2) |
| 126 | O | $(CH_2)_2$ | O | H | $CH_2CH=CH_2$ | (2)Cl | (4)$SO_2CH_3$ | Z2 | (I-2) |
| 127 | O | $(CH_2)_2$ | O | H | $CH_2CH=CH_2$ | (2)Cl | (4)$SO_2CH_3$ | Z1 | (I-2) |
| 167 | O | $(CH_2)_2$ | $SO_2$ | H | $C_3H_7$-n | (2)Cl | (4)Cl | Z2 | (I-2) logP = 2,15 [a)] |
| 168 | O | $(CH_2)_2$ | $SO_2$ | H | $C_3H_7$-n | (2)Cl | (4)Cl | Z1 | (I-2) logP = 2,67 [a)] |
| 169 | O | $(CH_2)_2$ | $SO_2$ | H | $C_3H_7$-n | (2)Cl | (4)$SO_2CH_3$ | Z2 | (I-2) |
| 170 | O | $(CH_2)_2$ | $SO_2$ | H | $C_3H_7$-n | (2)Cl | (4)$SO_2CH_3$ | Z1 | (I-2) |
| 171 | O | $(CH_2)_3$ | O | H | $CH_3$ | (2)Cl | (4)Cl | Z2 | (I-2) |
| 172 | O | $(CH_2)_3$ | O | H | $CH_3$ | (2)Cl | (4)Cl | Z1 | (I-2) |
| 173 | O | $(CH_2)_3$ | O | H | $CH_3$ | (2)Cl | (4)$SO_2CH_3$ | Z2 | (I-2) |
| 174 | O | $(CH_2)_3$ | O | H | $CH_3$ | (2)Cl | (4)$SO_2CH_3$ | Z1 | (I-2) |
| 175 | O | $(CH_2)_3$ | O | $CH_3$ | $CH_3$ | (2)Cl | (4)Cl | Z2 | (I-2) |
| 176 | O | $(CH_2)_3$ | O | $CH_3$ | $CH_3$ | (2)Cl | (4)Cl | Z1 | (I-2) |
| 177 | O | $(CH_2)_3$ | O | H | $C_2H_5$ | (2)Cl | (4)Cl | Z2 | (I-2) |
| 178 | O | $(CH_2)_3$ | O | H | $C_2H_5$ | (2)Cl | (4)Cl | Z1 | (I-2) |
| 179 | O | $(CH_2)_3$ | O | H | $C_2H_5$ | (2)Cl | (4)$SO_2CH_3$ | Z2 | (I-2) |
| 180 | O | $(CH_2)_3$ | O | H | $C_2H_5$ | (2)Cl | (4)$SO_2CH_3$ | Z1 | (I-2) |
| 181 | O | $(CH_2)_3$ | O | H | $C_3H_7$-i | (2)Cl | (4)Cl | Z2 | (I-2) |
| 182 | O | $(CH_2)_3$ | O | H | $C_3H_7$-i | (2)Cl | (4)Cl | Z1 | (I-2) |
| 183 | O | $(CH_2)_3$ | O | H | $C_3H_7$-i | (2)Cl | (4)$SO_2CH_3$ | Z2 | (I-2) |
| 184 | O | $(CH_2)_3$ | O | H | $C_3H_7$-i | (2)Cl | (4)$SO_2CH_3$ | Z1 | (I-2) |
| 185 | O | $(CH_2)_3$ | O | H | $CH_2CH=CH_2$ | (2)Cl | (4)Cl | Z2 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 186 | O | $(CH_2)_3$ | O | H | $CH_2CH=CH_2$ | (2)Cl | (4)Cl | Z1 | (I-2) |
| 187 | O | $(CH_2)_2$ | O | $C_2H_5$ | $CH_3$ | (2)Cl | (4)Cl | Z2 | (I-2) |
| 188 | O | $(CH_2)_2$ | O | $C_2H_5$ | $CH_3$ | (2)Cl | (4)Cl | Z1 | (I-2) |
| 189 | O | $(CH_2)_2$ | O | $C_2H_5$ | $CH_3$ | (2)Cl | $(4)SO_2CH_3$ | Z2 | (I-2) |
| 190 | O | $(CH_2)_2$ | O | $C_2H_5$ | $CH_3$ | (2)Cl | $(4)SO_2CH_3$ | Z1 | (I-2) |
| 191 | O | $(CH_2)_3$ | O | $C_2H_5$ | $CH_3$ | (2)Cl | (4)Cl | Z2 | (I-2) |
| 192 | O | $(CH_2)_3$ | O | $C_2H_5$ | $CH_3$ | (2)Cl | (4)Cl | Z1 | (I-2) |
| 193 | O | $(CH_2)_3$ | O | $C_2H_5$ | $CH_3$ | (2)Cl | $(4)SO_2CH_3$ | Z2 | (I-2) |
| 194 | O | $(CH_2)_3$ | O | $C_2H_5$ | $CH_3$ | (2)Cl | $(4)SO_2CH_3$ | Z1 | (I-2) |
| 230 | O | $(CH_2)_2$ | O | H | 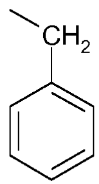 | (2)Cl | $(4)SO_2CH_3$ | Z1 | (I-2) |
| 231 | O | $(CH_2)_2$ | O | H | 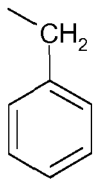 | (2)Cl | $(4)SO_2CH_3$ | Z2 | (I-2) |
| 232 | O | $(CH_2)_2$ | O | H | 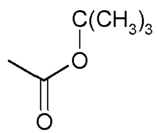 | (2)Cl | $(4)SO_2CH_3$ | Z1 | (I-2) |
| 233 | O | $(CH_2)_2$ | O | H | 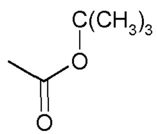 | (2)Cl | $(4)SO_2CH_3$ | Z2 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 234 | O | $(CH_2)_2$ | O | 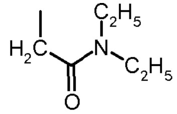 | $CH_3$ | (2)Cl | (4)$SO_2CH_3$ | Z1 | (I-2) |
| 235 | O | $(CH_2)_2$ | O | 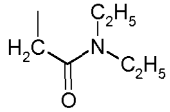 | $C_2H_5$ | (2)Cl | (4)$S0_2CH_3$ | Z1 | (I-2) |
| 236 | O | $(CH_2)_2$ | O | $C_3H_7$-i | $CH_3$ | (2)Cl | (4)$SO_2CH_3$ | Z1 | (I-2) |
| 237 | O | $(CH_2)_2$ | O | $C_3H_7$-i | $C_2H_5$ | (2)Cl | (4)$SO_2CH_3$ | Z1 | (I-2) |
| 238 | O | $(CH_2)_2$ | O | 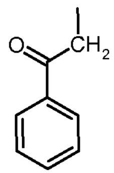 | $CH_3$ | (2)Cl | (4)$SO_2CH_3$ | Z1 | (I-2) |
| 239 | O | $(CH_2)_2$ | O | 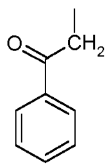 | $C_2H_5$ | (2)Cl | (4)$SO_2CH_3$ | Z1 | (I-2) |
| 240 | O | $(CH_2)_2$ | O | 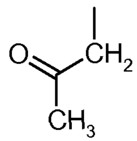 | $CH_3$ | (2)Cl | (4)$SO_2CH_3$ | Z1 | (I-2) |
| 241 | O | $(CH_2)_2$ | O | 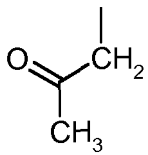 | $C_2H_5$ | (2)Cl | (4)$SO_2CH_3$ | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 242 | O | $(CH_2)_2$ | O | 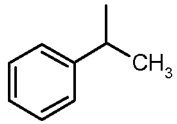 | $CH_3$ | (2)Cl | (4)$SO_2CH_3$ | Z1 | (I-2) |
| 243 | O | $(CH_2)_2$ | O | 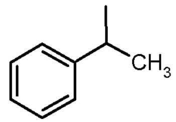 | $C_2H_5$ | (2)Cl | (4)$SO_2CH_3$ | Z1 | (I-2) |
| 244 | O | $(CH_2)_2$ | O | 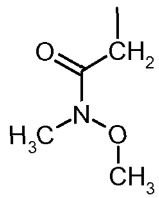 | $CH_3$ | (2)Cl | (4)$SO_2CH_3$ | Z1 | (I-2) |
| 245 | O | $(CH_2)_2$ | O | 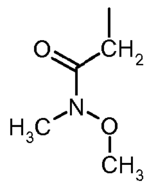 | $C_2H_5$ | (2)Cl | (4)$SO_2CH_3$ | Z1 | (I-2) |
| 246 | O | $(CH_2)_2$ | O | 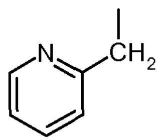 | $CH_3$ | (2)Cl | (4)$SO_2CH_3$ | Z1 | (I-2) |
| 247 | O | $(CH_2)_2$ | O | 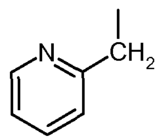 | $C_2H_5$ | (2)Cl | (4)$SO_2CH_3$ | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 248 | O | $(CH_2)_2$ | O | 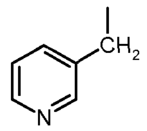 | $CH_3$ | (2)Cl | (4)$SO_2CH_3$ | Z1 | (I-2) |
| 249 | O | $(CH_2)_2$ | O | 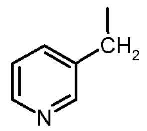 | $C_2H_5$ | (2)Cl | (4)$SO_2CH_3$ | Z1 | (I-2) |
| 250 | O | $(CH_2)_2$ | O | 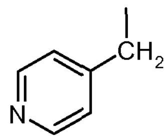 | $CH_3$ | (2)Cl | (4)$SO_2CH_3$ | Z1 | (I-2) |
| 251 | O | $(CH_2)_2$ | O | 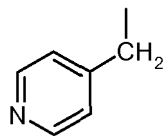 | $C_2H_5$ | (2)Cl | (4)$SO_2CH_3$ | Z1 | (I-2) |
| 252 | O | $(CH_2)_2$ | O | 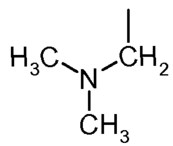 | $CH_3$ | (2)Cl | (4)$SO_2CH_3$ | Z1 | (I-2) |
| 253 | O | $(CH_2)_2$ | O | 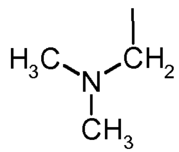 | $C_2H_5$ | (2)Cl | (4)$SO_2CH_3$ | Z1 | (I-2) |
| 254 | O | $(CH_2)_2$ | O | 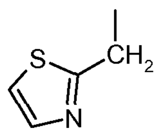 | $CH_3$ | (2)Cl | (4)$SO_2CH_3$ | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 255 | O | $(CH_2)_2$ | O | 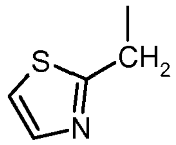 | $C_2H_5$ | (2)Cl | $(4)SO_2CH_3$ | Z1 | (I-2) |
| 256 | O | $(CH_2)_2$ | O | 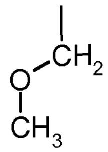 | $CH_3$ | (2)Cl | $(4)SO_2CH_3$ | Z1 | (I-2) |
| 257 | O | $(CH_2)_2$ | O | 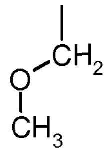 | $C_2H_5$ | (2)Cl | $(4)SO_2CH_3$ | Z1 | (I-2) |
| 258 | O | $(CH_2)_2$ | O | 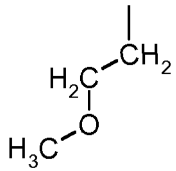 | $CH_3$ | (2)Cl | $(4)SO_2CH_3$ | Z1 | (I-2) |
| 259 | O | $(CH_2)_2$ | O | 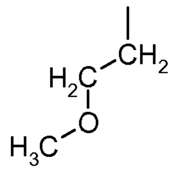 | $C_2H_5$ | (2)Cl | $(4)SO_2CH_3$ | Z1 | (I-2) |
| 260 | O | $(CH_2)_2$ | O | 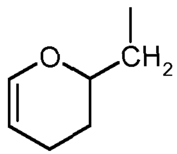 | $CH_3$ | (2)Cl | $(4)SO_2CH_3$ | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 261 | O | $(CH_2)_2$ | O | 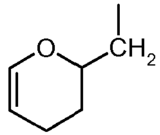 | $C_2H_5$ | (2)Cl | (4)$SO_2CH_3$ | Z1 | (I-2) |
| 262 | O | $(CH_2)_3$ | O | H | 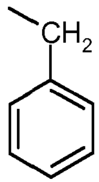 | (2)Cl | (4)$SO_2CH_3$ | Z1 | (I-2) |
| 263 | O | $(CH_2)_3$ | O | H | 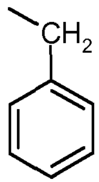 | (2)Cl | (4)$SO_2CH_3$ | Z2 | (I-2) |
| 264 | O | $(CH_2)_3$ | O | H | 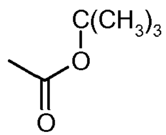 | (2)Cl | (4)$SO_2CH_3$ | Z1 | (I-2) |
| 265 | O | $(CH_2)_3$ | O | H | 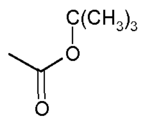 | (2)Cl | (4)$SO_2CH_3$ | Z2 | (I-2) |
| 266 | O | $(CH_2)_3$ | O | 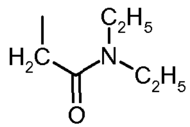 | $CH_3$ | (2)Cl | (4)$SO_2CH_3$ | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 267 | O | $(CH_2)_3$ | O | 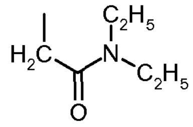 | $C_2H_5$ | (2)Cl | $(4)SO_2CH_3$ | Z1 | (I-2) |
| 268 | O | $(CH_2)_3$ | O | $C_3H_7$-i | $CH_3$ | (2)Cl | $(4)SO_2CH_3$ | Z1 | (I-2) |
| 269 | O | $(CH_2)_3$ | O | $C_3H_7$-i | $C_2H_5$ | (2)Cl | $(4)SO_2CH_3$ | Z1 | (I-2) |
| 270 | O | $(CH_2)_3$ | O | 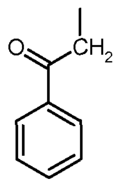 | $CH_3$ | (2)Cl | $(4)SO_2CH_3$ | Z1 | (I-2) |
| 271 | O | $(CH_2)_3$ | O | 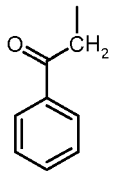 | $C_2H_5$ | (2)Cl | $(4)SO_2CH_3$ | Z1 | (I-2) |
| 272 | O | $(CH_2)_3$ | O | 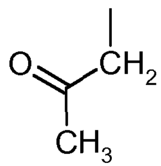 | $CH_3$ | (2)Cl | $(4)SO_2CH_3$ | Z1 | (I-2) |
| 273 | O | $(CH_2)_3$ | O | 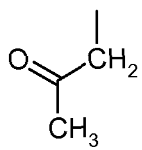 | $C_2H_5$ | (2)Cl | $(4)SO_2CH_3$ | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 274 | O | $(CH_2)_3$ | O | 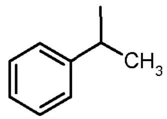 | $CH_3$ | (2)Cl | (4)$SO_2CH_3$ | Z1 | (I-2) |
| 278 | O | $(CH_2)_3$ | O | 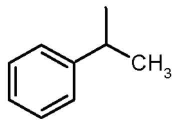 | $C_2H_5$ | (2) Cl | (4)$SO_2CH_3$ | Z1 | (I-2) |
| 279 | O | $(CH_2)_3$ | O | 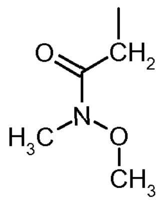 | $CH_3$ | (2)Cl | (4)$SO_2CH_3$ | Z1 | (I-2) |
| 280 | O | $(CH_2)_3$ | O | 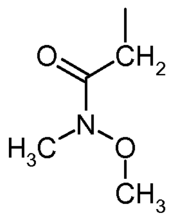 | $C_2H_5$ | (2)Cl | (4)$SO_2CH_3$ | Z1 | (I-2) |
| 281 | O | $(CH_2)_3$ | O | 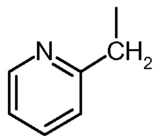 | $CH_3$ | (2)Cl | (4)$SO_2CH_3$ | Z1 | (I-2) |
| 282 | O | $(CH_2)_3$ | O | 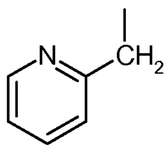 | $C_2H_5$ | (2)Cl | (4)$SO_2CH_3$ | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 283 | O | $(CH_2)_3$ | O | 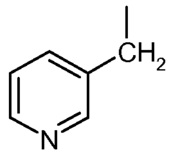 | $CH_3$ | (2)Cl | $(4)SO_2CH_3$ | Z1 | (I-2) |
| 284 | O | $(CH_2)_3$ | O | 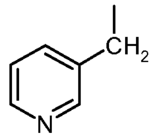 | $C_2H_5$ | (2)Cl | $(4)SO_2CH_3$ | Z1 | (I-2) |
| 285 | O | $(CH_2)_3$ | O | 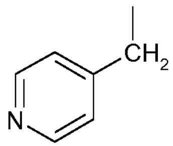 | $CH_3$ | (2)Cl | $(4)SO_2CH_3$ | Z1 | (I-2) |
| 286 | O | $(CH_2)_3$ | O | 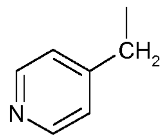 | $C_2H_5$ | (2)Cl | $(4)SO_2CH_3$ | Z1 | (I-2) |
| 287 | O | $(CH_2)_3$ | O | 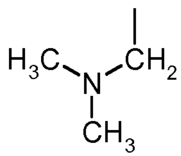 | $CH_3$ | (2)Cl | $(4)SO_2CH_3$ | Z1 | (I-2) |
| 289 | O | $(CH_2)_3$ | O | 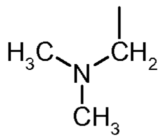 | $C_2H_5$ | (2)Cl | $(4)SO_2CH_3$ | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 290 | **O** | $(CH_2)_3$ | O | 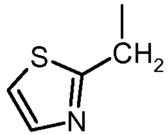 | $CH_3$ | (2)Cl | $(4)SO_2CH_3$ | Z1 | (I-2) |
| 291 | O | $(CH_2)_3$ | O | 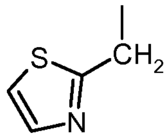 | $C_2H_5$ | (2)Cl | $(4)SO_2CH_3$ | Z1 | (I-2) |
| 292 | O | $(CH_2)_3$ | O | 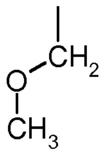 | $CH_3$ | (2)Cl | $(4)SO_2CH_3$ | Z1 | (I-2) |
| 293 | O | $(CH_2)_3$ | O | 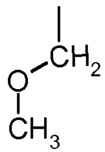 | $C_2H_5$ | (2)Cl | $(4)SO_2CH_3$ | Z1 | (I-2) |
| 294 | O | $(CH_2)_3$ | O | 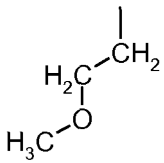 | $CH_3$ | (2) Cl | $(4)SO_2CH_3$ | Z1 | (I-2) |
| 295 | O | $(CH_2)_3$ | O | 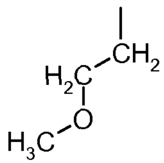 | $C_2H_5$ | (2)Cl | $(4)SO_2CH_3$ | Z1 | (I-2) |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 296 | O | $(CH_2)_3$ | O | 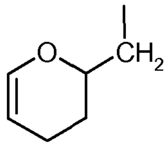 | $CH_3$ | (2)Cl | (4)$SO_2CH_3$ | Z1 | (I-2) |
| 297 | O | $(CH_2)$ | O | 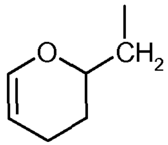 | $C_2H_5$ | (2)Cl | (4)$SO_2CH_3$ | Z1 | (I-2) |
| 353 | O | $(CH_2)_2$ | $SO_2$ | H | $CH_3$ | (2)Cl | (4)Cl | Z4 | (I-2) logP = 1,36 [a)] |
| 354 | O | $(CH_2)_2$ | $SO_2$ | H | $CH_3$ | (2)Cl | (4)Cl | Z2 | (I-2) logP = 1,54 [a)] |
| 355 | O | $(CH_2)_2$ | $SO_2$ | H | $CH_3$ | (2)Cl | (4)Cl | Z3 | (I-2) |
| | | | | | | | | | logP = 1,32 [a)] |
| 356 | O | $(CH_2)_2$ | $SO_2$ | $CH_3$ | 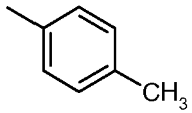 | (2)Cl | (4)Cl | Z2 | (I-2) |
| 357 | O | $(CH_2)_2$ | $SO_2$ | H | $CH_3$ | (2)Br | (4)Br | 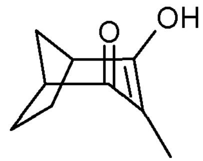 | (I-2) logP = 2,52 [a)] |
| 358 | O | $(CH_2)_2$ | $SO_2$ | H | $C_2H_5$ | (2) $CH_3$ | (4)Cl | Z2 | (I-2) logP = 2,03 [a)] |
| 359 | O | $(CH_2)_2$ | $SO_2$ | H | $C_2H_5$ | (2) $CH_3$ | (4)Cl | Z1 | (I-2) logP = 2,33 [a)] |
| 360 | O | $(CH_2)_2$ | $SO_2$ | H | $CH_3$ | (2) $CH_3$ | (4)Cl | Z2 | (I-2) logP = 1,82 [a)] |
| 361 | O | $(CH_2)_2$ | $SO_2$ | H | $CH_3$ | (2) $CH_3$ | (4)Cl | Z1 | (I-2) logP = 2,13 [a)] |
| 362 | O | $(CH_2)_2$ | $SO_2$ | H | $C_2H_5$ | (2) $CH_3$ | (4)Br | Z2 | (I-2) logP = 2,09 [a)] |
| 363 | O | $(CH_2)_2$ | $SO_2$ | H | $C_2H_5$ | (2) $CH_3$ | (4)Br | Z4 | (I-2) logP = 1,77 [a)] |

(fortgesetzt)

| Bsp.-Nr. | $A^1$ | $A^2$ | $A^3$ | $R^1$ | $R^2$ | (Position) X | (Position) Y | Z | Formel Physikal. Daten |
|---|---|---|---|---|---|---|---|---|---|
| 364 | O | $(CH_2)_2$ | $SO_2$ | H | $C_2H_5$ | (2) $CH_3$ | (4)Br | Z1 | (I-2) logP = 2,41 [a)] |
| 365 | O | $(CH_2)_2$ | $SO_2$ | H | $CH_3$ | (2) $CH_3$ | (4)Br | Z2 | (I-2) logP = 1,89 [a)] |
| 366 | O | $(CH_2)_2$ | $SO_2$ | H | $CH_3$ | (2) $CH_3$ | (4)Br | Z4 | (I-2) logP = 1,56 [a)] |
| 367 | O | $(CH_2)_2$ | $SO_2$ | H | $CH_3$ | (2) $CH_3$ | (4)Br | Z1 | (I-2) logP = 2,19 [a)] |
| 368 | O | $(CH_2)_2$ | $SO_2$ | H | $CH_3$ | (2)Cl | (4) $CH_3SO_2$ | Z4 | (I-2) logP = 0,95 [a)] |

* -$A^3$-$R^2$ stehen gemeinsam für den in Spalte $R^2$ aufgeführten Rest

Die Bestimmung der in der Tabelle angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.

(a) Eluenten für die Bestimmung im sauren Bereich: 0,1% wässrige Phosphorsäure, Acetonitril; linearer Gradient von 10% Acetonitril bis 90% Acetonitril - entsprechende Messergebnisse sind in Tabelle 1 mit [a)] markiert.

(b) Eluenten für die Bestimmung im neutralen Bereich: 0,01-molare wässrige Phosphatpuffer-Lösung, Acetonitril; linearer Gradient von 10% Acetonitril bis 90% Acetonitril - entsprechende Messergebnisse sind in Tabelle 1 mit [b)] markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

<u>Ausgangsstoffe der Formel (II):</u>

Beispiel (II-1) (Verfahren g)

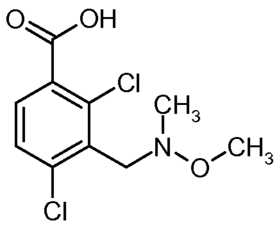

Eine Mischung aus 2,0 g (7,2 mMol) 2,4-Dichlor-3-(N-methoxy-N-methyl-aminomethyl)-benzoesäure-methylester, 0,32 g (7,9 mMol) Natriumhydroxid, 10 ml Wasser und 90 ml Tetrahydrofuran wird 4 Stunden bei Raumtemperatur (ca. 20°C) gerührt und anschließend unter vermindertem Druck eingeengt. Der Rückstand wird mit Wasser und Essigsäureethylester geschüttelt, die wässrige Phase abgetrennt und durch Zugabe von IN-Salzsäure der pH-Wert der Lösung auf ca. 3,5 eingestellt. Dann wird mit Essigsäureethylester geschüttelt, die organische Phase abgetrennt, mit Natriumsulfat getrocknet und filtriert. Das Filtrat wird unter vermindertem Druck eingeengt, der Rückstand mit Hexan digeriert und das hierbei kristallin angefallene Produkt durch Absaugen isoliert.
Man erhält 1,35 g (68 % der Theorie, 95,9 %iges Produkt) 2,4-Dichlor-3-(N-methoxy-N-methyl-amino-methyl)-benzoesäure. LogP = 1,99.

Beispiel (II-2) (Verfahren g)

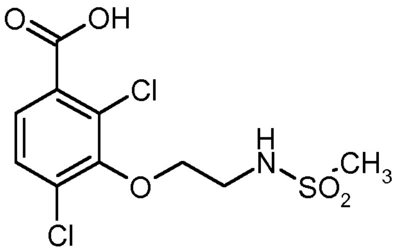

Eine Mischung aus 0,85 g (2,4 mMol) 2,4-Dichlor-3-(2-methylsulfonylamino-ethoxy)-benzoesäure-ethylester, 10 ml Ethanol und 1,5 g 10 %iger wässriger Natronlauge wird 3 Stunden bei Raumtemperatur (ca. 20°C) gerührt und anschließend mit 60 ml Diethylether digeriert. Die wässrige Phase wird abgetrennt, und die organische Phase mit 10 ml Wasser geschüttelt. Die vereinigten wässrigen Phasen werden mit konz. Salzsäure angesäuert und dann zweimal mit je 30 ml

Methylenchlorid extrahiert. Die vereinigten organischen Extraktionslösungen werden mit Natriumsulfat getrocknet und filtriert. Vom Filtrat wird das Lösungsmittel unter vermindertem Druck sorgfältig abdestilliert.
Man erhält 0,56 g (69 % der Theorie, 96 %iges Produkt) 2,4-Dichlor-3-(2-methylsulfonylamino-ethoxy)-benzoesäure. LogP = 1,36

Beispiel II-3 (Verfahren g)

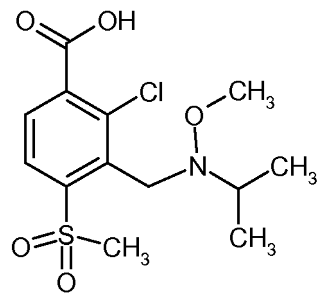

1,90 g (5,4 mMol) 2-Chlor-4-methylsulfonyl-3-(N-methoxy-N-(1-methylethyl)-aminomethyl)-benzoesäuremethylester werden in 20 ml Tetrahydrofuran vorgelegt. Dazu tropft man bei Raumtemperatur (ca. 20°C) eine Lösung von 0,22 g (5,4 mMol) Natriumhydroxid in 20 ml Wasser. Das Reaktionsgemisch wird 16 Stunden bei Raumtemperatur gerührt. Anschließend entfernt man das Tetrahydrofuran unter vermindertem Druck und wäscht die wäßrige Lösung mit Diethylether. Man säuert nun die wäßrige Phase mit Salzsäure an und extrahiert mehrmals mit Essigester. Die vereinigten Essigester-Phasen werden über Natriumsulfat getrocknet und unter vermindertem Druck eingeengt.
Man erhält 1,50 g (82% der Theorie, 87%iges Produkt nach HPLC) 2-Chlor-4-methylsulfonyl-3-(N-methoxy-N-(1-methylethyl)-aminomethyl)-benzoesäure als hellgelben Feststoff. logP = 2,09 (sauer)

Analog zu den Beispielen (II-1) und (II-2) können beispielsweise auch die in der nachstehenden Tabelle 2 aufgeführten Verbindungen der Formel (II) hergestellt werden.

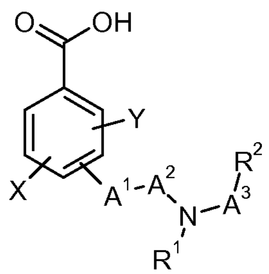

(II)

Tabelle 2: Beispiele für die Verbindungen der Formel II

| Bsp.-Nr. | (Position-) 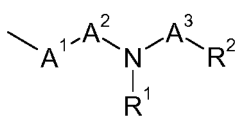 | (Position-) X | (Position-) Y | Physikal. Daten |
|---|---|---|---|---|
| II-4 | 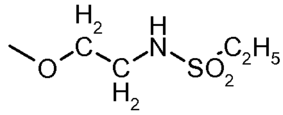 (3) | (2)Cl | (4)Cl | LogP = 1,58 a) |

(fortgesetzt)

| Bsp.-Nr. | (Position-) 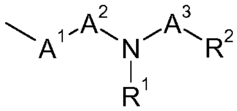 | (Position-) X | (Position-) Y | Physikal. Daten |
|---|---|---|---|---|
| II-5 | 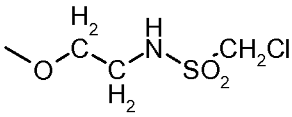 (3) | (2)Cl | (4)Cl | |
| II-6 | 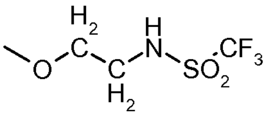 (3) | (2)Cl | (4)Cl | LogP = 2,43 [a)] |
| II-7 | 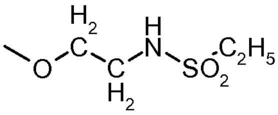 (3) | (2)Cl | (4)$SO_2CH_3$ | LogP = 0,81 [a)] |
| II-8 | 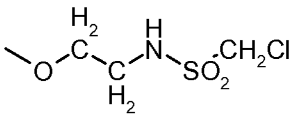 (3) | (2)Cl | (4)$SO_2CH_3$ | |
| II-9 | 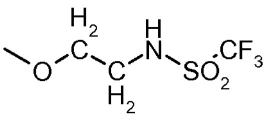 (3) | (2)Cl | (4)$SO_2CH_3$ | |
| II-10 | 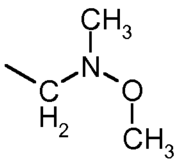 (3) | (2)Cl | (4)$SO_2CH_3$ | |
| II-11 | 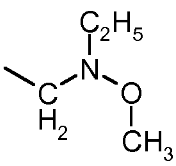 (3) | (2)Cl | (4)Cl | LogP=2,51 |
| II-12 | 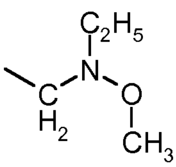 (3) | (2) Cl | (4)$SO_2CH_3$ | |

(fortgesetzt)

| Bsp.-Nr. | (Position-) 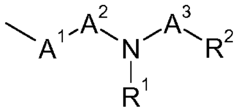 | (Position-) X | (Position-) Y | Physikal. Daten |
|---|---|---|---|---|
| II-13 | (3) 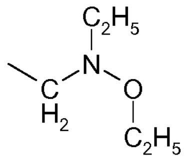 | (2) Cl | (4) Cl | LogP=2,51 |
| II-14 | (3) 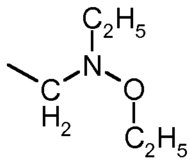 | (2)Cl | (4)$SO_2CH_3$ | LogP=2,04 |
| II-15 | (3) 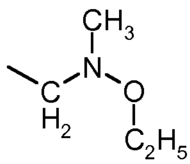 | (2)Cl | (4)Cl | |
| II-16 | (3) 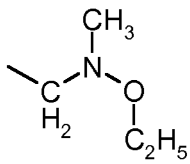 | (2)Cl | (4)$SO_2CH_3$ | |
| II-17 | (3) 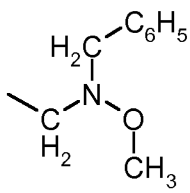 | (2)Cl | (4)Cl | |
| II-18 | (3) 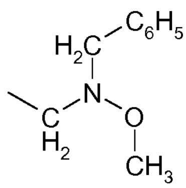 | (2)Cl | (4)$SO_2CH_3$ | |

(fortgesetzt)

| Bsp.-Nr. | (Position-) 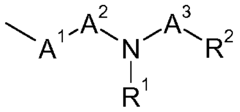 | (Position-) X | (Position-) Y | Physikal. Daten |
|---|---|---|---|---|
| II-19 | 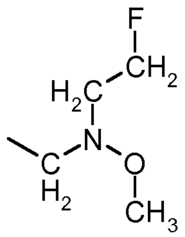 (3) | (2)Cl | (4)Cl | |
| II-20 | 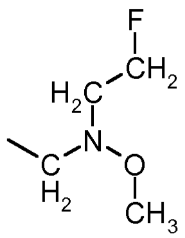 (3) | (2)Cl | (4)$SO_2CH_3$ | |
| II-21 | 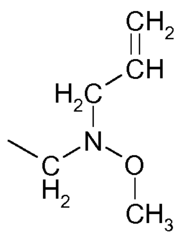 (3) | (2)Cl | (4)Cl | |
| II-22 | 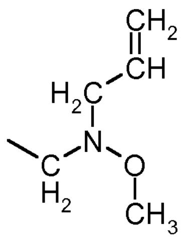 (3) | (2)Cl | (4)$SO_2CH_3$ | |
| II-23 | 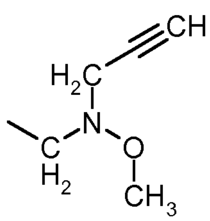 (3) | (2)Cl | (4)Cl | |

(fortgesetzt)

| Bsp.-Nr. | (Position-) 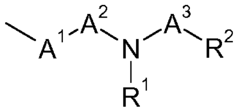 | (Position-) X | (Position-) Y | Physikal. Daten |
|---|---|---|---|---|
| II-24 | (3) 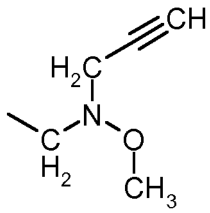 | (2)Cl | (4)$SO_2CH_3$ | |
| II-25 | (3) 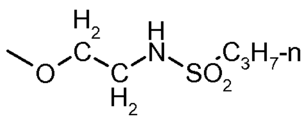 | (2)Cl | (4)Cl | LogP = 1,86 [a)] |
| II-26 | (3) 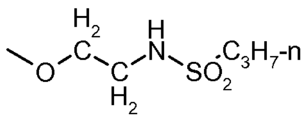 | (2)Cl | (4)$SO_2CH_3$ | |
| II-27 | (3) 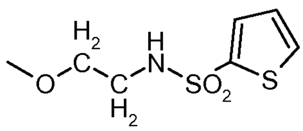 | (2)Cl | (4)Cl | LogP = 2,13 [a)] |
| II-28 | (3) 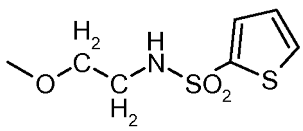 | (2)Cl | (4)$SO_2CH_3$ | |
| II-29 | (3) 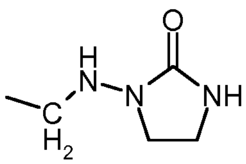 | (2)Cl | (4)Cl | Fp.: 223°C |
| II-30 | (3) 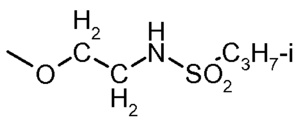 | (2)Cl | (4)Cl | LogP = 1,50 [a)] |
| II-31 | (3) 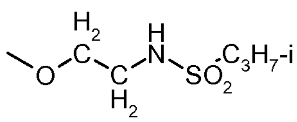 | (2)Cl | (4)$SO_2CH_3$ | |

(fortgesetzt)

| Bsp.-Nr. | (Position-) 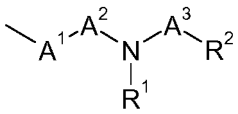 | (Position-) X | (Position-) Y | Physikal. Daten |
|---|---|---|---|---|
| II-32 | 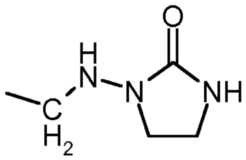 (3) | (2)Cl | (4)$SO_2CH_3$ | Fp.: 243°C |
| II-33 | 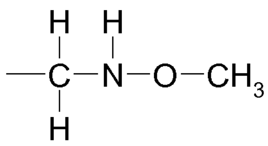 (3) | (2)Cl | (4)Cl | logP=1,38 |
| II-34 | 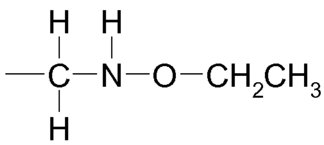 (3) | (2)Cl | (4)Cl | logP=1,76 |
| II-35 | 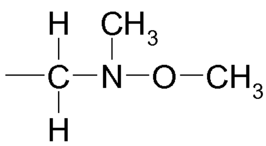 (3) | (2)Cl | (4)Cl | logP=1,99 |
| II-36 | 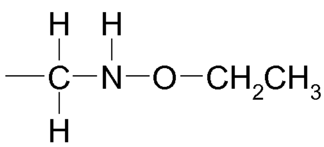 (3) | (2)Cl | (4)$SO_2Me$ | logP=0,98 |
| II-37 | 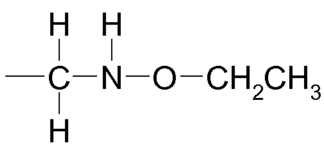 (3) | (2)$OCH_3$ | (4)Cl | logP=1,44 |

(fortgesetzt)

| Bsp.-Nr. | (Position-) 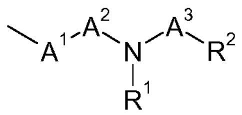 | (Position-) X | (Position-) Y | Physikal. Daten |
|---|---|---|---|---|
| II-38 | (3) 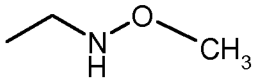 | (2)Cl | (4)$SO_2$Me | $logP_{sauer}$=0,58 |
| II-39 | (3) 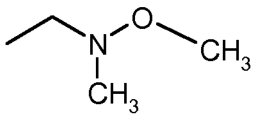 | (2)Cl | (4)$SO_2$Me | $logP_{sauer}$=1,37 |
| II-40 | (3) 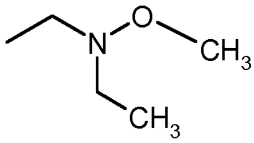 | (2)Cl | (4)$SO_2$Me | $logP_{sauer}$=1,77 |
| II-41 | (3) 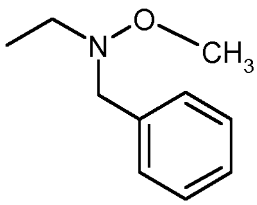 | (2)Cl | (4)$SO_2$Me | $logP_{sauer}$=2,43 |
| II-42 | 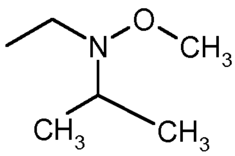 | (2)Cl | (4)$SO_2$Me | $logP_{sauer}$=2,09 |
| II-43 | (3) 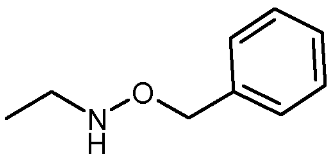 | (2)Cl | (4)$SO_2$Me | $logP_{saue}$=1,99 |
| II-44 | (3) 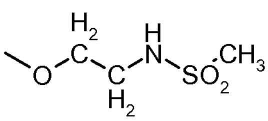 | (2)$CH_3$ | (4)Cl | $logP_{sauer}$=1,82 |
| II-45 | (3) 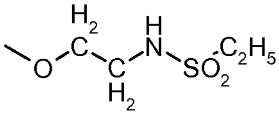 | (2)$CH_3$ | (4)Cl | $logP_{saue}$=1,69 |

(fortgesetzt)

| Bsp.-Nr. | (Position-) 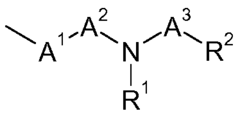 | (Position-) X | (Position-) Y | Physikal. Daten |
|---|---|---|---|---|
| II-46 | 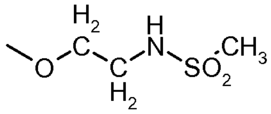 (3) | (2) $CH_3$ | (4) Br | $logP_{sauer}$=1,56 |
| II-47 | 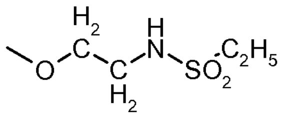 (3) | (2)$CH_3$ | (4) Br | $logP_{sauer}$=1,76 |
| II-48 | 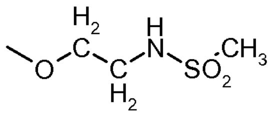 (3) | (2)Cl | (4) S02Me | $logP_{sauer}$=0,74 |
| II-49 | 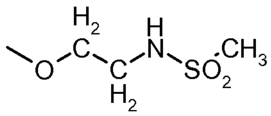 (3) | (2) Br | (4) Br | $logP_{saue}$=1,43 |
| II-50 | 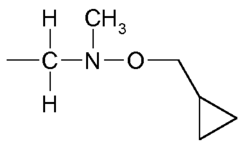 (3) | (2) Cl | (4) $SO_2Me$ | $logP_{sauer}$=2,00 |
| II-51 | 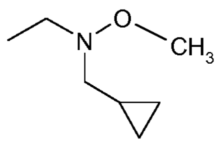 (3) | (2) Cl | (4) $SO_2Me$ | $logP_{sauer}$=2,18 |
| II-52 | 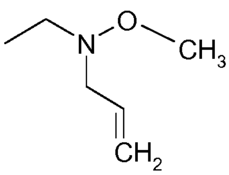 (3) | (2) Cl | (4) $SO_2Me$ | $logP_{sauer}$=1,91 |
| II-53 | 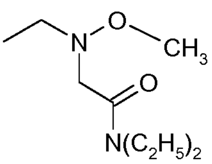 (3) | (2) Cl | (4) $SO_2Me$ | $logP_{sauer}$= 1,51 |

(fortgesetzt)

| Bsp.-Nr. | (Position-) 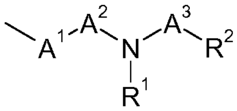 | (Position-) X | (Position-) Y | Physikal. Daten |
|---|---|---|---|---|
| II-54 | (3) 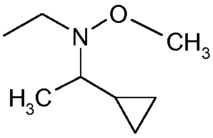 | (2) Cl | (4) $SO_2Me$ | $logP_{sauer}$=2,50 |
| II-55 | (3) 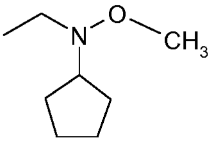 | (2) Cl | (4) $SO_2Me$ | $logP_{sauer}$=2,53 |
| II-56 | (3) 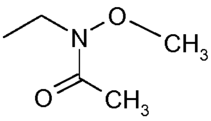 | (2) Cl | (4) $SO_2Me$ | $logP_{sauer}$=0,72 |
| II-57 | (3) 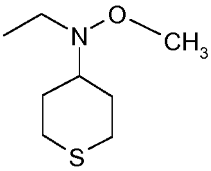 | (2) Cl | (4) $SO_2Me$ | $logP_{sauer}$=2,20 |
| II-58 | (3) 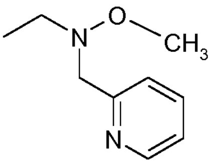 | (2) Cl | (4) $SO_2Me$ | $logP_{sauer}$=0,34 |
| II-59 | (3) 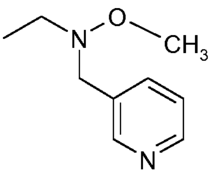 | (2) Cl | (4) $SO_2Me$ | $logP_{sauer}$=0,36 |
| II-60 | (3) 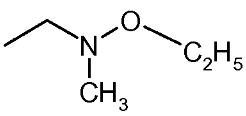 | (2) Cl | (4) $SO_2Me$ | $logP_{sauer}$=1,66 |
| II-61 | (3) 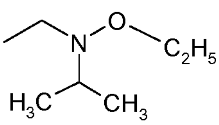 | (2) Cl | (4) $SO_2Me$ | $logP_{sauer}$=2,41 |

(fortgesetzt)

| Bsp.-Nr. | (Position-) 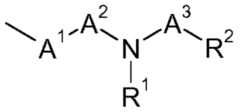 | (Position-) X | (Position-) Y | Physikal. Daten |
|---|---|---|---|---|
| II-62 | (3) 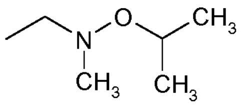 | (2) Cl | (4) $SO_2Me$ | $logP_{sauer}$=1,96 |
| II-63 | (3) 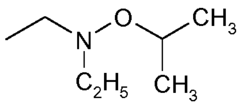 | (2) Cl | (4) $SO_2Me$ | $logP_{sauer}$=2,30 |
| II-64 | (3) 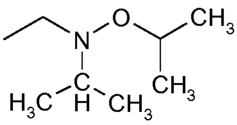 | (2) Cl | (4) $SO_2Me$ | $logP_{sauer}$=2,71 |
| II-65 | (3) 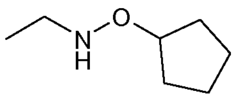 | (2) Cl | (4) $SO_2Me$ | $logP_{sauer}$=1,88 |
| II-66 | (3) 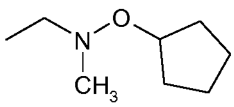 | (2) Cl | (4) $SO_2Me$ | $logP_{sauer}$=2,36 |
| II-67 | (3) 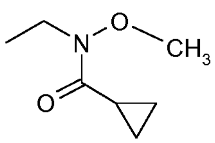 | (2) Cl | (4) $SO_2Me$ | $logP_{sauer}$=1,25 |
| II-68 | (3) 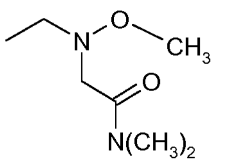 | (2) Cl | (4) $SO_2Me$ | $logP_{sauer}$=0,99 |
| II-69 | (3) 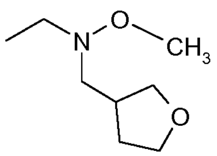 | (2) Cl | (4) $SO_2Me$ | $logP_{sauer}$=1,52 |
| II-70 | (3) 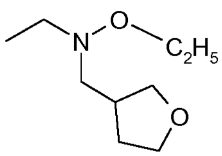 | (2) Cl | (4) $SO_2Me$ | $logP_{sauer}$=1,71 |

(fortgesetzt)

| Bsp.-Nr. | (Position-) 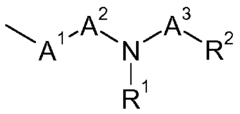 | (Position-) X | (Position-) Y | Physikal. Daten |
|---|---|---|---|---|
| II-71 | (3) 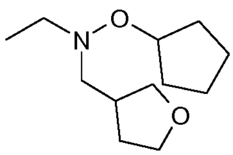 | (2) Cl | (4) $SO_2Me$ | $logP_{sauer}$=2,28 |
| II-72 | (3) 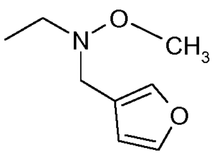 | (2) Cl | (4) $SO_2Me$ | $logP_{sauer}$=2,04 |
| II-73 | (3) 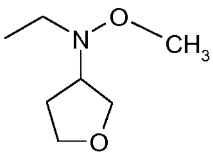 | (2) Cl | (4) $SO_2Me$ | $logP_{sauer}$=1,33 |
| II-74 | (3) 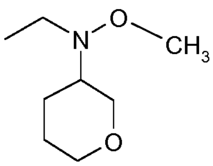 | (2) Cl | (4) $SO_2Me$ | $logP_{sauer}$=1,64 |
| II-75 | (3) 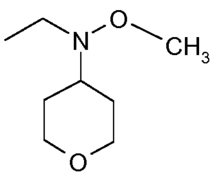 | (2) Cl | (4) S02Me | $logP_{sauer}$=1,49 |
| II-76 | (3) 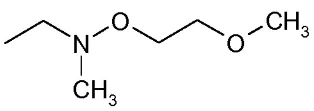 | (2) Cl | (4) S02Me | $logP_{sauer}$=1,40 |
| II-77 | (3) 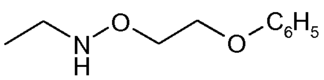 | (2) Cl | (4) S02Me | $logP_{sauer}$=2,09 |
| II-78 | (3) 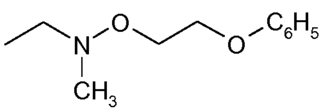 | (2) Cl | (4) S02Me | $logP_{sauer}$=2,43 |

(fortgesetzt)

| Bsp.-Nr. | (Position-) 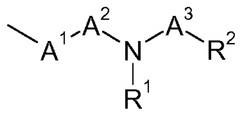 | (Position-) X | (Position-) Y | Physikal. Daten |
|---|---|---|---|---|
| II-79 | (3) 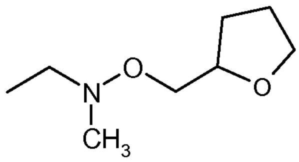 | (2) Cl | (4) S02Me | $logP_{sauer}$=1,63 |
| II-80 | (3) 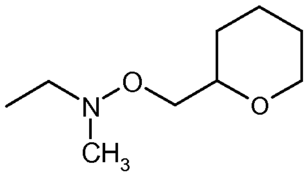 | (2) Cl | (4) S02Me | $logP_{sauer}$=1,91 |
| II-81 | (3) 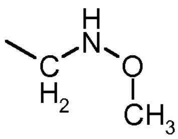 | (2) Cl | (4) Cl | $logP_{sauer}$=1,3 |
| II-82 | (3) 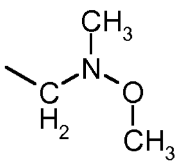 | (2) $OCH_3$ | (4) Cl | $logP_{sauer}$=1,81 |
| II-83 | (3) 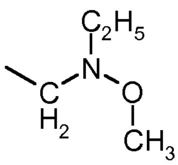 | (2) $OCH_3$ | (4) Cl | $logP_{sauer}$=2,29 |
| II-84 | (3) 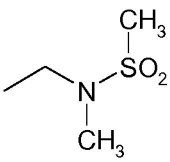 | (2) Cl | (4) Cl | $logP_{sauer}$=1,45 |
| | | | | |
| II-86 | (3) 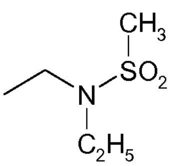 | (2) $OCH_3$ | (4) Cl | $logP_{sauer}$=1,73 |

(fortgesetzt)

| Bsp.-Nr. | (Position-) 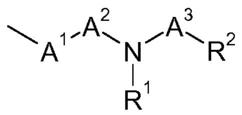 | (Position-) X | (Position-) Y | Physikal. Daten |
|---|---|---|---|---|
| II-87 | 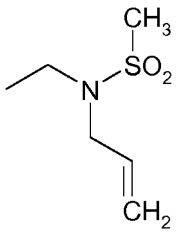 (3) | (2) Cl | (4) S02Me | $logP_{sauer}$=1,28 |
| II-88 | 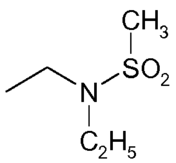 (3) | (2) Cl | (4) S02Me | $logP_{sauer}$=1,10 |
| II-89 | 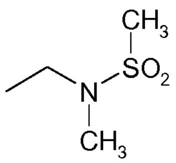 (3) | (2) $OCH_3$ | (4) Cl | $logP_{sauer}$=1,49 |

<u>Ausgangsstoffe der Formel (VIII)</u>

Beispiel (VIII-2)

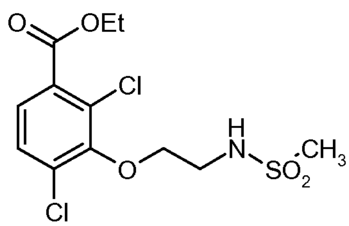

Stufe 1

Eine Mischung aus 4,8 g (20,4 mMol) 2,4-Dichlor-3-hydroxy-benzoesäure-ethylester, 11,6 g (36,8 mMol) N-[2-[(4-Methyl-phenyl)-sulfonyloxy]-ethyl]-O-t-butyl-carbamidsäureester, 5,6 g (40 mMol) Kaliumcarbonat und 140 ml Acetonitril wird 15 Stunden bei 75°C gerührt. Dann werden bei Raumtemperatur 200 ml Wasser dazu gegeben und die Mischung wird zweimal mit Methylenchlorid geschüttelt. Die vereinigten organischen Phasen werden mit Natriumsulfat getrocknet und filtriert. Vom Filtrat werden die flüchtigen Komponenten unter vermindertem Druck sorgfältig abdestilliert. Man erhält 8,0 g (84 % der Theorie, 81%iges Produkt) 2,4-Dichlor-3-[2-[(t-butoxycarbonyl)-amino]-ethoxy]-benzoesäure-ethylester. LogP = 3,79

Stufe 2

52 g (455 mMol) Trifluoressigsäure werden bei Raumtemperatur (ca. 20°C) unter Rühren zu 12,3 g (32,5 mMol)

2,4-Dichlor-3-[2-[(t-butoxycarbonyl)-amino]-ethoxy]-benzoesäure-ethylester tropfenweise gegeben. Die Mischung wird 15 Minuten bei Raumtemperatur gerührt und dann auf 200 ml Eiswasser gegeben. Dann wird zweimal mit je 150 ml Methylenchlorid extrahiert, die vereinigten organischen Phasen mit Natriumsulfat getrocknet und filtriert. Das Filtrat wird unter vermindertem Druck eingeengt, der Rückstand mit Petrolether/Diethylether digeriert und das kristallin angefallene Produkt durch Absaugen isoliert.
Man erhält 7,3 g (79 % der Theorie, 98%iges Produkt) 3-(2-Amino-ethoxy)-2,4-dichlor-benzoesäure-ethylester. LogP = 1,08

Stufe 3 (Verfahren i)

2,0 g (7,2 mMol) 3-(2-Amino-ethoxy)-2,4-dichlor-benzoesäure-ethylester werden in 24 ml Tetrahydrofuran gelöst und mit 0,29 g (7,2 mMol) Natriumhydrid (60%ig) versetzt. Die Mischung wird 60 Minuten bei Raumtemperatur (ca. 20°C) gerührt, dann mit 0,82 g (7,2 mMol) Methansulfonsäurechlorid versetzt und weitere 30 Minuten bei Raumtemperatur gerührt. Anschließend werden 50 ml Wasser dazu gegeben und die Mischung wird zweimal mit je 50 ml Essigsäureethylester extrahiert. Die vereinigten organischen Phasen werden mit Natriumsulfat getrocknet und filtriert. Vom Filtrat wird das Lösungsmittel unter vermindertem Druck sorgfältig abdestilliert.
Man erhält 0,75 g (25 % der Theorie, 86%iges Produkt) 2,4-Dichlor-3-(2-methylsulfonylamino-ethoxy)-benzoesäureethylester. LogP = 2,35

Beispiel (VIII-3)

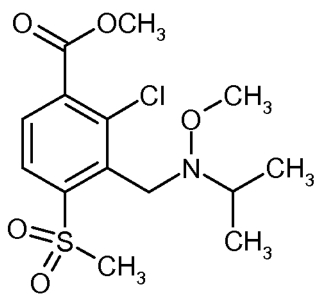

Stufe 1

Eine Lösung von 33,41 g (0,40 Mol) O-Methylhydroxylamin-Hydrochlorid in 100 ml Acetonitril wird bei Zimmertemperatur mit 55,20 g (0,40 Mol) Kaliumcarbonat versetzt. Nach 30 minütigem Rühren bei Zimmertemperatur gibt man eine Lösung von 68,32 g (0,20 Mol) 3-Brommethyl-2-chlor-4-methylsulfonyl-benzoesäuremethylester in 100 ml Acetonitril zu und läßt 18 Stunden bei Zimmertemperatur nachrühren. Zur Aufarbeitung wird das Reaktionsgemisch am Rotationsverdampfer vom Lösungsmittel befreit und der Rückstand zwischen Wasser und Dichlormethan verteilt. Die organische Phase wird abgetrennt, über Natriumsulfat getrocknet und unter vermindertem Druck eingeengt. Der Rückstand wird mit Diethylether verrührt und filtriert. Man erhält 36,6 g (55% der Theorie, 92%iges Produkt nach LC/MS) 2-Chlor-4-methylsulfonyl-3-(N-methoxy-aminomethyl)-benzoesäuremethylester als festen Rückstand.

Stufe 2 (Verfahren k)

4,00 g (13,0 mMol) 2-Chlor-4-methylsulfonyl-3-(N-methoxy-aminomethyl)-benzoesäuremethylester, 0,76 g (13,0 mMol) Aceton und 3,27 g (13,0 mMol) Pyridinium-*p*-toluolsulfonat werden in einer Mischung aus 50 ml Methanol und 15 ml Tetrahydrofuran bei Zimmertemperatur unter Schutzgasatmosphäre vorgelegt. Dazu tropft man bei Zimmertemperatur aus einer Spritze 1,3 ml (1,21 g, 13,0 mMol) Pyridin-Boran-Komplex zu und rührt noch 16 Stunden bei Zimmertemperatur nach.
Zur Aufarbeitung wird das Reaktionsgemisch mit Essigester versetzt und nacheinander mit 2 N wässriger Salzsäurelösung, gesättigter wässriger Natriumhydrogencarbonatlösung und gesättigter wässriger Natriumchloridlösung gewaschen. Die organische Phase wird anschließend über Natriumsulfat getrocknet und unter vermindertem Druck eingeengt. Man erhält 3,10 g (68% der Theorie, 90%iges Produkt nach HPLC,) 2-Chlor-4-methylsulfonyl-3-(N-methoxy-N-(1-methylethyl)-aminomethyl)-benzoesäuremethylester als gelbes hochviskoses Öl.

logP = 3,13 (sauer)

Analog zu den Beispielen (VIII-1) - (VIII-3) können beispielsweise auch die in der nachstehenden Tabelle 3 aufgeführten Verbindungen der Formel (VIII) hergestellt werden

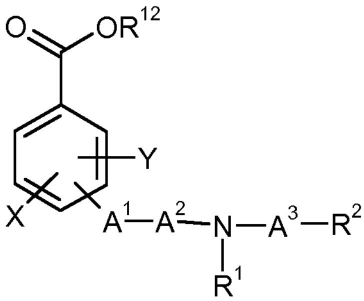

(VIII)

Tabelle 3 Beispiele für die Verbindungen der Formel (VIII)

| Bsp.- Nr | (Position) 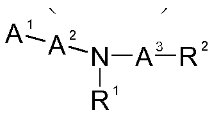 | $R^{12}$ | (Position) X | (Position) Y | Physik. Daten |
|---|---|---|---|---|---|
| VIII-4 | 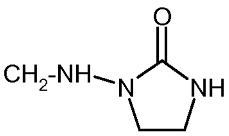 (3) | $CH_3$ | (2) Cl | (4) Cl | Fp.: 164°C |
| VIII-5 | 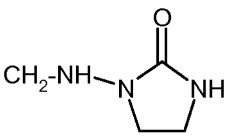 (3) | $CH_3$ | (2) Cl | (4) $SO_2CH_3$ | logP = 1,24 |
| VIII-6 | 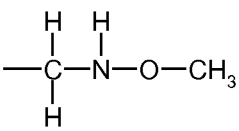 (3) | $CH_3$ | (2) Cl | (4) Cl | logP = 2,26 |
| VIII-7 | 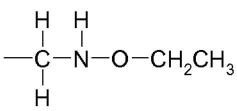 (3) | $CH_3$ | (2) Cl | (4) Cl | logP = 2,77 |
| VIII-8 | 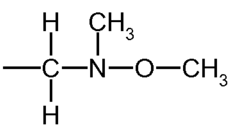 (3) | $CH_3$ | (2) Cl | (4) Cl | logP =3,08 |

(fortgesetzt)

| Bsp.- Nr | (Position)<br>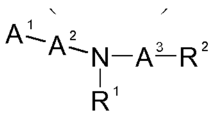 | $R^{12}$ | (Position) X | (Position) Y | Physik. Daten |
|---|---|---|---|---|---|
| VIII-9 | 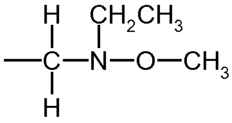 (3) | $CH_3$ | (2) Cl | (4) Cl | logP=3,72 |
| VIII-10 | (3) 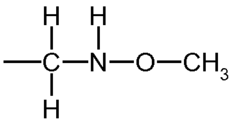 | $CH_3$ | (2) Cl | (4) $SO_2Me$ | logP=1,69 |
| VIII-11 | (3) 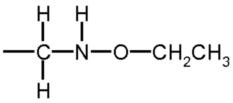 | $CH_3$ | (2) Cl | (4) $SO_2Me$ | logP=2,05 |
| VIII-12 | (3) 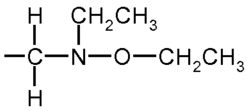 | $CH_3$ | (2) Cl | (4) $SO_2Me$ | logP=3,06 |
| VIII-13 | (3) 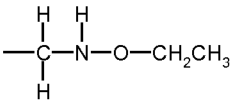 | $CH_3$ | (2) OMe | (4) Cl | logP=2,27 |
| VIII-14 | (3) 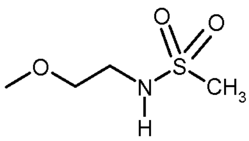 | $C_2H_5$ | (2) Cl | (4) Cl | logP=2,24 |
| VIII-15 | (3) 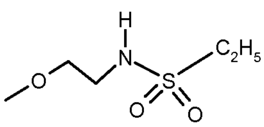 | $C_2H_5$ | (2) Cl | (4) Cl | logP=2,61 |
| VIII-16 | (3) 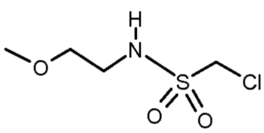 | $C_2H_5$ | (2) Cl | (4) Cl | logP=2,80 |
| VIII-17 | (3) 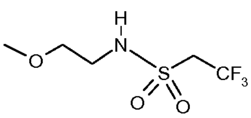 | $C_2H_5$ | (2) Cl | (4) Cl | logP=3,08 |
| VIII-18 | (3) 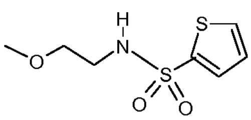 | $C_2H_5$ | (2) Cl | (4) Cl | logP=3,20 |

(fortgesetzt)

| Bsp.- Nr | (Position) 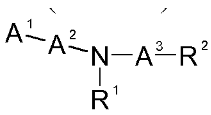 | $R^{12}$ | (Position) X | (Position) Y | Physik. Daten |
|---|---|---|---|---|---|
| VIII-19 | 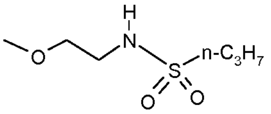 (3) | $C_2H_5$ | (2) Cl | (4) Cl | logP=2,92 |
| VIII-20 | 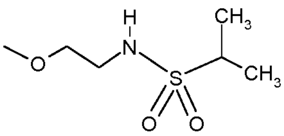 (3) | $C_2H_5$ | (2) Cl | (4) Cl | logP=2,86 |
| VIII-21 | 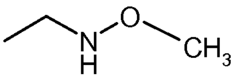 (3) | $CH_3$ | (2) Cl | (4) $SO_2Me$ | $logP_{sauer}$=1,69 |
| VIII-22 | 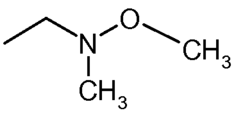 (3) | $CH_3$ | (2) Cl | (4) $SO_2Me$ | $logP_{sauer}$=2,31 |
| VIII-23 | 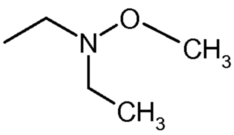 (3) | $CH_3$ | (2) Cl | (4) $SO_2Me$ | $logP_{sauer}$=2,74 |
| VIII-24 | 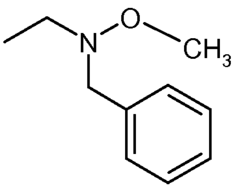 (3) | $CH_3$ | (2) Cl | (4) $SO_2Me$ | $logP_{sauer}$=3,42 |
| VIII-25 | 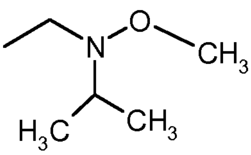 (3) | $CH_3$ | (2) Cl | (4) $SO_2Me$ | $logP_{sauer}$=3,13 |
| VIII-26 | 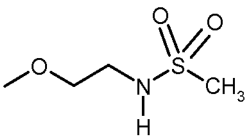 (3) | $CH_3$ | (2) $CH_3$ | (4) Cl | $logP_{sauer}$=2,14 |
| VIII-27 | 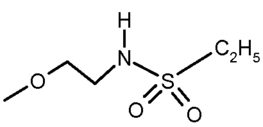 (3) | $CH_3$ | (2) $CH_3$ | (4) Cl | $logP_{sauer}$=2,37 |

(fortgesetzt)

| Bsp.- Nr | (Position) 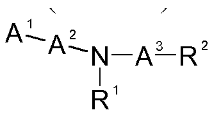 | $R^{12}$ | (Position) X | (Position) Y | Physik. Daten |
|---|---|---|---|---|---|
| VIII-28 | (3) 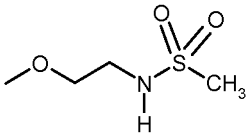 | $CH_3$ | (2) $CH_3$ | (4) Br | $logP_{sauer}$=2,20 |
| VIII-29 | (3) 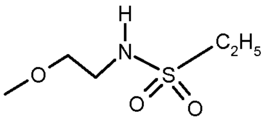 | $CH_3$ | (2) $CH_3$ | (4) Br | $logP_{saue}$=2,46 |
| VIII-30 | (3) 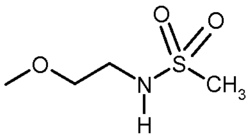 | $CH_3$ | (2) Cl | (4) $SO_2Me$ | $logP_{saue}$=1,56 |
| VIII-31 | (3) 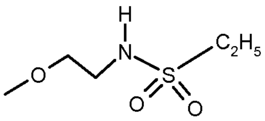 | $CH_3$ | (2) Cl | (4) $SO_2Me$ | $logP_{sauer}$=1,75 |
| VIII-32 | (3) 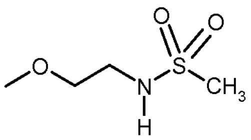 | $CH_3$ | (2) Br | (4) Br | $logP_{sauer}$=2,13 |
| VIII-33 | (3) 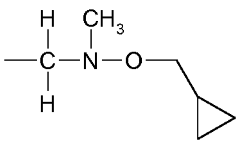 | $CH_3$ | (2) Cl | (4) $SO_2Me$ | $logP_{sauer}$=2,98 |
| VIII-34 | (3) 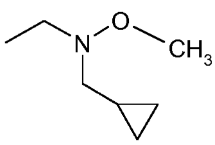 | $CH_3$ | (2) Cl | (4) $SO_2Me$ | $logP_{saue}$=3,18 |
| VIII-35 | (3) 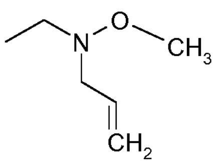 | $CH_3$ | (2) Cl | (4) $SO_2Me$ | $logP_{sauer}$=2,87 |

(fortgesetzt)

| Bsp.- Nr | (Position) 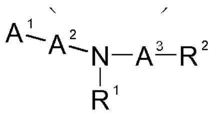 | $R^{12}$ | (Position) X | (Position) Y | Physik. Daten |
|---|---|---|---|---|---|
| VIII-36 | (3) 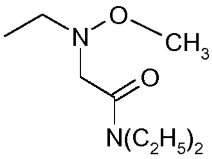 | $CH_3$ | (2) Cl | (4) $SO_2Me$ | $logP_{sauer}$=2,28 |
| VIII-37 | (3) 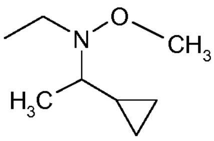 | $CH_3$ | (2) Cl | (4) $SO_2Me$ | $logP_{sauer}$=3,58 |
| VIII-38 | (3) 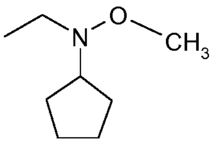 | $CH_3$ | (2) Cl | (4) $SO_2Me$ | $logP_{sauer}$=3,67 |
| VIII-39 | (3) 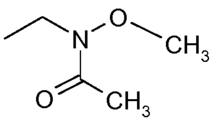 | $CH_3$ | (2) Cl | (4) $SO_2Me$ | $logP_{sauer}$=1,58 |
| VIII-40 | (3) 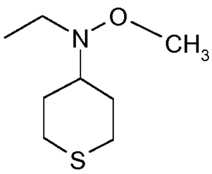 | $CH_3$ | (2) Cl | (4) $SO_2Me$ | $logP_{sauer}$=3,20 |
| VIII-41 | (3) 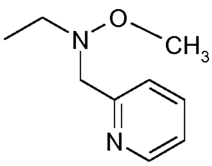 | $CH_3$ | (2) Cl | (4) $SO_2Me$ | $logP_{sauer}$=1,17 |
| VIII-42 | (3) 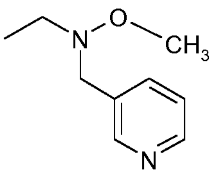 | $CH_3$ | (2) Cl | (4) $SO_2Me$ | $logP_{sauer}$=1,16 |
| VIII-43 | (3) 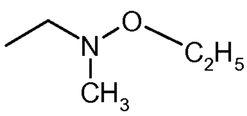 | $CH_3$ | (2) Cl | (4) $SO_2Me$ | $logP_{sauer}$=2,64 |

(fortgesetzt)

| Bsp.- Nr | (Position) 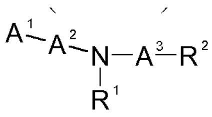 | $R^{12}$ | (Position) X | (Position) Y | Physik. Daten |
|---|---|---|---|---|---|
| VIII-44 | 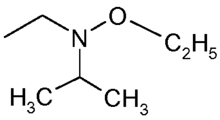 (3) | $CH_3$ | (2) Cl | (4) $SO_2Me$ | $logP_{sauer}$=3,52 |
| VIII-45 | 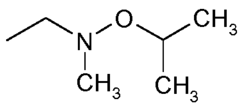 (3) | $CH_3$ | (2) Cl | (4) $SO_2Me$ | $logP_{sauer}$=2,97 |
| VIII-46 | 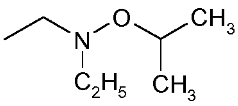 (3) | $CH_3$ | (2) Cl | (4) $SO_2Me$ | $logP_{sauer}$=3,39 |
| VIII-47 | 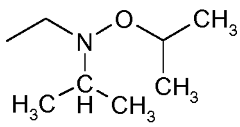 (3) | $CH_3$ | (2) Cl | (4) $SO_2Me$ | $logP_{sauer}$=3,86 |
| VIII-48 | 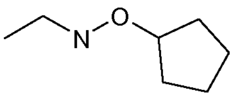 (3) | $CH_3$ | (2) Cl | (4) $SO_2Me$ | $logP_{sauer}$=2,93 |
| VIII-49 | 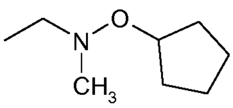 (3) | $CH_3$ | (2) Cl | (4) $SO_2Me$ | $logP_{sauer}$=3,48 |
| VIII-50 | 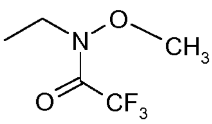 (3) | $CH_3$ | (2) Cl | (4) $SO_2Me$ | $logP_{sauer}$=2,60 |
| VIII-51 | 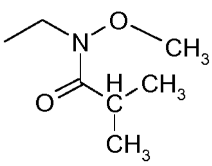 (3) | $CH_3$ | (2) Cl | (4) $SO_2Me$ | $logP_{sauer}$=2,21 |
| VIII-52 | 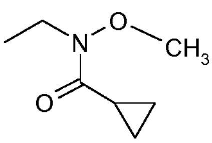 (3) | $CH_3$ | (2) Cl | (4) $SO_2Me$ | $logP_{sauer}$=2,02 |

(fortgesetzt)

| Bsp.- Nr | (Position) 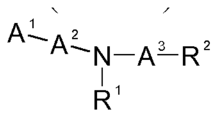 | $R^{12}$ | (Position) X | (Position) Y | Physik. Daten |
|---|---|---|---|---|---|
| VIII-53 | 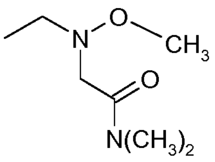 (3) | $CH_3$ | (2) Cl | (4) $SO_2Me$ | $logP_{sauer}$=1,74 |
| VIII-54 | 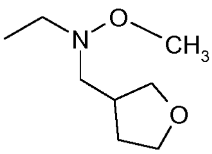 (3) | $CH_3$ | (2) Cl | (4) $SO_2Me$ | $logP_{sauer}$=2,35 |
| VIII-55 | 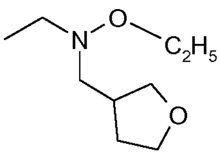 (3) | $CH_3$ | (2) Cl | (4) $SO_2Me$ | $logP_{sauer}$=2,60 |
| VIII-56 | 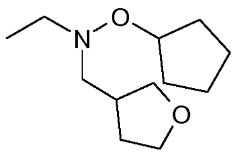 (3) | $CH_3$ | (2) Cl | (4) $SO_2Me$ | $logP_{sauer}$=3,30 |
| VIII-57 | 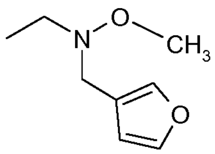 (3) | $CH_3$ | (2) Cl | (4) $SO_2Me$ | $logP_{sauer}$=2,91 |
| VIII-58 | 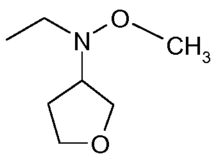 (3) | $CH_3$ | (2) Cl | (4) $SO_2Me$ | $logP_{sauer}$=2,17 |
| VIII-59 | 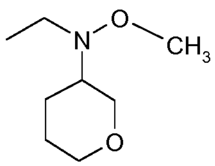 (3) | $CH_3$ | (2) Cl | (4) $SO_2Me$ | $logP_{sauer}$=2,55 |

(fortgesetzt)

| Bsp.- Nr | (Position) 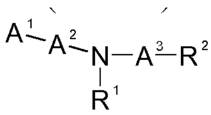 | $R^{12}$ | (Position) X | (Position) Y | Physik. Daten |
|---|---|---|---|---|---|
| VIII-60 | 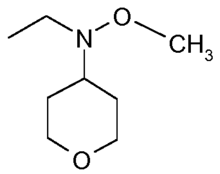 (3) | $CH_3$ | (2) Cl | (4) $S0_2Me$ | $logP_{sauer}$=2,38 |
| VIII-61 | (3) 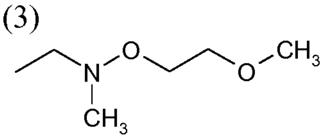 | $CH_3$ | (2) Cl | (4) $SO_2Me$ | $logP_{sauer}$=2,26 |
| VIII-62 | (3) 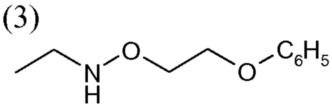 | $CH_3$ | (2) Cl | (4) $SO_2Me$ | $logP_{sauer}$=2,93 |
| VIII-63 | (3) 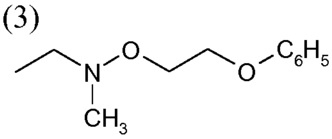 | $CH_3$ | (2) Cl | (4) $SO_2Me$ | $logP_{sauer}$=3,37 |
| VIII-64 | (3) | $CH_3$ | (2) Cl | (4) $SO_2Me$ | $logP_{sauer}$=2,51 |
| | 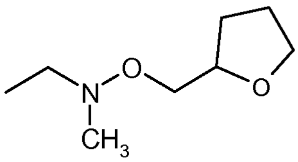 | | | | |
| VIII-65 | 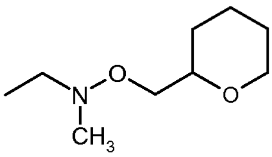 (3) | $CH_3$ | (2) Cl | (4) $SO_2Me$ | $logP_{sauer}$=2,87 |
| VIII-66 | 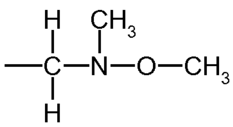 (3) | $CH_3$ | (2) $OCH_3$ | (4) Cl | $logP_{sauer}$=2,76 |
| VIII-67 | 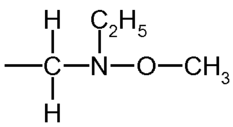 (3) | $CH_3$ | (2) $OCH_3$ | (4) Cl | $logP_{sauer}$=3,43 |

(fortgesetzt)

| Bsp.- Nr | (Position) 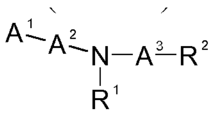 | $R^{12}$ | (Position) X | (Position) Y | Physik. Daten |
|---|---|---|---|---|---|
| VIII-68 | (3) 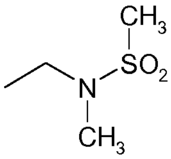 | $CH_3$ | (2) Cl | (4) Cl | $logP_{sauer}$=2,15 |
| VIII-69 | (3) 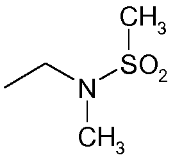 | $CH_3$ | (2) Cl | (4) $SO_2Me$ | $logP_{sauer}$=1,65 |
| VIII-70 | (3) 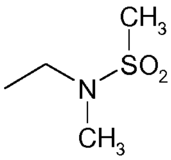 | $CH_3$ | (2) $OCH_3$ | (4) Cl | $logP_{sauer}$=2,09 |
| VIII-71 | (3) 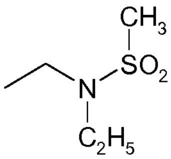 | $CH_3$ | (2) $OCH_3$ | (4) Cl | $logP_{sauer}$=2,38 |
| VIII-72 | (3) 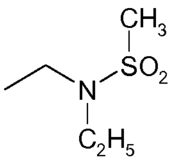 | $CH_3$ | (2) Cl | (4) $SO_2Me$ | $logP_{sauer}$=1,88 |
| VIII-73 | (3) 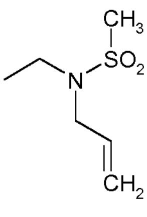 | $CH_3$ | (2) Cl | (4) $SO_2Me$ | $logP_{sauer}$=2,03 |

**Anwendungsbeispiele:**

**Beispiel A**

Pre-emergence-Test

Lösungsmittel: 5 Gewichtsteile Aceton

(fortgesetzt)

| | |
|---|---|
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.
Samen der Testpflanzen werden in normalen Boden ausgesät. Nach 24 Stunden wird der Boden so mit der Wirkstoffzubereitung besprüht, dass die jeweils gewünschte Wirkstoffmenge pro Flächeneinheit ausgebracht wird. Die Wirkstoffkonzentration in der Spritzbrühe wird so gewählt, dass in 1000 Liter Wasser pro Hektar die jeweils gewünschte Wirkstoffmenge ausgebracht wird.
Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigen beispielsweise die Verbindungen gemäß Herstellungsbeispiel 2, 4, 5 und 6 bei zum Teil guter Verträglichkeit gegenüber Kulturpflanzen, wie z.B. Mais und Soja, starke Wirkung gegen Unkräuter.

**Beispiel B**

Post-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.
Mit der Wirkstoffzubereitung spritzt man Testpflanzen, welche eine Höhe von 5-15 cm haben so, dass die jeweils gewünschten Wirkstoffmengen pro Flächeneinheit ausgebracht werden. Die Konzentration der Spritzbrühe wird so gewählt, dass in 1000 1 Wasser/ha die jeweils gewünschten Wirkstoffmengen ausgebracht werden.
Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)

100 % = totale Vernichtung

In diesem Test zeigen beispielsweise die Verbindungen gemäß Herstellungsbeispiel 2, 3, 4 und 5 bei zum Teil guter Verträglichkeit gegenüber Kulturpflanzen, wie z.B. Mais und Weizen, starke Wirkung gegen Unkräuter.

**Patentansprüche**

1. Verbindung der Formel (I-2)

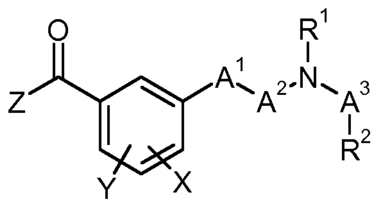

(I-2)

in welcher

$A^1$ für O (Sauerstoff)

steht, wobei

$A^3$ für O (Sauerstoff), S (Schwefel), die Gruppierung

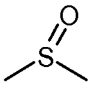 , 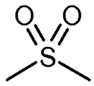 , 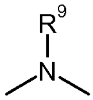 , 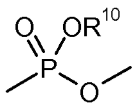 oder 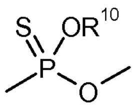

steht, wobei

Z für eine der nachstehenden Gruppierungen steht

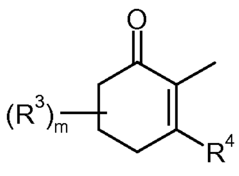 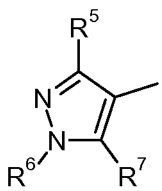

wobei

$A^2$ für Alkandiyl mit 1 bis 6 Kohlenstoffatomen, Alkendiyl oder Alkindiyl mit jeweils 2 bis 6 Kohlenstoffatomen steht;
$R^1$ für Wasserstoff, für gegebenenfalls durch Hydroxy, Amino, Cyano, Carbamoyl, Halogen, $C_1$-$C_4$-Alkoxy, $C_1$-$C_4$-Alkyl-carbonyl, $C_1$-$C_4$-Alkoxycarbonyl, $C_1$-$C_4$-Alkylamino-carbonyl, Di-($C_1$-$C_4$-alkyl)-amino, Di-($C_1$-$C_4$-alkyl)-amino-carbonyl oder N-($C_1$-$C_4$-Alkoxy)-N-($C_1$-$C_4$-alkyl)-amino-carbonyl substituiertes Alkyl mit 1 bis 6 Kohlenstoffatomen, für jeweils gegebenenfalls durch Halogen substituiertes Alkylthio, Alkylsulfinyl oder Alkylsulfonyl mit jeweils 1 bis 6 Kohlenstoffatomen, für jeweils gegebenenfalls durch Halogen substituiertes Alkenyl oder Alkinyl mit jeweils 2 bis 6 Kohlenstoffatomen, für jeweils gegebenenfalls durch Cyano, Halogen oder $C_1$-$C_4$-Alkyl substituiertes Cycloalkyl, Cycloalkylalkyl, mit jeweils 3 bis 6 Kohlenstoffatomen in den Cycloalkylgruppen und gegebenenfalls 1 bis 4 Kohlenstoffatomen im Alkylteil, für jeweils gegebenenfalls durch Cyano, Nitro, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Aryl, Arylalkyl, oder Arylcarbonylalkyl mit jeweils 6 oder 10 Kohlenstoffatomen in den Arylgruppen und gegebenenfalls 1 bis 4 Kohlenstoffatomen im Alkylteil, oder für durch jeweils gegebenenfalls Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Heterocyclyl, oder Heterocyclylalkyl, wobei jeweils die Heterocyclyl-Gruppierung bis zu 10 Kohlenstoffatome und zusätzlich mindestens ein Heteroatom ausgewählt aus der Reihe N (Stickstoff, höchstens jedoch 5 N-Atome), O (Sauerstoff, höchstens jedoch 2 O-Atome), S (Schwefel, höchstens jedoch 2 S-Atome), SO oder $SO_2$, sowie gegebenenfalls zusätzlich eine Gruppe ausgewählt aus Oxo (C=O), Thioxo (C=S), Imino (C=NH), Cyanoimino (C=N-CN), Nitroimino (C=N-$NO_2$) enthält, steht;
$R^2$ für Wasserstoff, für gegebenenfalls durch Cyano, Halogen, $C_1$-$C_4$-Alkoxy, $C_1$-$C_4$-Alkyl-carbonyl, $C_1$-$C_4$-Alkoxy-carbonyl, $C_1$-$C_4$-Alkyl-amino-carbonyl oder Di-($C_1$-$C_4$-alkyl)-amino-carbonyl substituiertes Alkyl mit 1 bis 6 Kohlenstoffatomen, für jeweils gegebenenfalls durch Cyano, Halogen oder $C_1$-$C_4$-Alkoxy substituiertes Alkylcarbonyl, Alkoxycarbonyl oder Alkylaminocarbonyl mit jeweils 1 bis 6 Kohlenstoffatomen in den Alkylgruppen, für Dialkylaminocarbonyl mit jeweils 1 bis 4 Kohlenstoffatomen in den Alkylgruppen, für jeweils gegebenenfalls

durch Halogen substituiertes Alkenyl, Alkenylcarbonyl, Alkenyloxycarbonyl, Alkinyl, Alkinylcarbonyl oder Alkinyloxycarbonyl mit jeweils 3 bis 6 Kohlenstoffatomen in den Alkenyl- bzw. Alkinyl-gruppen, für jeweils gegebenenfalls durch Cyano, Halogen oder $C_1$-$C_4$-Alkyl substituiertes Cycloalkyl, Cycloalkylcarbonyl, Cycloalkyloxycarbonyl, Cycloalkylalkyl, Cycloalkylalkylcarbonyl oder Cycloalkylalkoxycarbonyl mit jeweils 3 bis 6 Kohlenstoffatomen in den Cycloalkylgruppen und gegebenenfalls 1 bis 4 Kohlenstoffatomen im Alkylteil, für jeweils gegebenenfalls durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Aryl, Arylcarbonyl, Aryloxycarbonyl, Arylaminocarbonyl, Arylalkyl, Arylalkylcarbonyl, Arylalkoxycarbonyl oder Arylalkylaminocarbonyl mit jeweils 6 oder 10 Kohlenstoffatomen in den Arylgruppen und gegebenenfalls 1 bis 4 Kohlenstoffatomen im Alkylteil, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Heterocyclyl, Heterocyclylcarbonyl, oder Heterocyclylalkyl, wobei jeweils die Heterocyclyl-Gruppierung bis zu 10 Kohlenstoffatome und zusätzlich mindestens ein Heteroatom ausgewählt aus der Reihe N (Stickstoff, höchstens jedoch 5 N-Atome), O (Sauerstoff, höchstens jedoch 2 O-Atome), S (Schwefel, höchstens jedoch 2 S-Atome), SO oder $SO_2$, sowie gegebenenfalls zusätzlich eine Gruppe ausgewählt aus Oxo (C=O), Thioxo (C=S), Imino (C=NH), Cyanoimino (C=N-CN), Nitroimino (C=N-$NO_2$) enthält, steht;

$R^3$ für Wasserstoff, Halogen, für jeweils gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl oder Alkylthio mit jeweils 1 bis 6 Kohlenstoffatomen, oder für Phenyl, oder - für den Fall, dass m für 2 steht - gegebenenfalls auch zusammen mit einem zweiten Rest $R^3$ für Sauerstoff oder Alkandiyl (Alkylen) mit 3 bis 5 Kohlenstoffatomen steht;

$R^4$ für Hydroxy, Formyloxy, Halogen, für jeweils gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkoxy, Alkylthio, Alkylsulfinyl, Alkylsulfonyl, Alkylcarbonyloxy, Alkoxycarbonyloxy, Alkylaminocarbonyloxy oder Alkylsulfonyloxy mit jeweils 1 bis 6 Kohlenstoffatomen, für jeweils gegebenenfalls durch Halogen substituiertes Alkenyloxy oder Alkinyloxy mit jeweils 3 bis 6 Kohlenstoffatomen, für jeweils gegebenenfalls durch Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl substituiertes Aryloxy, Arylthio, Arylsulfinyl, Arylsulfonyl, Arylcarbonyloxy, Arylcarbonylalkoxy, Arylsulfonyloxy, Arylalkoxy, Arylalkylthio, Arylalkylsulfinyl oder Arylalkylsulfonyl mit jeweils 6 oder 10 Kohlenstoffatomen in der Arylgruppe und gegebenenfalls 1 bis 4 Kohlenstoffatomen im Alkylteil, für gegebenenfalls durch Cyano, Halogen, Oxo-, Hydroxy-, $C_1$-$C_4$-Alkoxy, $C_1$-$C_4$-Alkyl substituiertes Heterocyclyl mit 5 oder 6 Ringatome, wobei mindestens 1 Stickstoffatom und gegebenenfalls bis zu 2 Sauerstoffatome, Schwefelatome und 3 Stickstoffatome enthalten sind, wobei insgesamt nicht mehr als 4 Heteroatome vorliegen und der Heterocyclus über den Stickstoff gebunden ist, steht;

$R^5$ für Wasserstoff, Cyano, Carbamoyl, Thiocarbamoyl, Halogen, für jeweils gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl, Alkoxy, Alkylthio, Alkylsulfinyl, Alkylsulfonyl oder Alkoxycarbonyl mit jeweils 1 bis 6 Kohlenstoffatomen in den Alkylgruppen oder für gegebenenfalls durch Cyano, Halogen oder $C_1$-$C_4$-Alkyl substituiertes Cycloalkyl mit 3 bis 6 Kohlenstoffatomen steht;

$R^6$ für Wasserstoff, für gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl mit 1 bis 6 Kohlenstoffatomen, für jeweils gegebenenfalls durch Cyano oder Halogen substituiertes Alkenyl oder Alkinyl mit jeweils 3 bis 6 Kohlenstoffatomen, für jeweils gegebenenfalls durch Cyano, Halogen oder $C_1$-$C_4$-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy, $C_1$-$C_4$-Halogen-alkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogen-alkylsulfonyl substituiertes Aryl oder Arylalkyl mit jeweils 6 oder 10 Kohlenstoffatomen in der Arylgruppe und gegebenenfalls 1 bis 4 Kohlenstoffatomen im Alkylteil steht;

$R^7$ für Hydroxy, Formyloxy, für jeweils gegebenenfalls durch Alkyl, Cyano, Halogen oder $C_1$-$C_4$-Alkoxy substituiertes Alkoxy, Alkylcarbonyloxy, Alkoxycarbonyloxy, Alkoxycarbonylalkoxy, Alkylaminocarbonyloxy oder Alkylsulfonyloxy mit jeweils 1 bis 6 Kohlenstoffatomen in den Alkylgruppen, für jeweils gegebenenfalls durch Cyano oder Halogen substituiertes Alkenyloxy oder Alkinyloxy mit jeweils 3 bis 6 Kohlenstoffatomen, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl substituiertes Arylalkoxy, Arylcarbonyloxy, Arylcarbonylalkoxy oder Arylsulfonyloxy mit jeweils 6 oder 10 Kohlenstoffatomen in der Arylgruppe und gegebenenfalls 1 bis 4 Kohlenstoffatomen im Alkylteil steht;.

$R^9$ für Wasserstoff, für gegebenenfalls durch Hydroxy, Amino, Cyano, Carbamoyl, Halogen, $C_1$-$C_4$-Alkoxy, $C_1$-$C_4$-Alkyl-carbonyl, $C_1$-$C_4$-Alkoxycarbonyl, $C_1$-$C_4$-Alkylamino-carbonyl oder Di-($C_1$-$C_4$-alkyl)-aminocarbonyl

substituiertes Alkyl mit 1 bis 6 Kohlenstoffatomen, für jeweils gegebenenfalls durch Cyano, Halogen oder $C_1$-$C_4$-Alkoxy substituiertes Alkylcarbonyl oder Alkoxycarbonyl mit jeweils 1 bis 6 Kohlenstoffatomen in den Alkylgruppen, für jeweils gegebenenfalls durch Halogen substituiertes Alkylthio, Alkylsulfinyl oder Alkylsulfonyl mit jeweils 1 bis 6 Kohlenstoffatomen, für jeweils gegebenenfalls durch Halogen substituiertes Alkenyl, Alkenylcarbonyl, Alkenyloxycarbonyl, Alkinyl, Alkinylcarbonyl oder Alkinyloxycarbonyl mit jeweils 2 bis 6 Kohlenstoffatomen in den Alkenyl- bzw. Alkinyl-gruppen, für jeweils gegebenenfalls durch Cyano, Halogen oder $C_1$-$C_4$-Alkyl substituiertes Cycloalkyl, Cycloalkylcarbonyl, Cycloalkyloxycarbonyl, Cycloalkylalkyl, Cycloalkylalkylcarbonyl oder Cycloalkylalkoxycarbonyl mit jeweils 3 bis 6 Kohlenstoffatomen in den Cycloalkylgruppen und gegebenenfalls 1 bis 4 Kohlenstoffatomen im Alkylteil, für jeweils gegebenenfalls durch Cyano, Nitro, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Aryl, Arylcarbonyl, Aryloxycarbonyl, Arylalkyl, Arylalkylcarbonyl oder Arylalkoxycarbonyl mit jeweils 6 oder 10 Kohlenstoffatomen in den Arylgruppen und gegebenenfalls 1 bis 4 Kohlenstoffatomen im Alkylteil, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituiertes Heterocyclyl, Heterocyclylcarbonyl oder Heterocyclylalkyl, wobei jeweils die Heterocyclyl-Gruppierung bis zu 10 Kohlenstoffatome und zusätzlich mindestens ein Heteroatom ausgewählt aus der Reihe N (höchstens jedoch 5 N-Atome), O (höchstens jedoch 2 O-Atome), S (höchstens jedoch 2 S-Atome), SO oder $SO_2$, sowie gegebenenfalls zusätzlich eine Gruppe ausgewählt aus Oxo (C=O), Thioxo (C=S), Imino (C=NH), Cyanoimino (C=N-CN), Nitroimino (C=N-$NO_2$) enthält, oder gemeinsam mit $R^2$ und dem Stickstoff an welches sie gebunden sind für einen gegebenenfalls durch Nitro, Cyano, Halogen, $C_1$-$C_4$-Alkyl, $C_1$-$C_4$-Halogenalkyl, $C_1$-$C_4$-Alkoxy oder $C_1$-$C_4$-Halogenalkoxy substituierten Heterocyclus steht, welcher 1 Stickstoffatom und 1 bis 10 Kohlenstoffatome und gegebenenfalls ein weiteres Heteroatom aus der Reihe N (höchstens jedoch 4 weitere N-Atome), O (höchstens jedoch 2 O-Atome), S (höchstens jedoch 2 S-Atome), SO oder $SO_2$, enthält sowie gegebenenfalls zusätzlich eine Gruppe ausgewählt aus Oxo (C=O), Thioxo (C=S), Imino (C=NH), Cyanoimino (C=N-CN), Nitroimino (C=N-$NO_2$) steht;

$R^{10}$ für Wasserstoff, Formyl, für gegebenenfalls durch Cyano, Halogen, $C_1$-$C_4$-Alkoxy, $C_1$-$C_4$-Alkylcarbonyl, $C_1$-$C_4$-Alkoxy-carbonyl, $C_1$-$C_4$-Alkylamino-carbonyl oder Di-($C_1$-$C_4$-alkyl)-amino-carbonyl substituiertes Alkyl mit 1 bis 6 Kohlenstoffatomen steht;
X für Wasserstoff, Nitro, Cyano, Carboxy, Carbamoyl, Thiocarbamoyl, Halogen, oder für jeweils gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl, Alkoxy, Alkylthio, Alkylsulfinyl, Alkylsulfonyl, Alkylamino, Dialkylamino oder Dialkylaminosulfonyl mit jeweils 1 bis 6 Kohlenstoffatomen in den Alkylgruppen steht;
Y für Wasserstoff, Nitro, Cyano, Carboxy, Carbamoyl, Thiocarbamoyl, Halogen, oder für jeweils gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl, Alkoxy, Alkylthio, Alkylsulfinyl, Alkylsulfonyl, Alkylamino, Dialkylamino oder Dialkylaminosulfonyl mit jeweils 1 bis 6 Kohlenstoffatomen in den Alkylgruppen steht;
m für die Zahlen 0, 1, 2 oder 3 steht.

**2.** Verbindung gemäß Anspruch 1 in welcher

$A^2$ für Methylen (-$CH_2$-), Ethan-1,1-diyl-(-CH($CH_3$)-), Ethan-1,2-diyl (Di-methylen, -$CH_2CH_2$-), Propan-1,1-diyl-(-CH($C_2H_5$)-), Propan-1,2-diyl (-CH($CH_3$)$CH_2$-), Propan-1,3-diyl-(-$CH_2CH_2CH_2$-), Butan-1,3-diyl (-CH($CH_3$)$CH_2CH_2$-), Butan-1,4-diyl-(-$CH_2CH_2CH_2CH_2$-), Ethendiyl, Propendiyl, Butendiyl, Ethindiyl, Propindiyl oder Butindiyl.
$R^1$ für Wasserstoff, für jeweils gegebenenfalls durch Hydroxy, Amino, Cyano, Carbamoyl, Fluor, Chlor, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, Dimethylamino, Diethylamino, Dimethylaminocarbonyl, Diethylaminocarbonyl oder N-Methoxy-N-methyl-aminocarbonyl substituiertes Methyl, Ethyl, n- oder i-Propyl, n-, i- oder s-Butyl, n-, i- oder s-Pentyl, für jeweils gegebenenfalls durch Fluor und/oder Chlor substituiertes Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, für jeweils gegebenenfalls durch Fluor, Chlor und/oder Brom substituiertes Propenyl, Butenyl, Propinyl, oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl, oder Cyclohexylmethyl, für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, s- oder i-Propoxy, n-, i-, s- oder t-Butoxy, Difluormethoxy oder Trifluormethoxy substituiertes Phenyl, Benzoyl, Benzyl, Phenylethyl, oder Phenylcarbonylmethyl, oder für jeweils gegebenenfalls durch Nitro, Cyano, Fluor, Chlor, Brom, Methyl, Ethyl, n- oder i-Propyl, n-, i-, s- oder t-Butyl,

Trifluormethyl, Methoxy, Ethoxy, n- oder i-Propoxy, Difluormethoxy oder Trifluormethoxy substituiertes Heterocyclyl, oder Heterocyclylalkyl aus der Reihe Furyl, , Furylmethyl, Thienyl, Thienylmethyl, Pyrrolidinyl, Oxopyrrolidinyl, Pyrrolyl, Indolyl, Pyrrolylmethyl, Pyrazolyl, Pyrazolylmethyl, Oxazolyl, Oxazolylmethyl, Isoxazolyl, Thiazolyl, Thiazolylmethyl, Dihydropyranyl, Dihydropyranylmethyl, Piperidinyl, Oxopiperidinyl, Morpholinyl, Piperazinyl, Pyridinyl, Pyridinylcarbonyl oder Pyridinylmethyl steht;

$R^2$ für Wasserstoff, für jeweils gegebenenfalls durch Cyano, Fluor, Chlor, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, Dimethylaminocarbonyl oder Diethylaminocarbonyl substituiertes Methyl, Ethyl, n- oder i-Propyl, n-, i-, s- oder t-Butyl, n-, i-, s- oder t-Pentyl, für jeweils gegebenenfalls durch Cyano, Fluor, Chlor, Methoxy, Ethoxy, n- oder i-Propoxy substituiertes Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, für Dimethylaminocarbonyl, Diethylaminocarbonyl oder Dipropylaminocarbonyl, für jeweils gegebenenfalls durch Fluor, Chlor und/oder Brom substituiertes Propenyl, Butenyl, Propenylcarbonyl, Butenylcarbonyl, Propenyloxycarbonyl, Butenyloxycarbonyl, Propenylaminocarbonyl, Butenylaminocarbonyl, Propinyl, Butinyl, Propinylcarbonyl, Butinylcarbonyl, Propinyloxycarbonyl oder Butinyloxycarbonyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylcarbonyl, Cyclobutylcarbonyl, Cyclopentylcarbonyl, Cyclohexylcarbonyl, Cyclopropyloxycarbonyl, Cyclobutyloxycarbonyl, Cyclopentyloxycarbonyl, Cyclohexyloxycarbonyl, Cyclopropylmethyl, Cyclobutylmethyl, Cyclopentylmethyl, Cyclohexylmethyl, Cyclopropylmethylcarbonyl, Cyclobutylmethylcarbonyl, Cyclopentylmethylcarbonyl, Cyclohexylmethylcarbonyl, Cyclopropylmethoxycarbonyl, Cyclobutylmethoxycarbonyl, Cyclopentylmethoxycarbonyl, Cyclohexylmethoxycarbonyl, für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Naphthyl, Benzoyl, Phenoxycarbonyl, Phenylaminocarbonyl, Benzyl, Phenylethyl, Phenylmethylcarbonyl oder Phenylmethoxycarbonyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Furyl, Furylcarbonyl, Furylmethyl, Thienyl, Thienylcarbonyl, Thienylmethyl, Pyrrolidinyl, Oxopyrrolidinyl, Pyrrolyl, Indolyl, Pyrrolylmethyl, Pyrazolyl, Pyrazolylcarbonyl, Pyrazolylmethyl, Oxazolyl, Oxazolylmethyl, Isoxazolyl, Isoxazolylcarbonyl, Thiazolyl, Thiazolylmethyl, 2-Oxo-1,3-diaza-cyclopentyl (2-Oxo-imidazolidinyl), Piperidinyl, Oxopiperidinyl, 2-Oxo-1,3-diazacyclohexyl, Morpholinyl, Thiomorpholinyl, 3-Oxo-morpholinyl, 3-Oxothiomorpholinyl, Piperazinyl, Pyridinyl, Pyridinylcarbonyl oder Pyridinylmethyl steht;

$R^3$ für Wasserstoff, Fluor, Chlor oder Brom, für jeweils gegebenenfalls durch Cyano, Fluor, Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, Methylthio, Ethylthio, n- oder i-Propylthio, n-, i-, s- oder t-Butylthio, oder für Phenyl, oder - für den Fall, dass m für 2 steht - gegebenenfalls auch zusammen mit einem zweiten Rest $R^3$ für Sauerstoff, Propan-1,3-diyl oder Butan-1,4-diyl steht;

$R^4$ für Hydroxy, Formyloxy, Fluor oder Chlor, für jeweils gegebenenfalls durch Cyano, Fluor, Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Acetyloxy, Propionyloxy, n- oder i-Butyroyloxy, Methoxycarbonyloxy, Ethoxycarbonyloxy, n- oder i-Propoxycarbonyloxy, Methylaminocarbonyloxy, Ethylaminocarbonyloxy, n- oder i-Propylaminocarbonyloxy, Methylsulfonyloxy, Ethylsulfonyloxy, n- oder i-Propylsulfonyloxy, für jeweils gegebenenfalls durch Fluor, Chlor und/oder Brom substituiertes Propenyloxy, Butenyloxy, Propinyloxy oder Butinyloxy, für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n- oder i-Propylthio, Difluormethylthio, Trifluormethylthio, Methylsulfinyl, Ethylsulfinyl, Trifluormethylsulfinyl, Methylsulfonyl, Ethylsulfonyl oder Trifluormethylsulfonyl substituiertes Phenyloxy, Phenylthio, Phenylsulfinyl, Phenylsulfonyl, Phenylcarbonyloxy, Phenylcarbonylmethoxy, Phenylsulfonyloxy, Phenylmethoxy, Phenylmethylthio, Phenylmethylsulfinyl oder Phenylmethylsulfonyl, für jeweils gegebenenfalls durch Cyano, Oxo, Fluor, Chlor, Methyl, Ethyl, Methoxy, Ethoxy, Methylthio, Ethylthio substituiertes Pyrrolyl, Pyrrolinyl, Pyrrolidinyl, Pyrazolyl, Pyrazolinyl, Pyrazolidinyl, Imidazolyl, Imidazolinyl, Imidazolidinyl, Triazolyl, Triazolinyl, Triazolidinyl, Tetrazolyl, Tetrazolinyl, Tetrazolidinyl, Oxazolyl, Oxazolinyl, Oxazolidinyl, Isoxazolyl, Isoxazolinyl, Isoxazolidinyl, Thiazolyl, Thiazolinyl, Thiazolidinyl, Thiadiazolyl, Indolyl, Piperidinyl, Piperazinyl, Oxazinyl, Thiazinyl, Morpholinyl steht;

$R^5$ für Wasserstoff, Cyano, Carbamoyl, Thiocarbamoyl, Fluor, Chlor oder Brom, für jeweils gegebenenfalls durch Cyano, Fluor, Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl,

Ethylsulfinyl, Methylsulfonyl, Ethylsulfonyl, Methoxycarbonyl, Ethoxycarbonyl, n- oder i-Propoxycarbonyl, oder für jeweils gegebenenfalls durch Cyano, Fluor, Chlor, Methyl oder Ethyl substituiertes Cyclopropyl, Cyclobutyl, Cyclopentyl oder Cyclohexyl steht;

$R^6$ für Wasserstoff, für jeweils gegebenenfalls durch Cyano, Fluor, Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Cyano, Fluor, Chlor oder Brom substituiertes Propenyl, Butenyl, Propinyl oder Butinyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Methylthio, Ethylthio, n- oder i-Propylthio, n-, i-, s- oder t-Butylthio, Difluormethylthio, Trifluormethylthio, Methylsulfinyl, Ethylsulfinyl, Trifluormethylsulfinyl, Methylsulfonyl, Ethylsulfonyl oder Trifluormethylsulfonyl substituiertes Phenyl oder Phenylmethyl steht;

$R^7$ für Hydroxy, Formyloxy, für jeweils gegebenenfalls durch Alkyl, Cyano, Fluor, Chlor, Methoxy, Ethoxy, n- oder i-Propoxy substituiertes Methoxy, Ethoxy, n- oder i-Propoxy, Acetyloxy, Propionyloxy, n- oder i-Butyroyloxy, Methoxycarbonyloxy, Ethoxycarbonyloxy, n- oder i-Propoxycarbonyloxy, Methylaminocarbonyloxy, Ethylaminocarbonyloxy, n- oder i-Propylaminocarbonyloxy, Ethoxycarbonylmethoxy, Methoxycarbonylmethoxy, Methylsulfonyloxy, Ethylsulfonyloxy, n- oder i-Propylsulfonyloxy, für jeweils gegebenenfalls durch Cyano, Fluor, Chlor oder Brom substituiertes Propenyloxy, Butenyloxy, Propinyloxy oder Butinyloxy, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n-oder i-Propoxy, Difluormethoxy, Trifluormethoxy, Methylthio, Ethylthio, n- oder i-Propylthio, Difluormethylthio, Trifluormethylthio, Methylsulfinyl, Ethylsulfinyl, Trifluormethylsulfinyl, Methylsulfonyl, Ethylsulfonyl oder Trifluormethylsulfonyl substituiertes Phenylmethoxy, Phenylcarbonyloxy, Phenylcarbonylmethoxy oder Phenylsulfonyloxy steht;

$R^9$ für Wasserstoff, für jeweils gegebenenfalls durch Hydroxy, Amino, Cyano, Carbamoyl, Fluor, Chlor, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, Dimethylaminocarbonyl oder Diethylaminocarbonyl substituiertes Methyl, Ethyl, n- oder i-Propyl, n-, i- oder s-Butyl, n-, i- oder s-Pentyl, für jeweils gegebenenfalls durch Cyano, Fluor, Chlor, Methoxy, Ethoxy, n- oder i-Propoxy substituiertes Acetyl, Propionyl, n- oder i-Butyroyl, Methoxycarbonyl, Ethoxycarbonyl, n- oder i-Propoxycarbonyl, für jeweils gegebenenfalls durch Fluor und/oder Chlor substituiertes Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, für jeweils gegebenenfalls durch Fluor, Chlor und/oder Brom substituiertes Propenyl, Butenyl, Propenylcarbonyl, Butenylcarbonyl, Propenyloxycarbonyl, Butenyloxycarbonyl, Propinyl, Butinyl, Propinylcarbonyl, Butinylcarbonyl, Propinyloxycarbonyl oder Butinyloxycarbonyl, für jeweils gegebenenfalls durch Cyano, Fluor, Chlor, Methyl oder Ethyl substituiertes Cyclopropyl, Cyclobutyl, Cyclopentyl, Cyclohexyl, Cyclopropylcarbonyl, Cyclobutylcarbonyl, Cyclopentylcarbonyl, Cyclohexylcarbonyl, Cyclopropyloxycarbonyl, Cyclobutyloxycarbonyl, Cyclopentyloxycarbonyl, Cyclohexyloxycarbonyl, Cyclopropylmethyl, Cyclobutylmethyl, Cyclopentylmethyl, Cyclohexylmethyl, Cyclopropylmethylcarbonyl, Cyclobutylmethylcarbonyl, Cyclopentylmethylcarbonyl, Cyclohexylmethylcarbonyl, Cyclopropylmethoxycarbonyl, Cyclobutylmethoxycarbonyl, Cyclopentylmethoxycarbonyl, Cyclohexylmethoxycarbonyl, für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, s- oder i-Propoxy, n-, i-, s- oder t-Butoxy, Difluormethoxy oder Trifluormethoxy substituiertes Phenyl, Benzoyl, Phenoxycarbonyl, Benzyl, Phenylethyl, Phenylmethylcarbonyl oder Phenylmethoxycarbonyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Heterocyclyl, Heterocyclylcarbonyl oder Heterocyclylalkyl aus der Reihe Furyl, Furylcarbonyl, Furylmethyl, Thienyl, Thienylcarbonyl, Thienylmethyl, Pyrrolidinyl, Oxopyrrolidinyl, Pyrrolyl, Indolyl, Pyrrolylmethyl, Pyrazolyl, Pyrazolylcarbonyl, Pyrazolylmethyl, Oxazolyl, Oxazolylmethyl, Isoxazolyl, Isoxazolylcarbonyl, Thiazolyl, Thiazolylmethyl, Piperidinyl, Oxopiperidinyl, Morpholinyl, Piperazinyl, Pyridinyl, Pyridinylcarbonyl oder Pyridinylmethyl steht, oder gemeinsam mit $R^2$ und dem Stickstoff an welches sie gebunden sind für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes, Pyrrolidinyl, Oxopyrrolidinyl, Pyrrolyl, Indolyl, Pyrazolyl, Oxazolyl, Isoxazolyl, Dihydropyranyl, Piperidinyl, Thiomorpholinyl, 3-Oxo-morpholinyl, 3-Oxo-thiomorpholinyl, Oxopiperidinyl, Morpholinyl, Piperazinyl, Imidazolyl, Imidazolidinyl, Oxoimidazolidinyl, Triazol, Triazoliniyl, Tetrazolinyl oder Pyridinyl steht;

$R^{10}$ für Wasserstoff, Formyl, für gegebenenfalls durch Cyano, Halogen, $C_1$-$C_4$-Alkoxy, $C_1$-$C_4$-Alkylcarbonyl, $C_1$-$C_4$-Alkoxy-carbonyl, $C_1$-$C_4$-Alkylamino-carbonyl oder Di-($C_1$-$C_4$-alkyl)-amino-carbonyl substituiertes Alkyl

mit 1 bis 6 Kohlenstoffatomen steht;
X für Wasserstoff, Nitro, Cyano, Carboxy, Carbamoyl, Thiocarbamoyl, Fluor, Chlor, Brom, Iod, oder für jeweils gegebenenfalls durch Cyano, Fluor, Chlor, Methoxy, Ethoxy, Methylthio, Ethylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s-oder t-Butylamino, Dimethylamino, Diethylamino, Dimethylaminosulfonyl oder Diethylaminosulfonyl steht;
Y für Wasserstoff, Nitro, Cyano, Carboxy, Carbamoyl, Thiocarbamoyl, Fluor, Chlor, Brom, Iod, oder für jeweils gegebenenfalls durch Cyano, Fluor, Chlor, Methoxy, Ethoxy, Methylthio, Ethylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s-oder t-Butylamino, Dimethylamino, Diethylamino, Dimethylaminosulfonyl oder Diethylaminosulfonyl steht;
m für die Zahlen 0, 1 oder 2 steht.

**3.** Verbindung gemäß Anspruch 1 oder 2, in welcher

$A^2$ für Methylen ($-CH_2-$), Ethan-1,1-diyl ($-CH(CH_3)-$), Ethan-1,2-diyl (Di-methylen, $-CH_2CH_2-$), Propan-1,2-diyl ($-CH(CH_3)CH_2-$) oder Propan-1,3-diyl ($-CH_2CH_2CH_2-$) steht;
$R^1$ für Wasserstoff, für jeweils gegebenenfalls durch Cyano, Fluor, Chlor, Methoxy, Ethoxy, n- oder i-Propoxy, Acetyl, Propionyl, Methoxycarbonyl oder Ethoxycarbonyl substituiertes Methyl, Ethyl, n- oder i-Propyl, für jeweils gegebenenfalls durch Cyano, Fluor, Chlor, Methoxy, Ethoxy, n- oder i-Propoxy substituiertes Acetyl, Propionyl, Methoxycarbonyl oder Ethoxycarbonyl, für jeweils gegebenenfalls durch Fluor und/oder Chlor substituiertes Methylthio, Ethylthio, n- oder i-Propylthio, n-, i-, s- oder t-Butylthio, Methylsulfinyl, Ethylsulfinyl, Methylsulfonyl oder Ethylsulfonyl, für jeweils gegebenenfalls durch Fluor, Chlor und/oder Brom substituiertes Propenyl, Butenyl, Propinyl oder Butinyl, für jeweils gegebenenfalls durch Fluor, Chlor oder Methyl substituiertes Cyclopropyl, Cyclopropylcarbonyl oder Cyclopropylmethyl, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, s- oder i-Propoxy, Difluormethoxy oder Trifluormethoxy substituiertes Phenyl, Benzoyl, Phenoxycarbonyl, Benzyl, Phenylmethylcarbonyl oder Phenylmethoxycarbonyl steht;.
$R^2$ für Wasserstoff, für jeweils gegebenenfalls durch Cyano, Fluor, Chlor, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl, Ethyl, n- oder i-Propyl, n-, i-, s- oder t-Butyl, n-, i-, s- oder t-Pentyl, für jeweils gegebenenfalls durch Cyano, Fluor, Chlor, Methoxy, Ethoxy, n- oder i-Propoxy substituiertes Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, für Dimethylaminocarbonyl, für jeweils gegebenenfalls durch Fluor und/oder Chlor substituiertes Methylthio, Ethylthio, n- oder i-Propylthio, n-, i-, s- oder t-Butylthio, Methylsulfinyl, Ethylsulfinyl, Methylsulfonyl oder Ethylsulfonyl, für jeweils gegebenenfalls durch Fluor, Chlor und/oder Brom substituiertes Propenyl, Butenyl, Propinyl oder Butinyl, für jeweils gegebenenfalls durch Fluor, Chlor oder Methyl substituiertes Cyclopropyl, Cyclopropylcarbonyl oder Cyclopropylmethyl, für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Benzoyl, Phenoxycarbonyl, Phenylaminocarbonyl, Benzyl, Phenylmethylcarbonyl oder Phenylmethoxycarbonyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Heterocyclyl, Heterocyclylcarbonyl oder Heterocyclylalkyl aus der Reihe Furyl, Furylcarbonyl, Furylmethyl, Thienyl, Thienylcarbonyl, Thienylmethyl, Pyrrolidinyl, Pyrrolyl, Indolyl, Pyrrolylmethyl, Pyrazolyl, Pyrazolylmethyl, Isoxazolyl, Piperidinyl, Morpholinyl, Thiomorpholinyl, 3-Oxo-morpholinyl, 3-Oxothiomorpholinyl, Piperazinyl, Pyridinyl, Pyridinylmethyl steht;
$R^3$ für Wasserstoff, für jeweils gegebenenfalls durch Fluor und/oder Chlor substituiertes Methyl, Ethyl, n- oder i-Propyl, Methylthio, Ethylthio, n- oder i-Propylthio, oder für Phenyl, oder - für den Fall, dass m für 2 steht - gegebenenfalls auch zusammen mit einem zweiten Rest $R^3$ für Sauerstoff, Propan-1,3-diyl oder Butan-1,4-diyl steht;
$R^4$ für Hydroxy, für Formyloxy, für jeweils gegebenenfalls durch Fluor und/oder Chlor substituiertes Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Acetyloxy, Propionyloxy, n- oder i-Butyroyloxy, Methoxycarbonyloxy, Ethoxycarbonyloxy, n- oder

i-Propoxycarbonyloxy, Methylaminocarbonyloxy, Ethylaminocarbonyloxy, n- oder i-Propylaminocarbonyloxy, Methylsulfonyloxy, Ethylsulfonyloxy, n- oder i-Propylsulfonyloxy, für jeweils gegebenenfalls durch Fluor und/oder Chlor substituiertes Propenyloxy, Butenyloxy, Propinyloxy oder Butinyloxy, für jeweils gegebenenfalls durch Nitro, Cyano, Fluor, Chlor, Brom, Methyl, Ethyl, n- oder i-Propyl, Trifluormethyl, Methoxy, Ethoxy, n- oder i-Propoxy, Difluormethoxy oder Trifluormethoxy substituiertes Phenyloxy, Phenylthio, Phenylsulfinyl, Phenylsulfonyl, Phenylcarbonyloxy, Phenylcarbonylmethoxy, Phenylsulfonyloxy, Phenylmethoxy, Phenylmethylthio, Phenylmethylsulfinyl oder Phenylmethylsulfonyl, für jeweils gegebenenfalls durch Cyano, Oxo, Fluor, Chlor, Methyl, Ethyl, Methoxy, Ethoxy, Methylthio, Ethylthio substituiertes Pyrrolyl, Pyrrolinyl, Pyrrolidinyl, Pyrazolyl, Pyrazolinyl, Pyrazolidinyl, Imidazolyl, Imidazolinyl, Imidazolidinyl, Triazolyl, Triazolinyl, Triazolidinyl, Tetrazolyl, Tetrazolinyl, Tetrazolidinyl, Oxazolyl, Oxazolinyl, Oxazolidinyl, Isoxazolyl, Isoxazolinyl, Isoxazolidinyl, Thiazolyl, Thiazolinyl, Thiazolidinyl, Thiadiazolyl, Indolyl, Piperidinyl, Piperazinyl, Oxazinyl, Thiazinyl, Morpholinyl steht;

$R^5$ für Wasserstoff, Cyano, Fluor, Chlor, für jeweils gegebenenfalls durch Fluor und/oder Chlor substituiertes Methyl, Ethyl, n- oder i-Propyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Methoxycarbonyl, Ethoxycarbonyl, n- oder i-Propoxycarbonyl, oder für gegebenenfalls durch Cyano, Fluor, Chlor oder Methyl substituiertes Cyclopropyl steht;

$R^6$ für Wasserstoff, für jeweils gegebenenfalls durch Cyano, Fluor, Chlor, Methoxy oder Ethoxy substituiertes Methyl, Ethyl, n- oder i-Propyl, n-, i-, s- oder t-Butyl, für jeweils gegebenenfalls durch Fluor oder Chlor substituiertes Propenyl, Butenyl, Propinyl oder Butinyl, für jeweils gegebenenfalls durch Fluor, Chlor oder Methyl substituiertes Cyclopropyl, Cyclobutyl, Cyclopentyl oder Cyclohexyl, oder für jeweils gegebenenfalls durch Fluor, Chlor, Brom, Methyl, Ethyl, n- oder i-Propyl, Trifluormethyl, Methoxy, Ethoxy, n- oder i-Propoxy, Difluormethoxy oder Trifluormethoxy substituiertes Phenyl oder Phenylmethyl steht;

$R^7$ für Hydroxy, für Formyloxy, für jeweils gegebenenfalls durch Alkyl, Cyano, Fluor, Chlor, Methoxy, Ethoxy, n- oder i-Propoxy substituiertes Methoxy, Ethoxy, n- oder i-Propoxy, Acetyloxy, Propionyloxy, n- oder i-Butyroyloxy, Methoxycarbonyloxy, Ethoxycarbonyloxy, n- oder i-Propoxycarbonyloxy, Methylaminocarbonyloxy, Ethylaminocarbonyloxy, n- oder i-Propylaminocarbonyloxy, Ethoxycarbonylmethoxy, Methoxycarbonylmethoxy, Methylsulfonyloxy, Ethylsulfonyloxy, n- oder i-Propylsulfonyloxy, für jeweils gegebenenfalls durch Fluor und/oder Chlor substituiertes Propenyloxy, Butenyloxy, Propinyloxy oder Butinyloxy, oder für jeweils gegebenenfalls durch Nitro, Cyano, Fluor, Chlor, Brom, Methyl, Ethyl, n- oder i-Propyl, Trifluormethyl, Methoxy, Ethoxy, n- oder i-Propoxy, Difluormethoxy oder Trifluormethoxy substituiertes Phenylmethoxy, Phenylcarbonyloxy, Phenylcarbonylmethoxy oder Phenylsulfonyloxy steht;

$R^9$ für Wasserstoff, für jeweils gegebenenfalls durch Cyano, Fluor, Chlor, Methoxy, Ethoxy, n- oder i-Propoxy, Acetyl, Propionyl, Methoxycarbonyl oder Ethoxycarbonyl substituiertes Methyl, Ethyl, n- oder i-Propyl, für jeweils gegebenenfalls durch Cyano, Fluor, Chlor, Methoxy, Ethoxy, n- oder i-Propoxy substituiertes Acetyl, Propionyl, Methoxycarbonyl oder Ethoxycarbonyl, für jeweils gegebenenfalls durch Fluor und/oder Chlor substituiertes Methylthio, Ethylthio, n- oder i-Propylthio, n-, i-, s- oder t-Butylthio, Methylsulfinyl, Ethylsulfinyl, Methylsulfonyl oder Ethylsulfonyl, für jeweils gegebenenfalls durch Fluor, Chlor und/oder Brom substituiertes Propenyl, Butenyl, Propinyl oder Butinyl, für jeweils gegebenenfalls durch Fluor, Chlor oder Methyl substituiertes Cyclopropyl, Cyclopropylcarbonyl oder Cyclopropylmethyl, oder für jeweils gegebenenfalls durch Cyano, Nitro, Fluor, Chlor, Brom, Methyl, Ethyl, n- oder i-Propyl, n-, i-, s- oder t-Butyl, Trifluormethyl, Methoxy, Ethoxy, s- oder i-Propoxy, Difluormethoxy oder Trifluormethoxy substituiertes Phenyl, Benzoyl, Phenoxycarbonyl, Benzyl, Phenylmethylcarbonyl oder Phenylmethoxycarbonyl oder gemeinsam mit $R^2$ und dem Stickstoff an welches sie gebunden sind für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes, Pyrrolidinyl, Oxopyrrolidinyl, Pyrrolyl, Indolyl, Pyrazolyl, Oxazolyl, Isoxazolyl, Dihydropyranyl, Piperidinyl, Oxopiperidinyl, Morpholinyl, Thiomorpholinyl, 3-Oxo-morpholinyl, 3-Oxothiomorpholinyl, Piperazinyl, Imidazolyl, Imidazolidinyl, Oxoimidazolidinyl, Triazol, Triazolinyl, Tetrazolinyl oder Pyridinyl steht;

$R^{10}$ für Wasserstoff, Formyl, für jeweils gegebenenfalls durch Cyano, Fluor, Chlor, Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl, Ethyl, n- oder i-Propyl, n-, i-, s- oder t-Butyl, n-, i-, s- oder t-Pentyl, für jeweils gegebenenfalls durch Cyano, Fluor, Chlor, Methoxy, Ethoxy, n- oder i-Propoxy substituiertes Acetyl, Propionyl, n- oder i-Butyroyl, Methoxycarbonyl, Ethoxycarbonyl, n- oder i-Propoxycarbonyl, Methylaminocarbonyl, Ethylaminocarbonyl, n- oder i-Propylaminocarbonyl, für Dimethylaminocarbonyl, für jeweils gegebenenfalls durch Fluor und/oder Chlor substituiertes Methylthio, Ethylthio, n- oder i-Propylthio, n-, i-, s- oder t-Butylthio, Methylsulfinyl, Ethylsulfinyl, Methylsulfonyl oder Ethylsulfonyl, für jeweils gegebenenfalls durch Fluor, Chlor und/oder Brom substituiertes Propenyl, Butenyl, Propinyl oder Butinyl, für jeweils gegebenenfalls durch Fluor, Chlor oder Methyl substituiertes Cyclopropyl, Cyclopropylcarbonyl oder Cyclopropylmethyl, für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Benzoyl, Phenoxycarbonyl, Phenylaminocarbonyl, Benzyl, Phenylmethylcarbonyl oder Phenylmethoxycarbonyl, oder für jeweils gegebenenfalls durch Nitro, Cyano, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Heterocyclyl, Heterocyclylcarbonyl oder Heterocyclylalkyl aus der Reihe Furyl, Furylcarbonyl, Furylmethyl, Thienyl, Thienylcarbonyl, Thienylmethyl, Pyrrolidinyl, Pyrrolyl, Indolyl, Pyrrolylmethyl, Pyrazolyl, Pyrazolylmethyl, Isoxazolyl, Piperidinyl, Morpholinyl, Piperazinyl, Pyridinyl, Pyridinylmethyl steht;
X für Wasserstoff, Nitro, Cyano, Fluor, Chlor, Brom, Iod, Methyl, Ethyl, Difluormethyl, Trifluormethyl, Dichlormethyl, Trichlormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl steht;
Y für Wasserstoff, Nitro, Cyano, Fluor, Chlor, Brom, Iod, Methyl, Ethyl, Difluormethyl, Trifluormethyl, Dichlormethyl, Trichlormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl steht und
m für die Zahlen 0 oder 2 steht.

**4.** Verfahren zur Herstellung von Verbindungen der Formel (I-2) gemäß Anspruch 1 bis 3, erhältlich durch die Umsetzung von

a) Carbonsäuren der Formel (II)

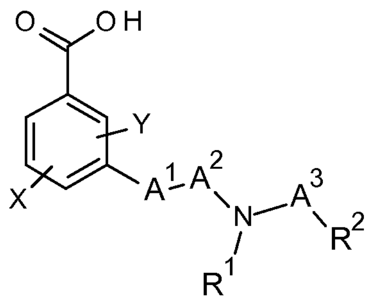

(II)

in welcher

$A^1$, $A^2$, $A^3$, $R^1$, $R^2$, X und Y die oben angegebene Bedeutung haben
- oder deren Alkali-, Erdalkali- oder Ammoniumsalze -
mit Verbindungen der allgemeinen Formel (III)

H-Z (III)

in welcher
Z die oben angegebene Bedeutung hat, oder

b) Carbonsäurederivaten der Formel (IX)

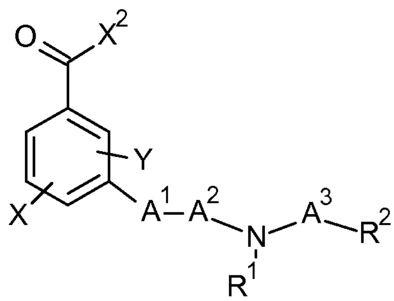

(IX)

in welcher

$A^1$, $A^2$, $A^3$, $R^1$, $R^2$, X und Y die oben angegebene Bedeutung haben und
$X^2$ für CN oder Halogen, bevorzugt Cl, Br, Imidazolyl oder Triazolyl steht

mit Verbindungen der Formel (III)

H-Z (III)

in welcher

Z die oben angegebene Bedeutung hat, oder

c) Verbindungen der Formel (XIII)

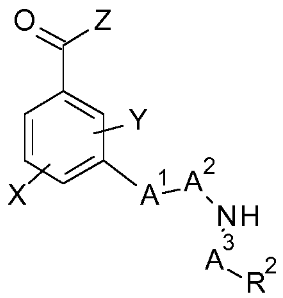

(XIII)

in welcher

$A^1$, $A^2$, $A^3$, $R^2$, X, Y und Z die oben angegebene Bedeutung haben
mit Verbindungen der Formel (XI)

$X^1$-$R^1$ (XI)

in welcher

$R^1$ die oben angegebene Bedeutung hat und
$X^1$ für Halogen, Arylsulfonat oder Alkylsulfonat, bevorzugt Chlor, Brom, Jod, Mesylat oder Tosylat steht, oder

d) Verbindungen der Formel (XIV)

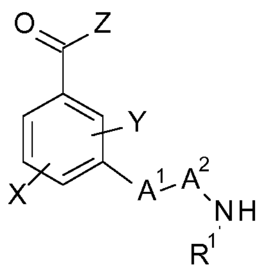

(XIV)

in welcher

$A^1$, $A^2$, $R^1$, X, Y und Z die oben angegebene Bedeutung haben
mit Verbindungen der Formel (V)

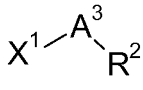

(V)

in welcher

$A^3$ und $R^2$ die oben angegebene Bedeutung haben und
$X^1$ für Halogen oder Tosylat, bevorzugt Chlor, Brom oder Tosylat steht, oder

e) Verbindungen der Formel (XV)

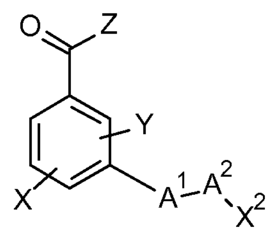

(XV)

in welcher

$A^1$, $A^2$, X, Y und Z die oben angegebene Bedeutung haben und
$X^2$ für Halogen oder Tosylat, bevorzugt Chlor, Brom oder Tosylat steht

mit Verbindungen der Formel (VII)

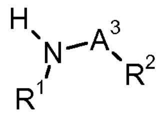

(VII)

in welcher

$A^3$, $R^1$ und $R^2$ die oben angegbene Bedeutung haben,
gegebenenfalls in Gegenwart eines oder mehrerer Reaktionshilfsmittel und gegebenenfalls in Gegenwart eines oder mehrerer Verdünnungsmittel, umsetzt.

**5.** Verbindung der Formel (IX)

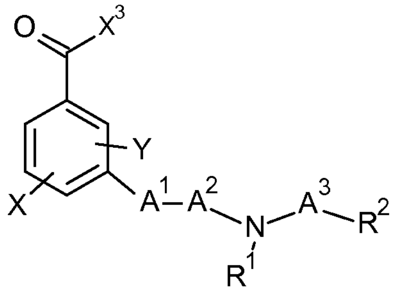

(IX)

in welcher

$A^1$, $A^2$, $A^3$, $R^1$, $R^2$, X und Y eine der in den Ansprüchen 1 bis 4 angegebenen Bedeutungen haben und
$X^3$ für Halogen, Cyano, Imidazolyl oder Triazolyl steht.

**6.** Verfahren zur Herstellung von Verbindungen der Formel (IX) erhältlich durch Umsetzung von Carbonsäuren der Formel (II)

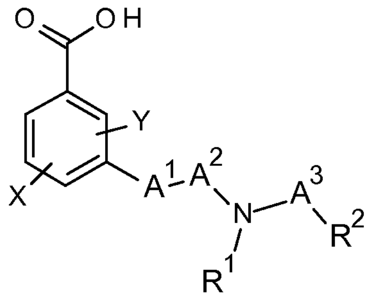

(II)

in welcher
$A^1$, $A^2$, $A^3$, $R^1$, $R^2$, X und Y die oben angegebene Bedeutung haben mit geeigneten Aktivierungsreagenzien, gegebenenfalls in Gegenwart eines oder mehrerer Reaktionshilfsmittel und gegebenenfalls in Gegenwart eines oder mehrerer Verdünnungsmittel.

**7.** Verbindung der Formel (II)

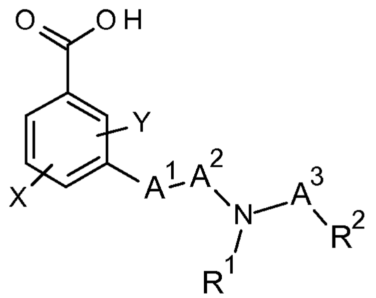

(II)

in welcher
X, Y, $A^1$, $A^2$, $A^3$, $R^1$ und $R^2$ eine der in den Ansprüchen 1 bis 4 angegebenen Bedeutungen haben.

**8.** Verbindung gemäß Anspruch 7, in welcher

$A^3$ für O, S, die Gruppierung

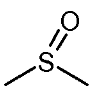
,
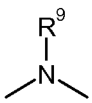
,
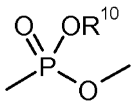
oder
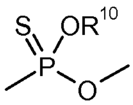

steht.

**9.** Verfahren zur Herstellung von Verbindungen der Formel (II) gemäß Anspruch 7 oder 8 erhältlich durch Umsetzung von Verbindungen (VIII)

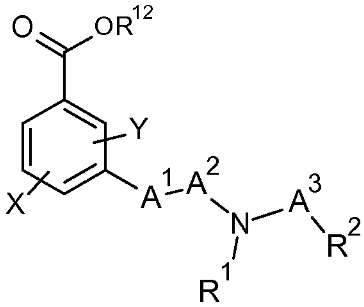

(VIII)

in welcher

$A^1$, $A^2$, $A^3$, $R^1$, $R^2$, X und Y eine der in den Ansprüchen 1 bis 4 angegebene Bedeutung haben und
$R^{12}$ für $C_1$-$C_4$-Alkyl, insbesondere Methyl, Ethyl, n-, i-Propyl, n-, s-, i-, t-Butyl, für Allyl oder Benzyl steht

unter reduktiven oder alkalischen Bedingungen in Gegenwart eines oder mehrerer Reaktionshilfsmittel und gegebenenfalls in Gegenwart eines oder mehrerer Verdünnungsmittel.

**10.** Verbindung der Formel (VIII)

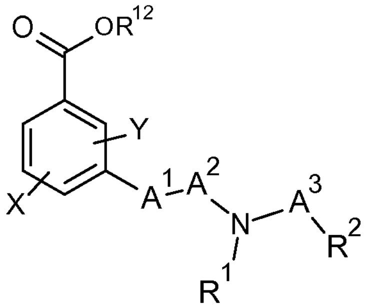

(VIII)

in welcher

$A^1$, $A^2$, $A^3$, $R^1$, $R^2$, X und Y eine der in den Ansprüchen 1 bis 4 angegebene Bedeutung haben und
$R^{12}$ für $C_1$-$C_4$-Alkyl, Allyl oder Benzyl steht.

**11.** Verfahren zur Herstellung von Verbindungen der Formel (VIII) gemäß Anspruch 10, erhältlich durch Umsetzung von

i) Verbindungen der Formel (IV)

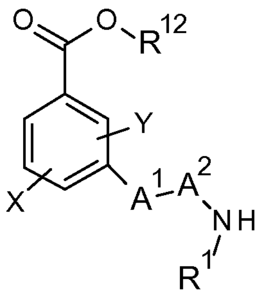

(IV)

in welcher

$A^1$, $A^2$, $R^1$, X und Y die oben angegebenen Bedeutungen haben und
$R^{12}$ für $C_1$-$C_4$ Alkyl, insbesondere Methyl, Ethyl, n-, i-Propyl, n-, s-, i-, t-Butyl, für Allyl oder Benzyl steht,

mit Verbindungen der Formel (V)

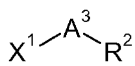

(V)

in welcher

$A^3$ und $R^2$ die oben angegebenen Bedeutungen haben und
$X^1$ für Halogen (vorzugsweise Fluor, Chlor, Brom oder Iod, insbesondere Chlor, Brom oder Iod) steht,

gegebenenfalls in Gegenwart eines oder mehrerer Reaktionshilfsmittel, und gegebenenfalls in Gegenwart eines oder mehrerer Verdünnungsmittel umsetzt,
oder

j) Verbindungen der Formel (X)

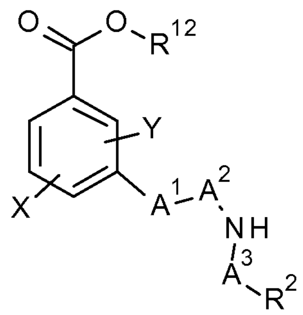

(X)

in welcher

$A^1$, $A^2$, $A^3$, $R^2$, X und Y die in den Ansprüchen 1 bis 4 angegebenen Bedeutungen haben und
$R^{12}$ für $C_1$-$C_4$-Alkyl, insbesondere Methyl, Ethyl, n-, i-Propyl, n-, s-, i-, t-Butyl, für Allyl oder Benzyl steht,

mit Verbindungen der Formel (XI)

$X^1$-$R^1$ (XI)

in welcher

$R^1$ die oben angegebene Bedeutung hat und
$X^1$ für Halogen (vorzugsweise Fluor, Chlor, Brom oder Iod, insbesondere Chlor, Brom oder Iod) steht,

gegebenenfalls in Gegenwart eines oder mehrerer Reaktionshilfsmittel, und gegebenenfalls in Gegenwart eines oder mehrerer Verdünnungsmittel umsetzt,
oder
k) Verbindungen der Formel (X)

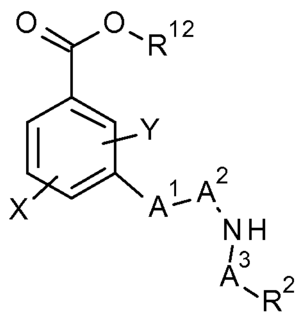

(X)

in welcher

$A^1$, $A^2$, $A^3$, $R^2$, X und Y die oben angegebenen Bedeutungen haben und
$R^{12}$ für $C_1$-$C_4$-Alkyl, insbesondere Methyl, Ethyl, n-, i-Propyl, n-, s-, i, t-Butyl, steht

mit Verbindungen der Formel (XII)

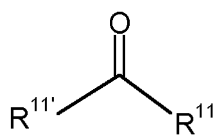

(XII)

in welcher

$R^{11}$ und $R^{11}$ unabhängig voneinander für Wasserstoff, für gegebenenfalls durch Cyano, Halogen, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes Alkyl mit 1 bis 6 Kohlenstoffatomen, für jeweils gegebenenfalls durch Cyano oder Halogen substituiertes Alkenyl oder Alkinyl mit jeweils 3 bis 6 Kohlenstoffatomen, für jeweils gegebenenfalls durch Cyano, Halogen oder $C_1$-$C_4$-Alkyl substituiertes Cycloalkyl oder Cycloalkylalkyl mit jeweils 3 bis 6 Kohlenstoffatomen in der Cycloalkylgruppe und gegebenenfalls 1 bis 4 Kohlenstoffatomen im Alkylteil, oder für jeweils gegebenenfalls durch Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl substituiertes Aryl oder Arylalkyl mit jeweils 6 oder 10 Kohlenstoffatomen in der Arylgruppe und gegebenenfalls 1 bis 4 Kohlenstoffatomen im Alkylteil, stehen,

in Gegenwart eines Reduktionsmittels, vorzugsweise eines Borans oder eines $BH_3$-Adduktes,
gegebenenfalls in Gegenwart eines oder mehrerer Reaktionshilfsmittel und gegebenenfalls in Gegenwart eines oder mehrerer Verdünnungsmittel umsetzt.

**12.** Schädlingsbekämpfungsmittel, **gekennzeichnet durch** einen Gehalt an mindestens einer Verbindung der Formel (I-2) gemäss Anspruch 1 bis 3 oder einer Verbindung der Formel (II) gemäss Anspruch 7 oder 8 oder einer Verbindung der Formel (VIII) gemäss Anspruch 10.

**13.** Verwendung von Verbindungen der Formel (I-2) gemäss Anspruch 1 bis 3, Verbindungen der Formel (II) gemäss Anspruch 7 oder 8 und/oder Verbindungen der Formel (VIII) gemäss Anspruch 10 zur Bekämpfung von Schädlingen.

**14.** Verfahren zur Bekämpfung von Schädlingen, **dadurch gekennzeichnet, dass** man Verbindungen der Formel (I-2) gemäss Anspruch 1 bis 3, Verbindungen der Formel (II) gemäss Anspruch 7 oder 8 und/oder Verbindungen der Formel (VIII) gemäss Anspruch 10 auf Schädlinge und/oder ihren Lebensraum einwirken lässt.

**15.** Verfahren zur Herstellung von Schädlingsbekämpfungsmitteln, **dadurch gekennzeichnet, dass** man Verbindungen der Formel (I-2) gemäss Anspruch 1 bis 3, Verbindungen der Formel (II) gemäss Anspruch Anspruch 7 oder 8 und/oder Verbindungen der Formel (VIII) gemäss Anspruch 10 mit Streckmitteln und/oder oberflächenaktiven Mitteln vermischt.

**16.** Verwendung von Verbindungen der Formel (I-2) gemäss Anspruch 1 bis 3, Verbindungen der Formel (II) gemäss Anspruch 7 oder 8 und/oder Verbindungen der Formel (VIII) gemäss Anspruch 10 zur Herstellung von Schädlingsbekämpfungsmitteln.

## Claims

**1.** Compound of the formula (1-2)

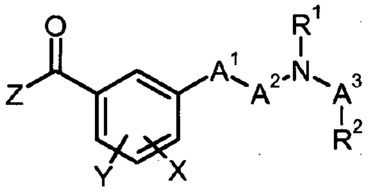

(I-2)

in which

$A^1$ represents O (oxygen) where
$A^3$ represents O (oxygen), S (sulphur), or the grouping

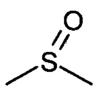 , 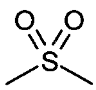 , 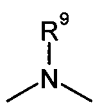 , 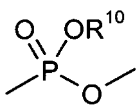 or 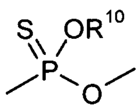

where

Z represents one of the groupings below

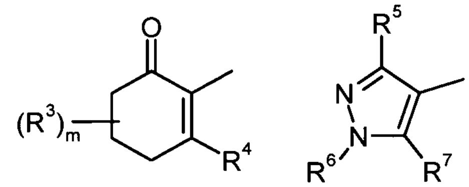

where

$A^2$ represents alkanediyl having 1 to 6 carbon atoms, alkenediyl or alkynediyl having in each case 2 to 6 carbon atoms;

$R^1$ represents hydrogen, represents optionally hydroxyl-, amino-, cyano-, carbamoyl-, halogen-, $C_1$-$C_4$-alkoxy-, $C_1$-$C_4$-alkyl-carbonyl-, $C_1$-$C_4$-alkoxycarbonyl-, $C_1$-$C_4$-alkylaminocarbonyl-, di($C_1$-$C_4$-alkyl) amino-, di ($C_1$-$C_4$-alkyl) amino-carbonylor N-($C_1$-$C_4$-alkoxy)-N-($C_1$-$C_4$-alkyl)aminocarbonyl-substituted alkyl having 1 to 6 carbon atoms, represents in each case optionally halogen-substituted alkylthio, alkylsulphinyl or alkylsulphonyl having in each case 1 to 6 carbon atoms, represents in each case optionally halogen-substituted alkenyl or alkynyl having in each case 2 to 6 carbon atoms, represents in each case optionally cyano-, halogen- or $C_1$-$C_4$-alkyl- substituted cycloalkyl, cycloalkylalkyl having in each case 3 to 6 carbon atoms in the cycloalkyl groups and, if appropriate, 1 to 4 carbon atoms in the alkyl moiety, represents in each case optionally cyano-, nitro-, halogen-, $C_1$-$C_4$-alkyl-, $C_1$-$C_4$-haloalkyl-, $C_1$-$C_4$-alkoxy- or $C_1$-$C_4$-haloalkoxy-substituted aryl, arylalkyl or arylcarbonylalkyl having in each case 6 or 10 carbon atoms in the aryl groups and, if appropriate, 1 to 4 carbon atoms in the alkyl moiety, or represents in each case optionally nitro-, cyano-, halogen-, $C_1$-$C_4$-alkyl-, $C_1$-$C_4$-haloalkyl-, $C_1$-$C_4$-alkoxy- or $C_1$-$C_4$-haloalkoxy-substituted heterocyclyl or heterocyclylalkyl where in each case the heterocyclyl grouping contains up to 10 carbon atoms and additionally at least one heteroatom selected from the group consisting of N (nitrogen, but at most 5 N atoms), O (oxygen, but at most 2 0 atoms), S (sulphur, but at most 2 S atoms), SO and $SO_2$ and optionally additionally one group selected from the group consisting of oxo (C=O), thioxo (C=S), imino (C=NH), cyanoimino (C=N-CN), nitroimino (C=N-$NO_2$);

$R^2$ represents hydrogen, represents optionally cyano-, halogen-, $C_1$-$C_4$-alkoxy-, $C_1$-$C_4$-alkyl-carbonyl-, $C_1$-$C_4$-alkoxycarbonyl-, $C_1$-$C_4$-alkyl-aminocarbonyl- or di($C_1$-$C_4$-alkyl)aminocarbonyl-substituted alkyl having 1 to 6 carbon atoms, represents in each case optionally cyano-, halogen- or $C_1$-$C_4$-alkoxy-substituted alkylcarbonyl, alkoxycarbonyl or alkylaminocarbonyl having in each case 1 to 6 carbon atoms in the alkyl groups, represents dialkylaminocarbonyl having in each case 1 to 4 carbon atoms in the alkyl groups, represents in each case optionally halogen-substituted alkenyl, alkenylcarbonyl, alkenyloxycarbonyl, alkynyl, alkynylcarbonyl or alkynyloxycarbonyl having in each case 3 to 6 carbon atoms in the alkenyl or alkynyl groups, represents in each case optionally cyano-, halogen- or $C_1$-$C_4$-alkyl-substituted cycloalkyl, cycloalkylcarbonyl, cycloalkyloxycarbonyl, cycloalkylalkyl, cycloalkylalkylcarbonyl or cycloalkylalkoxycarbonyl having in each case 3 to 6 carbon atoms in the cycloalkyl groups and, if appropriate, 1 to 4 carbon atoms in the alkyl moiety, represents in each case optionally nitro-, cyano-, halogen-, $C_1$-$C_4$-alkyl-, $C_1$-$C_4$-haloalkyl-, $C_1$-$C_4$-alkoxy- or $C_1$-$C_4$-haloalkoxy-substituted aryl, arylcarbonyl, aryloxycarbonyl, arylaminocarbonyl, arylalkyl, arylalkylcarbonyl, arylalkoxycarbonyl or arylalkylaminocarbonyl having in each case 6 or 10 carbon atoms in the aryl groups and, if appropriate, 1 to 4 carbon atoms in the alkyl moiety, or represents in each case optionally nitro-, cyano-, halogen-, $C_1$-$C_4$-alkyl-, $C_1$-$C_4$-haloalkyl-, $C_1$-$C_4$-alkoxy- or $C_1$-$C_4$-haloalkoxy-substituted heterocyclyl, heterocyclylcarbonyl or heterocyclylalkyl, where the heterocyclyl group contains in each case up to 10 carbon atoms and additionally at least one heteroatom selected from the group consisting of N (nitrogen, but at most 5 N atoms), O (oxygen, but at most 2 O atoms), S (sulphur, but at most 2 S atoms), SO and $SO_2$, and optionally additionally one group selected from the group consisting of oxo (C=O), thioxo (C=S), imino (C=NH), cyanoimino (C=N-CN), nitroimino (C=N-$NO_2$);

$R^3$ represents hydrogen, halogen, represents in each case optionally cyano-, halogen-, $C_1$-$C_4$-alkoxy-, $C_1$-$C_4$-alkylthio-, $C_1$-$C_4$-alkylsulphinyl or $C_1$-$C_4$-alkylsulphonyl-substituted alkyl or alkylthio having in each case 1 to 6 carbon atoms, or represents phenyl, or - if m represents 2 - optionally also together with a second radical $R^3$ represents oxygen or alkanediyl (alkylene) having 3 to 5 carbon atoms;

$R^4$ represents hydroxyl, formyloxy, halogen, represents in each case optionally cyano-, halogen-, $C_1$-$C_4$-alkoxy-, $C_1$-$C_4$-alkylthio-, $C_1$-$C_4$-alkylsulphinyl- or $C_1$-$C_4$-alkylsulphonyl-substituted alkoxy, alkylthio, alkylsulphinyl, alkylsulphonyl, alkylcarbonyloxy, alkoxycarbonyloxy, alkylaminocarbonyloxy or alkylsulphonyloxy having in each case 1 to 6 carbon atoms, represents in each case optionally halogen-substituted alkenyloxy or alkynyloxy having in each case 3 to 6 carbon atoms, represents in each case optionally nitro-, cyano-, halogen-, $C_1$-$C_4$-alkyl-, $C_1$-$C_4$-haloalkyl-, $C_1$-$C_4$-alkoxy-, $C_1$-$C_4$-haloalkoxy-, $C_1$-$C_4$-alkylthio-, $C_1$-$C_4$-haloalkylthio-, $C_1$-$C_4$-alkylsulphinyl-, $C_1$-$C_4$-haloalkylsulphinyl-, $C_1$-$C_4$-alkylsulphonyl- or $C_1$-$C_4$-haloalkylsulphonyl-substituted aryloxy, arylthio, arylsulphinyl, arylsulphonyl, aryl-carbonyloxy, arylcarbonylalkoxy, arylsulphonyloxy, arylalkoxy, arylalkylthio, arylalkylsulphinyl or arylalkylsulphonyl having in each case 6 or 10 carbon atoms in the aryl group and, if appropriate, 1 to 4 carbon atoms in the alkyl moiety, represents in each case optionally cyano-, halogen-, oxo-, hydroxyl-, $C_1$-$C_4$-alkoxy-, $C_1$-$C_4$-alkyl-substituted heterocyclyl having 5 or 6 ring atoms comprising at least 1 nitrogen atom and optionally up to 2 oxygen atoms, sulphur atoms and 3 nitrogen atoms, where in total not more than 4 heteroatoms are present and where the heterocycle is attached via the nitrogen;

$R^5$ represents hydrogen, cyano, carbamoyl, thiocarbamoyl, halogen, represents in each case optionally cyano-, halogen-, $C_1$-$C_4$-alkoxy-, $C_1$-$C_4$-alkylthio-, $C_1$-$C_4$-alkylsulphinyl- or $C_1$-$C_4$-alkylsulphonyl-substituted alkyl, alkoxy, alkylthio, alkylsulphinyl, alkylsulphonyl or alkoxycarbonyl having in each case 1 to 6 carbon atoms in the alkyl groups, or represents optionally cyano-, halogen- or $C_1$-$C_4$-alkyl-substituted cycloalkyl having 3 to 6 carbon atoms;

$R^6$ represents hydrogen, represents-optionally cyano-, halogen-, $C_1$-$C_4$-alkoxy-, $C_1$-$C_4$-alkylthio-, $C_1$-$C_4$-alkylsulphinyl- or $C_1$-$C_4$-alkylsulphonyl-substituted alkyl having 1 to 6 carbon atoms, represents in each case optionally cyano- or halogen-substituted alkenyl or alkynyl having in each case 3 to 6 carbon atoms, represents in each case optionally cyano-, halogen- or $C_1$-$C_4$-alkyl-substituted cycloalkyl or cycloalkylalkyl having in each case 3 to 6 carbon atoms in the cycloalkyl group and, if appropriate, 1 to 4 carbon atoms in the alkyl moiety, or represents in each case optionally nitro-, cyano-, halogen-, $C_1$-$C_4$-alkyl-, $C_1$-$C_4$-haloalkyl-, $C_1$-$C_4$-alkoxy-, $C_1$-$C_4$-haloalkoxy-, $C_1$-$C_4$-alkylthio-, $C_1$-$C_4$-haloalkylthio-, $C_1$-$C_4$-alkylsulphinyl-, $C_1$-$C_4$-haloalkylsulphinyl-, $C_1$-$C_4$-alkylsulphonyl- or $C_1$-$C_4$-haloalkyl-sulphonyl-substituted aryl or arylalkyl having in each case 6 or 10 carbon atoms in the aryl group and, if appropriate, 1 to 4 carbon atoms in the alkyl moiety;

$R^7$ represents hydroxyl, formyloxy, represents in each case optionally alkyl-, cyano-, halogen-or $C_1$-$C_4$-alkoxy-substituted alkoxy, alkyl-carbonyloxy, alkoxycarbonyloxy, alkoxycarbonylalkoxy, alkylaminocarbonyloxy or alkylsulphonyloxy having in each case 1 to 6 carbon atoms in the alkyl groups, represents in each case optionally cyano- or halogen-substituted alkenyloxy or alkynyloxy having in each case 3 to 6 carbon atoms, or represents in each case optionally nitro-, cyano-, halogen-, $C_1$-$C_4$-alkyl-, $C_1$-$C_4$-haloalkyl-, $C_1$-$C_4$-alkoxy-, $C_1$-$C_4$-haloalkoxy-, $C_1$-$C_4$-alkylthio-, $C_1$-$C_4$-haloalkylthio-, $C_1$-$C_4$-alkylsulphinyl-, $C_1$-$C_4$-haloalkylsulphinyl-, $C_1$-$C_4$-alkylsulphonyl- or $C_1$-$C_4$-haloalkyl-sulphonyl-substituted arylalkoxy, arylcarbonyloxy, arylcarbonylalkoxy or arylsulphonyloxy having in each case 6 or 10 carbon atoms in the aryl group and, if appropriate, 1 to 4 carbon atoms in the alkyl moiety;

$R^9$ represents hydrogen, represents optionally hydroxyl-, amino-, cyano-, carbamoyl-, halogen-, $C_1$-$C_9$-alkoxy-, $C_1$-$C_4$-alkyl-carbonyl-, $C_1$-$C_4$-alkoxycarbonyl-, $C_1$-$C_4$-alkylaminocarbonyl or di($C_1$-$C_4$-alkyl)aminocarbonyl-substituted alkyl having 1 to 6 carbon atoms, represents in each case optionally cyano-, halogen- or $C_1$-$C_4$-alkoxy-substituted alkylcarbonyl or alkoxycarbonyl having in each case 1 to 6 carbon atoms in the alkyl groups, represents in each case optionally halogen-substituted alkylthio, alkylsulphinyl or alkylsulphonyl having in each case 1 to 6 carbon atoms, represents in each case optionally halogen-substituted alkenyl, alkenylcarbonyl, alkenyloxycarbonyl, alkynyl, alkynylcarbonyl or alkynyloxycarbonyl having in each case 2 to 6 carbon atoms in the alkenyl or alkynyl groups, represents in each case optionally cyano-, halogen- or $C_1$-$C_4$-alkyl-substituted cycloalkyl, cycloalkylcarbonyl, cycloalkyloxycarbonyl, cycloalkylalkyl, cycloalkylalkylcarbonyl or cycloalkylalkoxycarbonyl having in each case 3 to 6 carbon atoms in the cycloalkyl groups and, if appropriate, 1 to 4 carbon atoms in the alkyl moiety, represents in each case optionally cyano-, nitro-, halogen-, $C_1$-$C_4$-alkyl-, $C_1$-$C_4$-haloalkyl-, $C_1$-$C_4$-alkoxy- or $C_1$-$C_4$-haloalkoxy-substituted aryl, arylcarbonyl, aryloxycarbonyl, arylalkyl, arylalkylcarbonyl or arylalkoxycarbonyl having in each case 6 or 10 carbon atoms in the aryl groups and, if appropriate, 1 to 4 carbon atoms in the alkyl moiety, or represents in each case optionally nitro-, cyano-, halogen-, $C_1$-$C_4$-alkyl-, $C_1$-$C_4$-haloalkyl-, $C_1$-$C_4$-alkoxy- or $C_1$-$C_4$-haloalkoxy-substituted heterocyclyl, heterocyclylcarbonyl or heterocyclylalkyl, where in each case the heterocyclyl grouping contains up to 10 carbon atoms and additionally at least one heteroatom selected from the group consisting of N (but at most 5 N atoms), 0 (but at most 2 O atoms), S (but at most 2 S atoms), SO and $SO_2$, and optionally additionally one group selected from the group consisting of oxo (C=O), thioxo (C=S), imino (C=NH), cyanoimino (C=N-CN), nitroimino (C=N-$NO_2$), or together with $R^2$ and the nitrogen to which they are attached represents an optionally nitro-, cyano-, halogen-, $C_1$-$C_4$-alkyl-, $C_1$-$C_4$-haloalkyl-, $C_1$-$C_4$-alkoxy- or $C_1$-$C_4$-haloalkoxy-substituted heterocycle which contains 1 nitrogen atom and 1 to 10 carbon atoms and optionally one further heteroatom from the group consisting of N (but at most 4 further N atoms), 0 (but at most 2 0 atoms), S (but at most 2 S atoms), SO and $SO_2$, and optionally additionally one group selected from the group consisting of oxo (C=O), thioxo (C=S), imino (C=NH), cyanoimino

(C=N-CN), nitroimino (C=N-$NO_2$);

$R^{10}$ represents hydrogen, formyl, represents optionally cyano-, halogen-, $C_1$-$C_4$-alkoxy-, $C_1$-$C_4$-alkyl-carbonyl-, $C_1$-$C_4$-alkoxycarbonyl-, $C_1$-$C_4$-alkylaminocarbonyl- or di-($C_1$-$C_4$-alkyl)aminocarbonyl-substituted alkyl having 1 to 6 carbon atoms;

X represents hydrogen, nitro, cyano, carboxyl, carbamoyl, thiocarbamoyl, halogen, or represents in each case optionally cyano-, halogen-, $C_1$-$C_4$-alkoxy-, $C_1$-$C_4$-alkylthio-, $C_1$-$C_4$-alkylsulphinyl- or $C_1$-$C_4$-alkylsulphonyl-substituted alkyl, alkoxy, alkylthio, alkyl-sulphinyl, alkylsulphonyl, alkylamino, dialkylamino or dialkylaminosulphonyl having in each case 1 to 6 carbon atoms in the alkyl groups;

Y represents hydrogen, nitro, cyano, carboxyl, carbamoyl, thiocarbamoyl, halogen, or represents in each case optionally cyano-, halogen-, $C_1$-$C_4$-alkoxy-, $C_1$-$C_4$-alkylthio-, $C_1$-$C_4$-alkylsulphinyl- or $C_1$-$C_4$-alkylsulphonyl-substituted alkyl, alkoxy, alkylthio, alkyl-sulphinyl, alkylsulphonyl, alkylamino, dialkylamino or dialkylaminosulphonyl having in each case 1 to 6 carbon atoms in the alkyl groups;

m represents the numbers 0, 1, 2 or 3.

**2.** Compound according to Claim 1 in which

$A^2$ represents methylene (-$CH_2$-), ethane-1,1-diyl-(-CH($CH_3$)-), ethane-1,2-diyl-(dimethylene, -$CH_2CH_2$-), propane-1,1-diyl-(-CH($C_2H_5$)-), propane-1,2-diyl-(-CH($CH_3$)$CH_2$-), propane-1,3-diyl-(-$CH_2CH_2CH_2$-), butane-1,3-diyl-(-CH($CH_3$)$CH_2CH_2$-), butane-1,4-diyl-(-$CH_2CH_2CH_2CH_2$-), ethenediyl, propenediyl, butenediyl, ethynediyl, propynediyl or butynediyl;

$R^1$ represents hydrogen, represents in each case optionally hydroxyl-, amino-, cyano-, carbamoyl-, fluorine-, chlorine-, methoxy-, ethoxy-, n- or i-propoxy-, acetyl-, propionyl-, n- or i-butyroyl-, methoxycarbonyl-, ethoxycarbonyl-, n- or i-propoxycarbonyl-, methylaminocarbonyl-, ethylaminocarbonyl-, n-or i-propylaminocarbonyl-, dimethylamino-, diethylamino-, dimethylaminocarbonyl-, diethylaminocarbonyl- or N-methoxy-N-methylaminocarbonyl-substituted methyl, ethyl, n- or i-propyl, n-, i- or s-butyl, n-, i- or s-pentyl, represents in each case optionally fluorine- and/or chlorine-substituted methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, represents in each case optionally fluorine-, chlorine- and/or bromine-substituted propenyl, butenyl, propynyl or butynyl, represents in each case optionally cyano-, fluorine-, chlorine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, represents in each case optionally cyano-, nitro-, fluorine-, chlorine-, bromine-, methyl-, ethyl-, n- or i-propyl-, n-, i-, s-or t-butyl-, trifluoromethyl-, methoxy-, ethoxy-, s- or i-propoxy-, n-, i-, s- or t-butoxy-, difluoromethoxy- or trifluoromethoxy-substituted phenyl, benzoyl, benzyl, phenylethyl or phenylcarbonylmethyl, or represents in each case optionally nitro-, cyano-, fluorine-, chlorine-, bromine-, methyl-, ethyl-, n- or i-propyl-, n-, i-, s-or t-butyl-, trifluoromethyl-, methoxy-, ethoxy-, n- or i-propoxy-, difluoromethoxy-or trifluoromethoxy-substituted heterocyclyl or heterocyclylalkyl from the group consisting of furyl, furylmethyl, thienyl, thienylmethyl, pyrrolidinyl, oxopyrrolidinyl, pyrrolyl, indolyl, pyrrolylmethyl, pyrazolyl, pyrazolylmethyl, oxazolyl, oxazolylmethyl, isoxazolyl, thiazolyl, thiazolylmethyl, dihydropyranyl, dihydropyranylmethyl, piperidinyl, oxopiperidinyl, morpholinyl, piperazinyl, pyridinyl, pyridinylcarbonyl or pyridinylmethyl;

$R^2$ represents hydrogen, represents in each case optionally cyano-, fluorine-, chlorine-, methoxy-, ethoxy-, n- or i-propoxy-, acetyl-, propionyl-, n- or i-butyroyl-, methoxycarbonyl-, ethoxycarbonyl-, n- or i-propoxycarbonyl-, methylaminocarbonyl-, ethylaminocarbonyl-, n-or i-propylaminocarbonyl-, dimethylaminocarbonyl- or diethylamino-carbonyl-substituted methyl, ethyl, n- or i-propyl, n-, i-, s- or t-butyl, n-, i-, s- or t-pentyl, represents in each case optionally cyano-, fluorine-, chlorine-, methoxy-, ethoxy-, n-or i-propoxy-substituted acetyl, propionyl, n- or i-butyroyl, methoxycarbonyl, ethoxycarbonyl, n- or i-propoxycarbonyl, methylaminocarbonyl, ethylaminocarbonyl, n-or i-propylaminocarbonyl, represents dimethylaminocarbonyl, diethylaminocarbonyl or dipropylaminocarbonyl, represents in each case optionally fluorine-, chlorine- and/or bromine-substituted propenyl, butenyl, propenylcarbonyl, butenylcarbonyl, propenyloxycarbonyl, butenyloxycarbonyl, propenylaminocarbonyl, butenylaminocarbonyl, propynyl, butynyl, propynylcarbonyl, butynylcarbonyl, propynyloxycarbonyl or butynyloxycarbonyl, represents in each case optionally cyano-, fluorine-, chlorine-, methyl- or ethyl-substituted cyclopropyl, cyclobutyl, cyclopentyl, cyclohexyl, cyclopropylcarbonyl, cyclobutylcarbonyl, cyclopentylcarbonyl, cyclohexylcarbonyl, cyclopropyloxycarbonyl, cyclobutyloxycarbonyl, cyclopentyloxycarbonyl, cyclohexyloxycarbonyl, cyclopropylmethyl, cyclobutylmethyl, cyclopentylmethyl, cyclohexylmethyl, cyclopropylmethylcarbonyl, cyclobutylmethylcarbonyl, cyclopentylmethyl-carbonyl, cyclohexylmethylcarbonyl, cyclopropylmethoxycarbonyl, cyclobutylmethoxycarbonyl, cyclopentylmethoxycarbonyl, cyclohexyl-methoxycarbonyl, represents in each case optionially nitro-, cyano-, fluorine-, chlorine-, bromine-, methyl-, ethyl-, n- or i-propyl-, n-, i-, s-or t-butyl-, trifluoromethyl-, methoxy-, ethoxy-, n- or i-propoxy-, difluoromethoxy-or trifluoromethoxy-substituted phenyl, naphthyl, benzoyl, phenoxycarbonyl, phenylaminocarbonyl, benzyl, phenylethyl, phenylmethylcarbonyl or phenylmethoxycarbonyl, or represents in each case optionally nitro-, cyano-, fluorine-, chlorine-, bromine-, methyl-, ethyl-, n- or i-propyl-, n-, i-, s-or t-butyl-, trifluoromethyl-, methoxy-, ethoxy-, n- or i-propoxy-, difluoromethoxy- or trifluoromethoxy-substituted furyl, furylcarbonyl, furylmethyl, thienyl, thienylcarbonyl, thienylmethyl, pyrrolidinyl, oxopyrrolidinyl, pyrrolyl, indolyl, pyrrolylmethyl, pyrazolyl, pyrazolylcarbonyl, pyrazolylmethyl, oxazolyl, oxazolylmethyl, isoxazolyl, isoxazolylcarbonyl, thiazolyl, thiazolylmethyl, 2-oxo-1,3-diazacyclopentyl (2-oxoimidazolidinyl), piperidinyl, oxopiperidinyl, 2-oxo-1,3-diazacyclohexyl, morpholinyl, thiomorpholinyl, 3-oxomorpholinyl, 3-oxothiomorpholinyl, piperazinyl, pyridinyl, pyridinylcarbonyl or pyridinylmethyl;

$R^3$ represents hydrogen, fluorine, chlorine or bromine, represents in each case optionally cyano-, fluorine-, chlorine-, methoxy-, ethoxy-, n- or i-propoxy-, methylthio-, ethylthio-, n- or i-propylthio-, methyl-sulphinyl-, ethylsulphinyl-, methylsulphonyl-or ethylsulphonyl-substituted methyl, ethyl, n- or i-propyl, n-, i-, s- or t-butyl, methylthio, ethylthio, n- or i-propylthio, n-, i-, s- or t-butylthio, or represents phenyl, or - if m represents 2 - optionally also together with a second radical $R^3$ represents oxygen, propane-1,3-diyl or butane-1,4-diyl;

$R^4$ represents hydroxyl, formyloxy, fluorine or chlorine, represents in each case optionally cyano-, fluorine-, chlorine-, methoxy-, ethoxy-, n- or i-propoxy-, methylthio-, ethylthio-, n- or i-propylthio-, methyl-sulphinyl-, ethylsulphinyl-, methylsulphonyl-or ethylsulphonyl-substituted methoxy, ethoxy, n- or i-propoxy, methylthio, ethylthio, n- or i-propylthio, methylsulphinyl, ethylsulphinyl, methylsulphonyl, ethylsulphonyl, acetyloxy, propionyloxy, n- or i-butyroyloxy, methoxycarbonyloxy, ethoxycarbonyloxy, n- or i-propoxycarbonyloxy, methylaminocarbonyloxy, ethylaminocarbonyloxy, n- or i-propyl-aminocarbonyloxy, methylsulphonyloxy, ethylsulphonyloxy, n- or i-propylsulphonyloxy, represents in each case optionally fluorine-, chlorine- and/or bromine-substituted propenyloxy, butenyloxy, propynyloxy or butynyloxy, represents in each case optionally nitro-, cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy-, trifluoromethoxy-, methylthio-, ethylthio-, n- or i-propylthio-, difluoromethylthio-, trifluoromethylthio-, methylsulphinyl-, ethylsulphinyl-, trifluoromethylsulphinyl-, methylsulphonyl-, ethylsulphonyl- or trifluoromethylsulphonyl-substituted phenyloxy, phenylthio, phenylsulphinyl, phenylsulphonyl, phenylcarbonyloxy, phenylcarbonylmethoxy, phenylsulphonyloxy, phenylmethoxy, phenylmethylthio, phenyl-methylsulphinyl or phenylmethylsulphonyl, represents in each case optionally cyano-, oxo-, fluorine-, chlorine-, methyl-, ethyl-, methoxy-, ethoxy-, methylthio-, ethylthio-substituted pyrrolyl, pyrrolinyl, pyrrolidinyl, pyrazolyl, pyrazolinyl, pyrazolidinyl, imidazolyl, imidazolinyl, imidazolidinyl, triazolyl, triazolinyl, triazolidinyl, tetrazolyl, tetrazolinyl, tetrazolidinyl, oxazolyl, oxazolinyl, oxazolidinyl, isoxazolyl, isoxazolinyl, isoxazolidinyl, thiazolyl, thiazolinyl, thiazolidinyl, thiadiazolyl, indolyl, piperidinyl, piperazinyl, oxazinyl, thiazinyl, morpholinyl;

$R^5$ represents hydrogen, cyano, carbamoyl, thiocarbamoyl, fluorine, chlorine or bromine, represents in each case optionally cyano-, fluorine-, chlorine-, methoxy-, ethoxy-, n-or i-propoxy-, methylthio-, ethylthio-, n- or i-propylthio-, methylsulphinyl-, ethylsulphinyl-, methylsulphonyl- or ethylsulphonyl-substituted methyl, ethyl, n-or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl, ethylsulphonyl, methoxycarbonyl, ethoxycarbonyl, n- or i-propoxycarbonyl, or represents in each case optionally cyano-, fluorine-, chlorine-, methyl- or ethyl-substituted cyclopropyl, cyclobutyl, cyclopentyl or cyclohexyl;

$R^6$ represents hydrogen, represents in each case optionally cyano-, fluorine-, chlorine-, methoxy-, ethoxy-, n- or i-propoxy-, methylthio-, ethylthio-, n- or i-propylthio-, methylsulphinyl-, ethylsulphinyl-, methylsulphonyl- or ethylsulphonyl-substituted methyl, ethyl, n- or i-propyl, n-, i-, s- or t-butyl, represents in each case optionally cyano-, fluorine-, chlorine-or bromine-substituted propenyl, butenyl, propynyl or butynyl, represents in each case optionally cyano-, fluorine-, chlorine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl, or represents in each case optionally nitro-, cyano-, fluorine-, chlorine-, bromine-, methyl-, ethyl-, n- or i-propyl-, n-, i-, s-or t-butyl-, trifluoromethyl-, methoxy-, ethoxy-, n- or i-propoxy-, difluoromethoxy-, trifluoromethoxy-, methylthio-, ethylthio-, n- or i-propylthio-, n-, i-, s- or t-butylthio-, difluoromethylthio-, trifluoromethylthio-, methylsulphinyl-, ethylsulphinyl-, trifluoromethylsulphinyl-, methylsulphonyl-, ethylsulphonyl- or trifluoromethylsulphonyl-substituted phenyl or phenylmethyl;

$R^7$ represents hydroxyl, formyloxy, represents in each case optionally alkyl-, cyano-, fluorine-, chlorine-, methoxy-, ethoxy-, n-or i-propoxy-substituted methoxy, ethoxy, n-or i-propoxy, acetyloxy, propionyloxy, n- or i-butyroyloxy, methoxycarbonyloxy, ethoxycarbonyloxy, n- or i-propoxycarbonyloxy, methylaminocarbonyloxy, ethylaminocarbonyloxy, n- or i-propylaminocarbonyloxy, ethoxycarbonyl-methoxy, methoxycarbonylmethoxy, methylsulphonyloxy, ethylsulphonyloxy, n- or i-propylsulphonyloxy, represents in each case optionally cyano-, fluorine-, chlorine- or bromine-substituted propenyloxy, butenyloxy, propynyloxy or butynyloxy, or represents in each case optionally nitro-, cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy-, trifluoromethoxy-, methylthio-, ethylthio-, n- or i-propylthio-, difluoromethylthio-, trifluoromethylthio-, methylsulphinyl-, ethylsulphinyl-, trifluoromethylsulphinyl-, methylsulphonyl-, ethylsulphonyl- or trifluoromethylsulphonyl-substituted phenylmethoxy,

phenylcarbonyloxy, phenylcarbonylmethoxy or phenylsulphonyloxy;

$R^9$ represents hydrogen, represents in each case optionally hydroxyl-, amino-, cyano-, carbamoyl-, fluorine-, chlorine-, methoxy-, ethoxy-, n- or i-propoxy-, acetyl-, propionyl-, n- or i-butyroyl-, methoxycarbonyl-, ethoxycarbonyl-, n- or i-propoxycarbonyl-, methylaminocarbonyl-, ethylaminocarbonyl-, n- or i-propylaminocarbonyl-, dimethylaminocarbonyl-or diethylaminocarbonyl-substituted methyl, ethyl, n- or i-propyl, n-, i- or s-butyl, n-, i- or s-pentyl, represents in each case optionally cyano-, fluorine-, chlorine-, methoxy-, ethoxy-, n-or i-propoxy-substituted acetyl, propionyl, n- or i-butyroyl, methoxycarbonyl, ethoxycarbonyl, n- or i-propoxycarbonyl, represents in each case optionally fluorine-and/or chlorine-substituted methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, represents in each case optionally fluorine-, chlorine- and/or bromine-substituted propenyl, butenyl, propenylcarbonyl, butenylcarbonyl, propenyloxycarbonyl, butenyloxycarbonyl, propynyl, butynyl, propynylcarbonyl, butynylcarbonyl, propynyloxycarbonyl or butynyloxycarbonyl, represents in each case optionally cyano-, fluorine-, chlorine-, methyl- or ethyl-substituted cyclopropyl, cyclobutyl, cyclopentyl, cyclohexyl, cyclopropylcarbonyl, cyclobutylcarbonyl, cyclopentylcarbonyl, cyclohexylcarbonyl, cyclopropyloxycarbonyl, cyclobutyloxycarbonyl, cyclopentyloxycarbonyl, cyclohexyloxycarbonyl, cyclopropylmethyl, cyclobutylmethyl, cyclopentylmethyl, cyclohexylmethyl, cyclopropylmethylcarbonyl, cyclobutylmethylcarbonyl, cyclopentylmethylcarbonyl, cyclohexylmethylcarbonyl, cyclopropylmethoxycarbonyl, cyclobutylmethoxycarbonyl, cyclopentylmethoxycarbonyl, cyclohexyl-methoxycarbonyl, represents in each case optionally cyano-, nitro-, fluorine-, chlorine-, bromine-, methyl-, ethyl-, n- or i-propyl-, n-, i-, s-or t-butyl-, trifluoromethyl-, methoxy-, ethoxy-, s- or i-propoxy-, n-, i-, s- or t-butoxy-, difluoromethoxy- or trifluoromethoxy-substituted phenyl, benzoyl, phenoxycarbonyl, benzyl, phenylethyl, phenylmethylcarbonyl or phenylmethoxycarbonyl, or represents in each case optionally nitro-, cyano-, fluorine-, chlorine-, bromine-, methyl-, ethyl-, n- or i-propyl-, n-, i-, s-or t-butyl-, trifluoromethyl-, methoxy-, ethoxy-, n- or i-propoxy-, difluoromethoxy-or trifluoromethoxy-substituted heterocyclyl, heterocyclylcarbonyl and heterocyclylalkyl from the group consisting of furyl, furylcarbonyl, furylmethyl, thienyl, thienylcarbonyl, thienylmethyl, pyrrolidinyl, oxopyrrolidinyl, pyrrolyl, indolyl, pyrrolylmethyl, pyrazolyl, pyrazolylcarbonyl, pyrazolylmethyl, oxazolyl, oxazolylmethyl, isoxazolyl, isoxazolylcarbonyl, thiazolyl, thiazolylmethyl, piperidinyl, oxopiperidinyl, morpholinyl, piperazinyl, pyridinyl, pyridinylcarbonyl or pyridinylmethyl, or together with $R^2$ and the nitrogen to which they are attached represents in each case optionally nitro-, cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy- or trifluoromethoxy-substituted pyrrolidinyl, oxopyrrolidinyl, pyrrolyl, indolyl, pyrazolyl, oxazolyl, isoxazolyl, dihydropyranyl, piperidinyl, thiomorpholinyl, 3-oxomorpholinyl, 3-oxothiomorpholinyl, oxopiperidinyl, morpholinyl, piperazinyl, imidazolyl, imidazolidinyl, oxo-imidazolidinyl, triazol, triazolinyl, tetrazolinyl or pyridinyl;

$R^{10}$ represents hydrogen, formyl, represents optionally cyano-, halogen-, $C_1$-$C_4$-alkoxy-, $C_1$-$C_4$-alkyl-carbonyl-, $C_1$-$C_4$-alkoxycarbonyl-, $C_1$-$C_4$-alkylaminocarbonyl- or di($C_1$-$C_4$-alkyl)aminocarbonyl-substituted alkyl having 1 to 6 carbon atoms;

X represents hydrogen, nitro, cyano, carboxyl, carbamoyl, thiocarbamoyl, fluorine, chlorine, bromine, iodine, or represents in each case optionally cyano-, fluorine-, chlorine-, methoxy-, ethoxy-, methylthio-, ethylthio-, methylsulphinyl-, ethylsulphinyl-, methyl-sulphonyl- or ethylsulphonyl-substituted methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, methylamino, ethylamino, n- or i-propylamino, n-, i-, s- or t-butylamino, dimethylamino, diethylamino, dimethylaminosulphonyl or diethylaminosulphonyl;

Y represents hydrogen, nitro, cyano, carboxyl, carbamoyl, thiocarbamoyl, fluorine, chlorine, bromine, iodine, or represents in each case optionally cyano-, fluorine-, chlorine-, methoxy-, ethoxy-, methylthio-, ethylthio-, methylsulphinyl-, ethylsulphinyl-, methyl-sulphonyl- or ethylsulphonyl-substituted methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, methylamino, ethylamino, n- or i-propylamino, n-, i-, s- or t-butylamino, dimethylamino, diethylamino, dimethylaminosulphonyl or diethylaminosulphonyl;

m represents the numbers 0, 1 or 2.

**3.** Compound according to Claim 1 or 2 in which

$A^2$ represents methylene ($-CH_2-$), ethane-1,1-diyl ($-CH(CH_3)-$), ethane-1,2-diyl (dimethylene, $-CH_2CH_2-$), propane-1,2-diyl ($-CH(CH_3)CH_2-$) or propane-1,3-diyl ($-CH_2CH_2CH_2-$) ;

$R^1$ represents hydrogen, represents in each case optionally cyano-, fluorine-, chlorine-, methoxy-, ethoxy-, n- or i-propoxy-, acetyl-, propionyl-, methoxy-carbonyl- or ethoxycarbonyl-substituted methyl, ethyl, n- or i-propyl,

represents in each case optionally cyano-, fluorine-, chlorine-, methoxy-, ethoxy-, n-or i-propoxy-substituted acetyl, propionyl, methoxycarbonyl or ethoxycarbonyl, represents in each case optionally fluorine- and/or chlorine-substituted methylthio, ethylthio, n- or i-propylthio, n-, i-, s- or t-butylthio, methylsulphinyl, ethylsulphinyl, methylsulphonyl or ethylsulphonyl, represents in each case optionally fluorine-, chlorine- and/or bromine-substituted propenyl, butenyl, propynyl or butynyl, represents in each case optionally fluorine-, chlorine- or methyl-substituted cyclopropyl, cyclopropylcarbonyl or cyclopropylmethyl, or represents in each case optionally cyano-, nitro-, fluorine-, chlorine-, bromine-, methyl-, ethyl-, n- or i-propyl-, n-, i-, s-or t-butyl-, trifluoromethyl-, methoxy-, ethoxy-, s- or i-propoxy-, difluoromethoxy-or trifluoromethoxy-substituted phenyl, benzoyl, phenoxycarbonyl, benzyl, phenylmethylcarbonyl or phenylmethoxycarbonyl;

$R^2$ represents hydrogen, represents in each case optionally cyano-, fluorine-, chlorine-, methoxy-, ethoxy-, n- or i-propoxy-, acetyl-, propionyl-, n- or i-butyroyl-, methoxycarbonyl-, ethoxycarbonyl-, n- or i-propoxycarbonyl-substituted methyl, ethyl, n- or i-propyl, n-, i-, s- or t-butyl, n-, i-, s- or t-pentyl, represents in each case optionally cyano-, fluorine-, chlorine-, methoxy-, ethoxy-, n-or i-propoxy-substituted acetyl, propionyl, n- or i-butyroyl, methoxycarbonyl, ethoxycarbonyl, n- or i-propoxycarbonyl, methylaminocarbonyl, ethylaminocarbonyl, n-or i-propylaminocarbonyl, represents dimethylaminocarbonyl, represents in each case optionally fluorine- and/or chlorine-substituted methylthio, ethylthio, n- or i-propylthio, n-, i-, s- or t-butylthio, methylsulphinyl, ethylsulphinyl, methylsulphonyl or ethylsulphonyl, represents in each case optionally fluorine-, chlorine- and/or bromine-substituted propenyl, butenyl, propynyl or butynyl, represents in each case optionally fluorine-, chlorine- or methyl-substituted cyclopropyl, cyclopropylcarbonyl or cyclopropylmethyl, represents in each case optionally nitro-, cyano-, fluorine-, chlorine-, bromine-, methyl-, ethyl-, n- or i-propyl-, n-, i-, s-or t-butyl-, trifluoromethyl-, methoxy-, ethoxy-, n- or i-propoxy-, difluoromethoxy-or trifluoromethoxy-substituted phenyl, benzoyl, phenoxycarbonyl, phenylaminocarbonyl, benzyl, phenylmethylcarbonyl or phenyl-methoxycarbonyl, or represents in each case optionally nitro-, cyano-, fluorine-, chlorine-, bromine-, methyl-, ethyl-, n- or i-propyl-, n-, i-, s-or t-butyl-, trifluoromethyl-, methoxy-, ethoxy-, n- or i-propoxy-, difluoromethoxy-or trifluoromethoxy-substituted heterocyclyl, heterocyclylcarbonyl or heterocyclylalkyl from the group consisting of furyl, furyl-carbonyl, furylmethyl, thienyl, thienylcarbonyl, thienylmethyl, pyrrolidinyl, pyrrolyl, indolyl, pyrrolylmethyl, pyrazolyl, pyrazolylmethyl, isoxazolyl, piperidinyl, morpholinyl, thiomorpholinyl, 3-oxo-morpholinyl, 3-oxothiomorpholinyl, piperazinyl, pyridinyl, pyridinylmethyl;

$R^3$ represents hydrogen, represents in each case optionally fluorine- and/or chlorine-substituted methyl, ethyl, n- or i-propyl, methylthio, ethylthio, n- or i-propylthio, or represents phenyl, or - if m represents 2 - optionally also together with a second radical $R^3$ represents oxygen, propane-1,3-diyl or butane-1,4-diyl;

$R^4$ represents hydroxyl, represents formyloxy, represents in each case optionally fluorine- and/or chlorine-substituted methoxy, ethoxy, n- or i-propoxy, methylthio, ethylthio, n- or i-propylthio, methylsulphinyl, ethylsulphinyl, methylsulphonyl, ethylsulphonyl, acetyloxy, propionyloxy, n-or i-butyroyloxy, methoxycarbonyloxy, ethoxycarbonyloxy, n- or i-propoxycarbonyloxy, methylaminocarbonyloxy, ethylaminocarbonyloxy, n- or i-propylaminocarbonyloxy, methylsulphonyloxy, ethylsulphonyloxy, n- or i-propylsulphonyloxy, represents in each case optionally fluorine- and/or chlorine-substituted propenyloxy, butenyloxy, propynyloxy or butynyloxy, represents in each case optionally nitro-, cyano-, fluorine-, chlorine-, bromine-, methyl-, ethyl-, n- or i-propyl-, trifluoromethyl-, methoxy-, ethoxy-, n- or i-propoxy-, difluoromethoxy-or trifluoromethoxy-substituted phenyloxy, phenylthio, phenylsulphinyl, phenylsulphonyl, phenylcarbonyloxy, phenylcarbonylmethoxy, phenylsulphonyloxy, phenylmethoxy, phenyl-methylthio, phenylmethylsulphinyl or phenylmethylsulphonyl, represents in each case optionally cyano-, oxo-, fluorine-, chlorine-, methyl-, ethyl-, methoxy-, ethoxy-, methylthio-, ethylthio-substituted pyrrolyl, pyrrolinyl, pyrrolidinyl, pyrazolyl, pyrazolinyl, pyrazolidinyl, imidazolyl, imidazolinyl, imidazolidinyl, triazolyl, triazolinyl, triazolidinyl, tetrazolyl, tetrazolinyl, tetrazolidinyl, oxazolyl, oxazolinyl, oxazolidinyl, isoxazolyl, isoxazolinyl, isoxazolidinyl, thiazolyl, thiazolinyl, thiazolidinyl, thiadiazolyl, indolyl, piperidinyl, piperazinyl, oxazinyl, thiazinyl, morpholinyl;

$R^5$ represents hydrogen, cyano, fluorine, chlorine, represents in each case optionally fluorine- and/or chlorine-substituted methyl, ethyl, n- or i-propyl, methoxy, ethoxy, n- or i-propoxy, methylthio, ethylthio, n- or i-propylthio, methylsulphinyl, ethylsulphinyl, methylsulphonyl, ethylsulphonyl, methoxycarbonyl, ethoxycarbonyl, n- or i-propoxycarbonyl, or represents optionally cyano-, fluorine-, chlorine- or methyl-substituted cyclopropyl;

$R^6$ represents hydrogen, represents in each case optionally cyano-, fluorine-, chlorine-, methoxy- or ethoxy-substituted methyl, ethyl, n- or i-propyl, n-, i-, s- or t-butyl, represents in each case optionally fluorine-or chlorine-substituted propenyl, butenyl, propynyl or butynyl, represents in each case optionally fluorine-, chlorine- or methyl-substituted cyclopropyl, cyclobutyl, cyclopentyl or cyclohexyl, or represents in each case optionally fluorine-, chlorine-, bromine-, methyl-, ethyl-, n- or i-propyl-, trifluoromethyl-, methoxy-, ethoxy-, n- or i-propoxy-, difluoromethoxy- or trifluoromethoxy-substituted phenyl or phenylmethyl;

$R^7$ represents hydroxyl, represents formyloxy, represents in each case optionally alkyl-, cyano-, fluorine-, chlorine-, methoxy-, ethoxy-, n- or i-propoxy-substituted methoxy, ethoxy, n- or i-propoxy, acetyloxy, propionyloxy,

n- or i-butyroyloxy, methoxycarbonyloxy, ethoxycarbonyloxy, n- or i-propoxycarbonyloxy, methylaminocarbonyloxy, ethylaminocarbonyloxy, n- or i-propylaminocarbonyloxy, ethoxycarbonylmethoxy, methoxycarbonylmethoxy, methylsulphonyloxy, ethylsulphonyloxy, n- or i-propylsulphonyloxy, represents in each case optionally fluorine- and/or chlorine-substituted propenyloxy, butenyloxy, propynyloxy or butynyloxy, or represents in each case optionally nitro-, cyano-, fluorine-, chlorine-, bromine-, methyl-, ethyl-, n- or i-propyl-, trifluoromethyl-, methoxy-, ethoxy-, n- or i-propoxy-, difluoromethoxy- or trifluoromethoxy-substituted phenylmethoxy, phenylcarbonyloxy, phenylcarbonylmethoxy or phenylsulphonyloxy;

$R^9$ represents hydrogen, represents in each case optionally cyano-, fluorine-, chlorine-, methoxy-, ethoxy-, n- or i-propoxy-, acetyl-, propionyl-, methoxycarbonyl- or ethoxycarbonyl-substituted methyl, ethyl, n-or i-propyl, represents in each case optionally cyano-, fluorine-, chlorine-, methoxy-, ethoxy-, n-or i-propoxy-substituted acetyl, propionyl, methoxycarbonyl or ethoxycarbonyl, represents in each case optionally fluorine- and/or chlorine-substituted methylthio, ethylthio, n- or i-propylthio, n-, i-, s- or t-butylthio, methylsulphinyl, ethylsulphinyl, methylsulphonyl or ethylsulphonyl, represents in each case optionally fluorine-, chlorine- and/or bromine-substituted propenyl, butenyl, propynyl or butynyl, represents in each case optionally fluorine-, chlorine- or methyl-substituted cyclopropyl, cyclopropylcarbonyl or cyclopropylmethyl, or represents in each case optionally cyano-, nitro-, fluorine-, chlorine-, bromine-, methyl-, ethyl-, n- or i-propyl-, n-, i-, s-or t-butyl-, trifluoromethyl-, methoxy-, ethoxy-, s- or i-propoxy-, difluoromethoxy-or trifluoromethoxy-substituted phenyl, benzoyl, phenoxycarbonyl, benzyl, phenylmethylcarbonyl or phenylmethoxycarbonyl or together with $R^2$ and the nitrogen to which they are attached represents in each case optionally nitro-, cyano-, fluorine-, chlorine-, bromine-, methyl-, ethyl-, n- or i-propyl-, n-, i-, s- or t-butyl-, trifluoromethyl-, methoxy-, ethoxy-, n- or i-propoxy-, difluoromethoxy- or trifluoromethoxy-substituted pyrrolidinyl, oxopyrrolidinyl, pyrrolyl, indolyl, pyrazolyl, oxazolyl, isoxazolyl, dihydropyranyl, piperidinyl, oxopiperidinyl, morpholinyl, thiomorpholinyl, 3-oxomorpholinyl, 3-oxo-thiomorpholinyl, piperazinyl, imidazolyl, imidazolidinyl, oxo-imidazolidinyl, triazol, triazolinyl, tetrazolinyl or pyridinyl;

$R^{10}$ represents hydrogen, formyl, represents in each case optionally cyano-, fluorine-, chlorine-, methoxy-, ethoxy-, n-or i-propoxy-, acetyl-, propionyl-, n- or i-butyroyl-, methoxycarbonyl-, ethoxycarbonyl-, n- or i-propoxycarbonyl-substituted methyl, ethyl, n- or i-propyl, n-, i-, s- or t-butyl, n-, i-, s- or t-pentyl, represents in each case optionally cyano-, fluorine-, chlorine-, methoxy-, ethoxy-, n-or i-propoxy-substituted acetyl, propionyl, n- or i-butyroyl, methoxycarbonyl, ethoxycarbonyl, n- or i-propoxycarbonyl, methylaminocarbonyl, ethylaminocarbonyl, n-or i-propylaminocarbonyl, represents dimethylaminocarbonyl, represents in each case optionally fluorine-and/or chlorine-substituted methylthio, ethylthio, n- or i-propylthio, n-, i-, s- or t-butylthio, methylsulphinyl, ethylsulphinyl, methylsulphonyl or ethylsulphonyl, represents in each case optionally fluorine-, chlorine- and/or bromine-substituted propenyl, butenyl, propynyl or butynyl, represents in each case optionally fluorine-, chlorine- or methyl-substituted cyclopropyl, cyclopropylcarbonyl or cyclopropylmethyl, represents in each case optionally nitro-, cyano-, fluorine-, chlorine-, bromine-, methyl-, ethyl-, n- or i-propyl-, n-, i-, s-or t-butyl-, trifluoromethyl-, methoxy-, ethoxy-, n- or i-propoxy-, difluoromethoxy-or trifluoromethoxy-substituted phenyl, benzoyl, phenoxycarbonyl, phenylaminocarbonyl, benzyl, phenylmethylcarbonyl or phenyl-methoxycarbonyl, or represents in each case optionally nitro-, cyano-, fluorine-, chlorine-, bromine-, methyl-, ethyl, n- or i-propyl-, n-, i-, s-or t-butyl-, trifluoromethyl, methoxy-, ethoxy-, n- or i-propoxy-, difluoromethoxy-or trifluoromethoxy-substituted heterocyclyl, heterocyclylcarbonyl or heterocyclylalkyl from the group consisting of furyl, furylcarbonyl, furylmethyl, thienyl, thienylcarbonyl, thienylmethyl, pyrrolidinyl, pyrrolyl, indolyl, pyrrolylmethyl, pyrazolyl, pyrazolylmethyl, isoxazolyl, piperidinyl, morpholinyl, piperazinyl, pyridinyl, pyridinylmethyl;

X represents hydrogen, nitro, cyano, fluorine, chlorine, bromine, iodine, methyl, ethyl, difluoromethyl, trifluoromethyl, dichloromethyl, trichloromethyl, methoxymethyl, methylthiomethyl, methylsulphinylmethyl, methylsulphonylmethyl, methoxy, ethoxy, difluoromethoxy, trifluoromethoxy, methylthio, ethylthio, methylsulphinyl, ethylsulphinyl, methylsulphonyl, ethylsulphonyl or dimethylaminosulphonyl;

Y represents hydrogen, nitro, cyano, fluorine, chlorine, bromine, iodine, methyl, ethyl, difluoromethyl, trifluoromethyl, dichloromethyl, trichloromethyl, methoxymethyl, methylthiomethyl, methylsulphinylmethyl, methylsulphonylmethyl, methoxy, ethoxy, difluoromethoxy, trifluoromethoxy, methylthio, ethylthio, methylsulphinyl, ethylsulphinyl, methylsulphonyl, ethylsulphonyl or dimethylaminosulphonyl and

m represents the number 0 or 2.

**4.** Process for preparing compounds of the formula (I-2) according to any of Claims 1 to 3, by reacting

a) carboxylic acids of the formula (II)

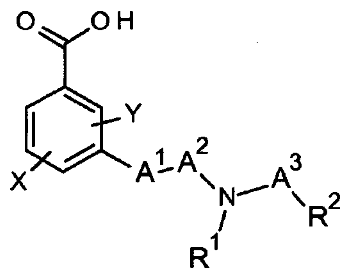

(II)

in which

$A^1$, $A^2$, $A^3$, $R^1$, $R^2$, X and Y are as defined above
- or alkali metal, alkaline earth metal or ammonium salts thereof -
with compounds of the general formula (III)

H-Z (III)

in which

Z is as defined above, or

b) carboxylic acid derivatives of the formula (IX)

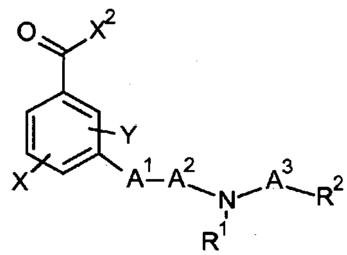

(IX)

in which

$A^1$, $A^2$, $A^3$, $R^1$, $R^2$, X and Y are as defined above and
$X^2$ represents CN or halogen, preferably Cl, Br, imidazolyl or triazolyl

with compounds of the formula (III)

H-Z (III)

in which

Z is as defined above, or

c) compounds of the formula (XIII)

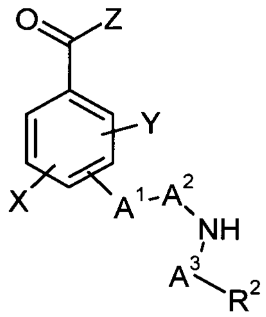

(XIII)

in which

$A^1$, $A^2$, $A^3$, $R^2$, X, Y and Z are as defined above with compounds of the formula (XI)

**$X^1$-$R^1$** **(XI)**

in which

$R^1$ is as defined above and
$X^1$ represents halogen, arylsulphonate or alkylsulphonate, preferably chlorine, bromine, iodine, mesylate or tosylate, or

d) compounds of the formula (XIV)

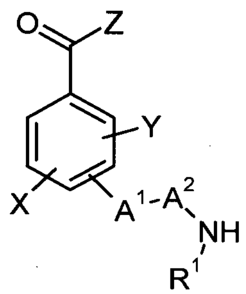

(XIV)

in which

$A^1$, $A^2$, $R^1$, X, Y and Z are as defined above with compounds of the formula (V)

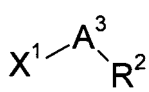

(V)

in which

$A^3$ and $R^2$ are as defined above and
$X^1$ represents halogen or tosylate, preferably chlorine, bromine or tosylate, or

e) compounds of the formula (XV)

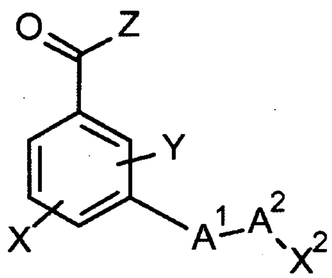

(XV)

in which

$A^1$, $A^2$, X, Y and Z are as defined above and
$X^2$ represents halogen or tosylate, preferably chlorine, bromine or tosylate

with compounds of the formula (VII)

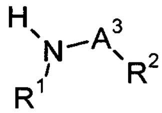

(VII)

in which

$A^3$, $R^1$ and $R^2$ are as defined above, if appropriate in the presence of one or more reaction auxiliaries and if appropriate in the presence of one or more diluents.

**5.** Compound of the formula (IX)

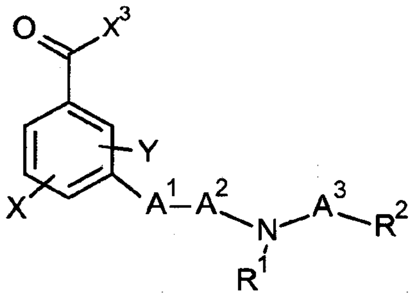

(IX)

in which

$A^1$, $A^2$, $A^3$, $R^1$, $R^2$, X and Y are as defined in any of Claims 1 to 4 and
$X^3$ represents halogen, cyano, imidazolyl or triazolyl.

**6.** Process for preparing compounds of the formula (IX) by reacting carboxylic acids of the formula (II)

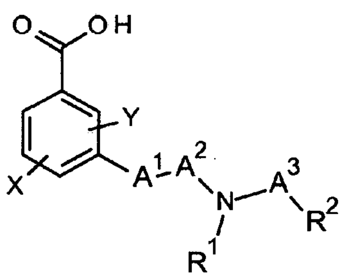

(II)

in which $A^1$, $A^2$, $A^3$, $R^1$, $R^2$, X and Y are as defined above with suitable activating reagents,

if appropriate in the presence of one or more reaction auxiliaries and if appropriate in the presence of one or more diluents.

**7.** Compound of the formula (II)

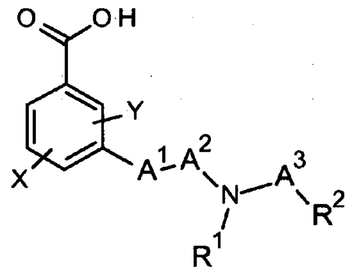

(II)

in which
X, Y, $A^1$, $A^2$, $A^3$, $R^1$ and $R^2$ are as defined in any of Claims 1 to 4.

**8.** Compound according to Claim 7 in which $A^3$ represents O, S, the grouping

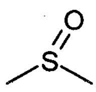 , 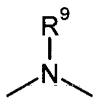 , 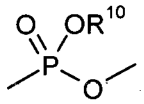 or 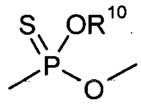 .

**9.** Process for preparing compounds of the formula (II) according to Claim 7 or 8 by reacting compounds (VIII)

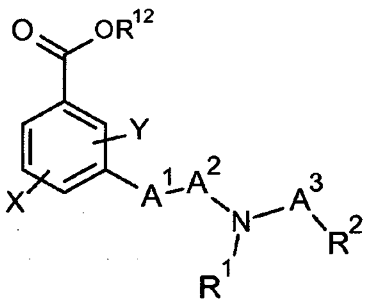

(VIII)

in which

$A^1$, $A^2$, $A^3$, $R^1$, $R^2$, X and Y are as defined in any of Claims 1 to 4 and
$R^{12}$ represents $C_1$-$C_4$-alkyl, in particular methyl, ethyl, n-, i-propyl, n-, s-, i-, t-butyl, represents allyl or benzyl,

under reductive or alkaline conditions in the presence of one or more reaction auxiliaries and if appropriate in the presence of one or more diluents.

**10.** Compound of the formula (VIII)

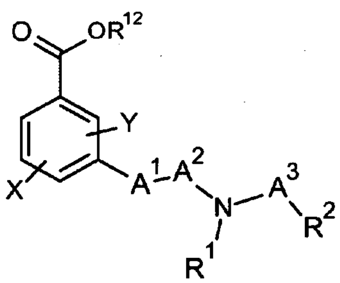

(VIII)

in which

$A^1$, $A^2$, $A^3$, $R^1$, $R^2$, X and Y are as defined in any of Claims 1 to 4 and
$R^{12}$ represents $C_1$-$C_4$-alkyl, allyl or benzyl.

**11.** Process for preparing compounds of the formula (VIII) according to Claim 10 by reacting

i) compounds of the formula (IV)

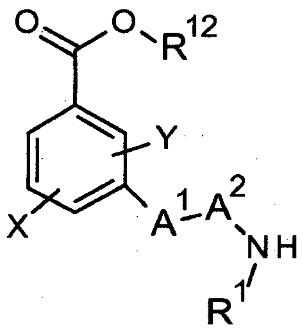

(IV)

in which

$A^1$, $A^2$, $R^1$, X and Y are as defined above and
$R^{12}$ represents $C_1$-$C_4$-alkyl, in particular methyl, ethyl, n-, i-propyl, n-, s-, i-, t-butyl, represents allyl or benzyl,

with compounds of the formula (V)

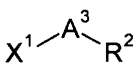

(V)

in which

$A^3$ and $R^2$ are as defined above and
$X^1$ represents halogen (preferably fluorine, chlorine, bromine or iodine, in particular chlorine, bromine or iodine), if appropriate in the presence of one or more reaction auxiliaries and if appropriate in the presence of one or more diluents,

or
j) compounds of the formula (X)

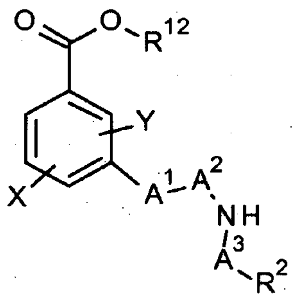

(X)

in which

$A^1$, $A^2$, $A^3$, $R^2$, X and Y are as defined in any of Claims 1 to 4 and
$R^{12}$ represents $C_1$-$C_4$-alkyl, in particular methyl, ethyl, n-, i-propyl, n-, s-, i-, t-butyl, represents allyl or benzyl,

with compounds of the formula (XI)

**$X^1$-$R^1$** **(XI)**

in which

$R^1$ is as defined above and
$X^1$ represents halogen (preferably fluorine, chlorine, bromine or iodine, in particular chlorine, bromine or iodine),

if appropriate in the presence of one or more reaction auxiliaries and if appropriate in the presence of one or more diluents,
or
k) compounds of the formula (X)

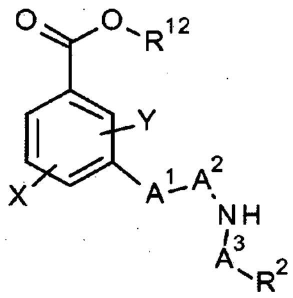

(X)

in which

$A^1$, $A^2$, $A^3$, $R^2$, X and Y are as defined above and
$R^{12}$ represents $C_1$-$C_4$-alkyl, in particular methyl, ethyl, n-, i-propyl, n-, s-, i-, t-butyl

with compounds of the formula (XII)

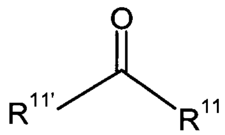

(XII)

in which

$R^{11'}$ and $R^{11}$ independently of one another represent hydrogen, represent optionally cyano-, halogen-, $C_1$-$C_4$-alkoxy-, $C_1$-$C_4$-alkylthio-, $C_1$-$C_4$-alkylsulphinyl- or $C_1$-$C_4$-alkylsulphonyl-substituted alkyl having 1 to 6 carbon atoms, represent in each case optionally cyano- or halogen-substituted alkenyl or alkynyl having in each case 3 to 6 carbon atoms, represent in each case optionally cyano-, halogen- or $C_1$-$C_4$-alkyl-substituted cycloalkyl or cycloalkylalkyl having in each case 3 to 6 carbon atoms in the cycloalkyl group and, if appropriate, 1 to 4 carbon atoms in the alkyl moiety, or represent in each case optionally nitro-, cyano-, halogen-, $C_1$-$C_4$-alkyl-, $C_1$-$C_4$-haloalkyl-, $C_1$-$C_4$-alkoxy-, $C_1$-$C_4$-haloalkoxy-, $C_1$-$C_4$-alkylthio-, $C_1$-$C_4$-haloalkylthio-, $C_1$-$C_4$-alkylsulphinyl-, $C_1$-$C_4$-haloalkyl-sulphinyl-, $C_1$-$C_4$-alkylsulphonyl- or $C_1$-$C_4$-haloalkylsulphonyl-substituted aryl or arylalkyl having in each case 6 or 10 carbon atoms in the aryl group and, if appropriate, 1 to 4 carbon atoms in the alkyl moiety,

in the presence of a reducing agent, preferably a borane or a $BH_3$ adduct,
if appropriate in the presence of one or more reaction auxiliaries and if appropriate in the presence of one or more diluents.

**12.** Pesticides, **characterized in that** they comprise at least one compound of the formula (1-2) according to any of Claims 1 to 3 or a compound of the formula (II) according to Claim 7 or 8 or a compound of the formula (VIII)

according to Claim 10.

**13.** Use of compounds of the formula (1-2) according to any of Claims 1 to 3, compounds of the formula (II) according to Claim 7 or 8 and/or compounds of the formula (VIII) according to Claim 10 for controlling pests.

**14.** Method for controlling pests, **characterized in that** compounds of the formula (1-2) according to any of Claims 1 to 3, compounds of the formula (II) according to Claim 7 or 8 and/or compounds of the formula (VIII) according to Claim 10 are allowed to act on pests and/or their habitat.

**15.** Process for preparing pesticides, **characterized in that** compounds of the formula (1-2) according to any of Claims 1 to 3, compounds of the formula (II) according to Claim 7 or 8 and/or compounds of the formula (VIII) according to Claim 10 are mixed with extenders and/or surfactants.

**16.** Use of compounds of the formula (1-2) according to any of Claims 1 to 3, compounds of the formula (II) according to Claim 7 or 8 and/or compounds of the formula (VIII) according to Claim 10 for preparing pesticides.

**Revendications**

**1.** Composé de formule (1-2)

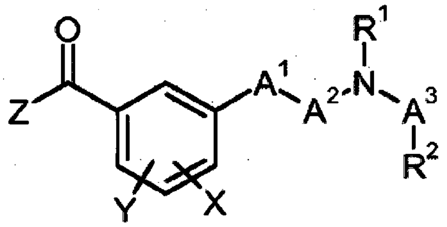

(I-2)

dans laquelle

$A^1$ représente 0 (oxygène),
$A^3$ représente O (oxygène), S (soufre), le groupement

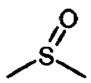 , 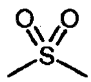 , 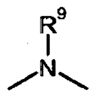 , 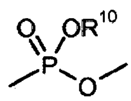 ou 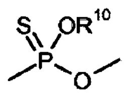

Z représente l'un des groupes ci-après

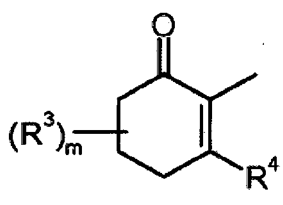 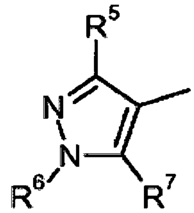

où
$A^2$ représente un groupe alcanediyle ayant 1 à 6 atomes de carbone, alcènediyle ou alcynediyle ayant chacun 2 à 6 atomes de carbone ;
$R^1$ représente un atome d'hydrogène,
un groupe alkyle ayant 1 à 6 atomes de carbone, éventuellement substitué par un ou plusieurs substituants hydroxy, amino, cyano, carbamoyle, halogéno, alcoxy en $C_1$-$C_4$, (alkyle en $C_1$-$C_4$)-carbonyle, (alcoxy en $C_1$-$C_4$)-carbonyle, (alkylamino en $C_1$-$C_4$)-carbonyle, di(alkyle en $C_1$-$C_4$)-amino, di(alkyle en $C_1$-$C_4$)-amino-carbonyle ou N-(alcoxy en $C_1$-$C_4$)-N-(alkyle en $C_1$-$C_4$)-amino-carbonyle ; un groupe alkylthio, alkylsulfinyle ou

alkylsulfonyle ayant chacun 1 à 6 atomes de carbone, chacun éventuellement substitué par un ou plusieurs substituants halogéno ; un groupe alcényle ou alcynyle ayant 2 à 6 atomes de carbone, chacun éventuellement substitué par un ou plusieurs substituants halogéno,

un groupe cycloalkyle, cycloalkylalkyle ayant chacun 3 à 6 atomes de carbone dans les groupes cycloalkyle et éventuellement 1 à 4 atomes de carbone dans la partie alkyle, chacun éventuellement substitué par un ou plusieurs substituants cyano, halogéno ou alkyle en $C_1$-$C_4$,

un groupe aryle, arylalkyle ou arylcarbonylalkyle ayant chacun 6 ou 10 atomes de carbone dans les groupes aryle et éventuellement 1 à 4 atomes de carbone dans la partie alkyle, chacun éventuellement substitué par un ou plusieurs substituants cyano, nitro, halogéno, alkyle en $C_1$-$C_4$, halogènalkyle en $C_1$-$C_4$, alcoxy en $C_1$-$C_4$ ou halogènalcoxy en $C_1$-$C_4$, ou

un groupe hétérocyclyle ou hétérocyclylalkyle chacun éventuellement substitué par un ou plusieurs substituants nitro, cyano, halogéno, alkyle en $C_1$-$C_4$, halogènalkyle en $C_1$-$C_4$, alcoxy en $C_1$-$C_4$ ou halogènalcoxy en $C_1$-$C_4$, chaque groupement hétérocyclyle contenant jusqu'à 10 atomes de carbone et en outre au moins un hétéroatome choisi dans la série N (azote, mais au plus 5 atomes d'azote), O (oxygène, mais au plus 2 atomes d'oxygène), S (soufre, mais au plus 2 atomes de soufre), SO ou $SO_2$, ainsi qu'éventuellement en outre un groupe choisi parmi les groupes oxo (C=O), thioxo (C=S), imino (C=NH), cyanoimino (C=N-CN), nitroimino (C=N-$NO_2$) ;

$R^2$ représente un atome d'hydrogène, un groupe alkyle ayant 1 à 6 atomes de carbone, éventuellement substitué par un ou plusieurs substituants cyano, halogéno, alcoxy en $C_1$-$C_4$, (alkyle en $C_1$-$C_4$)-carbonyle, (alcoxy en $C_1$-$C_4$)-carbonyle, (alkyle en $C_1$-$C_4$)-amino-carbonyle ou di(alkyle en $C_1$-$C_4$)-amino-carbonyle,

un groupe alkylcarbonyle, alcoxycarbonyle ou alkylaminocarbonyle ayant chacun 1 à 6 atomes de carbone dans les groupes alkyle, chacun éventuellement substitué par un ou plusieurs substituants cyano, halogéno ou alcoxy en $C_1$-$C_4$,

un groupe dialkylaminocarbonyle, ayant chacun 1 à 4 atomes de carbone dans les groupes alkyle,

un groupe alcényle, alcényloxy, alcényloxycarbonyle, alcynyle, alcynylcarbonyle ou alcynyloxycarbonyle, chacun éventuellement substitué par un ou plusieurs substituants halogéno, ayant chacun 3 à 6 atomes de carbone dans les groupes alcényle ou alcynyle ; un groupe cycloalkyle, cycloalkylcarbonyle, cycloalkyloxycarbonyle, cycloalkylalkyle, cycloalkylalkylcarbonyle ou cycloalkylalcoxycarbonyle, chacun éventuellement substitué par un ou plusieurs substituants cyano, halogéno ou alkyle en $C_1$-$C_4$, ayant chacun 3 à 6 atomes de carbone dans les groupes cycloalkyle et éventuellement 1 à 4 atomes de carbone dans la partie alkyle,

un groupe aryle, arylcarbonyle, aryloxycarbonyle, arylaminocarbonyle, arylalkyle, arylalkylcarbonyle, arylalcoxycarbonyle ou arylalkylaminocarbonyle, ayant chacun 6 à 10 atomes de carbone dans les groupes aryle et éventuellement 1 à 4 atomes de carbone dans la partie alkyle, chacun éventuellement substitué par un ou plusieurs substituants nitro, cyano, halogéno, alkyle en $C_1$-$C_4$, halogènalkyle en $C_1$-$C_4$, alcoxy en $C_1$-$C_4$ ou halogènalcoxy en $C_1$-$C_4$, ou

un groupe hétérocyclyle, hétérocyclylcarbonyle ou hétérocyclylalkyle, chacun éventuellement substitué par un ou plusieurs substituants nitro, cyano, halogéno, alkyle en $C_1$-$C_4$, halogènalkyle en $C_1$-$C_4$, alcoxy en $C_1$-$C_4$ ou halogènalcoxy en $C_1$-$C_4$, chaque groupement hétérocyclyle contenant jusqu'à 10 atomes de carbone et en outre au moins un hétéroatome choisi dans la série N (azote, mais au plus 5 atomes d'azote), O (oxygène, mais au plus 2 atomes d'oxygène), S (soufre, mais au plus 2 atomes de soufre), SO ou $SO_2$, ainsi qu'éventuellement en outre un groupe choisi parmi les groupes oxo (C=O), thioxo (C=S), imino (C=NH), cyanoimino (C=N-CN), nitroimino (C=N-$NO_2$) ;

$R^3$ représente un atome d'hydrogène, un groupe halogéno, un groupe alkyle ou alkylthio ayant chacun 1 à 6 atomes de carbone, chacun éventuellement substitué par un ou plusieurs substituants cyano, halogéno, alcoxy en $C_1$-$C_4$, alkylthio en $C_1$-$C_4$, alkylsulfinyle en $C_1$-$C_4$ ou alkylsulfonyle en $C_1$-$C_4$, ou le groupe phényle, ou - dans le cas dans lequel m vaut 2 - éventuellement aussi, avec un deuxième radical $R^3$, un atome d'oxygène ou un groupe alcanediyle (alkylène) ayant 3 à 5 atomes de carbone ;

$R^4$ représente un groupe hydroxy, formyloxy, halogéno ; un groupe alcoxy, alkylthio, alkylsulfinyle, alkylsulfonyle, alkylcarbonyloxy, alcoxycarbonyloxy, alkylaminocarbonyloxy ou alkylsulfonyloxy ayant chacun 1 à 6 atomes de carbone, chacun éventuellement substitué par un ou plusieurs substituants cyano, halogéno, alcoxy en $C_1$-$C_4$, alkylthio en $C_1$-$C_4$, alkylsulfinyle en $C_1$-$C_4$, ou alkylsulfonyle en $C_1$-$C_4$ ; un groupe alcényloxy ou alcynyloxy ayant chacun 3 à 6 atomes de carbone, chacun éventuellement substitué par un ou plusieurs substituants halogéno ; un groupe aryloxy, arylthio, arylsulfinyle, arylsulfonyle, arylcarbonyloxy, arylcarbonylalcoxy, arylsulfonyloxy, arylalcoxy, arylalkylthio, arylalkylsulfinyle ou arylalkylsulfonyle ayant chacun 6 ou 10 atomes de carbone dans le groupe aryle et éventuellement 1 à 4 atomes de carbone dans la partie alkyle, chacun éventuellement substitué par un ou plusieurs substituants nitro, cyano, halogéno, alkyle en $C_1$-$C_4$, halogènalkyle en $C_1$-$C_4$, alcoxy en $C_1$-$C_4$, halogènalcoxy en $C_1$-$C_4$, alkylthio en $C_1$-$C_4$, halogènalkylthio en $C_1$-$C_4$, alkylsulfinyle en $C_1$-$C_4$, halogènalkylsulfinyle en $C_1$-$C_4$, alkylsulfonyle en $C_1$-$C_4$ ou halogènalkylsulfonyle en $C_1$-$C_4$ ; un groupe hétérocyclyle ayant 5 ou 6 atomes nucléaires, éventuellement substitué par un ou plusieurs substituants cyano,

halogéno, oxo, hydroxy, alcoxy en $C_1$-$C_4$, alkyle en $C_1$-$C_4$, au moins 1 atome d'azote et éventuellement jusqu'à 2 atomes d'oxygène, atomes de soufre et 3 atomes d'azote étant présents, en tout pas plus de 4 hétéroatomes étant présents, et l'hétérocycle étant lié par l'intermédiaire de l'azote ;

$R^5$ représente un atome d'hydrogène, un groupe cyano, carbamoyle, thiocarbamoyle, halogéno ; un groupe alkyle, alcoxy, alkylthio, alkylsulfinyle, alkylsulfonyle ou alcoxycarbonyle, ayant chacun 1 à 6 atomes de carbone dans les groupes alkyle, chacun éventuellement substitué par un ou plusieurs substituants cyano, halogéno, alcoxy en $C_1$-$C_4$, alkylthio en $C_1$-$C_4$, alkylsulfinyle en $C_1$-$C_4$ ou alkylsulfonyle en $C_1$-$C_4$ ; ou un groupe cycloalkyle ayant 3 à 6 atomes de carbone, éventuellement substitué par un ou plusieurs substituants cyano, halogéno ou alkyle en $C_1$-$C_4$ ;

$R^6$ représente un atome d'hydrogène ; un groupe alkyle ayant 1 à 6 atomes de carbone, éventuellement substitué par un ou plusieurs substituants cyano, halogéno, alcoxy en $C_1$-$C_4$, alkylthio en $C_1$-$C_4$, alkylsulfinyle en $C_1$-$C_4$ ou alkylsulfonyle en $C_1$-$C_4$ ; un groupe alcényle ou alcynyle ayant chacun 3 à 6 atomes de carbone, chacun éventuellement substitué par un ou plusieurs substituants cyano ou halogéno ; un groupe cycloalkyle ou cycloalkylalkyle ayant chacun 3 à 6 atomes de carbone dans le groupe cycloalkyle et éventuellement 1 à 4 atomes de carbone dans la partie alkyle, chacun éventuellement substitué par un ou plusieurs substituants cyano, halogéno ou alkyle en $C_1$-$C_4$ ; ou un groupe aryle ou aralkyle ayant chacun 6 ou 10 atomes de carbone dans le groupe aryle et éventuellement 1 à 4 atomes de carbone dans la partie alkyle, chacun éventuellement substitué par un ou plusieurs substituants nitro, cyano, halogéno, alkyle en $C_1$-$C_4$, halogènalkyle en $C_1$-$C_4$, alcoxy en $C_1$-$C_4$, halogènalcoxy en $C_1$-$C_4$, alkylthio en $C_1$-$C_4$, halogènalkylthio en $C_1$-$C_4$, alkylsulfinyle en $C_1$-$C_4$, halogènalkylsulfinyle en $C_1$-$C_4$, alkylsulfonyle en $C_1$-$C_4$ ou halogènalkylsulfonyle en $C_1$-$C_4$ ;

$R^7$ représente un groupe hydroxy, formyloxy ; alcoxy, alkylcarbonyloxy, alcoxycarbonyloxy, alcoxycarbonylalcoxy, alkylaminocarbonyloxy ou alkylsulfonyloxy ayant chacun 1 à 6 atomes de carbone dans les groupes alkyle, chacun éventuellement substitué par un ou plusieurs substituants alkyle, cyano, halogéno ou alcoxy en $C_1$-$C_4$ ; un groupe alcényloxy ou alcynyloxy ayant chacun 3 à 6 atomes de carbone, chacun éventuellement substitué par un ou plusieurs substituants cyano ou halogéno ; ou un groupe aryloxy, arylcarbonyloxy, arylcarbonylalcoxy ou arylsulfonyloxy ayant chacun 6 ou 10 atomes de carbone dans le groupe aryle et éventuellement 1 à 4 atomes de carbone dans la partie alkyle, chacun éventuellement substitué par un ou plusieurs substituants nitro, cyano, halogéno, alkyle en $C_1$-$C_4$, halogènalkyle en $C_1$-$C_4$, alcoxy en $C_1$-$C_4$, halogènalcoxy en $C_1$-$C_4$, alkylthio en $C_1$-$C_4$, halogènalkylthio en $C_1$-$C_4$, alkylsulfinyle en $C_1$-$C_4$, halogènalkylsulfinyle en $C_1$-$C_4$, alkylsulfonyle en $C_1$-$C_4$ ou halogènalkylsulfonyle en $C_1$-$C_4$ ;

$R^9$ représente un atome d'hydrogène,

un groupe alkyle ayant 1 à 6 atomes de carbone, éventuellement substitué par un ou plusieurs substituants hydroxy, amino, cyano, carbamoyle, halogéno, alcoxy en $C_1$-$C_4$, (alkyle en $C_1$-$C_4$)-carbonyle, (alcoxy en $C_1$-$C_4$)-carbonyle, (alkylamino en $C_1$-$C_4$)-carbonyle ou di(alkyle en $C_1$-$C_4$)-amino-carbonyle,

un groupe alkylcarbonyle ou alcoxycarbonyle ayant chacun 1 à 6 atomes de carbone dans les groupes alkyle, chacun éventuellement substitué par un ou plusieurs substituants cyano, halogéno ou alcoxy en $C_1$-$C_4$,

un groupe alkylthio, alkylsulfinyle ou alkylsulfonyle ayant chacun 1 à 6 atomes de carbone, chacun éventuellement substitué par un ou plusieurs substituants halogéno,

un groupe alcényle, alcénylcarbonyle, alcényloxycarbonyle, alcynyle, alcynylcarbonyle ou alcynyloxycarbonyle ayant chacun 2 à 6 atomes de carbone dans les groupes respectivement alcényle et alcynyle, chacun éventuellement substitué par un ou plusieurs substituants halogéno,

un groupe cycloalkyle, cycloalkylcarbonyle, cycloalkyloxycarbonyle, cycloalkylalkyle, cycloalkylalkylcarbonyle ou cycloalkylalcoxycarbonyle ayant chacun 3 à 6 atomes de carbone dans les groupes cycloalkyle et éventuellement 1 à 4 atomes de carbone dans la partie alkyle, chacun éventuellement substitué par un ou plusieurs substituants cyano, halogéno ou alkyle en $C_1$-$C_4$,

un groupe aryle, arylcarbonyle, aryloxycarbonyle, arylalkyle, arylalkylcarbonyle ou arylalcoxycarbonyle ayant chacun 6 ou 10 atomes de carbone dans les groupes aryle et éventuellement 1 à 4 atomes de carbone dans la partie alkyle, chacun éventuellement substitué par un ou plusieurs substituants cyano, nitro, halogéno, alkyle en $C_1$-$C_4$, halogènalkyle en $C_1$-$C_4$, alcoxy en $C_1$-$C_4$ ou halogènalcoxy en $C_1$-$C_4$, ou

un groupe hétérocyclyle, hétérocyclylcarbonyle ou hétérocyclylalkyle, chacun éventuellement substitué par un ou plusieurs substituants nitro, cyano, halogéno, alkyle en $C_1$-$C_4$, halogènalkyle en $C_1$-$C_4$, alcoxy en $C_1$-$C_4$ ou halogènalcoxy en $C_1$-$C_4$, chaque groupement hétéroaryle contenant jusqu'à 10 atomes de carbone et en outre au moins un hétéroatome choisi dans la série N (mais au plus 5 atomes d'azote), 0 (mais au plus 2 atomes d'oxygène), S (mais au plus 2 atomes de soufre), SO ou $SO_2$, ainsi qu'éventuellement en outre un groupe choisi parmi les groupes oxo (C=O), thioxo (C=S), imino (C=NH), cyanoimino (C=N-CN), nitroimino (C=N-$NO_2$), ou ensemble avec $R^2$ et l'atome d'azote auquel ils sont liés, un groupe hétérocyclique éventuellement substitué par un ou plusieurs substituants nitro, cyano, halogéno, alkyle en $C_1$-$C_4$, halogènalkyle en $C_1$-$C_4$, alcoxy en $C_1$-$C_4$ ou halogènalcoxy en $C_1$-$C_4$, qui contient 1 atome d'azote et 1 à 10 atomes de carbone et éventuellement

un autre hétéroatome de la série N (mais au plus 4 autres atomes d'azote), O (mais au plus 2 atomes d'oxygène), S (mais au plus 2 atomes de soufre), SO ou $SO_2$, ainsi qu'éventuellement en outre un groupe choisi parmi les groupes oxo (C=O), thioxo (C=S), imino (C=NH), cyanoimino (C=N-CN), nitroimino ($C=N-NO_2$) ;

$R^{10}$ représente un atome d'hydrogène, le groupe formyle, un groupe alkyle ayant 1 à 6 atomes de carbone, éventuellement substitué par un ou plusieurs substituants cyano, halogéno, alcoxy en $C_1$-$C_4$, (alkyle en $C_1$-$C_4$)-carbonyle, (alcoxy en $C_1$-$C_4$)-carbonyle, (alkylamino en $C_1$-$C_4$)-carbonyle ou di (alkyle en $C_1$-$C_4$)-aminocarbonyle ;

X représente un atome d'hydrogène, un groupe nitro, cyano, carboxy, carbamoyle, thiocarbamoyle, halogéno, ou un groupe alkyle, alcoxy, alkylthio, alkylsulfinyle, alkylsulfonyle, alkylamino, dialkylamino ou dialkylaminosulfonyle, ayant chacun 1 à 6 atomes de carbone dans les groupes alkyle, chacun éventuellement substitué par un ou plusieurs groupes cyano, halogéno, alcoxy en $C_1$-$C_4$, alkylthio en $C_1$-$C_4$, alkylsulfinyle en $C_1$-$C_4$ ou alkylsulfonyle en $C_1$-$C_4$ ;

Y représente un atome d'hydrogène, un groupe nitro, cyano, carboxy, carbamoyle, thiocarbamoyle, halogéno, ou un groupe alkyle, alcoxy, alkylthio, alkylsulfinyle, alkylsulfonyle, alkylamino, dialkylamino ou dialkylaminosulfonyle ayant chacun 1 à 6 atomes de carbone dans les groupes alkyle, chacun éventuellement substitué par un ou plusieurs substituants cyano, halogéno, alcoxy en $C_1$-$C_4$, alkylthio en $C_1$-$C_4$, alkylsulfinyle en $C_1$-$C_4$ ou alkylsulfonyle en $C_1$-$C_4$ ;

m représente les nombres 0, 1, 2 ou 3.

**2.** Composé selon la revendication 1, dans lequel

$A^2$ représente le groupe méthylène ($-CH_2-$), éthane-1,1-diyl-($-CH(CH_3)-$), éthane-1,2-diyle (diméthylène, $-CH_2CH_2-$), propane-1,1-diyle- ($-CH(C_2H_5)-$), propane-1,2-diyle ($-CH(CH_3)CH_2-$), propane-1,3-diyle-($-CH_2CH_2CH_2-$), butane-1,3-diyle ($-CH(CH_3)CH_2CH_2-$), butane-1,4-diyle-($-CH_2CH_2CH_2CH_2-$), éthènediyle, propènediyle, butènediyle, éthynediyle, propynediyle ou butynediyle,

$R^1$ représente un atome d'hydrogène,

un groupe méthyle, éthyle, n- ou isopropyle, n-, iso- ou sec-butyle, n-, iso- ou sec-pentyle, chacun éventuellement substitué par un ou plusieurs substituants hydroxy, amino, cyano, carbamoyle, fluoro, chloro, méthoxy, éthoxy, n- ou isopropoxy, acétyle, propionyle, n- ou iso-butyroyle, méthoxycarbonyle, éthoxycarbonyle, n- ou iso-propoxycarbonyle, méthylaminocarbonyle, éthylaminocarbonyle, n- ou isopropylaminocarbonyle, diméthylamino, diéthylamino, diméthylaminocarbonyle, diéthylaminocarbonyle ou N-méthoxy-N-méthyl-amino-carbonyle,

un groupe méthylthio, éthylthio, n- ou isopropylthio, n-, iso-, sec- ou tert-butylthio, méthylsulfinyle, éthylsulfinyle, n- ou isopropylsulfinyle, méthylsulfonyle, éthylsulfonyle, n- ou

isopropylsulfonyle, chacun éventuellement substitué par un ou plusieurs substituants fluoro et/ou chloro,

un groupe propényle, butényle, propynyle ou butynyle, chacun éventuellement substitué par un ou plusieurs substituants fluoro, chloro et/ou bromo,

un groupe cyclopropyle, cyclobutyle, cyclopentyle, cyclohexyle, cyclopropylméthyle, cyclobutylméthyle, cyclopentylméthyle ou cyclohexylméthyle, chacun éventuellement substitué par un ou plusieurs substituants cyano, fluoro, chloro, méthyle ou éthyle, un groupe phényle, benzoyle, benzyle, phényléthyle ou phénylcarbonylméthyle, chacun éventuellement substitué par un ou plusieurs substituants cyano, nitro, fluoro, chloro, bromo, méthyle, éthyle, n- ou isopropyle, n-, iso-, sec- ou tert-butyle, trifluorométhyle, méthoxy, éthoxy, sec- ou isopropoxy, n-, iso-, sec-, ou tert-butoxy, difluorométhoxy ou trifluorométhoxy, ou

un groupe hétérocyclyle, ou hétérocycylalkyle de la série furyle, furylméthyle, thiényle, thiénylméthyle, pyrrolidinyle, oxopyrrolidinyle, pyrrolyle, indolyle, pyrrolylméthyle, pyrazolyle, pyrazolylméthyle, oxazolyle, oxazolylméthyle, isoxazolyle, thiazolyle, thiazolylméthyle, dihydropyrannyle, dihydropyrannylméthyle, pipéridinyle, oxopipéridinyle, morpholinyle, pipérazinyle, pyridinyle, pyridinylcarbonyle ou pyridinylméthyle, chacun éventuellement substitué par un ou plusieurs substituants nitro, cyano, fluoro, chloro, bromo, méthyle, éthyle n- ou isopropyle, n-, iso-, sec- ou tert-butyle, trifluorométhyle, méthoxy, éthoxy, n- ou isopropoxy, difluorométhoxy ou trifluorométhoxy ;

$R^2$ représente un atome d'hydrogène,

un groupe méthyle, éthyle, n- ou isopropyle, n-, iso-, sec- ou tert-butyle, n-, iso-, sec- ou tert-pentyle, chacun éventuellement substitué par un ou plusieurs substituants cyano, fluoro, chloro, méthoxy, éthoxy, n- ou isopropoxy, acétyle, propionyle, n- ou isobutyroyle, méthoxycarbonyle, éthoxycarbonyle, n- ou isopropoxycarbonyle, méthylaminocarbonyle, éthylaminocarbonyle, n- ou isopropylaminocarbonyle, diméthylaminocarbonyle ou diéthylaminocarbonyle,

un groupe acétyle, propionyle, n- ou isobutyroyle, méthoxycarbonyle, éthoxycarbonyle, n- ou isopropoxycarbonyle, méthylaminocarbonyle, éthylaminocarbonyle, n- ou isopropylaminocarbonyle, chacun éventuellement substitué par un ou plusieurs substituants cyano, fluoro, chloro, méthoxy, éthoxy, n- ou isopropoxy,

un groupe diméthylaminocarbonyle, diéthylaminocarbonyle ou dipropylaminocarbonyle,

un groupe propényle, butényle, propénylcarbonyle, buténylcarbonyle, propényloxycarbonyle, buténylоxycarbonyle, propénylaminocarbonyle, buténylaminocarbonyle, propynyle, butynyle, propynylcarbonyle, butynylcarbonyle, propynyloxycarbonyle ou butynyloxycarbonyle, chacun éventuellement substitué par un ou plusieurs substituants fluoro, chloro et/ou bromo,

un groupe cyclopropyle, cyclobutyle, cyclopentyle, cyclohexyle, cyclopropylcarbonyle, cyclobutylcarbonyle, cyclopentylcarbonyle, cyclohexylcarbonyle, cyclopropyloxycarbonyle, cyclobutyloxycarbonyle, cyclopentyloxycarbonyle, cyclohexyloxycarbonyle, cyclopropylméthyle, cyclobutylméthyle, cyclopentylméthyle, cyclohexylméthyle, cyclopropylméthylcarbonyle, cyclobutylméthylcarbonyle, cyclopentylméthylcarbonyle, cyclohexylméthylcarbonyle, cyclopropylméthoxycarbonyle, cyclobutylméthoxycarbonyle, cyclopentylméthoxycarbonyle, cyclohexylméthoxycarbonyle, chacun éventuellement substitué par un ou plusieurs substituants cyano, fluoro, chloro, méthyle ou éthyle,

un groupe phényle, naphtyle, benzoyle, phénoxycarbonyle, phénylaminocarbonyle, benzyle, phényléthyle, phénylméthylcarbonyle ou phénylméthoxycarbonyle, chacun éventuellement substitué par un ou plusieurs substituants nitro, cyano, fluoro, chloro, bromo, méthyle, éthyle, n- ou isopropyle, n-, iso-, sec- ou tert-butyle, trifluorométhyle, méthoxy, éthoxy, n- ou isopropoxy, difluorométhoxy ou trifluorométhoxy, ou

un groupe furyle, furylcarbonyle, furylméthyle, thiényle, thiénylcarbonyle, thiénylméthyle, pyrrolidinyle, oxopyrrolidinyle, pyrrolyle, indolyle, pyrrolylméthyle, pyrazolyle, pyrazolylcarbonyle, pyrazolylméthyle, oxazolyle, oxazolylméthyle, isoxazolyle, isoxazolylcarbonyle, thiazolyle, thiazolylméthyle, 2-oxo-1,3-diaza-cyclopentyl(2-oxoimidazolidinyle), pipéridinyle, oxopipéridinyle, 2-oxo-1,3-diaza-cyclohexyle, morpholinyle, thiomorpholinyle, 3-oxo-morpholinyle, 3-oxo-thiomorpholinyle, pipérazinyle, pyridinyle, pyridinylcarbonyle ou pyridinylméthyle, chacun éventuellement substitué par un ou plusieurs substituants nitro, cyano, fluoro, chloro, bromo, méthyle, éthyle, n- ou isopropyle, n-, iso-, sec- ou tert-butyle, trifluorométhyle, méthoxy, éthoxy, n- ou isopropoxy, difluorométhoxy ou trifluorométhoxy ;

$R^3$ représente un groupe méthyle, éthyle, n- ou isopropyle, n-, iso-, sec- ou tert-butyle, méthylthio, éthylthio, n- ou isopropylthio, n-, iso-, sec- ou tert-butylthio, chacun éventuellement substitué par un ou plusieurs substituants cyano, fluoro, chloro, méthoxy, éthoxy, n- ou isopropoxy, méthylthio, éthylthio, n- ou isopropylthio, méthylsulfinyle, éthylsulfinyle, méthylsulfonyle ou éthylsulfonyle, ou phényle, ou - dans le cas dans lequel m représente 2 - éventuellement aussi, ensemble avec un deuxième radical $R^3$, un atome d'oxygène, le groupe propane-1,3-diyle ou butane-1,4-diyle ;

$R^4$ représente un groupe hydroxy, formyloxy, fluoro ou chloro ; un groupe méthoxy, éthoxy, n- ou isopropoxy, méthylthio, éthylthio, n- ou isopropylthio, méthylsulfinyle, éthylsulfinyle, méthylsulfonyle, éthylsulfonyle, acétyloxy, propionyloxy, n- ou isobutyroyloxy, méthoxycarbonyloxy, éthoxycarbonyloxy, n- ou isopropoxycarbonyloxy, méthylaminocarbonyloxy, éthylaminocarbonyloxy, n- ou isopropylaminocarbonyloxy, méthylsulfonyloxy, éthylsulfonyloxy, n- ou isopropylsulfonyloxy, chacun éventuellement substitué par un ou plusieurs substituants cyano, fluoro, chloro, méthoxy, éthoxy, n- ou isopropoxy, méthylthio, éthylthio, n- ou isopropylthio, méthylsulfinyle, éthylsulfinyle, méthylsulfonyle ou éthylsulfonyle ; un groupe propényloxy, buténylоxy, propynyloxy et butynyloxy, chacun éventuellement substitué par un ou plusieurs substituants fluoro, chloro et/ou bromo ; un groupe phényloxy, phénylthio, phénylsulfinyle, phénylsulfonyle, phénylcarbonyloxy, phénylcarbonylméthoxy, phénylsulfonyloxy, phénylméthoxy, phénylméthylthio, phénylméthylsulfinyle ou phénylméthylsulfonyle, chacun éventuellement substitué par un ou plusieurs substituants nitro, cyano, fluoro, chloro, bromo, méthyle, éthyle, n- ou isopropyle, n-, iso-, sec- ou tert-butyle, trifluorométhyle, méthoxy, éthoxy, n- ou isopropoxy, difluorométhoxy, trifluorométhoxy, méthylthio, éthylthio, n- ou isopropylthio, difluorométhylthio, trifluorométhylthio, méthylsulfinyle, éthylsulfinyle, trifluorométhylsulfinyle, méthylsulfonyle, éthylsulfonyle ou trifluorométhylsulfonyle ; un groupe pyrrolyle, pyrrolinyle, pyrrolidinyle, pyrazolyle, pyrazolinyle, pyrazolidinyle, imidazolyle, imidazolinyle, imidazolidinyle, triazolyle, triazolinyle, triazolidinyle, tétrazolyle, tétrazolinyle, tétrazolidinyle, oxazolyle, oxazolinyle, oxazolidinyle, isoxazolyle, isoxazolinyle, isoxazolidinyle, thiazolyle, thiazolinyle, thiazolidinyle, thiadiazolyle, indolyle, pipéridinyle, pipérazinyle, oxazinyle, thiazinyle, morpholinyle, chacun éventuellement substitué par un ou plusieurs substituants cyano, oxo, fluoro, chloro, méthyle, éthyle, méthoxy, éthoxy, méthylthio, éthylthio ;

$R^5$ représente un atome d'hydrogène, un groupe cyano, carbamoyle, thiocarbamoyle, fluoro, chloro ou bromo ; un groupe méthyle, éthyle, n- ou isopropyle, n-, iso-, sec- ou tert-butyle, méthoxy, éthoxy, n- ou isopropoxy, méthylthio, éthylthio, n- ou isopropylthio, méthylsulfinyle, éthylsulfinyle, méthylsulfonyle, éthylsulfonyle, méthoxycarbonyle, éthoxycarbonyle, n- ou isopropoxycarbonyle, chacun éventuellement substitué par un ou plusieurs substituants cyano, fluoro, chloro, méthoxy, éthoxy, n- ou isopropoxy, méthylthio, éthylthio, n- ou isopropylthio, méthylsulfinyle, éthylsulfinyle, méthylsulfonyle ou éthylsulfonyle, ou un groupe cyclopropyle, cyclobutyle, cyclopentyle ou cyclohexyle, chacun éventuellement substitué par un ou plusieurs substituants cyano, fluoro, chloro, méthyle ou éthyle ;

$R^6$ représente un atome d'hydrogène ; un groupe méthyle, éthyle, n- ou isopropyle, n-, iso-, sec- ou tert-butyle,

chacun éventuellement substitué par un ou plusieurs substituants cyano, fluoro, chloro, méthoxy, éthoxy, n- ou isopropoxy, méthylthio, éthylthio, n- ou isopropylthio, méthylsulfinyle, éthylsulfinyle, méthylsulfonyle ou éthylsulfonyle ; un groupe propényle, butényle, propynyle ou butynyle, chacun éventuellement substitué par un ou plusieurs substituants cyano, fluoro, chloro ou bromo ; un groupe cyclopropyle, cyclobutyle, cyclopentyle, cyclohexyle, cyclopropylméthyle, cyclobutylméthyle, cyclopentylméthyle ou cyclohexylméthyle, chacun éventuellement substitué par un ou plusieurs substituants cyano, fluoro, chloro, méthyle ou éthyle ; ou un groupe phényle ou phénylméthyle, chacun éventuellement substitué par un ou plusieurs substituants nitro, cyano, fluoro, chloro, bromo, méthyle, éthyle, n- ou isopropyle, n-, iso-, sec- ou tert-butyle, trifluorométhyle, méthoxy, éthoxy, n- ou isopropoxy, difluorométhoxy, trifluorométhoxy, méthylthio, éthylthio, n- ou isopropylthio, n-, iso-, sec- ou tert-butylthio, difluorométhylthio, trifluorométhylthio, méthylsulfinyle, éthylsulfinyle, trifluorométhylsulfinyle, méthylsulfonyle, éthylsulfonyle ou trifluorométhylsulfonyle ;

$R^7$ représente un groupe hydroxy, formyloxy ; un groupe méthoxy, éthoxy, n- ou isopropoxy, acétyloxy, propionyloxy, n- ou isobutyroyloxy, méthoxycarbonyloxy, éthoxycarbonyloxy, n- ou isopropoxycarbonyloxy, méthylaminocarbonyloxy, éthylaminocarbonyloxy, n- ou isopropylaminocarbonyloxy, éthoxycarbonylméthoxy, méthoxycarbonylméthoxy, méthylsulfonyloxy, éthylsulfonyloxy, n- ou isopropylsulfonyloxy, chacun éventuellement substitué par un ou plusieurs substituants alkyle, cyano, fluoro, chloro, méthoxy, éthoxy, n- ou isopropoxy ; un groupe propionyloxy, buténylоxy, propynyloxy ou butynyloxy, chacun éventuellement substitué par un ou plusieurs substituants cyano, fluoro, chloro ou bromo ; ou un groupe phénylméthoxy, phénylcarbonyloxy, phénylcarbonylméthoxy ou phénylsulfonyloxy, chacun éventuellement substitué par un ou plusieurs substituants nitro, cyano, fluoro, chloro, bromo, méthyle, éthyle, n- ou isopropyle, n-, iso-, sec- ou tert-butyle, trifluorométhyle, méthoxy, éthoxy, n- ou isopropoxy, difluorométhoxy, trifluorométhoxy, méthylthio, éthylthio, n- ou isopropylthio, difluorométhylthio, trifluorométhylthio, méthylsulfinyle, éthylsulfinyle, trifluorométhylsulfinyle, méthylsulfonyle, éthylsulfonyle ou trifluorométhylsulfonyle ;

$R^9$ représente un atome d'hydrogène ; un groupe méthyle, éthyle, n- ou isopropyle, n-, iso- ou sec-butyle, n-, iso- ou sec-butyle, chacun éventuellement substitué par un ou plusieurs substituants hydroxy, amino, cyano, carbamoyle, fluoro, chloro, méthoxy, éthoxy, n- ou isopropoxy, acétyle, propionyle, n- ou isobutyroyle, méthoxycarbonyle, éthoxycarbonyle, n- ou isopropoxycarbonyle, méthylaminocarbonyle, éthylaminocarbonyle, n- ou isopropylaminocarbonyle, diméthylaminocarbonyle ou diéthylaminocarbonyle,

un groupe acétyle, propionyle, n- ou isobutyroyle, méthoxycarbonyle, éthoxycarbonyle, n- ou isopropoxycarbonyle, chacun éventuellement substitué par un ou plusieurs substituants cyano, fluoro, chloro, méthoxy, éthoxy, n- ou isopropoxy,

un groupe méthylthio, éthylthio, n- ou isopropylthio, n-, iso-, sec- ou tert-butylthio, méthylsulfinyle, éthylsulfinyle, n- ou isopropylsulfinyle, méthylsulfonyle, éthylsulfonyle, n- ou isopropylsulfonyle, chacun éventuellement substitué par un ou plusieurs substituants fluoro et/ou chloro,

un groupe propényle, butényle, propénylcarbonyle, buténylcarbonyle, propényloxycarbonyle, buténylоxycarbonyle, propynyle, butynyle, propynylcarbonyle, butynylcarbonyle, propynyloxycarbonyle ou butynyloxycarbonyle, chacun éventuellement substitué par un ou plusieurs substituants fluoro, chloro et/ou bromo,

un groupe cyclopropyle, cyclobutyle, cyclopentyle, cyclohexyle, cyclopropylcarbonyle, cyclobutylcarbonyle, cyclopentylcarbonyle, cyclohexylcarbonyle, cyclopropyloxycarbonyle, cyclobutyloxycarbonyle, cyclopentyloxycarbonyle, cylohexyloxycarbonyle, cyclopropylméthyle, cyclobutylméthyle, cyclopentylméthyle, cyclohexylméthyle, cyclopropylméthylcarbonyle, cyclobutylméthylcarbonyle, cyclopentylméthylcarbonyle, cyclohexylméthylcarbonyle, cyclopropylméthoxycarbonyle, cyclobutylméthoxycarbonyle, cyclopentylméthoxycarbonyle, cyclohexylméthoxycarbonyle, chacun éventuellement substitué par un ou plusieurs substituants cyano, fluoro, chloro, méthyle ou éthyle,

un groupe phényle, benzoyle, phénoxycarbonyle, benzyle, phényléthyle, phénylméthylcarbonyle ou phénylméthoxycarbonyle, chacun éventuellement substitué par un ou plusieurs substituants cyano, nitro, fluoro, chloro, bromo, méthyle, éthyle, n- ou isopropyle, n-, iso-, sec- ou tert-butyle, trifluorométhyle, méthoxy, éthoxy, sec- ou isopropoxy, n-, iso-, sec- ou tert-butoxy, difluorométhoxy ou trifluorométhoxy, ou

un groupe hétérocyclyle, hétérocyclylcarbonyle, ou hétérocyclylalkyle de la série furyle, furylcarbonyle, furylméthyle, thiényle, thiénylcarbonyle, thiénylméthyle, pyrrolidinyle, oxopyrrolidinyle, pyrrolyle, indolyle, pyrrolylméthyle, pyrazolyle, pyrazolylcarbonyle, pyrazolylméthyle, oxazolyle, oxazolylméthyle, isoxazolyle, isoxazolylcarbonyle, thiazolyle, thiazolylméthyle, pipéridinyle, oxopipéridinyle, morpholinyle, pipérazinyle, pyridinyle, pyridinylcarbonyle ou pyridinylméthyle, chacun éventuellement substitué par un ou plusieurs substituants nitro, cyano, fluoro, chloro, bromo, méthyle, éthyle, n- ou isopropyle, n-, iso-, sec- ou tert-butyle, trifluorométhyle, méthoxy, éthoxy, n- ou isopropoxy, difluorométhoxy ou trifluorométhoxy, ou ensemble avec $R^2$ et l'atome d'azote auxquels ils sont liés, un groupe pyrrolidinyle, oxopyrrolidinyle, pyrrolyle, indolyle, pyrazolyle, oxazolyle, isoxazolyle, dihydropyrannyle, pipéridinyle, thiomorpholinyle, 3-oxo-morpholinyle, 3-oxo-thiomorpholinyle, oxopipéridinyle, morpholinyle, pipérazinyle, imidazolyle, imidazolidinyle, oxoimidazolidinyle, triazole, triazolinyle, tétrazolinyle ou piridinyle, chacun éventuellement substitué par un ou plusieurs substituants nitro, cyano, fluoro, chloro, bromo, méthyle, éthyle, n- ou isopropyle, n-, iso-, sec- ou tert-butyle, trifluorométhyle, méthoxy, éthoxy, n- ou isopropoxy, difluorométhoxy ou trifluorométhoxy ;

$R^{10}$ représente un atome d'hydrogène, un groupe formyle,

un groupe alkyle ayant 1 à 6 atomes de carbone, éventuellement substitué par un ou plusieurs substituants cyano, halogéno, alcoxy en $C_1$-$C_4$ (alkyle en $C_1$-$C_4$)-carbonyle, (alcoxy en $C_1$-$C_4$)-carbonyle, (alkylamino en $C_1$-$C_4$)-carbonyle ou di (alkyle en $C_1$-$C_4$)-amino-carbonyle ;

X représente un atome d'hydrogène, un groupe nitro, cyano, carboxy, carbamoyle, thiocarbamoyle, fluoro, chloro, bromo, iodo ; ou un groupe méthyle, éthyle, n- ou isopropyle, n-, iso-, sec- ou tert-butyle, méthoxy, éthoxy, n- ou isopropoxy, n-, iso-, sec- ou tert-butoxy, méthylthio, éthylthio, n- ou isopropylthio, n-, iso-, sec- ou tert-butylthio, méthylsulfinyle, éthylsulfinyle, n- ou isopropylsulfinyle, méthylsulfonyle, éthylsulfonyle, n- ou isopropylsulfonyle, méthylamino, éthylamino, n- ou isopropylamino, n-, iso-, sec- ou tert-butylamino, diméthylamino, diéthylamino, diméthylaminosulfonyle ou diéthylaminosulfonyle, chacun éventuellement substitué par un ou plusieurs substituants cyano, fluoro, chloro, méthoxy, éthoxy, méthylthio, éthylthio, méthylsulfinyle, éthylsulfinyle, méthylsulfonyle ou éthylsulfonyle ;

Y représente un atome d'hydrogène, un groupe nitro, cyano, carboxy, carbamoyle, thiocarbamoyle, fluoro, chloro, bromo, iodo ; ou un groupe méthyle, éthyle, n- ou isopropyle, n-, iso-, sec- ou tert-butyle, méthoxy, éthoxy, n- ou isopropoxy, n-, iso-, sec- ou tert-butoxy, méthylthio, éthylthio, n- ou isopropylthio, n-, iso-, sec- ou tert-butylthio, méthylsulfinyle, éthylsulfinyle, n- ou isopropylsulfinyle, méthylsulfonyle, éthylsulfonyle, n- ou isopropylsulfonyle, méthylamino, éthylamino, n- ou isopropylamino, n-, iso-, sec- ou tert-butylamino, diméthylamino, diéthylamino, diméthylaminosulfonyle ou diéthylaminosulfonyle, chacun éventuellement substitué par un ou plusieurs substituants cyano, fluoro, chloro, méthoxy, éthoxy, méthylthio, éthylthio, méthylsulfinyle, éthylsulfinyle, méthylsulfonyle ou éthylsulfonyle ;

m représente les nombres 0, 1 ou 2.

**3.** Composé selon la revendication 1 ou 2, dans lequel

$A^2$ représente un groupe méthylène ($-CH_2-$), éthane-1,1-diyle ($-CH(CH_3)-$), éthane-1,2-diyle (diméthylène, $-CH_2CH_2-$), propane-1,2-diyle ($-CH(CH_3)CH_2-$) ou propane-1,3-diyle ($-CH_2CH_2CH_2-$) ;

$R^1$ représente un atome d'hydrogène,

un groupe méthyle, éthyle ou n-isopropyle, chacun éventuellement substitué par un ou plusieurs substituants cyano, fluoro, chloro, méthoxy, éthoxy, n- ou isopropoxy, acétyle, propionyle, méthoxycarbonyle ou éthoxycarbonyle,

un groupe acétyle, propionyle, méthoxycarbonyle ou éthoxycarbonyle, chacun éventuellement substitué par un ou plusieurs substituants cyano, fluoro, chloro, méthoxy, éthoxy, n- ou isopropoxy,

un groupe méthylthio, éthylthio, n- ou isopropylthio, n-, iso-, sec- ou tert-butylthio, méthylsulfinyle, éthylsulfinyle, méthylsulfonyle ou éthylsulfonyle, chacun éventuellement substitué par un ou plusieurs substituants fluoro et/ou chloro,

un groupe propényle, butényle, propynyle ou butynyle, chacun éventuellement substitué par un ou plusieurs substituants fluoro, chloro et/ou bromo,

un groupe cyclopropyle, cyclopropylcarbonyle ou cyclopropylméthyle, chacun éventuellement substitué par un ou plusieurs substituants fluoro, chloro ou méthyle, ou

un groupe phényle, benzoyle, phénoxycarbonyle, benzyle, phénylméthylcarbonyle ou phénylméthoxycarbonyle, chacun éventuellement substitué par un ou plusieurs substituants cyano, nitro, fluoro, chloro, bromo, méthyle, éthyle, n- ou isopropyle, n-, iso-, sec- ou tert-butyle, trifluorométhyle, méthoxy, éthoxy, sec- ou isopropoxy, difluorométhoxy ou trifluorométhoxy ;

$R^2$ représente un atome d'hydrogène,

un groupe méthyle, éthyle, n- ou isopropyle, n-, iso-, sec- ou tert-butyle, n-, iso-, sec- ou tert-pentyle, chacun éventuellement substitué par un ou plusieurs substituants cyano, fluoro, chloro, méthoxy, éthoxy, n- ou isopropoxy, acétyle, propionyle, n- ou isobutyroyle, méthoxycarbonyle, éthoxycarbonyle, n- ou isopropoxycarbonyle,

un groupe acétyle, propionyle, n- ou isobutyroyle, méthoxycarbonyle, éthoxycarbonyle, n- ou isopropoxycarbonyle, méthylaminocarbonyle, éthylaminocarbonyle, n- ou isopropylaminocarbonyle, chacun éventuellement substitué par un ou plusieurs substituants cyano, fluoro, chloro, méthoxy, éthoxy, n- ou isopropoxy ; un groupe diméthylaminocarbonyle,

un groupe méthylthio, éthylthio, n- ou isopropylthio, n-, iso-, sec- ou tert-butylthio, méthylsulfinyle, éthylsulfinyle, méthylsulfonyle ou éthylsulfonyle, chacun éventuellement substitué par un ou plusieurs substituants fluoro et/ou chloro ; un groupe propényle, butényle, propynyle ou butynyle, chacun éventuellement substitué par un ou plusieurs substituants fluoro, chloro et/ou bromo,

un groupe cyclopropyle, cyclopropylcarbonyle ou cyclopropylméthyle, chacun éventuellement substitué par un ou plusieurs substituants fluoro, chloro ou méthyle, un groupe phényle, benzoyle, phénoxycarbonyle, phénylaminocarbonyle, benzyle, phénylméthylcarbonyle ou phénylméthoxycarbonyle, chacun éventuellement substitué par un ou plusieurs substituants nitro, cyano, fluoro, chloro, bromo, méthyle, éthyle, n- ou isopropyle, n-, iso-, sec- ou tert-butyle, trifluorométhyle, méthoxy, éthoxy, n- ou isopropoxy, difluorométhoxy et trifluorométhoxy, ou

un groupe hétérocyclyle, hétérocyclylcarbonyle ou hétérocyclylalkyle de la série furyle, furylcarbonyle, furylméthyle, thiényle, thiénylcarbonyle, thiénylméthyle, pyrrolidinyle, pyrrolyle, indolyle, pyrrolylméthyle, pyrazolyle, pyrazolylméthyle, isoxazolyle, pipéridinyle, morpholinyle, thiomorpholinyle, 3-oxo-morpholinyle, 3-oxo-thiomorpholinyle, pipérazinyle, pyridinyle, pyridinylméthyle, chacun éventuellement substitué par un ou plusieurs substituants nitro, cyano, fluoro, chloro, bromo, méthyle, éthyle, n- ou isopropyle, n-, iso-, sec- ou tert-butyle, trifluorométhyle, méthoxy, éthoxy, n- ou isopropoxy, difluorométhoxy ou trifluorométhoxy ;

$R^3$ représente un atome d'hydrogène ; un groupe méthyle, éthyle, n- ou isopropyle, méthylthio, éthylthio, n- ou isopropylthio, chacun éventuellement substitué par un ou plusieurs substituants fluoro et/ou chloro ; ou un groupe phényle ; ou - dans le cas dans lequel m vaut 2 - éventuellement aussi ensemble avec un deuxième radical $R^3$, un atome d'oxygène, un groupe propane-1,3-diyle ou butane-1,4-diyle ;

$R^4$ représente un groupe hydroxy ; un groupe formyloxy ; un groupe méthoxy, éthoxy, n- ou isopropoxy, méthylthio, éthylthio, n- ou isopropylthio, méthylsulfinyle, éthylsulfinyle, méthylsulfonyle, éthylsulfonyle, acétyloxy, propionyloxy, n- ou isobutyroyloxy, méthoxycarbonyloxy, éthoxycarbonyloxy, n- ou isopropoxycarbonyloxy, méthylaminocarbonyloxy, éthylaminocarbonyloxy, n- ou isopropylaminocarbonyloxy, méthylsulfonyloxy, éthylsulfonyloxy, n- ou isopropylsulfonyloxy, chacun éventuellement substitué par un ou plusieurs substituants fluoro et/ou chloro ; un groupe propényloxy, butényloxy, propynyloxy ou butynyloxy, chacun éventuellement substitué par un ou plusieurs substituants fluoro et/ou chloro ; un groupe phényloxy, phénylthio, phénylsulfinyle, phénylsulfonyle, phénylcarbonyloxy, phénylcarbonylméthoxy, phénylsulfonyloxy, phénylméthoxy, phénylméthylthio, phénylméthylsulfinyle ou phénylméthylsulfonyle, chacun éventuellement substitué par un ou plusieurs substituants nitro, cyano, fluoro, chloro, bromo, méthyle, éthyle, n- ou isopropyle, trifluorométhyle, méthoxy, éthoxy, n- ou isopropoxy, difluorométhoxy ou trifluorométhoxy ; un groupe pyrrolyle, pyrrolinyle, pyrrolidinyle, pyrazolyle, pyrazolinyle, pyrazolidinyle, imidazolyle, imidazolinyle, imidazolidinyle, triazolyle, triazolinyle, triazolidinyle, tétrazolyle, tétrazolinyle, tétrazolidinyle, oxazolyle, oxazolinyle, oxazolidinyle, isoxazolyle, isoxazolinyle, isoxazolidinyle, thiazolyle, thiazolinyle, thiazolidinyle, thiadiazolyle, indolyle, pipéridinyle, pipérazinyle, oxazinyle, thiazinyle, morpholinyle, chacun éventuellement substitué par un ou plusieurs substituants cyano, oxo, fluoro, chloro, méthyle, éthyle, méthoxy, éthoxy, méthylthio, éthylthio ;

$R^5$ représente un atome d'hydrogène, un groupe cyano, fluoro, chloro ; un groupe méthyle, éthyle, n- ou isopropyle, méthoxy, éthoxy, n- ou isopropoxy, méthylthio, éthylthio, n- ou isopropylthio, méthylsulfinyle, éthylsulfinyle, méthylsulfonyle, éthylsulfonyle, méthoxycarbonyle, éthoxycarbonyle, n- ou isopropoxycarbonyle, chacun éventuellement substitué par un ou plusieurs substituants fluoro et/ou chloro ; ou un groupe cyclopropyle, éventuellement substitué par un ou plusieurs substituants cyano, fluoro, chloro ou méthyle ;

$R^6$ est un atome d'hydrogène ; un groupe méthyle, éthyle, n- ou isopropyle, n-, iso-, sec- ou tert-butyle, chacun éventuellement substitué par un ou plusieurs substituants cyano, fluoro, chloro, méthoxy ou éthoxy ; un groupe propényle, butényle, propynyle ou butynyle, chacun éventuellement substitué par un ou plusieurs substituants fluoro ou chloro ; un groupe cyclopropyle, cyclobutyle, cyclopentyle ou cyclohexyle, chacun éventuellement substitué par un ou plusieurs substituants fluoro, chloro ou méthyle ; ou un groupe phényle ou phénylméthyle, chacun éventuellement substitué par un ou plusieurs substituants fluoro, chloro, bromo, méthyle, éthyle, n- ou isopropyle, trifluorométhyle, méthoxy, éthoxy, n- ou isopropoxy, difluorométhoxy ou trifluorométhoxy ;

$R^7$ représente un groupe hydroxy ; un groupe formyloxy ; un groupe méthoxy, éthoxy, n- ou isopropoxy, acétyloxy, propionyloxy, n- ou isobutyroyloxy, méthoxycarbonyloxy, éthoxycarbonyloxy, n- ou isopropoxycarbonyloxy, méthylaminocarbonyloxy, éthylaminocarbonyloxy, n- ou isopropylaminocarbonyloxy, éthoxycarbonylméthoxy, méthoxycarbonylméthoxy, méthylsulfonyloxy, éthylsulfonyloxy, n- ou isopropylsulfonyloxy, chacun éventuellement substitué par un ou plusieurs substituants alkyle, cyano, fluoro, chloro, méthoxy, éthoxy, n- ou isopropoxy ; un groupe propényloxy, butényloxy, propynyloxy ou butynyloxy, chacun éventuellement substitué par un ou plusieurs substituants fluoro et/ou chloro ; ou un groupe phénylméthoxy, phénylcarbonyloxy, phénylcarbonylméthoxy ou phénylsulfonyloxy, chacun éventuellement substitué par un ou plusieurs substituants nitro, cyano, fluoro, chloro, bromo, méthyle, éthyle, n- ou isopropyle, trifluorométhyle, méthoxy, éthoxy, n- ou isopropoxy, difluorométhoxy ou trifluorométhoxy ;

$R^9$ représente un atome d'hydrogène; un groupe méthyle, éthyle, n- ou isopropyle, chacun éventuellement substitué par un ou plusieurs substituants cyano, fluoro, chloro, méthoxy, éthoxy, n- ou isopropoxy, acétyle, propionyle, méthoxycarbonyle ou éthoxycarbonyle,

un groupe acétyle, propionyle, méthoxycarbonyle ou éthoxycarbonyle, chacun éventuellement substitué par

un ou plusieurs substituants cyano, fluoro, chloro, méthoxy, éthoxy, n- ou isopropoxy,
un groupe méthylthio, éthylthio, n- ou isopropylthio, n-, iso-, sec- ou tert-butylthio, méthylsulfinyle, éthylsulfinyle, méthylsulfonyle et éthylsulfonyle, chacun éventuellement substitué par un ou plusieurs substituants fluoro et/ou chloro,
un groupe propényle, butényle, propynyle ou butynyle, chacun éventuellement substitué par un ou plusieurs substituants fluoro, chloro et/ou bromo ; un groupe cyclopropyle, cyclopropylcarbonyle ou cyclopropylméthyle, chacun éventuellement substitué par un ou plusieurs substituants fluoro, chloro ou méthyle, ou
un groupe phényle, benzoyle, phénoxycarbonyle, benzyle, phénylméthylcarbonyle ou phénylméthoxycarbonyle, chacun éventuellement substitué par un ou plusieurs substituants cyano, nitro, fluoro, chloro, bromo, méthyle, éthyle, n- ou isopropyle, n-, iso-, sec- ou tert-butyle, trifluorométhyle, méthoxy, éthoxy, sec- ou isopropoxy, difluorométhoxy ou trifluorométhoxy, ou ensemble avec $R^2$ et l'atome d'azote auquel ils sont liés, un groupe pyrrolidinyle, oxopyrrolidinyle, pyrrolyle, indolyle, pyrazolyle, oxazolyle, isoxazolyle, dihydropyrannyle, pipéridinyle, oxopipéridinyle, morpholinyle, thiomorpholinyle, 3-oxo-morpholinyle, 3-oxo-thiomorpholinyle, pipérazinyle, imidazolyle, imidazolidinyle, oxo-imidazolidinyle, triazole, triazolinyle, tétrazolinyle ou pyridinyle, chacun éventuellement substitué par un ou plusieurs substituants nitro, cyano, fluoro, chloro, bromo, méthyle, éthyle, n- ou isopropyle, n-, iso-, sec- ou tert-butyle, trifluorométhyle, méthoxy, éthoxy, n- ou isopropoxy, difluorométhoxy ou trifluorométhoxy ;
$R^{10}$ représente un atome d'hydrogène, le groupe formyle, un groupe méthyle, éthyle, n- ou isopropyle, n-, iso-, sec- ou tert-butyle, n-, iso-, sec- ou tert-pentyle, chacun éventuellement substitué par un ou plusieurs substituants cyano, fluoro, chloro, méthoxy, éthoxy, n- ou isopropoxy, acétyle, propionyle, n- ou isobutyroyle, méthoxycarbonyle, éthoxycarbonyle, n- ou isopropoxycarbonyle,
un groupe acétyle, propionyle, n- ou isobutyroyle, méthoxycarbonyle, éthoxycarbonyle, n- ou isopropoxycarbonyle, méthylaminocarbonyle, éthylaminocarbonyle, n- ou isopropylaminocarbonyle, chacun éventuellement substitué par un ou plusieurs substituants cyano, fluoro, chloro, méthoxy, éthoxy, n- ou isopropoxy ; un groupe diméthylaminocarbonyle,
un groupe méthylthio, éthylthio, n- ou isopropylthio, n-, iso-, sec- ou tert-butylthio, méthylsulfinyle, éthylsulfinyle, méthylsulfonyle ou éthylsulfonyle, chacun éventuellement substitué par un ou plusieurs substituants fluoro et/ou chloro ; un groupe propényle, butényle, propynyle ou butynyle, chacun éventuellement substitué par un ou plusieurs substituants fluoro, chloro et/ou bromo,
un groupe cyclopropyle, cyclopropylcarbonyle, cyclopropylméthyle, chacun éventuellement substitué par un ou plusieurs substituants fluoro, chloro ou méthyle, un groupe phényle, benzoyle, phénoxycarbonyle, phénylaminocarbonyle, benzyle, phénylméthylcarbonyle ou phénylméthoxycarbonyle, chacun éventuellement substitué par un ou plusieurs substituants nitro, cyano, fluoro, chloro, bromo, méthyle, éthyle, n- ou isopropyle, n-, iso-, sec- ou tert-butyle, trifluorométhyle, méthoxy, éthoxy, n- ou isopropoxy, difluorométhoxy ou trifluorométhoxy, ou un groupe hétérocyclyle, hétérocyclylcarbonyle ou hétérocyclylalkyle de la série furyle, furylcarbonyle, furylméthyle, thiényle, thiénylcarbonyle, thiénylméthyle, pyrrolidinyle, pyrrolyle, indolyle, pyrrolylméthyle, pyrazolyle, pyrazolylméthyle, isoxazolyle, pipéridinyle, morpholinyle, pipérazinyle, pyridinyle, pyridinylméthyle, chacun éventuellement substitué par un ou plusieurs substituants nitro, cyano, fluoro, chloro, bromo, méthyle, éthyle, n- ou isopropyle, n-, iso-, sec- ou tert-butyle, trifluorométhyle, méthoxy, éthoxy, n- ou isopropoxy, difluorométhoxy ou trifluorométhoxy ;
X représente un atome d'hydrogène, un groupe nitro, cyano, fluoro, chloro, bromo, iodo, méthyle, éthyle, difluorométhyle, trifluorométhyle, dichlorométhyle, trichlorométhyle, méthoxyméthyle, méthylthiométhyle, méthylsulfinylméthyle, méthylsulfonylméthyle, méthoxy, éthoxy, difluorométhoxy, trifluorométhoxy, méthylthio, éthylthio, méthylsulfinyle, éthylsulfinyle, méthylsulfonyle, éthylsulfonyle ou diméthylaminosulfonyle ;
Y représente un atome d'hydrogène, un groupe nitro, cyano, fluoro, chloro, bromo, iodo, méthyle, éthyle, difluorométhyle, trifluorométhyle, dichlorométhyle, trichlorométhyle, méthoxyméthyle, méthylthiométhyle, méthylsulfinylméthyle, méthylsulfonylméthyle, méthoxy, éthoxy, difluorométhoxy, trifluorométhoxy, méthylthio, éthylthio, méthylsulfinyle, éthylsulfinyle, méthylsulfonyle, éthylsulfonyle ou diméthylaminosulfonyle, et
m représente le nombre 0 ou 2.

**4.** Procédé de préparation de composés de formule (I-2) selon les revendications 1 à 3, pouvant être obtenus par la réaction

a) d'acides carboxyliques de formule (II)

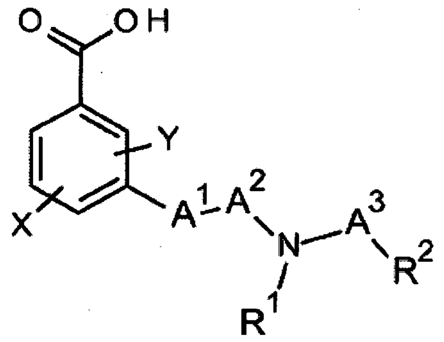

(II)

dans laquelle
$A^1$, $A^2$, $A^3$, $R^1$, $R^2$, X et Y ont les significations données ci-dessus, - ou leurs sels de métaux alcalins, de métaux alcalino-terreux ou d'ammonium -, avec des composés de formule générale (III)

H-Z (III)

dans laquelle
Z a les significations données ci-dessus, ou
b) de dérivés d'un acide carboxylique de formule (IX)

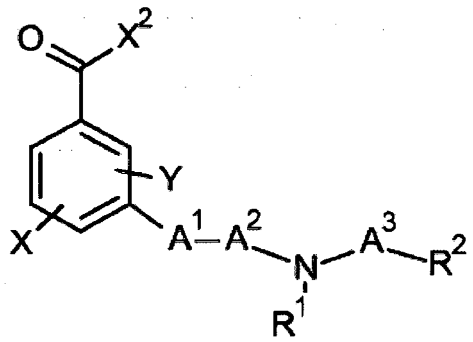

(IX)

dans laquelle

$A^1$, $A^2$, $A^3$, $R^1$, $R^2$, X et Y ont les significations données ci-dessus, et
$X^2$ représente CN ou un groupe halogéno, de préférence Cl, Br, imidazolyle ou triazolyle, avec des composés de formule (III)

H-Z (III)

dans laquelle
Z a les significations données ci-dessus, ou

c) de composés de formule (XIII)

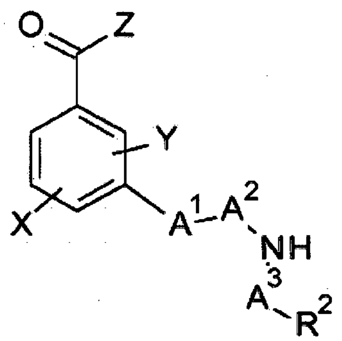

(XIII)

dans laquelle
$A^1$, $A^2$, $A^3$, $R^2$, X, Y et Z ont les significations données ci-dessus,
avec des composés de formule (XI)

$X^1$-$R^1$ (XI)

dans laquelle

$R^1$ a les significations données ci-dessus, et
$X^1$ représente un groupe halogéno, un groupe arylsulfonate ou alkylsulfonate, de préférence chloro, bromo, iodo, mésylate ou tosylate, ou

d) de composés de formule (XIV)

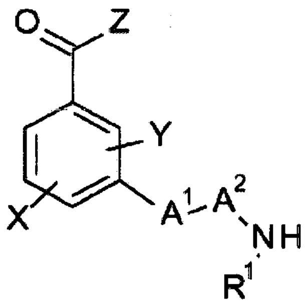

(XIV)

dans laquelle
$A^1$, $A^2$, $R^1$, X, Y et Z ont les significations données ci-dessus,
avec des composés de formule (V)

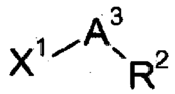

(V)

dans laquelle
$A^3$ et $R^2$ ont les significations données ci-dessus, et $X^1$ représente un groupe halogéno ou tosylate, de préférence chloro, bromo ou tosylate, ou
e) de composés de formule (XV)

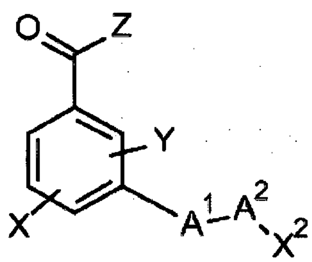

(XV)

dans laquelle

$A^1$, $A^2$, X, Y et Z ont les significations données ci-dessus, et
$X^2$ représente un groupe halogéno ou tosylate, de préférence chloro, bromo ou tosylate,
avec des composés de formule (VII)

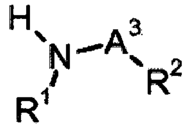

(VII)

dans laquelle

$A^3$, $R^1$ et $R^2$ ont les significations données ci-dessus, éventuellement en présence d'un ou plusieurs auxiliaires de réaction et éventuellement en présence d'un ou plusieurs diluants.

**5.** Composé de formule (IX)

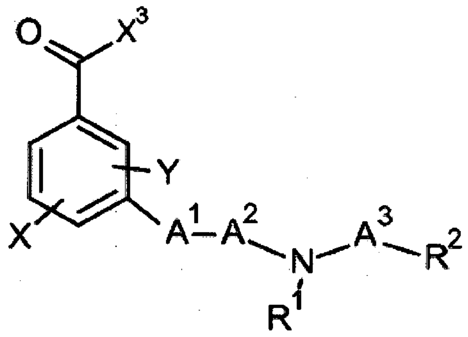

(IX)

dans laquelle

$A^1$, $A^2$, $A^3$, $R^1$, $R^2$, X et Y ont l'une des significations données dans les revendications 1 à 4, et $X^3$ représente un groupe halogéno, cyano, imidazolyle ou triazolyle.

**6.** Procédé de préparation de composés de formule (IX), pouvant être obtenus par réaction d'acides carboxyliques de formule (II)

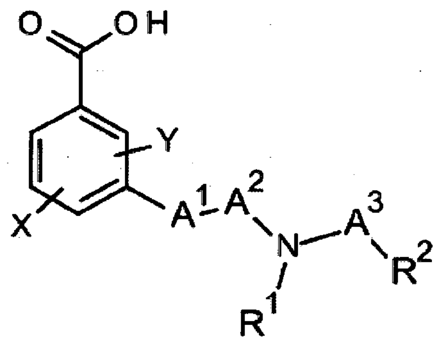

(II)

dans laquelle

$A^1$, $A^2$, $A^3$, $R^1$, $R^2$, X et Y ont les significations données ci-dessus, avec des réactifs d'activation appropriés, éventuellement en présence d'un ou plusieurs auxiliaires de réaction et éventuellement en présence d'un ou plusieurs diluants.

**7.** Composé de formule (II)

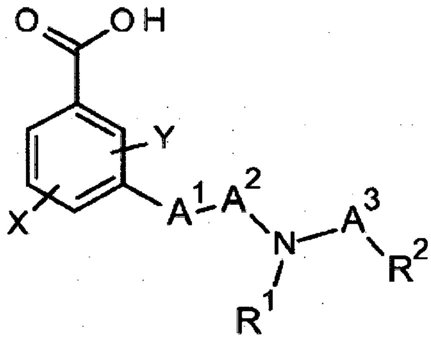

(II)

dans laquelle
X, Y, $A^1$, $A^2$, $A^3$, $R^1$ et $R^2$ ont l'une des significations données dans les revendications 1 à 4.

**8.** Composé selon la revendication 7, dans lequel A3 représente O, S, le groupement

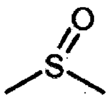

, 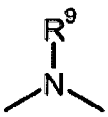

, 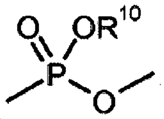

ou 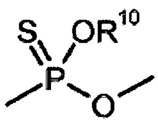

**9.** Procédé de préparation de composés de formule (II) selon la revendication 7 ou 8, pouvant être obtenus par la réaction de composés (VIII)

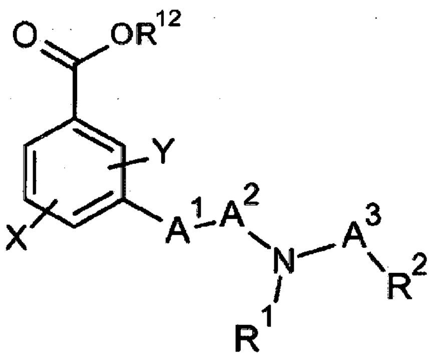

(VIII)

dans laquelle

$A^1$, $A^2$, $A^3$, $R^1$, $R^2$, X et Y ont l'une des significations données dans les revendications 1 à 4, et
$R^{12}$ représente un groupe alkyle en $C_1$-$C_4$, en particulier méthyle, éthyle, n-, isopropyle, n-, sec-, iso-, tert-butyle, un groupe allyle ou benzyle,
dans des conditions réductrices ou alcalines, en présence d'un ou plusieurs auxiliaires de réaction et éventuellement en présence d'un ou plusieurs diluants.

**10.** Composé de formule (VIII)

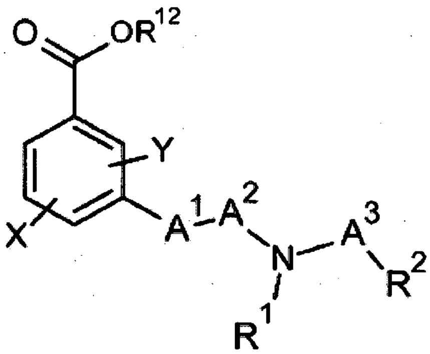

(VIII)

dans laquelle

$A^1$, $A^2$, $A^3$, $R^1$, $R^2$, X et Y ont l'une des significations données dans les revendications 1 à 4, et $R^{12}$ représente un groupe alkyle en $C_1$-$C_4$, allyle ou benzyle.

**11.** Procédé de préparation de composés de formule (VIII) selon la revendication 10, pouvant être obtenus par la réaction

i) de composés de formule (IV)

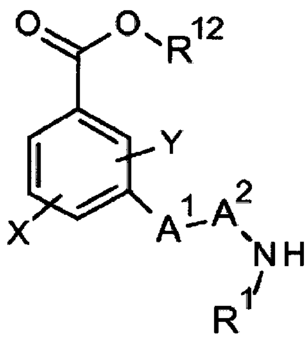

(IV)

dans laquelle

$A^1$, $A^2$, $R^1$, X et Y ont les significations données ci-dessus, et
$R^{12}$ représente un groupe alkyle en $C_1$-$C_4$, en particulier méthyle, éthyle, n-, isopropyle, n-, sec-, iso-, tert-butyle, un groupe allyle ou benzyle,
avec des composés de formule (V)

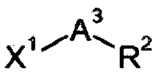

(V)

dans laquelle

$A^3$ et $R^2$ ont les significations données ci-dessus, et $X^1$ représente un groupe halogéno (de préférence fluoro, chloro, bromo ou iodo, en particulier chloro, bromo ou iodo),
éventuellement en présence d'un ou plusieurs auxiliaires de réaction, et éventuellement en présence d'un ou plusieurs diluants, ou

j) de composés de formule (X)

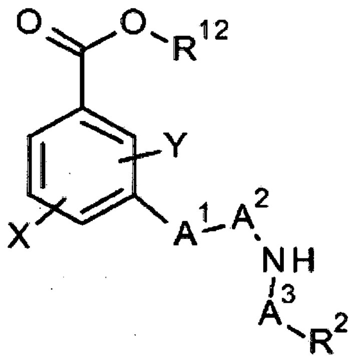

(X)

dans laquelle

$A^1$, $A^2$, $A^3$, $R^2$, X et Y ont les significations données dans les revendications 1 à 4, et
$R^{12}$ représente un groupe alkyle en $C_1$-$C_4$, en particulier méthyle, éthyle, n-, isopropyle, n-, sec-, iso-, tert-butyle, un groupe allyle ou benzyle,
avec des composés de formule (XI)

$X^1$ - $R^1$ (XI)

dans laquelle

$R^1$ a les significations données ci-dessus, et
$X^1$ représente un groupe halogéno (de préférence fluoro, chloro, bromo ou iodo, en particulier chloro, bromo ou iodo),
éventuellement en présence d'un ou plusieurs auxiliaires de réaction, et éventuellement en présence d'un ou plusieurs diluants,
ou

k) de composés de formule (X)

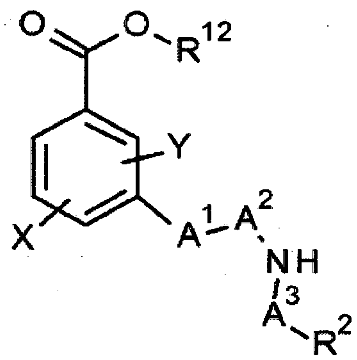

(X)

dans laquelle

$A^1$, $A^2$, $A^3$, $R^2$, X et Y ont les significations données ci-dessus, et
$R^{12}$ représente un groupe alkyle en $C_1$-$C_4$, en particulier méthyle, éthyle, n-, isopropyle, n-, sec-, iso-, tert-butyle,
avec des composés de formule (XII)

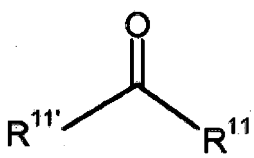

(XII)

dans laquelle

$R^{11'}$ et $R^{11}$ représentent chacun indépendamment de l'autre un atome d'hydrogène ; un groupe alkyle ayant 1 à 6 atomes de carbone, éventuellement substitué par un ou plusieurs substituants cyano, halogéno, alcoxy en $C_1$-$C_4$, alkylthio en $C_1$-$C_4$, alkylsulfinyle en $C_1$-$C_4$ ou alkylsulfonyle en $C_1$-$C_4$ ; un groupe alcényle ou alcynyle, ayant chacun 3 à 6 atomes de carbone, chacun éventuellement substitué par un ou plusieurs substituants cyano ou halogéno ; un groupe cycloalkyle ou cycloalkylalkyle ayant chacun 3 à 6 atomes de carbone dans le groupe cycloalkyle et éventuellement 1 à 4 atomes de carbone dans la partie alkyle, chacun éventuellement substitué par un ou plusieurs substituants cyano, halogéno ou alkyle en $C_1$-$C_4$ ; ou un groupe aryle ou arylalkyle, ayant chacun 6 ou 10 atomes de carbone dans le groupe aryle et éventuellement 1 à 4 atomes de carbone dans la partie alkyle, chacun éventuellement substitué par un ou plusieurs substituants nitro, cyano, halogéno, alkyle en $C_1$-$C_4$, halogènalkyle en $C_1$-$C_4$, alcoxy en $C_1$-$C_4$, halogènalcoxy en $C_1$-$C_4$, alkylthio en $C_1$-$C_4$, halogènalkylthio en $C_1$-$C_4$, alkylsulfinyle en $C_1$-$C_4$, halogènalkylsulfinyle en $C_1$-$C_4$, alkylsulfonyle en $C_1$-$C_4$ ou halogènalkylsulfonyle en $C_1$-$C_4$,
en présence d'un réducteur, de préférence d'un borane ou d'un adduit de $BH_3$,
éventuellement en présence d'un ou plusieurs auxiliaires de réaction et éventuellement en présence d'un ou plusieurs diluants.

**12.** Produit pesticide, **caractérisé en ce qu'**il contient au moins un composé de formule (I-2) selon les revendications 1 à 3 ou un composé de formule (II) selon la revendication 7 ou 8 ou un composé de formule (VIII) selon la revendication 10.

**13.** Utilisation de composés de formule (1-2) selon les revendications 1 à 3, de composés de formule (II) selon la revendication 7 ou 8 et/ou de composés de formule (VIII) selon la revendication 10, pour lutter contre les ravageurs.

**14.** Procédé de lutte contre les ravageurs, **caractérisé en ce qu'**on fait agir sur les ravageurs et/ou leur habitat des composés de formule (1-2) selon les revendications 1 à 3, des composés de formule (II) selon la revendication 7 ou 8 et/ou de composés de formule (VIII) selon la revendication 10.

**15.** Procédé de préparation de produits pesticides, **caractérisé en ce qu'**on mélange à des diluants et/ou des agents tensioactifs des composés de formule (1-2) selon les revendications 1 à 3, des composés de formule (II) selon la revendication 7 ou 8, et/ou des composés de formule (VIII) selon la revendication 10.

**16.** Utilisation de composés de formule (1-2) selon les revendications 1 à 3, de composés de formule (II) selon la revendication 7 ou 8 et/ou de composés de formule (VIII) selon la revendication 10, pour fabriquer des produits pesticides.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 090262 A
- EP 135191 A
- EP 186118 A
- EP 186119 A
- EP 186120 A
- EP 319075 A
- EP 352543 A
- EP 418175 A
- EP 487357 A
- EP 527036 A
- EP 527037 A
- EP 560483 A
- EP 609797 A
- EP 609798 A
- EP 625505 A
- EP 625508 A
- EP 636622 A
- US 5804532 A
- US 5834402 A
- US 5846906 A
- US 5863865 A
- WO 9531446 A
- WO 9626192 A
- WO 9626193 A
- WO 9626200 A
- WO 9626206 A
- WO 9727187 A
- WO 9735850 A
- WO 9741105 A
- WO 9741116 A
- WO 9741117 A
- WO 9741118 A
- WO 9743270 A
- WO 9746530 A
- WO 9828981 A
- WO 9831681 A
- WO 9831682 A
- WO 9903856 A
- WO 9907688 A
- WO 9907697 A
- WO 9910327 A
- WO 9910328 A
- WO 0005221 A
- WO 0021924 A
- WO 9928282 A
- WO 0153275 A
- DE 10122445 A